# EUROPEAN PATENT SPECIFICATION

(11) **EP 1 539 785 B1**
(45) Date of publication and mention of the grant of the patent: **06.05.2009**
(21) Application number: 03762172.9
(22) Date of filing: 26.06.2003
(51) Int. Cl.: C07H 21/00, C12N 15/00

(54) **GENE REGULATION IN TRANSGENIC ANIMALS USING A TRANSPOSON-BASED VECTOR**
GENREGULATION IN TRANSGENEN TIEREN UNTER VERWENDUNG EINES VEKTORS AUF TRANSPOSONBASIS
REGULATION D'UN GENE DANS DES ANIMAUX TRANSGENIQUES AU MOYEN D'UN VECTEUR A BASE D'UN TRANSPOSON

(30) Priority: 26.06.2002 US 392415 P; 21.01.2003 US 441377 P; 21.01.2003 US 441502 P; 21.01.2003 US 441405 P; 21.01.2003 US 441447 P; 21.01.2003 US 441392 P; 21.01.2003 US 441381 P
(43) Date of publication of application: 15.06.2005
(73) Proprietor: Transgenrx, Inc., Dallas, TX 75234 (US); THE BOARD OF SUPERVISORS OF LOUISIANA STATE UNIVERSITY AND AGRICULTURAL AND MECHANICAL COLLEGE, Baton Rouge, LA 70894-5055 (US)
(72) Inventor: COOPER, Richard K., Baton Rouge, LA 70810 (US); CADD, Gary G., Grapevine, TX 76051 (US); FIORETTI, William C., Grapevine, TX 76051 (US); DEBOER, Kenneth F., Ryegate, MT 59074 (US)
(74) Representative: Dey, Michael
(86) International application number: PCT/US2003/020389
(87) International publication number: WO 2004/003157

(56) References cited:
- WO-A-01/83786
- WO-A1-01/14537
- WO-A1-97/47739
- WO-A1-99/09817
- US-A- 5 719 055
- US-B1- 6 218 185
- ALEXEYEV M F ET AL: "Mini-Tn10 transposon derivatives for insertion mutagenesis and gene delivery into the chromosome of Gram-negative bacteria" GENE, ELSEVIER BIOMEDICAL PRESS. AMSTERDAM, NL, vol. 160, no. 1, 4 July 1995 (1995-07-04), pages 59-62, XP004042178 ISSN: 0378-1119
- HERRERO M ET AL: "TRANSPOSON VECTORS CONTAINING NON-ANTIBIOTIC RESISTANCE SELECTION MARKERS FOR CLONING AND STABLE CHROMOSOMAL INSERTION OF FOREIGN GENES IN GRAM-NETATIVE BACTERIA" JOURNAL OF BACTERIOLOGY, WASHINGTON, DC, US, vol. 172, no. 11, 1 November 1990 (1990-11-01), pages 6557-6567, XP000572232 ISSN: 0021-9193
- SHERMAN A. ET AL.: 'Transposition of the Drosophila element mariner into the chicken germ line' NATURE BIOTECHNOLOGY vol. 16, November 1998, pages 1050 - 1053, XP002942479
- SCHNEIDER S. ET AL.: 'An epitope tagged mammalian/prokaryotic expression vector with positive selection of cloned inserts' GENE vol. 197, 1997, pages 337 - 341, XP004126435
- KOZAK M.: 'At least six nucleotides preceding the AUG initiator codon enhance translation in mammalian cells' JOURNAL OF MOL. BIOL. vol. 196, 1987, pages 947 - 950, XP002975779

## Description

The U.S. Government has certain rights in this invention. The development of this invention was partially funded by the United States Government under a HATCH grant from the United States Department of Agriculture, partially funded by the United States Government with Formula 1433 funds from the United States Department of Agriculture and partially funded by the United States Government under contract DAAD 19-02016 awarded by the Army.

### FIELD OF THE INVENTION

The present invention relates generally to cell-specific gene regulation in transgenic animals. Animals may be made transgenic through administration of a transposon-based vector through any method of administration including pronuclear injection, or intraembryonic, intratesticular, intraoviductal or intravenous administration. These transgenic animals contain the gene of interest in all cells, including germ cells. Animals may also be made transgenic by targeting specific cells for uptake and gene incorporation of the transposon-based vectors. Stable incorporation of a gene of interest into cells of the transgenic animals is demonstrated by expression of the gene of interest in a cell, wherein expression is regulated by a promoter sequence. The promoter sequence may be provided as a transgene along with the gene of interest or may be endogenous to the cell. The promoter sequence may be constitutive or inducible, wherein inducible promoters include tissue-specific promoters, developmentally regulated promoters and chemically inducible promoters.

### BACKGROUND OF THE INVENTION

Transgenic animals are desirable for a variety of reasons, including their potential as biological factories to produce desired molecules for pharmaceutical, diagnostic and industrial uses. This potential is attractive to the industry due to the inadequate capacity in facilities used for recombinant production of desired molecules and the increasing demand by the pharmaceutical industry for use of these facilities. Numerous attempts to produce transgenic animals have met several problems, including low rates of gene incorporation and unstable gene incorporation. Accordingly, improved gene technologies are needed for the development of transgenic animals for the production of desired molecules.

Improved gene delivery technologies are also needed for the treatment of disease in animals and humans. Many diseases and conditions can be treated with gene-delivery technologies, which provide a gene of interest to a patient suffering from the disease or the condition. An example of such disease is Type 1 diabetes. Type 1 diabetes is an autoimmune disease that ultimately results in destruction of the insulin producing β-cells in the pancreas. Although patients with Type 1 diabetes may be treated adequately with insulin injections or insulin pumps, these therapies are only partially effective. Insulin replacement, such as via insulin injection or pump administration, cannot fully reverse the defect in the vascular endothelium found in the hyperglycemic state (Pieper et al., 1996. Diabetes Res. Clin. Pract. Suppl. S157-S162). In addition, hyper- and hypoglycemia occurs frequently despite intensive home blood glucose monitoring. Finally, careful dietary constraints are needed to maintain an adequate ratio of consumed calories consumed. This often causes major psychosocial stress for many diabetic patients. Development of gene therapies providing delivery of the insulin gene into the pancreas of diabetic patients could overcome many of these problems and result in improved life expectancy and quality of life.

Several of the prior art gene delivery technologies employed viruses that are associated with potentially undesirable side effects and safety concerns. The majority of current gene-delivery technologies useful for gene therapy rely on virus-based delivery vectors, such as adeno and adeno-associated viruses, retroviruses, and other viruses, which have been attenuated to no longer replicate. (Kay, M.A., et al. 2001. Nature Medicine 7:33-40).

There are multiple problems associated with the use or viral vectors. First, they are not tissue-specific. In fact, a gene therapy trial using adenovirus was recently halted because the vector was present in the patient's sperm (Gene trial to proceed despite fears that therapy could change child's genetic makeup. The New York Times, December 23, 2001). Second, viral vectors are likely to be transiently incorporated, which necessitates re-treating a patient at specified time intervals. (Kay, M.A., et al. 2001. Nature Medicine 7:33-40). Third, there is a concern that a viral-based vector could revert to its virulent form and cause disease. Fourth, viral-based vectors require a dividing cell for stable integration. Fifth, viral-based vectors indiscriminately integrate into various cells and tissues, which can result in undesirable germline integration. Sixth, the required high titers needed to achieve the desired effect have resulted in the death of one patient and they are believed to be responsible for induction of cancer in a separate study. (Science, News of the Week, October 4, 2002).

Accordingly, what is needed is a new vector to produce transgenic animals and humans with stably incorporated genes, which vector does not cause disease or other unwanted side effects. There is also a need for DNA constructs that would be stably incorporated into the tissues and cells of animals and humans, including cells in the resting state, which are not replicating. There is a further recognized need in the art for DNA constructs capable of delivering genes to specific tissues and cells of animals and humans.

When incorporating a gene of interest into an animal for the production of a desired protein or when incorporating a gene of interest in an animal or human for the treatment of a disease, it is often desirable to selectively activate incorporated genes using inducible promoters. These inducible promoters are regulated by substances either produced or recognized by the transcription control elements within the cell in which the gene is incorporated. In many instances, control of gene expression is desired in transgenic animals or humans so that incorporated genes are selectively activated at desired times and/or under the influence of specific substances. Accordingly, what is needed is a means to selectively activate genes introduced into the genome of cells of a transgenic animal or human. This can be taken a step further to cause incorporation to be tissue-specific, which prevents widespread gene incorporation throughout a patient's body (animal or human). This decreases the amount of DNA needed for a treatment, decreases the chance of incorporation in gametes, and targets gene delivery, incorporation, and expression to the desired tissue where the gene is needed to function.

US-A-5,719,055 discloses a transposon-based vector for the integration of DNA into a host genome.

US-B1-6,218,185 relates to nucleic acid and amino acid sequences for transformation constructs containing specific transposable elements.

Kozak et al. (Journal of Mol. Biol., vol. 196, 1987, pp. 947-950) discloses that sequences flanking the AUG initiator codon influence its recognition by eukaryotic ribosomes. At least six nucleotides preceding the AUG initiator codon enhance translation in mammalian cells.

### SUMMARY OF THE INVENTION

The present invention addresses the problems described above by providing a vector and the use of this vector for producing transgenic animals.

Transgenic animals include all egg-laying animals and milk-producing animals. Transgenic animals further include but are not limited to avians, fish, amphibians, reptiles, insects, mammals and humans. In a preferred embodiment, the animal is an avian animal. In another preferred embodiment, the animal is a milk-producing animal, including but not limited to bovine, porcine, ovine and equine animals. Animals are made transgenic through administration of a composition comprising a transposon-based vector designed for stable incorporation of a gene of interest for production of a desired protein, together with an acceptable carrier. A transfection reagent is optionally added to the composition before administration.

The transposon-based vectors of the present invention include a) a modified transposase gene operably linked to a first promoter, wherein the nucleic acid sequence 3' to the first promoter comprises the sequence as set forth in SEQ ID NO: 13, wherein SEQ ID NO: 13 contains the Kozak sequence and a start codon for the transposase, and wherein at least one of the first twenty codons of the transposase gene are modified from the wild-type sequence by changing a nucleotide at a third base position of the codon to an adenine or thymine without modifying the amino acid encoded by the codon, and b) one or more genes of interest operably-linked to one or more additional promoters, and wherein the one or more genes of interest and their operably-linked promoters are flanked by transposase insertion sequences recognized by the transposase encoded by the modified transposase gene, wherein the promoter which is operably linked to the gene of interest is selected from the group consisting of an ovalbumin promoter, a conalbumin promoter, a vitellogenin promoter or an ovomucoid promoter. The transposon-based vector also includes the following characteristics: a) one or more modified Kozak sequences comprising ACCATG (SEQ ID NO:13) at the 3' end of the first promoter to enhance expression of the transposase; b) modifications of the codons for the first several N-terminal amino acids of the transposase, wherein the nucleotide at the third base position of each codon was changed to an A or a T without changing the corresponding amino acid; c) addition of one or more stop codons to enhance the termination of transposase synthesis; and/or, d) addition of an effective polyA sequence operably-linked to the transposase to further enhance expression of the transposase gene.

Use of the vectors and compositions, respectively, of the present invention results in highly efficient and stable incorporation of a gene of interest into the genome of transfected animals. For example, transgenic avians have been mated and produce transgenic progeny in the G1 generation. The transgenic progeny have been mated and produce transgenic progeny in the G2 generation.

The present invention also provides for tissue-specific incorporation and/or expression of a gene of interest. Tissue-specific incorporation of a gene of interest may be achieved by placing the transposase gene under the control of a tissue-specific promoter, whereas tissue-specific expression of a gene of interest may be achieved by placing the gene of interest under the control of a tissue-specific promoter. According to the invention the promoter which is operably linked to the gene of interest is selected from the group consisting of an ovalbumin promoter, a conalbumin promoter, a vitellogenin promoter or an ovomucoid promoter. In some embodiments, the gene of interest is transcribed under the influence of an ovalbumin, or other oviduct specific, promoter. Linking the gene of interest to an oviduct specific promoter in an egg-laying animal results in synthesis of a desired molecule and deposition of the desired molecule in a developing egg. The present invention further provides for stable incorporation and expression of genes in the epithelial cells of the mammary gland in milk-producing animals. Transcription of the gene of interest in the epithelial cells of the mammary gland results in synthesis of a desired molecule and deposition of the desired molecule in the milk. A preferred molecule is a protein. In some embodiments, the desired molecule deposited in the milk is an antiviral protein, an antibody, or a serum protein.

In other embodiments, specific incorporation of the proinsulin gene into liver cells of a diabetic animal results in the improvement of the animal's condition. Such improvement is achieved by placing a transposase gene under the control of a liver-specific promoter, which drives integration of the gene of interest in liver cells of the diabetic animal.

The present invention advantageously produces a high number of transgenic animals having a gene of interest stably incorporated. These transgenic animals successfully pass the desired gene to their progeny. The transgenic animals of the present invention also produce large amounts of a desired molecule encoded by the transgene. Transgenic egg-laying animals, particularly avians, produce large amounts of a desired protein that is deposited in the egg for rapid harvest and purification. Transgenic milk-producing animals produce large amounts of a desired protein that is deposited in the milk for rapid harvest and purification.

Any desired gene may be incorporated into the novel transposon-based vectors of the present invention in order to synthesize a desired molecule in the transgenic animals. Proteins, peptides and nucleic acids are preferred desired molecules to be produced by the transgenic animals of the present invention. Particularly preferred proteins are antibody proteins.

This invention provides a composition useful for the production of transgenic hens capable of producing substantially high amounts of a desired protein or peptide. Entire flocks of transgenic birds may be developed very quickly in order to produce industrial amounts of desired molecules. The present invention solves the problems inherent in the inadequate capacity of fermentation facilities used for bacterial production of molecules and provides a more efficient and economical way to produce desired molecules. Accordingly, the present invention provides a means to produce large amounts of therapeutic, diagnostic and reagent molecules.

Transgenic chickens are excellent in terms of convenience and efficiency of manufacturing molecules such as proteins and peptides. Starting with a single transgenic rooster, thousands of transgenic offspring can be produced within a year. (In principle, up to forty million offspring could be produced in just three

It is another object of the present invention to provide novel transposon-based vectors that encode for the production of desired proteins or peptides in cells.

It is an object of the present invention to produce transgenic animals through administration of a transposon-based vector.

Another object of the present invention is to produce transgenic animals through administration of a transposon-based vector, wherein the transgenic animals produce desired proteins or peptides.

Yet another object of the present invention is to produce transgenic animals through administration of a transposon-based vector, wherein the transgenic animals produce desired proteins or peptides and deposit the proteins or peptides in eggs or milk.

It is a further object of the present invention to produce transgenic animals through intraembryonic, intratesticular or intraoviductal administration of a transposon-based vector.

It is a further object of the present invention to provide the use of a vector according to the invention for producing a transgenic animal which is capable of producing transgenic progeny.

Yet another object of the present invention relates to the use of a vector according to the invention to produce transgenic animals through administration of a vector according to the invention that are capable of producing a desired molecule, such as a protein, peptide or nucleic acid.

Another object of the present invention relates to the use of a vector according to the invention to produce transgenic animals through administration of a vector according to the invention, wherein such administration results in modulation of endogenous gene expression.

It is another object of the present invention to provide transposon-vectors useful for cell- or tissue-specific expression of a gene of interest in an animal or human with the purpose of gene therapy.

Yet another object of the present invention relates to the use of a vector according to the invention to produce transgenic avians through administration of a vector according to the invention that are capable of producing proteins, peptides or nucleic acids.

It is another object of the present invention to produce transgenic animals through administration of a transposon-based vector encoding an antibody or a fragment thereof. generations). Each transgenic female is expected to lay at least 250 eggs/year, each potentially containing hundreds of milligrams of the selected protein. Flocks of chickens numbering in the hundreds of thousands are readily handled through established commercial systems. The technologies for obtaining eggs and fractionating them are also well known and widely accepted. Thus, for each therapeutic, diagnostic, or other protein of interest, large amounts of a substantially pure material can be produced at relatively low incremental cost.

A wide range of recombinant peptides and proteins can be produced in transgenic egg-laying animals and milk-producing animals. Enzymes, hormones, antibodies, growth factors, serum proteins, commodity proteins, biological response modifiers, peptides and designed proteins may all be made through practice of the present invention. For example, rough estimates suggest that it is possible to produce in bulk growth hormone, insulin, or Factor VIII, and deposit them in transgenic egg whites, for an incremental cost in the order of one dollar per gram. At such prices it is feasible to consider administering such medical agents by inhalation or even orally, instead of through injection. Even if bioavailability rates through these avenues were low, the cost of a much higher effective-dose would not be prohibitive.

In one embodiment, the egg-laying transgenic animal is an avian. The use of a vector according to the invention may be done with respect to avians including Ratites, Psittaciformes, Falconiformes, Piciformes, Strigiformes, Passeriformes, Coraciformes, Ralliformes, Cuculiformes, Columbiformes, Galliformes, Anseriformes, and Herodiones. Preferably, the egg-laying transgenic animal is a poultry bird. More preferably, the bird is a chicken, turkey, duck, goose or quail. Another preferred bird is a ratite, such as, an emu, an ostrich, a rhea, or a cassowary. Other preferred birds are partridge, pheasant, kiwi, parrot, parakeet, macaw, falcon, eagle, hawk, pigeon, cockatoo, song birds, jay bird, blackbird, finch, warbler, canary, toucan, mynah, or sparrow.

In another embodiment, the transgenic animal is a milk-producing animal, including but not limited to bovine, ovine, porcine, equine, and primate animals. Milk-producing animals include but are not limited to cows, goats, horses, pigs, buffalo, rabbits, non-human primates, and humans.

Accordingly, it is an object of the present invention to provide novel transposon-based vectors. Still another object of the present invention relates to the use of a vector according to the invention to produce transgenic avians through administration of a vector according to the invention that are capable of producing proteins or peptides and depositing these proteins or peptides in the egg.

Another object of the present invention is to provide transgenic avians that contain a stably incorporated transgene.

Still another object of the present invention is to provide eggs containing desired proteins or peptides encoded by a transgene incorporated into the transgenic avian that produces the egg.

A further object of the present invention relates to the use of a vector according to the invention to produce transgenic milk-producing animals through administration of a vector according to the invention that are capable of producing proteins, peptides or nucleic acids.

Still another object of the present invention relates to the use of a vector according to the invention to produce transgenic milk-producing animals through administration of a vector according to the invention that are capable of producing proteins or peptides and depositing these proteins or peptides in their milk.

Another object of the present invention is to provide transgenic milk-producing animals that contain a stably incorporated transgene.

Another object of the present invention is to provide transgenic milk-producing animals that are capable of producing proteins or peptides and depositing these proteins or peptides in their milk.

Yet another object of the present invention is to provide milk containing desired molecules encoded by a transgene incorporated into the transgenic milk-producing animals that produce the milk.

Still another object of the present invention is to provide milk containing desired proteins or peptides encoded by a transgene incorporated into the transgenic milk-producing animals that produce the milk.

A further object of the present invention relates to the use of a vector according to the invention to produce transgenic sperm through administration of a vector according to the invention to an animal.

A further object of the present invention to provide transgenic sperm that contain a stably incorporated transgene.

An advantage of the present invention is that transgenic animals are produced with higher efficiencies than observed in the prior art.

Another advantage of the present invention is that these transgenic animals possess high copy numbers of the transgene.

Another advantage of the present invention is that the transgenic animals produce large amounts of desired molecules encoded by the transgene.

Still another advantage of the present invention is that desired molecules are produced by the transgenic animals much more efficiently and economically than prior art methods, thereby providing a means for large scale production of desired molecules, particularly proteins and peptides.

These and other objects, features and advantages of the present invention will become apparent after a review of the following detailed description of the disclosed embodiments and claims.

### BRIEF DESCRIPTION OF THE FIGURES

Figure 1 depicts schematically a transposon-based vector containing a transposase operably linked to a first promoter and a gene of interest operably-linked to a second promoter, wherein the gene of interest and its operably-linked promoter are flanked by insertion sequences (IS) recognized by the transposase. "Pro" designates a promoter. In this and subsequent figures, the size of the actual nucleotide sequence is not necessarily proportionate to the box representing that sequence.
Figure 2 depicts schematically a transposon-based vector for targeting deposition of a polypeptide in an egg white wherein Ov pro is the ovalbumin promoter, Ov protein is the ovalbumin protein and PolyA is a polyadenylation sequence. The TAG sequence includes a spacer, the gp41 hairpin loop from HIV I and a protein cleavage site.
Figure 3 depicts schematically a transposon-based vector for targeting deposition of a polypeptide in an egg white wherein Ovo pro is the ovomucoid promoter and Ovo SS is the ovomucoid signal sequence. The TAG sequence includes a spacer, the gp41 hairpin loop from HIV I and a protein cleavage site.
Figure 4 depicts schematically a transposon-based vector for targeting deposition of a polypeptide in an egg yolk wherein Vit pro is the vitellogenin promoter and Vit targ is the vitellogenin targeting sequence.
Figure 5 depicts schematically a transposon-based vector for expression of antibody heavy and light chains. Prepro indicates a prepro sequence from cecropin and pro indicates a pro sequence from cecropin.
Figure 6 depicts schematically a transposon-based vector for expression of antibody heavy and light chains. Ent indicates an enterokinase cleavage sequence.

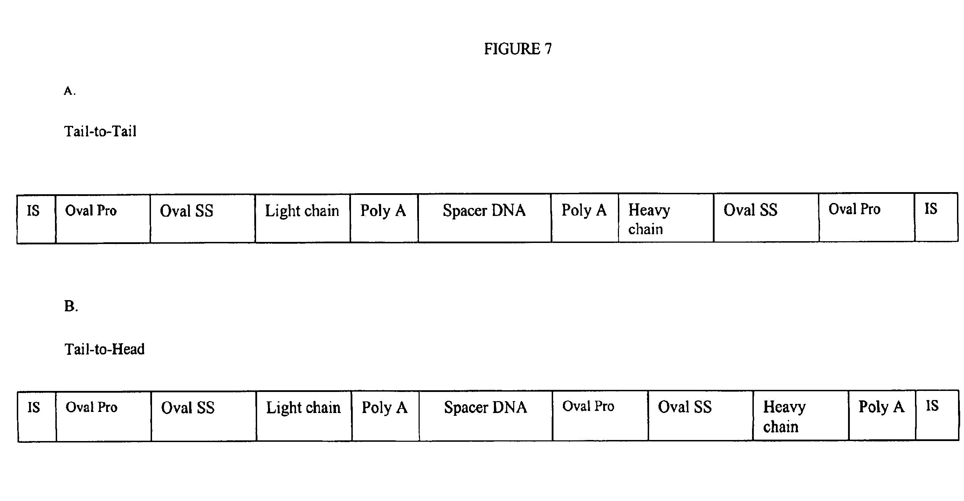

Figure 7 depicts schematically egg white targeted expression of antibody heavy and light chains from one vector in either tail-to-tail (Figure 7A) or tail-to-head (Figure 7B) configuration. In the tail-to-tail configuration, the ovalbumin signal sequence adjacent to the gene for the light chain contains on its 3' end an enterokinase cleavage site (not shown) to allow cleavage of the signal sequence from the light chain, and the ovalbumin signal sequence adjacent to the gene for the heavy chain contains on its 5' end an enterokinase cleavage site (not shown) to allow cleavage of the signal sequence from the heavy chain. In the tail-to-head configuration, the ovalbumin signal sequence adjacent to the gene for the heavy chain and the light chain contains on its 3' end an enterokinase cleavage site (not shown) to allow cleavage of the signal sequence from the heavy or light chain.

### DETAILED DESCRIPTION OF THE INVENTION

The present invention provides the use of a vector according to the invention for producing transgenic animals, particularly egg-laying animals and milk-producing animals, through administration of a composition comprising a vector according to the invention designed for stable incorporation of a gene of interest for production of a desired molecule.

### Definitions

It is to be understood that as used in the specification and in the claims, "a" or "an" can mean one or more, depending upon the context in which it is used. Thus, for example, reference to "a cell" can mean that at least one cell can be utilized.

The term "antibody" is used interchangeably with the term "immunoglobulin" and is defined herein as a protein synthesized by an animal or a cell of the immune system in response to the presence of a foreign substance commonly referred to as an "antigen" or an "immunogen". The term antibody includes fragments of antibodies. Antibodies are characterized by specific affinity to a site on the antigen, wherein the site is referred to an "antigenic determinant" or an "epitope". Antigens can be naturally occurring or artificially engineered. Artificially engineered antigens include but are not limited to small molecules, such as small peptides, attached to haptens such as macromolecules, for example proteins, nucleic acids, or polysaccharides. Artificially designed or engineered variants of naturally occurring antibodies and artificially designed or engineered antibodies not occurring in nature are all included in the current definition. Such variants include conservatively substituted amino acids and other forms of substitution as described in the section concerning proteins and polypeptides.

As used herein, the term "egg-laying animal" includes all amniotes such as birds, turtles, lizards and monotremes. Monotremes are egg-laying mammals and include the platypus and echidna. The term "bird" or "fowl," as used herein, is defined as a member of the Aves class of animals which are characterized as warm-blooded, egg-laying vertebrates primarily adapted for flying. Avians include, without limitation, Ratites, Psittaciformes, Falconiformes, Piciformes, Strigiformes, Passeriformes, Coraciformes, Ralliformes, Cuculiformes, Columbiformes, Galliformes, Anseriformes, and Herodiones. The term "Ratite," as used herein, is defined as a group of flightless, mostly large, running birds comprising several orders and including the emus, ostriches, kiwis, and cassowaries. The term "Psittaciformes", as used herein, includes parrots and refers to a monofamilial order of birds that exhibit zygodactylism and have a strong hooked bill. A "parrot" is defined as any member of the avian family Psittacidae (the single family of the Psittaciformes), distinguished by the short, stout, strongly hooked beak. The term "chicken" as used herein denotes chickens used for table egg production, such as egg-type chickens, chickens reared for public meat consumption, or broilers, and chickens reared for both egg and meat production ("dual-purpose" chickens). The term "chicken" also denotes chickens produced by primary breeder companies, or chickens that are the parents, grandparents, great-grandparents, etc. of those chickens reared for public table egg, meat, or table egg and meat consumption.

The term "egg" is defined herein as a large female sex cell enclosed in a porous, calcarous or leathery shell, produced by birds and reptiles. The term "ovum" is defined as a female gamete, and is also known as an egg. Therefore, egg production in all animals other than birds and reptiles, as used herein, is defined as the production and discharge of an ovum from an ovary, or "ovulation". Accordingly, it is to be understood that the term "egg" as used herein is defined as a large female sex cell enclosed in a porous, calcarous or leathery shell, when a bird or reptile produces it, or it is an ovum when it is produced by all other animals.

The term "milk-producing animal" refers herein to mammals including, but not limited to, bovine, ovine, porcine, equine, and primate animals. Milk-producing animals include but are not limited to cows, llamas, camels, goats, reindeer, zebu, water buffalo, yak, horses, pigs, rabbits, non-human primates, and humans.

The term "gene" is defined herein to include a coding region for a protein, peptide or polypeptide.

The term "vector" is used interchangeably with the terms "construct", "DNA construct" and "genetic construct" to denote synthetic nucleotide sequences used for manipulation of genetic material, including but not limited to cloning, subcloning, sequencing, or introduction of exogenous genetic material into cells, tissues or organisms, such as birds. It is understood by one skilled in the art that vectors may contain synthetic DNA sequences, naturally occurring DNA sequences, or both. The vectors of the present invention are transposon-based vectors as described herein.

When referring to two nucleotide sequences, one being a regulatory sequence, the term "operably-linked" is defined herein to mean that the two sequences are associated in a manner that allows the regulatory sequence to affect expression of the other nucleotide sequence. It is not required that the operably-linked sequences be directly adjacent to one another with no intervening sequence(s).

The term "regulatory sequence" is defined herein as including promoters, enhancers and other expression control elements such as polyadenylation sequences, matrix attachment sites, insulator regions for expression of multiple genes on a single construct, ribosome entry/attachment sites, introns that are able to enhance expression, and silencers.

### Transposon-Based Vectors

While not wanting to be bound by the following statement, it is believed that the nature of the DNA construct is an important factor in successfully producing transgenic animals. The "standard" types of plasmid and viral vectors that have previously been almost universally used for transgenic work in all species, especially avians, have low efficiencies and may constitute a major reason for the low rates of transformation previously observed. The DNA (or RNA) constructs previously used often do not integrate into the host DNA, or integrate only at low frequencies. Other factors may have also played a part, such as poor entry of the vector into target cells. The present invention provides transposon-based vectors that can be administered to an animal that overcome the prior art problems relating to low transgene integration frequencies. Two preferred transposon-based vectors of the present invention in which a tranposase, gene of interest and other polynucleotide sequences may be introduced are termed pTnMCS (SEQ ID NO:36) and pTnMod (SEQ ID NO:1).

The transposon-based vectors of the present invention produce integration frequencies an order of magnitude greater than has been achieved with previous vectors. More specifically, intratesticular injections performed with a prior art transposon-based vector (described in U.S. Patent No. 5,719,055) resulted in 41% sperm positive roosters whereas intratesticular injections performed with the novel transposon-based vectors of the present invention resulted in 77% sperm positive roosters. Actual frequencies of integration were estimated by either or both comparative strength of the PCR signal from the sperm and histological evaluation of the testes and sperm by quantitative PCR.

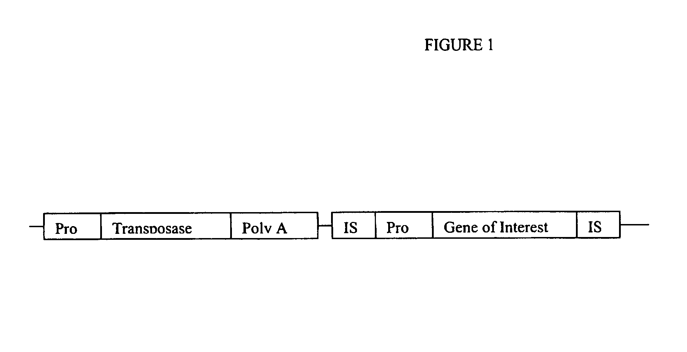

The transposon-based vectors of the present invention include a) a modified transposase gene operably linked to a first promoter, wherein the nucleic acid sequence 3' to the first promoter comprises the sequence as set forth in SEQ ID NO: 13, wherein SEQ ID NO: 13 contains the Kozak sequence and a start codon for the transposase, and wherein at least one of the first twenty codons of the transposase gene are modified from the wild-type sequence by changing a nucleotide at a third base position of the codon to an adenine or thymine without modifying the amino acid encoded by the codon, and b) one or more genes of interest operably-linked to one or more additional promoters, and wherein the one or more genes of interest and their operably-linked promoters are flanked by transposase insertion sequences recognized by the transposase encoded by the modified transposase gene, wherein the promoter which is operably linked to the gene of interest is selected from the group consisting of an ovalbumin promoter, a conalbumin promoter, a vitellogenin promoter or an ovomucoid promoter. The transposon-based vector also includes one or more of the following characteristics: a) one or more modified Kozak sequences comprising ACCATG (SEQ ID NO:13) at the 3' end of the first promoter to enhance expression of the transposase; b) modifications of the codons for the first several N-terminal amino acids of the transposase, wherein the third base of each codon was changed to an A or a T without changing the corresponding amino acid; c) addition of one or more stop codons to enhance the termination of transposase synthesis; and, d) addition of an effective polyA sequence operably-linked to the transposase to further enhance expression of the transposase gene. Figure 1 shows a schematic representation of several components of the transposon-based vector. The present invention further includes vectors containing more than one gene of interest, wherein a second or subsequent gene of interest is operably-linked to the second promoter or to a different promoter. It is also to be understood that the transposon-based vectors shown in the Figures are representational of the present invention and that the order of the vector elements may be different than that shown in the Figures, that the elements may be present in various orientations, and that the vectors may contain additional elements not shown in the Figures.

### Transposases and Insertion Sequences

In a further embodiment of the present invention, the transposase found in the transposase-based vector is an altered target site (ATS) transposase and the insertion sequences are those recognized by the ATS transposase. However, the transposase located in the transposase-based vectors is not limited to a modified ATS transposase and can be derived from any transposase. Transposases known in the prior art include those found in AC7, Tn5SEQ1, Tn916, Tn951, Tn1721, Tn 2410, Tn1681, Tn1, Tn2, Tn3, Tn4, Tn5, Tn6, Tn9, Tn10, Tn30, Tn101, Tn903, Tn501, Tn1000 (γδ), Tn1681, Tn2901, AC transposons, Mp transposons, Spm transposons, En transposons, Dotted transposons, Mu transposons, Ds transposons, dSpm transposons and I transposons. According to the present invention, these transposases and their regulatory sequences are modified for improved functioning as follows: a) the addition one or more modified Kozak sequences comprising ACCATG (SEQ ID NO:13) at the 3' end of the promoter operably-linked to the transposase; b) a change of the codons for the first several amino acids of the transposase, wherein the third base of each codon was changed to an A or a T without changing the corresponding amino acid; c) the addition of one or more stop codons to enhance the termination of transposase synthesis; and/or, d) the addition of an effective polyA sequence operably-linked to the transposase to further enhance expression of the transposase gene.

Although not wanting to be bound by the following statement, it is believed that the modifications of the first several N-terminal codons of the transposase gene increase transcription of the transposase gene, in part, by increasing strand dissociation. It is preferable that between approximately 1 and 20, more preferably 3 and 15, and most preferably between 4 and 12 of the first N-terminal codons of the transposase are modified such that the third base of each codon is changed to an A or a T without changing the encoded amino acid. In one embodiment, the first ten N-terminal codons of the transposase gene are modified in this manner. It is also preferred that the transposase contain mutations that make it less specific for preferred insertion sites and thus increases the rate of transgene insertion as discussed in U.S. Patent No. 5,719,055.

In some embodiments, the transposon-based vectors are optimized for expression in a particular host by changing the methylation patterns of the vector DNA. For example, prokaryotic methylation may be reduced by using a methylation deficient organism for production of the transposon-based vector. The transposon-based vectors may also be methylated to resemble eukaryotic DNA for expression in a eukaryotic host.

Transposases and insertion sequences from other analogous eukaryotic transposon-based vectors that can also be modified and used are, for example, the Drosophila P element derived vectors disclosed in U.S. Patent No. 6,291,243; the Drosophila mariner element described in Sherman et al. (1998); or the sleeping beauty transposon. See also Hackett et al. (1999); D. Lampe et al., 1999. Proc. Natl. Acad. Sci. USA, 96:11428-11433; S. Fischer et al., 2001. Proc. Natl. Acad. Sci. USA, 98:6759-6764; L. Zagoraiou et al., 2001. Proc. Natl. Acad. Sci. USA, 98:11474-11478; and D. Berg et al. (Eds.), Mobile DNA, Amer. Soc. Microbiol. (Washington, D.C., 1989). However, it should be noted that bacterial transposon-based elements are preferred, as there is less likelihood that a eukaryotic transposase in the recipient species will recognize prokaryotic insertion sequences bracketing the transgene.

Many transposases recognize different insertion sequences, and therefore, it is to be understood that a transposase-based vector will contain insertion sequences recognized by the particular transposase also found in the transposase-based vector. In a preferred embodiment of the invention, the insertion sequences have been shortened to about 70 base pairs in length as compared to those found in wild-type transposons that typically contain insertion sequences of well over 100 base pairs.

While the examples provided below incorporate a "cut and insert" Tn10 based vector that is destroyed following the insertion event, the present invention also encompasses the use of a "rolling replication" type transposon-based vector. Use of a rolling replication type transposon allows multiple copies of the transposon/transgene to be made from a single transgene construct and the copies inserted. This type of transposon-based system thereby provides for insertion of multiple copies of a transgene into a single genome. A rolling replication type transposon-based vector may be preferred when the promoter operably-linked to gene of interest is endogenous to the host cell and present in a high copy number or highly expressed. However, use of a rolling replication system may require tight control to limit the insertion events to non-lethal levels. Tn1, Tn2, Tn3, Tn4, Tn5, Tn9, Tn21, Tn501, Tn551, Tn951, Tn1721, Tn2410 and Tn2603 are examples of a rolling replication type transposon, although Tn5 could be both a rolling replication and a cut and insert type transposon.

### Stop Codons and PolyA Sequences

In one embodiment, the transposon-based vector contains two stop codons operably-linked to the transposase and/or to the gene of interest. In an alternate embodiment, one stop codon of UAA or UGA is operably linked to the transposase and/or to the gene of interest. As used herein an "effective polyA sequence" refers to either a synthetic or non-synthetic sequence that contains multiple and sequential nucleotides containing an adenine base (an A polynucleotide string) and that increases expression of the gene to which it is operably-linked. A polyA sequence may be operably-linked to any gene in the transposon-based vector including, but not limited to, a transposase gene and a gene of interest. In one embodiment, a polyA sequence comprises the polynucleotide sequence provided in SEQ ID NO:28. A preferred polyA sequence is optimized for use in the host animal or human. In one embodiment, the polyA sequence is optimized for use in a bird, and more specifically, a chicken. The chicken optimized polyA sequence generally contains a minimum of 60 base pairs, and more preferably between approximately 60 and several hundred base pairs, that precede the A polynucleotide string and thereby separate the stop codon from the A polynucleotide string. A chicken optimized polyA sequence may also have a reduced amount of CT repeats as compared to a synthetic polyA sequence. In one embodiment of the present invention, the polyA sequence comprises a conalbumin polyA sequence as provided in SEQ ID NO:33 and as taken from GenBank accession # Y00407, base pairs 10651-11058.

### Promoters and Enhancers

The first promoter operably-linked to the transposase gene can be a constitutive promoter or an inducible promoter. The second promoter operably-linked to the gene of interest is selected from the group consisting of an ovalbumin promoter, a conalbumin promoter, a vitellogenin promoter or an ovomucoid promoter. Constitutive promoters include, but are not limited to, immediate early cytomegalovirus (CMV) promoter, herpes simplex virus 1 (HSV1) immediate early promoter, SV40 promoter, lysozyme promoter, early and late CMV promoters, early and late HSV promoters, β-actin promoter, tubulin promoter, Rous-Sarcoma virus (RSV) promoter, and heat-shock protein (HSP) promoter. Inducible promoters include tissue-specific promoters, developmentally-regulated promoters and chemically inducible promoters. Examples of tissue-specific promoters include the glucose 6 phosphate (G6P) promoter, vitellogenin promoter, ovalbumin promoter, ovomucoid promoter, conalbumin promoter, ovotransferrin promoter, prolactin promoter, kidney uromodulin promoter, and placental lactogen promoter. In one embodiment, the vitellogenin promoter includes a polynucleotide sequence of SEQ ID NO:17. The G6P promoter sequence may be deduced from a rat G6P gene untranslated upstream region provided in GenBank Accession number U57552.1. Examples of developmentally-regulated promoters include the homeobox promoters and several hormone induced promoters. Examples of chemically inducible promoters include reproductive hormone induced promoters and antibiotic inducible promoters such as the tetracycline inducible promoter and the zinc-inducible metallothionine promoter.

Other inducible promoter systems include the Lac operator repressor system inducible by IPTG (isopropyl beta-D-thiogalactoside) (Cronin, A. et al. 2001. Genes and Development, v. 15), ecdysone-based inducible systems (Hoppe, U. C. et al. 2000. Mol. Ther. 1:159-164); estrogen-based inducible systems (Braselmann, S. et al. 1993. Proc. Natl. Acad. Sci. 90:1657-1661); progesterone-based inducible systems using a chimeric regulator, GLVP, which is a hybrid protein consisting of the GAL4 binding domain and the herpes simplex virus transcriptional activation domain, VP16, and a truncated form of the human progesterone receptor that retains the ability to bind ligand and can be turned on by RU486 (Wang, et al. 1994. Proc. Natl. Acad. Sci. 91:8180-8184); CID-based inducible systems using chemical inducers of dimerization (CIDs) to regulate gene expression, such as a system wherein rapamycin induces dimerization of the cellular proteins FKBP12 and FRAP (Belshaw, P. J. et al. 1996. J. Chem. Biol. 3:731-738; Fan, L. et al. 1999. Hum. Gene Ther. 10:2273-2285; Shariat, S.F. et al. 2001. Cancer Res. 61:2562-2571; Spencer, D.M. 1996. Curr. Biol. 6:839-847). Chemical substances that activate the chemically inducible promoters can be administered to the animal containing the transgene of interest via any method known to those of skill in the art.

Other examples of cell or tissue-specific and constitutive promoters include but are not limited to smooth-muscle SM22 promoter, including chimeric SM22alpha/telokin promoters (Hoggatt A.M. et al., 2002. Circ Res. 91(12):1151-9); ubiquitin C promoter (Biochim Biophys Acta, 2003. Jan. 3;1625(1):52-63); Hsf2 promoter; murine COMP (cartilage oligomeric matrix protein) promoter; early B cell-specific mb-1 promoter (Sigvardsson M., et al., 2002. Mol. Cell Biol. 22(24):8539-51); prostate specific antigen (PSA) promoter (Yoshimura I. et al., 2002, J. Urol. 168(6):2659-64); exorh promoter and pineal expression-promoting element (Asaoka Y., et al., 2002. Proc. Natl. Acad. Sci. 99(24):15456-61); neural and liver ceramidase gene promoters (Okino N. et al., 2002. Biochem. Biophys. Res. Commun. 299(1):160-6); PSP94 gene promoter/enhancer (Gabril M.Y. et al., 2002. Gene Ther. 9(23):1589-99); promoter of the human FAT/CD36 gene (Kuriki C., et al., 2002. Biol. Pharm. Bull. 25(11):1476-8); VL30 promoter (Staplin W.R. et al., 2002. Blood October 24, 2002); IL-10 promoter (Brenner S., et al., 2002. J. Biol. Chem. December 18, 2002).

Examples of avian promoters include, but are not limited to, promoters controlling expression of egg white proteins, such as ovalbumin, ovotransferrin (conalbumin), ovomucoid, lysozyme, ovomucin, g2 ovoglobulin, g3 ovoglobulin, ovoflavoprotein, ovostatin (ovomacroglobin), cystatin, avidin, thiamine-binding protein, glutamyl aminopeptidase minor glycoprotein 1, minor glycoprotein 2; and promoters controlling expression of egg-yolk proteins, such as vitellogenin, very low-density lipoproteins, low density lipoprotein, cobalamin-binding protein, riboflavin-binding protein, biotin-binding protein (Awade, 1996. Z. Lebensm. Unters. Forsch. 202:1-14). An advantage of using the vitellogenin promoter is that it is active during the egg-laying stage of an animal's life-cycle, which allows for the production of the protein of interest to be temporally connected to the import of the protein of interest into the egg yolk when the protein of interest is equipped with an appropriate targeting sequence.

Liver-specific promoters of the present invention include, but are not limited to, the following promoters, vitellogenin promoter, G6P promoter, cholesterol-7-alpha-hydroxylase (CYP7A) promoter, phenylalanine hydroxylase (PAH) promoter, protein C gene promoter, insulin-like growth factor I (IGF-I) promoter, bilirubin UDP-glucuronosyltransferase promoter, aldolase B promoter, furin promoter, metallothioneine promoter, albumin promoter, and insulin promoter.

Also included in the present invention are promoters that can be used to target expression of a protein of interest into the milk of a milk-producing animal including, but not limited to, β lactoglobin promoter, whey acidic protein promoter, lactalbumin promoter and casein promoter.

Promoters associated with cells of the immune system may also be used. Acute phase promoters such as interleukin (IL)-1 and IL-2 may be employed. Promoters for heavy and light chain Ig may also be employed. The promoters of the T cell receptor components CD4 and CD8, B cell promoters and the promoters of CR2 (complement receptor type 2) may also be employed. Immune system promoters are preferably used when the desired protein is an antibody protein.

Also included in this invention are modified promoters/enhancers wherein elements of a single promoter are duplicated, modified, or otherwise changed. In one embodiment, steroid hormone-binding domains of the ovalbumin promoter are moved from about -6.5 kb to within approximately the first 1000 base pairs of the gene of interest. Modifying an existing promoter with promoter/enhancer elements not found naturally in the promoter, as well as building an entirely synthetic promoter, or drawing promoter/enhancer elements from various genes together on a non-natural backbone, are all encompassed by the current invention.

Accordingly, it is to be understood that the promoters contained within the transposon-based vectors of the present invention may be entire promoter sequences or fragments of promoter sequences. For example, in one embodiment, the promoter operably linked to a gene of interest is an approximately 900 base pair fragment of a chicken ovalbumin promoter (SEQ ID NO:40). The constitutive and inducible promoters contained within the transposon-based vectors may also be modified by the addition of one or more modified Kozak sequences of ACCATG (SEQ ID NO:13).

As indicated above, the present invention includes transposon-based vectors containing one or more enhancers. These enhancers may or may not be operably-linked to their native promoter and may be located at any distance from their operably-linked promoter. A promoter operably-linked to an enhancer is referred to herein as an "enhanced promoter." The enhancers contained within the transposon-based vectors are preferably enhancers found in birds, and more preferably, an ovalbumin enhancer, but are not limited to these types of enhancers. In one embodiment, an approximately 675 base pair enhancer element of an ovalbumin promoter is cloned upstream of an ovalbumin promoter with 300 base pairs of spacer DNA separating the enhancer and promoter. In one embodiment, the enhancer used as a part of the present invention comprises base pairs 1-675 of a Chicken Ovalbumin enhancer from GenBank accession #S82527.1. The polynucleotide sequence of this enhancer is provided in SEQ ID NO:37.

Also included in some of the transposon-based vectors of the present invention are cap sites and fragments of cap sites. In one embodiment, approximately 50 base pairs of a 5' untranslated region wherein the capsite resides are added on the 3' end of an enhanced promoter or promoter. An exemplary 5' untranslated region is provided in SEQ ID NO:38. A putative cap-site residing in this 5' untranslated region preferably comprises the polynucleotide sequence provided in SEQ ID NO: 39.

In one embodiment of the present invention, the first promoter operably-linked to the transposase gene is a constitutive promoter and the second promoter operably-linked to the gene of interest is a tissue-specific promoter. In this embodiment, use of the first constitutive promoter allows for constitutive activation of the transposase gene and incorporation of the gene of interest into virtually all cell types, including the germline of the recipient animal. Although the gene of interest is incorporated into the germline generally, the gene of interest is only expressed in a tissue-specific manner. It should be noted that cell- or tissue-specific expression as described herein does not require a complete absence of expression in cells or tissues other than the preferred cell or tissue. Instead, "cell-specific" or "tissue-specific" expression refers to a majority of the expression of a particular gene of interest in the preferred cell or tissue, respectively.

When incorporation of the gene of interest into the germline is not preferred, the first promoter operably-linked to the transposase gene can be a tissue-specific promoter. For example, transfection of a transposon-based vector containing a transposase gene operably-linked to a liver-specific promoter such as the G6P promoter or vitellogenin promoter provides for activation of the transposase gene and incorporation of the gene of interest in the cells of the liver but not into the germline and other cells generally. In this second embodiments, the second promoter operably-linked to the gene of interest can be an ovalbumin promoter, a conalbumin promoter, a vitellogenin promoter or an ovomucoid promoter. In embodiments wherein tissue-specific expression or incorporation is desired, it is preferred that the transposon-based vector is administered directly to the tissue of interest or to an artery leading to the tissue of interest.

Accordingly, cell specific promoters may be used to enhance transcription in selected tissues. In birds, for example, promoters that are found in cells of the fallopian tube, such as ovalbumin, conalbumin, ovomucoid and/or lysozyme, are used in the vectors to ensure transcription of the gene of interest in the epithelial cells and tubular gland cells of the fallopian tube, leading to synthesis of the desired protein encoded by the gene and deposition into the egg white. In mammals, promoters specific for the epithelial cells of the alveoli of the mammary gland, such as prolactin, insulin, beta lactoglobin, whey acidic protein, lactalbumin, casein, and/or placental lactogen, are used in the design of vectors used for transfection of these cells for the production of desired proteins for deposition into the milk. In liver cells, the G6P promoter may be employed to drive transcription of the gene of interest for protein production. Proteins made in the liver of birds may be delivered to the egg yolk.

In order to achieve higher or more efficient expression of the transposase gene, the promoter and other regulatory sequences operably-linked to the transposase gene may be those derived from the host. These host specific regulatory sequences can be tissue specific as described above or can be of a constitutive nature. For example, an avian actin promoter and its associated polyA sequence can be operably-linked to a transposase in a transposase-based vector for transfection into an avian. Examples of other host specific promoters that .could be operably-linked to the transposase include the myosin and DNA or RNA polymerase promoters.

### Directing Sequences

In some embodiments of the present invention, the gene of interest is operably-linked to a directing sequence or a sequence that provides proper conformation to the desired protein encoded by the gene of interest. As used herein, the term "directing sequence" refers to both signal sequences and targeting sequences. An egg directing sequence includes, but is not limited to, an ovomucoid signal sequence, an ovalbumin signal sequence and a vitellogenin targeting sequence. The term "signal sequence" refers to an amino acid sequence, or the polynucleotide sequence that encodes the amino acid sequence, that directs the protein to which it is linked to the endoplasmic reticulum in a eukaryote, and more preferably the translocational pores in the endoplasmic reticulum, or the plasma membrane in a prokaryote, or mitochondria, such us for the purpose of gene therapy of mitochondrial diseases. Signal and targeting sequences can be used to direct a desired protein into, for example, the milk, when the transposon-based vectors are administered to a milk-producing animal.

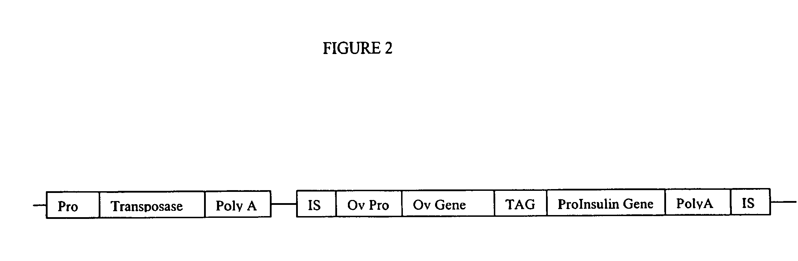

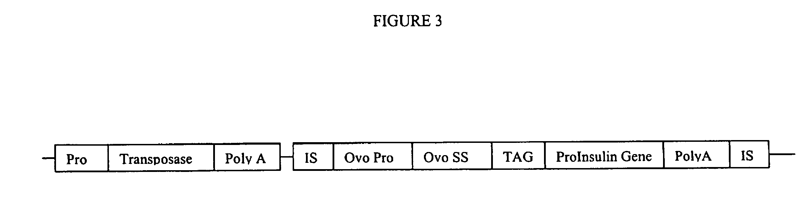

Signal sequences can also be used to direct a desired protein into, for example, a secretory pathway for incorporation into the egg yolk or the egg white, when the transposon-based vectors are administered to a bird or other egg-laying animal. One example of such a transposon-based vector is provided in Figure 3 wherein the gene of interest is operably linked to the ovomucoid signal sequence. The present invention also includes a gene of interest operably-linked to a second gene containing a signal sequence. An example of such an embodiment is shown in Figure 2 wherein the gene of interest is operably-linked to the ovalbumin gene that contains an ovalbumin signal sequence. Other signal sequences that can be included in the transposon-based vectors include, but are not limited to the ovotransferrin and lysozyme signal sequences.

As also used herein, the term "targeting sequence" refers to an amino acid sequence, or the polynucleotide sequence encoding the amino acid sequence, which amino acid sequence is recognized by a receptor located on the exterior of a cell. Binding of the receptor to the targeting sequence results in uptake of the protein or peptide operably-linked to the targeting sequence by the cell. One example of a targeting sequence is a vitellogenin targeting sequence that is recognized by a vitellogenin receptor (or the low density lipoprotein receptor) on the exterior of an oocyte. In one embodiment, the vitellogenin targeting sequence includes the polynucleotide sequence of SEQ ID NO: 18. In another embodiment, the vitellogenin targeting sequence includes all or part of the vitellogenin gene. Other targeting sequences include VLDL and Apo E, which are also capable of binding the vitellogenin receptor. Since the ApoE protein is not endogenously expressed in birds, its presence may be used advantageously to identify birds carrying the transposon-based vectors of the present invention.

### Genes of Interest Encoding Desired Proteins

A gene of interest selected for stable incorporation is designed to encode any desired protein or peptide or to regulate any cellular response. In some embodiments, the desired proteins or peptides are deposited in an egg or in milk. It is to be understood that the present invention encompasses transposon-based vectors containing multiple genes of interest. The multiple genes of interest may each be operably-linked to a separate promoter and other regulatory sequence(s) or may all be operably-linked to the same promoter and other regulatory sequences(s). In one embodiment, multiple gene of interest are linked to a single promoter and other regulatory sequence(s) and each gene of interest is separated by a cleavage site or a pro portion of a signal sequence.

Protein and peptide hormones are a preferred class of proteins in the present invention. Such protein and peptide hormones are synthesized throughout the endocrine system and include, but are not limited to, hypothalamic hormones and hypophysiotropic hormones, anterior, intermediate and posterior pituitary hormones, pancreatic islet hormones, hormones made in the gastrointestinal system, renal hormones, thymic hormones, parathyroid hormones, adrenal cortical and medullary hormones. Specifically, hormones that can be produced using the present invention include, but are not limited to, chorionic gonadotropin, corticotropin, erythropoietin, glucagons, IGF-1, oxytocin, platelet-derived growth factor, calcitonin, follicle-stimulating hormone, leutinizing hormone, thyroid-stimulating hormone, insulin, gonadotropin-releasing hormone and its analogs, vasopressin, octreotide, somatostatin, prolactin, adrenocorticotropic hormone, antidiuretic hormone, thyrotropin-releasing hormone (TRH), growth hormone-releasing hormone (GHRH), dopamine, melatonin, thyroxin (T₄), parathyroid hormone (PTH), glucocorticoids such as cortisol, mineralocorticoids such as aldosterone, androgens such as testosterone, adrenaline (epinephrine), noradrenaline (norepinephrine), estrogens such as estradiol, progesterone, glucagons, calcitrol, calciferol, atrial-natriuretic peptide, gastrin, secretin, cholecystokinin (CCK), neuropeptide Y, ghrelin, PYY₃₋₃₆, angiotensinogen, thrombopoietin, and leptin. By using appropriate polynucleotide sequences, species-specific hormones may be made by transgenic animals.

In one embodiment of the present invention, the gene of interest is a proinsulin gene and the desired molecule is insulin. Proinsulin consists of three parts: a C-peptide and two long strands of amino acids (called the alpha and beta chains) that later become linked together to form the insulin molecule. Figures 2 and 3 are schematics of transposon-based vector constructs containing a proinsulin gene operably-linked to an ovalbumin promoter and ovalbumin protein or an ovomucoid promoter and ovomucoid signal sequence, respectively. In these embodiments, proinsulin is expressed in the oviduct tubular gland cells and then deposited in the egg white. One example of a proinsulin polynucleotide sequence is shown in SEQ ID NO:21, wherein the C-peptide cleavage site spans from Arg at position 31 to Arg at position 65.

Serum proteins including lipoproteins such as high density lipoprotein (HDL), HDL-Milano and low density lipoprotein, albumin, clotting cascade factors, factor VIII, factor IX, fibrinogen, and globulins are also included in the group of desired proteins of the present invention. Immunoglobulins are one class of desired globulin molecules and include but are not limited to IgG, IgM, IgA, IgD, IgE, IgY, lambda chains, kappa chains and fragments thereof; Fc fragments, and Fab fragments. Desired antibodies include, but are not limited to, naturally occurring antibodies, human antibodies, humanized antibodies, and hybrid antibodies. Genes encoding modified versions of naturally occurring antibodies or fragments thereof and genes encoding artificially designed antibodies or fragments thereof may be incorporated into the transposon-based vectors of the present invention. Desired antibodies also include antibodies with the ability to bind specific ligands, for example, antibodies against proteins associated with cancer-related molecules, such as anti-her 2, or anti-CA125. Accordingly, the present invention encompasses a transposon-based vector containing one or more genes encoding a heavy immunoglobulin (Ig) chain and a light Ig chain. Further, more than one gene encoding for more than one antibody may be administered in one or more transposon-based vectors of the present invention. In this manner, an egg may contain more than one type of antibody in the egg white, the egg yolk or both.

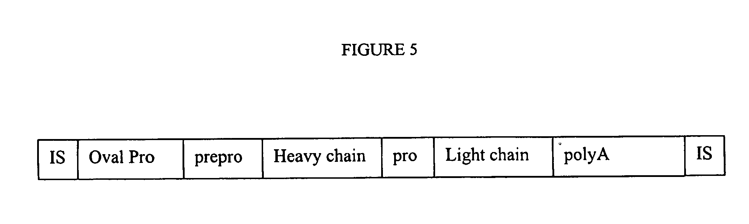

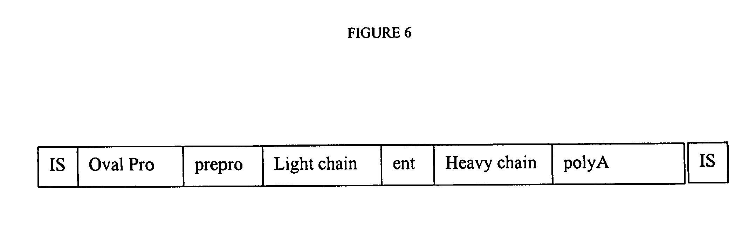

In one embodiment, a transposon-based vector contains a heavy Ig chain and a light Ig chain, both operably linked to a promoter. Figures 5 and 6 schematically depict exemplary constructs of this embodiment. More specifically, Figure 5 shows a construct containing a cecropin pre-pro sequence and a cecropin pro sequence, wherein the pre sequence functions to direct the resultant protein into the endoplasmic reticulum and the pro sequences and the pro sequences are cleaved upon secretion of the protein from a cell into which the construct has been transfected. Figure 6 shows a construct containing an enterokinase cleavage site. In this embodiment, it may be required to further remove several additional amino acids from the light chain following cleavage by enterokinase. In another embodiment, the transposon-based vector comprises a heavy Ig chain operably-linked to one promoter and a light Ig chain operably-linked to another promoter. Figure 7 schematically depicts an exemplary construct of this embodiment. The present invention also encompasses a transposon-based vector containing genes encoding portions of a heavy Ig chain and/or portions of a light Ig chain. The present invention further includes a transposon-based vector containing a gene that encodes a fusion protein comprising a heavy and/or light Ig chain, or portions thereof.

Antibodies used as therapeutic reagents include but are not limited to antibodies for use in cancer immunotherapy against specific antigens, or for providing passive immunity to an animal or a human against an infectious disease or a toxic agent. Antibodies used as diagnostic reagents include, but are not limited to antibodies that may be labeled and detected with a detector, for example antibodies with a fluorescent label attached that may be detected following exposure to specific wavelengths. Such labeled antibodies may be primary antibodies directed to a specific antigen, for example, rhodamine-labeled rabbit anti-growth hormone, or may be labeled secondary antibodies, such as fluorescein-labeled goat-anti chicken IgG. Such labeled antibodies are known to one of ordinary skill in the art. Labels useful for attachment to antibodies are also known to one of ordinary skill in the art. Some of these labels are described in the "Handbook of Fluorescent Probes and Research Products", ninth edition, Richard P. Haugland (ed) Molecular Probes, Inc. Eugene, OR), which is incorporated herein in its entirety.

Antibodies produced with using the present invention may be used as laboratory reagents for numerous applications including radioimmunoassay, western blots, dot blots, ELISA, immunoaffinity columns and other procedures requiring antibodies as known to one of ordinary skill in the art. Such antibodies include primary antibodies, secondary antibodies and tertiary antibodies, which may be labeled or unlabeled.

Antibodies that may be made with the practice of the present invention include, but are not limited to primary antibodies, secondary antibodies, designer antibodies, anti-protein antibodies, anti-peptide antibodies, anti-DNA antibodies, anti-RNA antibodies, anti-hormone antibodies, anti-hypophysiotropic peptides, antibodies against non-natural antigens, anti-anterior pituitary hormone antibodies, anti-posterior pituitary hormone antibodies, anti-venom antibodies, anti-tumor marker antibodies, antibodies directed against epitopes associated with infectious disease, including, antiviral, anti-bacterial, anti-protozoal, anti-fungal, anti-parasitic, anti-receptor, anti-lipid, anti-phospholipid, anti-growth factor, anti-cytokine, anti-monokine, anti-idiotype, and anti-accessory (presentation) protein antibodies. Antibodies made with the present invention, as well as light chains or heavy chains, may also be used to inhibit enzyme activity.

Antibodies that may be produced using the present invention include, but are not limited to, antibodies made against the following proteins: Bovine γ-Globulin, Serum; Bovine IgG, Plasma; Chicken γ-Globulin, Serum; Human γ-Globulin, Serum; Human IgA, Plasma; Human IgA₁, Myeloma; Human IgA₂, Myeloma; Human IgA₂, Plasma; Human IgD, Plasma; Human IgE, Myeloma; Human IgG, Plasma; Human IgG, Fab Fragment, Plasma; Human IgG, F(ab')₂ Fragment, Plasma; Human IgG, Fc Fragment, Plasma; Human IgG₁, Myeloma; Human IgG₂, Myeloma; Human IgG₃, Myeloma; Human IgG₄, Myeloma; Human IgM, Myeloma; Human IgM, Plasma; Human Immunoglobulin, Light Chain κ, Urine; Human Immunoglobulin, Light Chains κ and λ, Plasma; Mouse γ-Globulin, Serum; Mouse IgG, Serum; Mouse IgM, Myeloma; Rabbit γ-Globulin, Serum; Rabbit IgG, Plasma; and Rat γ-Globulin, Serum. In one embodiment, the transposon-based vector comprises the coding sequence of light and heavy chains of a murine monoclonal antibody that shows specificity for human seminoprotein (GenBank Accession numbers AY129006 and AY129304 for the light and heavy chains, respectively).

A further non-limiting list of antibodies that recognize other antibodies is as follows: Anti-Chicken IgG, heavy (H) & light (L) Chain Specific (Sheep); Anti-Goat γ-Globulin (Donkey); Anti-Goat IgG, Fc Fragment Specific (Rabbit); Anti-Guinea Pig γ-Globulin (Goat); Anti-Human Ig, Light Chain, Type κ Specific; Anti-Human Ig, Light Chain, Type λ Specific; Anti-Human IgA, α-Chain Specific (Goat); Anti-Human IgA, Fab Fragment Specific; Anti-Human IgA, Fc Fragment Specific; Anti-Human IgA, Secretory; Anti-Human IgE, ε-Chain Specific (Goat); Anti-Human IgE, Fc Fragment Specific; Anti-Human IgG, Fc Fragment Specific (Goat); Anti-Human IgG, γ-Chain Specific (Goat); Anti-Human IgG, Fc Fragment Specific; Anti-Human IgG, Fd Fragment Specific; Anti-Human IgG, H & L Chain Specific (Goat); Anti-Human IgG₁, Fc Fragment Specific; Anti-Human IgG₂, Fc Fragment Specific; Anti-Human IgG₂, Fd Fragment Specific; Anti-Human IgG₃, Hinge Specific; Anti-Human IgG₄, Fc Fragment Specific; Anti-Human IgM, Fc Fragment Specific; Anti-Human IgM, µ-Chain Specific; Anti-Mouse IgE, ε-Chain Specific; Anti-Mouse γ-Globulin (Goat); Anti-Mouse IgG, γ-Chain Specific (Goat); Anti-Mouse IgG, γ-Chain Specific (Goat) F(ab')₂ Fragment; Anti-Mouse IgG, H & L Chain Specific (Goat); Anti-Mouse IgM, µ-Chain Specific (Goat); Anti-Mouse IgM, H & L Chain Specific (Goat); Anti-Rabbit γ-Globulin (Goat); Anti-Rabbit IgG, Fc Fragment Specific (Goat); Anti-Rabbit IgG, H & L Chain Specific (Goat); Anti-Rat γ-Globulin (Goat); Anti-Rat IgG, H & L Chain Specific; Anti-Rhesus Monkey γ-Globulin (Goat); and, Anti-Sheep IgG, H & L Chain Specific.

Another non-limiting list of the antibodies that may be produced using the present invention is provided in product catalogs of companies such as Phoenix Pharmaceuticals, Inc. (www.phoenixpeptide.com; 530 Harbor Boulevard, Belmont, CA), Peninsula Labs San Carlos CA, SIGMA, St.Louis, MO www.sigma-aldrich.com, Cappel ICN, Irvine, California, www.icnbiomed.com, and Calbiochem, La Jolla, California, www.calbiochem.com, which are all incorporated herein by reference in their entirety. The polynucleotide sequences encoding these antibodies may be obtained from the scientific literature, from patents, and from databases such as GenBank. Alternatively, one of ordinary skill in the art may design the polynucleotide sequence to be incorporated into the genome by choosing the codons that encode for each amino acid in the desired antibody. Antibodies made by the transgenic animals of the present invention include antibodies that may be used as therapeutic reagents, for example in cancer immunotherapy against specific antigens, as diagnostic reagents and as laboratory reagents for numerous applications including immunoneutralization, radioimmunoassay, western blots, dot blots, ELISA, immunoprecipitation and immunoaffinity columns. Some of these antibodies include, but are not limited to, antibodies which bind the following ligands: adrenomedulin, amylin, calcitonin, amyloid, calcitonin gene-related peptide, cholecystokinin, gastrin, gastric inhibitory peptide, gastrin releasing peptide, interleukin, interferon, cortistatin, somatostatin, endothelin, sarafotoxin, glucagon, glucagon-like peptide, insulin, atrial natriuretic peptide, BNP, CNP, neurokinin, substance P, leptin, neuropeptide Y, melanin concentrating hormone, melanocyte stimulating hormone, orphanin, endorphin, dynorphin, enkephalin, enkephalin, leumorphin, peptide F, PACAP, PACAP-related peptide, parathyroid hormone, urocortin, corticotrophin releasing hormone, PHM, PHI, vasoactive intestinal polypeptide, secretin, ACTH, angiotensin, angiostatin, bombesin, endostatin, bradykinin, FMRF amide, galanin, gonadotropin releasing hormone (GnRH) associated peptide, GnRH, growth hormone releasing hormone, inhibin, granulocyte-macrophage colony stimulating factor (GM-CSF), motilin, neurotensin, oxytocin, vasopressin, osteocalcin, pancreastatin, pancreatic polypeptide, peptide YY, proopiomelanocortin, transforming growth factor, vascular endothelial growth factor, vesicular monoamine transporter, vesicular acetylcholine transporter, ghrelin, NPW, NPB, C3d, prokinetican, thyroid stimulating hormone, luteinizing hormone, follicle stimulating hormone, prolactin, growth hormone, beta-lipotropin, melatonin, kallikriens, kinins, prostaglandins, erythropoietin, p146 (SEQ ID NO:18 amino acid sequence, SEQ ID NO:19, nucleotide sequence), estrogen, testosterone, corticosteroids, mineralocorticoids, thyroid hormone, thymic hormones, connective tissue proteins, nuclear proteins, actin, avidin, activin, agrin, albumin, and prohormones, propeptides, splice variants, fragments and analogs thereof.

The following is yet another non-limiting of antibodies that can be produced by the methods of present invention: abciximab (ReoPro), abciximab anti-platelet aggregation monoclonal antibody, anti-CD11a (hu1124), anti-CD18 antibody, anti-CD20 antibody, anti-cytomegalovirus (CMV) antibody, anti-digoxin antibody, anti-hepatitis B antibody, anti-HER-2 antibody, anti-idiotype antibody to GD3 glycolipid, anti-IgE antibody, anti-IL-2R antibody, antimetastatic cancer antibody (mAb 17-1 A), anti-rabies antibody, anti-respiratory syncytial virus (RSV) antibody, anti-Rh antibody, anti-TCR, anti-TNF antibody, anti-VEGF antibody and fab fragment thereof, rattlesnake venom antibody, black widow spider venom antibody, coral snake venom antibody, antibody against very late antigen-4 (VLA-4), C225 humanized antibody to EGF receptor, chimeric (human & mouse) antibody against TNFα, antibody directed against GPIIb/IIIa receptor on human platelets, gamma globulin, anti-hepatitis B immunoglobulin, human anti-D immunoglobulin, human antibodies against *S aureus,* human tetanus immunoglobulin, humanized antibody against the epidermal growth receptor-2, humanized antibody against the α subunit of the interleukin-2 receptor, humanized antibody CTLA4IG, humanized antibody to the IL-2 R α-chain, humanized anti-CD40-ligand monoclonal antibody (5c8), humanized mAb against the epidermal growth receptor-2, humanized mAb to rous sarcoma virus, humanized recombinant antibody (IgGlk) against respiratory syncytial virus (RSV), lymphocyte immunoglobulin (anti-thymocyte antibody), lymphocyte immunoglobulin, mAb against factor VII, MDX-210 bi-specific antibody against HER-2, MDX-22, MDX-220 bi-specific antibody against TAG-72 on tumors, MDX-33 antibody to FcγR1 receptor, MDX-447 bi-specific antibody against EGF receptor, MDX-447 bispecific humanized antibody to EGF receptor, MDX-RA immunotoxin (ricin A linked) antibody, Medi-507 antibody (humanized form of BTI-322) against CD2 receptor on T-cells, monoclonal antibody LDP-02, muromonab-CD3(OKT3) antibody, OKT3 ("muromomab-CD3") antibody, PRO 542 antibody, ReoPro ("abciximab") antibody, and TNF-IgG fusion protein.

The antibodies prepared using the methods of the present invention may also be designed to possess specific labels that may be detected through means known to one of ordinary skill in the art. The antibodies may also be designed to possess specific sequences useful for purification through means known to one of ordinary skill in the art. Specialty antibodies designed for binding specific antigens may also be made in transgenic animals using the transposon-based vectors of the present invention.

Production of a monoclonal antibody using the transposon-based vectors of the present invention can be accomplished in a variety of ways. In one embodiment, two vectors may be constructed: one that encodes the light chain, and a second vector that encodes the heavy chain of the monoclonal antibody. These vectors may then be incorporated into the genome of the target animal by methods disclosed herein. In an alternative embodiment, the sequences encoding light and heavy chains of a monoclonal antibody may be included on a single DNA construct. For example, the coding sequence of light and heavy chains of a murine monoclonal antibody that show specificity for human seminoprotein can be expressed using transposon-based constructs of the present invention (GenBank Accession numbers AY129006 and AY 129304 for the light and heavy chains, respectively).

Further included in the present invention are proteins and peptides synthesized by the immune system including those synthesized by the thymus, lymph nodes, spleen, and the gastrointestinal associated lymph tissues (GALT) system. The immune system proteins and peptides proteins that can be made in transgenic animals using the transposon-based vectors of the present invention include, but are not limited to, alpha-interferon, beta-interferon, gamma-interferon, alpha-interferon A, alpha-interferon 1, G-CSF, GM-CSF, interlukin-1 (IL-1), IL-2, IL-3, IL-4, IL-5, IL-6, IL-7, IL-8, IL-9, IL-10, IL-11, IL-12, IL-13, TNF-α, and TNF-β. Other cytokines included in the present invention include cardiotrophin, stromal cell derived factor, macrophage derived chemokine (MDC), melanoma growth stimulatory activity (MGSA), macrophage inflammatory proteins 1 alpha (MIP-1 alpha), 2, 3 alpha, 3 beta, 4 and 5.

Lytic peptides such as p146 are also included in the desired molecules of the present invention. In one embodiment, the p146 peptide comprises an amino acid sequence of SEQ ID NO:19. The present invention also encompasses a transposon-based vector comprising a p146 nucleic acid comprising a polynucleotide sequence of SEQ ID NO:20.

Enzymes are another class of proteins that may be made through the use of the transposon-based vectors of the present invention. Such enzymes include but are not limited to adenosine deaminase, alpha-galactosidase, cellulase, collagenase, dnaseI, hyaluronidase, lactase, L-asparaginase, pancreatin, papain, streptokinase B, subtilisin, superoxide dismutase, thrombin, trypsin, urokinase, fibrinolysin, glucocerebrosidase and plasminogen activator. In some embodiments wherein the enzyme could have deleterious effects, additional amino acids and a protease cleavage site are added to the carboxy end of the enzyme of interest in order to prevent expression of a functional enzyme. Subsequent digestion of the enzyme with a protease results in activation of the enzyme.

Extracellular matrix proteins are one class of desired proteins that may be made through the use of the present invention. Examples include but are not limited to collagen, fibrin, elastin, laminin, and fibronectin and subtypes thereof. Intracellular proteins and structural proteins are other classes of desired proteins in the present invention.

Growth factors are another desired class of proteins that may be made through the use of the present invention and include, but are not limited to, transforming growth factor-α ("TGF-α"), transforming growth factor-β (TGF-β), platelet-derived growth factors (PDGF), fibroblast growth factors (FGF), including FGF acidic isoforms 1 and 2, FGF basic form 2 and FGF 4, 8, 9 and 10, nerve growth factors (NGF) including NGF 2.5s, NGF 7.0s and beta NGF and neurotrophins, brain derived neurotrophic factor, cartilage derived factor, growth factors for stimulation of the production of red blood cells, growth factors for stimulation of the production of white blood cells, bone growth factors (BGF), basic fibroblast growth factor, vascular endothelial growth factor (VEGF), granulocyte colony stimulating factor (G-CSF), insulin like growth factor (IGF) I and II, hepatocyte growth factor, glial neurotrophic growth factor (GDNF), stem cell factor (SCF), keratinocyte growth factor (KGF), transforming growth factors (TGF), including TGFs alpha, beta, betal, beta2, beta3, skeletal growth factor, bone matrix derived growth factors, bone derived growth factors, erythropoietin (EPO) and mixtures thereof.

Another desired class of proteins that may be made may be made through the use of the present invention include but are not limited to leptin, leukemia inhibitory factor (LIF), tumor necrosis factor alpha and beta, ENBREL, angiostatin, endostatin, thrombospondin, osteogenic protein-1, bone morphogenetic proteins 2 and 7, osteonectin, somatomedin-like peptide, and osteocalcin.

A non-limiting list of the peptides and proteins that may be made may be made through the use of the present invention is provided in product catalogs of companies such as Phoenix Pharmaceuticals, Inc. (www.phoenixpeptide.com; 530 Harbor Boulevard • Belmont, CA), Peninsula Labs San Carlos CA, SIGMA, St.Louis, MO www.sigma-aldrich.com, Cappel ICN, Irvine, California, www.icnbiomed.com, and Calbiochem, La Jolla, California, www.calbiochem.com. The polynucleotide sequences encoding these proteins and peptides of interest may be obtained from the scientific literature, from patents, and from databases such as GenBank. Alternatively, one of ordinary skill in the art may design the polynucleotide sequence to be incorporated into the genome by choosing the codons that encode for each amino acid in the desired protein or peptide.

Some of these desired proteins or peptides that may be made through the use of the present invention include but are not limited to the following: adrenomedulin, amylin, calcitonin, amyloid, calcitonin gene-related peptide, cholecystokinin, gastrin, gastric inhibitory peptide, gastrin releasing peptide, interleukin, interferon, cortistatin, somatostatin, endothelin, sarafotoxin, glucagon, glucagon-like peptide, insulin, atrial natriuretic peptide, BNP, CNP, neurokinin, substance P, leptin, neuropeptide Y, melanin concentrating hormone, melanocyte stimulating hormone, orphanin, endorphin, dynorphin, enkephalin, leumorphin, peptide F, PACAP, PACAP-related peptide, parathyroid hormone, urocortin, corticotrophin releasing hormone, PHM, PHI, vasoactive intestinal polypeptide, secretin, ACTH, angiotensin, angiostatin, bombesin, endostatin, bradykinin, FMRF amide, galanin, gonadotropin releasing hormone (GnRH) associated peptide, GnRH, growth hormone releasing hormone, inhibin, granulocyte-macrophage colony stimulating factor (GM-CSF), motilin, neurotensin, oxytocin, vasopressin, osteocalcin, pancreastatin, pancreatic polypeptide, peptide YY, proopiomelanocortin, transforming growth factor, vascular endothelial growth factor, vesicular monoamine transporter, vesicular acetylcholine transporter, ghrelin, NPW, NPB, C3d, prokinetican, thyroid stimulating hormone, luteinizing hormone, follicle stimulating hormone, prolactin, growth hormone, beta-lipotropin, melatonin, kallikriens, kinins, prostaglandins, erythropoietin, p146 (SEQ ID NO:19, amino acid sequence, SEQ ID NO:20, nucleotide sequence), thymic hormones, connective tissue proteins, nuclear proteins, actin, avidin, activin, agrin, albumin, and prohormones, propeptides, splice variants, fragments and analogs thereof.

Other desired proteins that may be made by the transgenic animals of the present invention include bacitracin, polymixin b, vancomycin, cyclosporine, anti-RSV antibody, alpha-1 antitrypsin (AAT), anti-cytomegalovirus antibody, anti-hepatitis antibody, anti-inhibitor coagulant complex, anti-rabies antibody, anti-Rh(D) antibody, adenosine deaminase, anti-digoxin antibody, antivenin crotalidae (rattlesnake venom antibody), antivenin latrodectus (black widow spider venom antibody), antivenin micrurus (coral snake venom antibody), aprotinin, corticotropin (ACTH), diphtheria antitoxin, lymphocyte immune globulin (anti-thymocyte antibody), protamine, thyrotropin, capreomycin, α-galactosidase, gramicidin, streptokinase, tetanus toxoid, tyrothricin, IGF-1, proteins of varicella vaccine, anti-TNF antibody, anti-IL-2r antibody, anti-HER-2 antibody, OKT3 ("muromonab-CD3") antibody, TNF-IgG fusion protein, ReoPro ("abciximab") antibody, ACTH fragment 1-24, desmopressin, gonadotropin-releasing hormone, histrelin, leuprolide, lypressin, nafarelin, peptide that binds GPIIb/GPIIIa on platelets (integrilin), goserelin, capreomycin, colistin, anti-respiratory syncytial virus, lymphocyte immune globulin (Thymoglovin, Atgam), panorex, alpha-antitrypsin, botulinin, lung surfactant protein, tumor necrosis receptor-IgG fusion protein (enbrel), gonadorelin, proteins of influenza vaccine, proteins of rotavirus vaccine, proteins of haemophilus b conjugate vaccine, proteins of poliovirus vaccine, proteins of pneumococcal conjugate vaccine, proteins of meningococcal C vaccine, proteins of influenza vaccine, megakaryocyte growth and development factor (MGDF), neuroimmunophilin ligand-A (NIL-A), brain-derived neurotrophic factor (BDNF), glial cell line-derived neurotrophic factor (GDNF), leptin (native), leptin B, leptin C, IL-1RA (interleukin-1RA), R-568, novel erythropoiesis-stimulating protein (NESP), humanized mAb to rous sarcoma virus (MEDI-493), glutamyl-tryptophan dipeptide IM862, LFA-3TIP immunosuppressive, humanized anti-CD40-ligand monoclonal antibody (5c8), gelsonin enzyme, tissue factor pathway inhibitor (TFPI), proteins of meningitis B vaccine, antimetastatic cancer antibody (mAb 17-1A), chimeric (human & mouse) mAb against TNFα, mAb against factor VII, relaxin, capreomycin, glycopeptide (LY333328), recombinant human activated protein C (rhAPC), humanized mAb against the epidermal growth receptor-2, altepase, anti-CD20 antigen, C2B8 antibody, insulin-like growth factor-1, atrial natriuretic peptide (anaritide), tenectaplase, anti-CD11a antibody (hu 1124), anti-CD18 antibody, mAb LDP-02, anti-VEGF antibody, fab fragment of anti-VEGF Ab, APO2 ligand (tumor necrosis factor-related apoptosis-inducing ligand), rTGF-β (transforming growth factor-β), alpha-antitrypsin, ananain (a pineapple enzyme), humanized mAb CTLA4IG, PRO 542 (mAb), D2E7 (mAb), calf intestine alkaline phosphatase, α-L-iduronidase, α-L-galactosidase (humanglutamic acid decarboxylase, acid sphingomyelinase, bone morphogenetic protein-2 (rhBMP-2), proteins of HIV vaccine, T cell receptor (TCR) peptide vaccine, TCR peptides, V beta 3 and V beta 13.1. (IR502), (IR501), BI 1050/1272 mAb against very late antigen-4 (VLA-4), C225 humanized mAb to EGF receptor, anti-idiotype antibody to GD3 glycolipid, antibacterial peptide against *H. pylori,* MDX-447 bispecific humanized mAb to EGF receptor, anti-cytomegalovirus (CMV), Medi-491 B19 parvovirus vaccine, humanized recombinant mAb (IgGlk) against respiratory syncytial virus (RSV), urinary tract infection vaccine (against "pili" on *Escherechia coli* strains), proteins of lyme disease vaccine against *B. burgdorferi* protein (DbpA), proteins of Medi-501 human papilloma virus-11 vaccine (HPV), *Streptococcus pneumoniae* vaccine, Medi-507 mAb (humanized form of BTI-322) against CD2 receptor on T-cells, MDX-33 mAb to FcγR1 receptor, MDX-RA immunotoxin (ricin A linked) mAb, MDX-210 bi-specific mAb against HER-2, MDX-447 bi-specific mAb against EGF receptor, MDX-22, MDX-220 bi-specific mAb against TAG-72 on tumors, colony-stimulating factor (CSF) (molgramostim), humanized mAb to the IL-2 R α-chain (basiliximab), mAb to IgE (IGE 025A), myelin basic protein-altered peptide (MSP771A), humanized mAb against the epidermal growth receptor-2, humanized mAb against the α subunit of the interleukin-2 receptor, low molecular weight heparin, anti-hemophillic factor, and bactericidal/permeability-increasing protein (r-BPI).

The peptides and proteins made using the present invention may be labeled using labels and techniques known to one of ordinary skill in the art. Some of these labels are described in the "Handbook of Fluorescent Probes and Research Products", ninth edition, Richard P. Haugland (ed) Molecular Probes, Inc. Eugene, OR), which is incorporated herein in its entirety. Some of these labels may be genetically engineered into the polynucleotide sequence for the expression of the selected protein or peptide. The peptides and proteins may also have label-incorporation "handles" incorporated to allow labeling of an otherwise difficult or impossible to label protein.

It is to be understood that the various classes of desired peptides and proteins, as well as specific peptides and proteins described in this section may be modified as described below by inserting selected codons for desired amino acid substitutions into the gene incorporated into the transgenic animal.

The present invention may also be used to produce desired molecules other than proteins and peptides including, but not limited to, lipoproteins such as high density lipoprotein (HDL), HDL-Milano, and low density lipoprotein, lipids, carbohydrates, siRNA and ribozymes. In these embodiments, a gene of interest encodes a nucleic acid molecule or a protein that directs production of the desired molecule.

The present invention further encompasses the use of inhibitory molecules to inhibit endogenous (i.e., non-vector) protein production. These inhibitory molecules include antisense nucleic acids, siRNA and inhibitory proteins. In one embodiment, a transposon-based vector containing an ovalbumin DNA sequence, that upon transcription forms a double stranded RNA molecule, is transfected into an animal such as a bird and the bird's production of endogenous ovalbumin protein is reduced by the interference RNA mechanism (RNAi). Additionally, inducible knockouts or knockdowns of the endogenous protein may be created to achieve a reduction or inhibition of endogenous protein production.

### Modified Desired Proteins and Peptides

"Proteins", "peptides," "polypeptides" and "oligopeptides" are chains of amino acids (typically L-amino acids) whose alpha carbons are linked through peptide bonds formed by a condensation reaction between the carboxyl group of the alpha carbon of one amino acid and the amino group of the alpha carbon of another amino acid. The terminal amino acid at one end of the chain (i.e., the amino terminal) has a free amino group, while the terminal amino acid at the other end of the chain (i.e., the carboxy terminal) has a free carboxyl group. As such, the term "amino terminus" (abbreviated N-terminus) refers to the free alpha-amino group on the amino acid at the amino terminal of the protein, or to the alpha-amino group (imino group when participating in a peptide bond) of an amino acid at any other location within the protein. Similarly, the term "carboxy terminus" (abbreviated C-terminus) refers to the free carboxyl group on the amino acid at the carboxy terminus of a protein, or to the carboxyl group of an amino acid at any other location within the protein.

Typically, the amino acids making up a protein are numbered in order, starting at the amino terminal and increasing in the direction toward the carboxy terminal of the protein. Thus, when one amino acid is said to "follow" another, that amino acid is positioned closer to the carboxy terminal of the protein than the preceding amino acid.

The term "residue" is used herein to refer to an amino acid (D or L) or an amino acid mimetic that is incorporated into a protein by an amide bond. As such, the amino acid may be a naturally occurring amino acid or, unless otherwise limited, may encompass known analogs of natural amino acids that function in a manner similar to the naturally occurring amino acids (i.e., amino acid mimetics). Moreover, an amide bond mimetic includes peptide backbone modifications well known to those skilled in the art.

Furthermore, one of skill will recognize that, as mentioned above, individual substitutions, deletions or additions which alter, add or delete a single amino acid or a small percentage of amino acids (typically less than about 5%, more typically less than about 1%) in an encoded sequence are conservatively modified variations where the alterations result in the substitution of an amino acid with a chemically similar amino acid. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

A conservative substitution is a substitution in which the substituting amino acid (naturally occurring or modified) is structurally related to the amino acid being substituted, i.e., has about the same size and electronic properties as the amino acid being substituted. Thus, the substituting amino acid would have the same or a similar functional group in the side chain as the original amino acid. A "conservative substitution" also refers to utilizing a substituting amino acid which is identical to the amino acid being substituted except that a functional group in the side chain is protected with a suitable protecting group.

Suitable protecting groups are described in Green and Wuts, "Protecting Groups in Organic Synthesis", John Wiley and Sons, Chapters 5 and 7, 1991, the teachings of which are incorporated herein by reference. Preferred protecting groups are those which facilitate transport of the peptide through membranes, for example, by reducing the hydrophilicity and increasing the lipophilicity of the peptide, and which can be cleaved, either by hydrolysis or enzymatically (Ditter et al., 1968. J. Pharm. Sci. 57:783; Ditter et al., 1968. J. Pharm. Sci. 57:828; Ditter et al., 1969. J. Pharm. Sci. 58:557; King et al., 1987. Biochemistry 26:2294; Lindberg et al., 1989. Drug Metabolism and Disposition 17:311; Tunek et al., 1988. Biochem. Pharm. 37:3867; Anderson et al., 1985 Arch. Biochem. Biophys. 239:538; and Singhal et al., 1987. FASEB J. 1:220). Suitable hydroxyl protecting groups include ester, carbonate and carbamate protecting groups. Suitable amine protecting groups include acyl groups and alkoxy or aryloxy carbonyl groups, as described above for N-terminal protecting groups. Suitable carboxylic acid protecting groups include aliphatic, benzyl and aryl esters, as described below for C-terminal protecting groups. In one embodiment, the carboxylic acid group in the side chain of one or more glutamic acid or aspartic acid residues in a peptide of the present invention is protected, preferably as a methyl, ethyl, benzyl or substituted benzyl ester, more preferably as a benzyl ester.

Provided below are groups of naturally occurring and modified amino acids in which each amino acid in a group has similar electronic and steric properties. Thus, a conservative substitution can be made by substituting an amino acid with another amino acid from the same group. It is to be understood that these groups are non-limiting, i.e. that there are additional modified amino acids which could be included in each group.
Group I includes leucine, isoleucine, valine, methionine and modified amino acids having the following side chains: ethyl, n-propyl n-butyl. Preferably, Group I includes leucine, isoleucine, valine and methionine.
Group II includes glycine, alanine, valine and a modified amino acid having an ethyl side chain. Preferably, Group II includes glycine and alanine.
Group III includes phenylalanine, phenylglycine, tyrosine, tryptophan, cyclohexylmethyl glycine, and modified amino residues having substituted benzyl or phenyl side chains. Preferred substituents include one or more of the following: halogen, methyl, ethyl, nitro, -NH₂, methoxy, ethoxy and CN. Preferably, Group III includes phenylalanine, tyrosine and tryptophan.
Group IV includes glutamic acid, aspartic acid, a substituted or unsubstituted aliphatic, aromatic or benzylic ester of glutamic or aspartic acid (e.g., methyl, ethyl, n-propyl iso-propyl, cyclohexyl, benzyl or substituted benzyl), glutamine, asparagine, -CO-NH- alkylated glutamine or asparagines (e.g., methyl, ethyl, n-propyl and iso-propyl) and modified amino acids having the side chain -(CH₂)₃-COOH, an ester thereof (substituted or unsubstituted aliphatic, aromatic or benzylic ester), an amide thereof and a substituted or unsubstituted N-alkylated amide thereof. Preferably, Group IV includes glutamic acid, aspartic acid, methyl aspartate, ethyl aspartate, benzyl aspartate and methyl glutamate, ethyl glutamate and benzyl glutamate, glutamine and asparagine.
Group V includes histidine, lysine, ornithine, arginine, N-nitroarginine, β-cycloarginine, γ-hydroxyarginine, N-amidinocitruline and 2-amino-4-guanidinobutanoic acid, homologs of lysine, homologs of arginine and homologs of ornithine. Preferably, Group V includes histidine, lysine, arginine and ornithine. A homolog of an amino acid includes from 1 to about 3 additional or subtracted methylene units in the side chain.
Group VI includes serine, threonine, cysteine and modified amino acids having C1-C5 straight or branched alkyl side chains substituted with -OH or -SH, for example, -CH₂CH₂OH, -CH₂CH₂CH₂OH or -CH₂CH₂OHCH₃. Preferably, Group VI includes serine, cysteine or threonine.

In another aspect, suitable substitutions for amino acid residues include "severe" substitutions. A "severe substitution" is a substitution in which the substituting amino acid (naturally occurring or modified) has significantly different size and/or electronic properties compared with the amino acid being substituted. Thus, the side chain of the substituting amino acid can be significantly larger (or smaller) than the side chain of the amino acid being substituted and/or can have functional groups with significantly different electronic properties than the amino acid being substituted. Examples of severe substitutions of this type include the substitution of phenylalanine or cyclohexylmethyl glycine for alanine, isoleucine for glycine, a D amino acid for the corresponding L amino acid, or NH-CH[(-CH₂)₅-COOH]-CO- for aspartic acid. Alternatively, a functional group may be added to the side chain, deleted from the side chain or exchanged with another functional group. Examples of severe substitutions of this type include adding of valine, leucine or isoleucine, exchanging the carboxylic acid in the side chain of aspartic acid or glutamic acid with an amine, or deleting the amine group in the side chain of lysine or ornithine. In yet another alternative, the side chain of the substituting amino acid can have significantly different steric and electronic properties that the functional group of the amino acid being substituted. Examples of such modifications include tryptophan for glycine, lysine for aspartic acid and - (CH₂)₄COOH for the side chain of serine. These examples are not meant to be limiting.

In another embodiment, for example in the synthesis of a peptide 26 amino acids in length, the individual amino acids may be substituted according in the following manner:
AA₁ is serine, glycine, alanine, cysteine or threonine;
AA₂ is alanine, threonine, glycine, cysteine or serine;
AA₃ is valine, arginine, leucine, isoleucine, methionine, ornithine, lysine, N-nitroarginine, β-cycloarginine, γ-hydroxyarginine, N-amidinocitruline or 2-amino-4-guanidinobutanoic acid;
AA₄ is proline, leucine, valine, isoleucine or methionine;
AA₅ is tryptophan, alanine, phenylalanine, tyrosine or glycine;
AA₆ is serine, glycine, alanine, cysteine or threonine;
AA₇ is proline, leucine, valine, isoleucine or methionine;
AA₈ is alanine, threonine, glycine, cysteine or serine;
AA₉ is alanine, threonine, glycine, cysteine or serine;
AA₁₀ is leucine, isoleucine, methionine or valine;
AA₁₁ is serine, glycine, alanine, cysteine or threonine;
AA₁₂ is leucine, isoleucine, methionine or valine;
AA₁₃ is leucine, isoleucine, methionine or valine;
AA₁₄ is glutamine, glutamic acid, aspartic acid, asparagine, or a substituted or unsubstituted aliphatic or aryl ester of glutamic acid or aspartic acid;
AA₁₅ is arginine, N-nitroarginine, β-cycloarginine, γ-hydroxy-arginine, N-amidinocitruline or 2-amino-4-guanidino-butanoic acid
AA₁₆ is proline, leucine, valine, isoleucine or methionine;
AA₁₇ is serine, glycine, alanine, cysteine or threonine;
AA₁₈ is glutamic acid, aspartic acid, asparagine, glutamine or a substituted or unsubstituted aliphatic or aryl ester of glutamic acid or aspartic acid;
AA₁₉ is aspartic acid, asparagine, glutamic acid, glutamine, leucine, valine, isoleucine, methionine or a substituted or unsubstituted aliphatic or aryl ester of glutamic acid or aspartic acid;
AA₂₀ is valine, arginine, leucine, isoleucine, methionine, ornithine, lysine, N-nitroarginine, β-cycloarginine, γ-hydroxyarginine, N-amidinocitruline or 2-amino-4-guanidinobutanoic acid;
AA₂₁ is alanine, threonine, glycine, cysteine or serine;
AA₂₂ is alanine, threonine, glycine, cysteine or serine;
AA₂₃ is histidine, serine, threonine, cysteine, lysine or ornithine;
AA₂₄ is threonine, aspartic acid, serine, glutamic acid or a substituted or unsubstituted aliphatic or aryl ester of glutamic acid or aspartic acid;
AA₂₅ is asparagine, aspartic acid" glutamic acid, glutamine, leucine, valine, isoleucine, methionine or a substituted or unsubstituted aliphatic or aryl ester of glutamic acid or aspartic acid; and
AA₂₆ is cysteine, histidine, serine, threonine, lysine or ornithine.

It is to be understood that these amino acid substitutions may be made for longer or shorter peptides than the 26 mer in the preceding example above, and for proteins.

In one embodiment of the present invention, codons for the first several N-terminal amino acids of the transposase are modified such that the third base of each codon is changed to an A or a T without changing the corresponding amino acid. It is preferable that between approximately 1 and 20, more preferably 3 and 15, and most preferably between 4 and 12 of the first N-terminal codons of the gene of interest are modified such that the third base of each codon is changed to an A or a T without changing the corresponding amino acid. In one embodiment, the first ten N-terminal codons of the gene of interest are modified in this manner.

When several desired proteins, protein fragments or peptides are encoded in the gene of interest to be incorporated into the genome, one of skill in the art will appreciate that the proteins, protein fragments or peptides may be separated by a spacer molecule such as, for example, a peptide, consisting of one or more amino acids. Generally, the spacer will have no specific biological activity other than to join the desired proteins, protein fragments or peptides together, or to preserve some minimum distance or other spatial relationship between them. However, the constituent amino acids of the spacer may be selected to influence some property of the molecule such as the folding, net charge, or hydrophobicity. The spacer may also be contained within a nucleotide sequence with a purification handle or be flanked by proteolytic cleavage sites.

Such polypeptide spacers may have from about 5 to about 40 amino acid residues. The spacers in a polypeptide are independently chosen, but are preferably all the same. The spacers should allow for flexibility of movement in space and are therefore typically rich in small amino acids, for example, glycine, serine, proline or alanine. Preferably, peptide spacers contain at least 60%, more preferably at least 80% glycine or alanine. In addition, peptide spacers generally have little or no biological and antigenic activity. Preferred spacers are (Gly-Pro-Gly-Gly)ₓ (SEQ ID NO:5) and (Gly₄-Ser)_{y}, wherein x is an integer from about 3 to about 9 and y is an integer from about 1 to about 8. Specific examples of suitable spacers include
(Gly-Pro-Gly-Gly)₃

SEQ ID NO:6 Gly Pro Gly Gly Gly Pro Gly Gly Gly Pro Gly Gly
(Gly4-Ser)3

SEQ ID NO:7 Gly Gly Gly Gly Ser Gly Gly Gly Gly Ser Gly Gly Gly Gly Ser
or (Gly4-Ser)4

SEQ ID NO:8 Gly Gly Gly Gly Ser Gly Gly Gly Gly Ser Gly Gly Gly Gly Ser
Gly Gly Gly Gly Ser.

Nucleotide sequences encoding for the production of residues which may be useful in purification of the expressed recombinant protein may also be built into the vector. Such sequences are known in the art and include the glutathione binding domain from glutathione S-transferase, polylysine, hexa-histidine or other cationic amino acids, thioredoxin, hemagglutinin antigen and maltose binding protein.

Additionally, nucleotide sequences may be inserted into the gene of interest to be incorporated so that the protein or peptide can also include from one to about six amino acids that create signals for proteolytic cleavage. In this manner, if a gene is designed to make one or more peptides or proteins of interest in the transgenic animal, specific nucleotide sequences encoding for amino acids recognized by enzymes may be incorporated into the gene to facilitate cleavage of the large protein or peptide sequence into desired peptides or proteins or both. For example, nucleotides encoding a proteolytic cleavage site can be introduced into the gene of interest so that a signal sequence can be cleaved from a protein or peptide encoded by the gene of interest. Nucleotide sequences encoding other amino acid sequences which display pH sensitivity or chemical sensitivity may also be added to the vector to facilitate separation of the signal sequence from the peptide or protein of interest.

In one embodiment of the present invention, a TAG sequence is linked to the gene of interest. The TAG sequence serves three purposes: 1) it allows free rotation of the peptide or protein to be isolated so there is no interference from the native protein or signal sequence, i.e. vitellogenin, 2) it provides a "purification handle" to isolate the protein using column purification, and 3) it includes a cleavage site to remove the desired protein from the signal and purification sequences. Accordingly, as used herein, a TAG sequence includes a spacer sequence, a purification handle and a cleavage site. The spacer sequences in the TAG proteins contain one or more repeats shown in SEQ ID NO:25. A preferred spacer sequence comprises the sequence provided in SEQ ID NO:26. One example of a purification handle is the gp41 hairpin loop from HIV I. Exemplary gp41 polynucleotide and polypeptide sequences are provided in SEQ ID NO:24 and SEQ ID NO:23, respectively. However, it should be understood that any antigenic region may be used as a purification handle, including any antigenic region of gp41. Preferred purification handles are those that elicit highly specific antibodies. Additionally, the cleavage site can be any protein cleavage site known to one of ordinary skill in the art and includes an enterokinase cleavage site comprising the Asp Asp Asp Asp Lys sequence (SEQ ID NO:9) and a furin cleavage site. Constructs containing a TAG sequence are shown in Figures 2 and 3. In one embodiment of the present invention, the TAG sequence comprises a polynucleotide sequence of SEQ ID NO:22.

### Methods of Administering Transposon-Based Vectors

In addition to the transposon-based vectors described above, the present invention also includes the use of a vector according to the invention for producing a transgenic animal and makes reference to methods of administering the transposon-based vectors to an animal, methods of producing a transgenic animal wherein a gene of interest is incorporated into the germline of the animal and methods of producing a transgenic animal wherein a gene of interest is incorporated into cells other than the germline cells of the animal. The transposon-based vectors of the present invention may be administered to an animal via any method known to those of skill in the art, including, but not limited to, intraembryonic, intratesticular, intraoviduct, intraperitoneal, intraarterial, intravenous, topical, oral, nasal, and pronuclear injection methods of administration, or any combination thereof. The transposon-based vectors may also be administered within the lumen of an organ, into an organ, into a body cavity, into the cerebrospinal fluid, through the urinary system or through any route to reach the desired cells.

The transposon-based vectors may be delivered through the vascular system to be distributed to the cells supplied by that vessel. For example, the compositions may be placed in the artery supplying the ovary or supplying the fallopian tube to transfect cells in those tissues. In this manner, follicles could be transfected to create a germline transgenic animal. Alternatively, supplying the compositions through the artery leading to the oviduct would preferably transfect the tubular gland and epithelial cells. Such transfected cells could manufacture a desired protein or peptide for deposition in the egg white. Administration of the compositions through the portal vein would target uptake and transformation of hepatic cells. Administration through the urethra and into the bladder would target the transitional epithelium of the bladder. Administration through the vagina and cervix would target the lining of the uterus. Administration through the internal mammary artery would transfect secretory cells of the lactating mammary gland to perform a desired function, such as to synthesize and secrete a desired protein or peptide into the milk.

In a preferred embodiment, the animal is an egg-laying animal, and more preferably, an avian. In one embodiment, between approximately 1 and 50 µg, preferably between 1 and 20 µg, and more preferably between 5 and 10 µg of transposon-based vector DNA is administered to the oviduct of a bird. Optimal ranges depending upon the type of bird and the bird's stage of sexual maturity. Intraoviduct administration of the transposon-based vectors of the present invention result in a PCR positive signal in the oviduct tissue, whereas intravascular administration results in a PCR positive signal in the liver. In other embodiments, the transposon-based vector is administered to an artery that supplies the oviduct or the liver. These methods of administration may also be combined with any methods for facilitating transfection, including without limitation, electroporation, gene guns, injection of naked DNA, and use of dimethyl sulfoxide (DMSO).

The present invention includes the use of a vector according to the invention for intraembryonic administration of a vector according to the invention to an avian embryo comprising the following steps: 1) incubating an egg on its side at room temperature for two hours to allow the embryo contained therein to move to top dead center (TDC); 2) drilling a hole through the shell without penetrating the underlying shell membrane; 3) injecting the embryo with the transposon-based vector in solution; 4) sealing the hole in the egg; and 5) placing the egg in an incubator for hatching. Administration of the transposon-based vector can occur anytime between immediately after egg lay (when the embryo is at Stage X) and hatching. Preferably, the transposon-based vector is administered between 1 and 7 days after egg lay, more preferably between 1 and 2 days after egg lay. The transposon-based vectors may be introduced into the embryo in amounts ranging from about 5.0 µg to 10 pg, preferably 1.0 µg to 100 pg. Additionally, the transposon-based vector solution volume may be between approximately 1 µl to 75 µl in quail and between approximately 1 µl to 500 µl in chicken.

The present invention also includes the use of a vector according to the invention for intratesticular administration of a transposon-based vector including injecting a bird with a composition comprising the transposon-based vector, an appropriate carrier and an appropriate transfection reagent. In one embodiment, the bird is injected before sexual maturity, preferably between approximately 4-14 weeks, more preferably between approximately 6-14 weeks and most preferably between 8-12 weeks old. In another embodiment, a mature bird is injected with a transposon-based vector an appropriate carrier and an appropriate transfection reagent. The mature bird may be any type of bird, but in one example the mature bird is a quail.

A bird is preferably injected prior to the development of the blood-testis barrier, which thereby facilitates entry of the transposon-based vector into the seminiferous tubules and transfection of the spermatogonia or other germline cells. At and between the ages of 4, 6, 8, 10, 12, and 14 weeks, it is believed that the testes of chickens are likely to be most receptive to transfection. In this age range, the blood/testis barrier has not yet formed, and there is a relatively high number of spermatogonia relative to the numbers of other cell types, e.g., spermatids, etc. See J. Kumaran et al., 1949. Poultry Sci., 29:511-520. See also E. Oakberg, 1956. Am. J. Anatomy, 99:507-515; and P. Kluin et al., 1984. Anat. Embryol., 169:73-78.

The transposon-based vectors may be introduced into a testis in an amount ranging from about 0.1 µg to 10 µg, preferably 1 µg to 10 µg, more preferably 3 µg to 10 µg. In a quail, about 5 µg is a preferred amount. In a chicken, about 5 µg to 10 µg per testis is preferred. These amounts of vector DNA may be injected in one dose or multiple doses and at one site or multiple sites in the testis. In a preferred embodiment, the vector DNA is administered at multiple sites in a single testis, both testes being injected in this manner. In one embodiment, injection is spread over three injection sites: one at each end of the testis, and one in the middle. Additionally, the transposon-based vector solution volume may be between approximately 1 µl to 75 µl in quail and between approximately 1 µl to 500 µl in chicken. In a preferred embodiment, the transposon-based vector solution volume may be between approximately 20 µl to 60 µl in quail and between approximately 50 µl to 250 µl in chicken. Both the amount of vector DNA and the total volume injected into each testis may be determined based upon the age and size of the bird.

According to the present invention, the transposon-based vector is to be administered in conjunction with an acceptable carrier and/or transfection reagent. Acceptable carriers include, but are not limited to, water, saline, Hanks Balanced Salt Solution (HBSS), Tris-EDTA (TE) and lyotropic liquid crystals. Transfection reagents commonly known to one of ordinary skill in the art that may be employed include, but are not limited to, the following: cationic lipid transfection reagents, cationic lipid mixtures, polyamine reagents, liposomes and combinations thereof; SUPERFECT®, Cytofectene, BioPORTER®, GenePORTER®, NeuroPORTER®, and perfectin from Gene Therapy Systems; lipofectamine, cellfectin, DMRIE-C oligofectamine, and PLUS reagent from InVitrogen; Xtreme gene, fugene, DOSPER and DOTAP from Roche; Lipotaxi and Genejammer from Strategene; and Escort from SIGMA. In one embodiment, the transfection reagent is SUPERFECT®. The ratio of DNA to transfection reagent may vary based upon the method of administration. In one embodiment, the transposon-based vector is administered intratesticularly and the ratio of DNA to transfection reagent can be from 1:1.5 to 1:15, preferably 1:2 to 1:10, all expressed as wt/vol. Transfection may also be accomplished using other means known to one of ordinary skill in the art, including without limitation electroporation, gene guns, injection of naked DNA, and use of dimethyl sulfoxide (DMSO).

Depending upon the cell or tissue type targeted for transfection, the form of the transposon-based vector may be important. Plasmids harvested from bacteria are generally closed circular supercoiled molecules, and this is the preferred state of a vector for gene delivery because of the ease of preparation. In some instances, transposase expression and insertion may be more efficient in a relaxed, closed circular configuration or in a linear configuration. In still other instances, a purified transposase protein may be co-injected with a transposon-based vector containing the gene of interest for more immediate insertion. This could be accomplished by using a transfection reagent complexed with both the purified transposase protein and the transposon-based vector.

### Testing for and Breeding Animals Carrying the Transience

Following administration of a transposon-based vector to an animal, DNA is extracted from the animal to confirm integration of the gene of interest. Actual frequencies of integration are estimated both by comparative strength of the PCR signal, and by histological evaluation of the tissues by quantitative PCR. Another method for estimating the rate of transgene insertion is the so-called primed in situ hybridization technique (PRINS). This method determines not only which cells carry a transgene of interest, but also into which chromosome the gene has inserted, and even what portion of the chromosome. Briefly, labeled primers are annealed to chromosome spreads (affixed to glass slides) through one round of PCR, and the slides are then developed through normal in situ hybridization procedures. This technique combines the best features of in situ PCR and fluorescence in situ hybridization (FISH) to provide distinct chromosome location and copy number of the gene in question. The 28s rRNA gene will be used as a positive control for spermatogonia to confirm that the technique is functioning properly. Using different fluorescent labels for the transgene and the 28s gene causes cells containing a transgene to fluoresce with two different colored tags.

Breeding experiments are also conducted to determine if germline transmission of the transgene has occurred. In a general bird breeding experiment performed according to the present invention, each male bird was exposed to 2-3 different adult female birds for 3-4 days each. This procedure was continued with different females for a total period of 6-12 weeks. Eggs were collected daily for up to 14 days after the last exposure to the transgenic male, and each egg was incubated in a standard incubator. In the first series of experiments the resulting embryos were examined for transgene presence at day 3 or 4 using PCR.

Any male producing a transgenic embryo was bred to additional females. Eggs from these females were incubated, hatched, and the chicks tested for the exogenous DNA. Any embryos that died were necropsied and examined directly for the transgene or protein encoded by the transgene, either by fluorescence or PCR. The offspring that hatched and were found to be positive for the exogenous DNA were raised to maturity. These birds were bred to produce further generations of transgenic birds, to verify efficiency of the transgenic procedure and the stable incorporation of the transgene into the germ line. The resulting embryos were examined for transgene presence at day 3 or 4 using PCR.

It is to be understood that the above procedure can be modified to suit animals other than birds and that selective breeding techniques may be performed to amplify gene copy numbers and protein output.

### Production of Desired Proteins or Peptides in Egg White

In one embodiment, the transposon-based vectors of the present invention may be administered to a bird for production of desired proteins or peptides in the egg white. These trasnposon-based vectors preferably contain one or more of an ovalbumin promoter, an ovomucoid promoter, an ovalbumin signal sequence and an ovomucoid signal sequence. Oviduct-specific ovalbumin promoters are described in B. O'Malley et al., 1987. EMBO J., vol. 6, pp. 2305-12; A. Qiu et al., 1994. Proc. Nat. Acad. Sci. (USA), vol. 91, pp. 4451-4455; D. Monroe et al., 2000. Biochim. Biophys. Acta, 1517 (1):27-32; H. Park et al., 2000. Biochem., 39:8537-8545; and T. Muramatsu et al., 1996. Poult. Avian Biol. Rev., 6:107-123. Examples of transposon-based vectors designed for production of a desired protein in an egg white are shown in Figures 2 and 3.

### Production of Desired Proteins or Peptides in Egg Yolk

The present invention is particularly advantageous for production of recombinant peptides and proteins of low solubility in the egg yolk. Such proteins include, but are not limited to, membrane-associated or membrane-bound proteins, lipophilic compounds; attachment factors, receptors, and components of second messenger transduction machinery. Low solubility peptides and proteins are particularly challenging to produce using conventional recombinant protein production techniques (cell and tissue cultures) because they aggregate in water-based, hydrophilic environments. Such aggregation necessitates denaturation and re-folding of the recombinantly-produced proteins, which may deleteriously affect their structure and function. Moreover, even highly soluble recombinant peptides and proteins may precipitate and require denaturation and renaturation when produced in sufficiently high amounts in recombinant protein production systems. The present invention provides an advantageous resolution of the problem of protein and peptide solubility during production of large amounts of recombinant proteins.

In one embodiment of the present invention, deposition of a desired protein into the egg yolk is accomplished by attaching a sequence encoding a protein capable of binding to the yolk vitellogenin receptor to a gene of interest that encodes a desired protein. This transposon-based vector can be used for the receptor-mediated uptake of the desired protein by the oocytes. In a preferred embodiment, the sequence ensuring the binding to the vitellogenin receptor is a targeting sequence of a vitellogenin protein. The invention encompasses various vitellogenin proteins and their targeting sequences. In a preferred embodiment, a chicken vitellogenin protein targeting sequence is used, however, due to the high degree of conservation among vitellogenin protein sequences and known cross-species reactivity of vitellogenin targeting sequences with their egg-yolk receptors, other vitellogenin targeting sequences can be substituted. One example of a construct for use in the transposon-based vectors of the present invention and for deposition of an insulin protein in an egg yolk is provided in SEQ ID NO:27. In this embodiment, the transposon-based vector contains a vitellogenin promoter, a vitellogenin targeting sequence, a TAG sequence, a pro-insulin sequence and a synthetic polyA sequence. The present invention includes, but is not limited to, vitellogenin targeting sequences residing in the N-terminal domain of vitellogenin, particularly in lipovitellin I. In one embodiment, the vitellogenin targeting sequence contains the polynucleotide sequence of SEQ ID NO: 18.

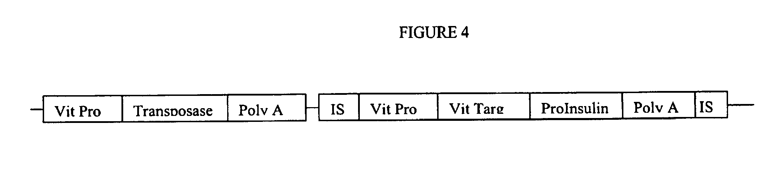

In a preferred embodiment, the transposon-based vector contains a transposase gene operably-linked to a liver-specific promoter and a gene of interest operably-linked to a promoter selected from the group consisting of an ovalbumin promoter, a conalbumin promoter, a vitellogenin promoter or an ovomucoid promoter and a vitellogenin targeting sequence. Figure 4 shows an example of such a construct. In another preferred embodiment, the transposon-based vector contains a transposase gene operably-linked to a constitutive promoter and a gene of interest operably-linked to a promoter selected from the group consisting of an ovalbumin promoter, a conalbumin promoter, a vitellogenin promoter or an ovomucoid promoter and a vitellogenin targeting sequence.

### Isolation and Purification of Desired Protein or Peptide

For large-scale production of protein, an animal breeding stock that is homozygous for the transgene is preferred. Such homozygous individuals are obtained and identified through, for example, standard animal breeding procedures or PCR protocols.

Once expressed, peptides, polypeptides and proteins can be purified according to standard procedures known to one of ordinary skill in the art, including ammonium sulfate precipitation, affinity columns, column chromatography, gel electrophoresis, high performance liquid chromatography, immunoprecipitation and the like. Substantially pure compositions of about 50 to 99% homogeneity are preferred, and 80 to 95% or greater homogeneity are most preferred for use as therapeutic agents.

In one embodiment of the present invention, the animal in which the desired protein is produced is an egg-laying animal. In a preferred embodiment of the present invention, the animal is an avian and a desired peptide, polypeptide or protein is isolated from an egg white. Egg white containing the exogenous protein or peptide is separated from the yolk and other egg constituents on an industrial scale by any of a variety of methods known in the egg industry. See, e.g., W. Stadelman et al. (Eds.), Egg Science & Technology, Haworth Press, Binghamton, NY (1995). Isolation of the exogenous peptide or protein from the other egg white constituents is accomplished by any of a number of polypeptide isolation and purification methods well known to one of ordinary skill in the art. These techniques include, for example, chromatographic methods such as gel permeation, ion exchange, affinity separation, metal chelation, HPLC, and the like, either alone or in combination. Another means that may be used for isolation or purification, either in lieu of or in addition to chromatographic separation methods, includes electrophoresis. Successful isolation and purification is confirmed by standard analytic techniques, including HPLC, mass spectroscopy, and spectrophotometry. These separation methods are often facilitated if the first step in the separation is the removal of the endogenous ovalbumin fraction of egg white, as doing so will reduce the total protein content to be further purified by about 50%.

To facilitate or enable purification of a desired protein or peptide, transposon-based vectors may include one or more additional epitopes or domains. Such epitopes or domains include DNA sequences encoding enzymatic or chemical cleavage sites including, but not limited to, an enterokinase cleavage site; the glutathione binding domain from glutathione S-transferase; polylysine; hexa-histidine or other cationic amino acids; thioredoxin; hemagglutinin antigen; maltose binding protein; a fragment of gp41 from HIV; and other purification epitopes or domains commonly known to one of skill in the art.

In one representative embodiment, purification of desired proteins from egg white utilizes the antigenicity of the ovalbumin carrier protein and particular attributes of a TAG linker sequence that spans ovalbumin and the desired protein. The TAG sequence is particularly useful in this process because it contains 1) a highly antigenic epitope, a fragment of gp41 from HIV, allowing for stringent affinity purification, and, 2) a recognition site for the protease enterokinase immediately juxtaposed to the desired protein. In a preferred embodiment, the TAG sequence comprises approximately 50 amino acids. A representative TAG sequence is provided below.

Pro Ala Asp Asp Ala Pro Ala Asp Asp Ala Pro Ala Asp Asp Ala Pro Ala Asp Asp
Ala Pro Ala Asp Asp Ala Pro Ala Asp Asp Ala Thr Thr Cys Ile Leu Lys Gly Ser Cys
Gly Trp Ile Gly Leu Leu Asp Asp Asp Asp Lys (SEQ ID NO:22)

The underlined sequences were taken from the hairpin loop domain of HIV gp-41 (SEQ ID NO:23). Sequences in italics represent the cleavage site for enterokinase (SEQ ID NO:9). The spacer sequence upstream of the loop domain was made from repeats of (Pro Ala Asp Asp Ala) (SEQ ID NO:25) to provide free rotation and promote surface availability of the hairpin loop from the ovalbumin carrier protein.

Isolation and purification of a desired protein is performed as follows:
1. Enrichment of the egg white protein fraction containing ovalbumin and the transgenic ovalbumin-TAG-desired protein.
2. Size exclusion chromatography to isolate only those proteins within a narrow range of molecular weights (a further enrichment of step 1).
3. Ovalbumin affinity chromatography. Highly specific antibodies to ovalbumin will eliminate virtually all extraneous egg white proteins except ovalbumin and the transgenic ovalbumin-TAG-desired protein.
4. gp41 affinity chromatography using anti-gp41 antibodies. Stringent application of this step will result in virtually pure transgenic ovalbumin-TAG-desired protein.
5. Cleavage of the transgene product can be accomplished in at least one of two ways:
   a. The transgenic ovalbumin-TAG-desired protein is left attached to the gp41 affinity resin (beads) from step 4 and the protease enterokinase is added. This liberates the transgene target protein from the gp41 affinity resin while the ovalbumin-TAG sequence is retained. Separation by centrifugation (in a batch process) or flow through (in a column purification), leaves the desired protein together with enterokinase in solution. Enterokinase is recovered and reused.
   b. Alternatively, enterokinase is immobilized on resin (beads) by the addition of poly-lysine moieties to a non-catalytic area of the protease. The transgenic ovalbumin-TAG-desired protein eluted from the affinity column of step 4 is then applied to the protease resin. Protease action cleaves the ovalbumin-TAG sequence from the desired protein and leaves both entities in solution. The immobilized enterokinase resin is recharged and reused.
   c. The choice of these alternatives is made depending upon the size and chemical composition of the transgene target protein.
6. A final separation of either of these two (5a or 5b) protein mixtures is made using size exclusion, or enterokinase affinity chromatography. This step allows for desalting, buffer exchange and/or polishing, as needed.

Cleavage of the transgene product (ovalbumin-TAG-desired protein) by enterokinase, then, results in two products: ovalbumin-TAG and the desired protein. More specific methods for isolation using the TAG label is provided in the Examples. Some desired proteins may require additions or modifications of the above-described approach as known to one of ordinary skill in the art. The method is scaleable from the laboratory bench to pilot and production facility largely because the techniques applied are well documented in each of these settings.

It is believed that a typical chicken egg produced by a transgenic animal of the present invention will contain at least 0.001 mg, from about 0.001 to 1.0 mg, or from about 0.001 to 100.0 mg of exogenous protein, peptide or polypeptide, in addition to the normal constituents of egg white (or possibly replacing a small fraction of the latter).

One of skill in the art will recognize that after biological expression or purification, the desired proteins, fragments thereof and peptides may possess a conformation substantially different than the native conformations of the proteins, fragments thereof and peptides. In this case, it is often necessary to denature and reduce protein and then to cause the protein to re-fold into the preferred conformation. Methods of reducing and denaturing proteins and inducing re-folding are well known to those of skill in the art.

### Production of Protein or Peptide in Milk

In addition to the use of a vector according to the invention for producing eggs containing transgenic proteins or peptides, the present invention encompasses the use of a vector according to the invention for the production of milk containing transgenic proteins or peptides. Each use includes the administration of a transposon-based vector described above to a mammal. In one embodiment, the transposon-based vector contains a transposase operably-linked to a constitutive Promoter and a gene of interest operably-linked to mammary specific promoter. Genes of interest can include, but are not limited to antiviral and antibacterial proteins and immunoglobulins.

### Treatment of Disease and Animal Improvement

In addition to production and isolation of desired molecules, the transposon-based vectors of the present invention can be used for the treatment of various genetic disorders. For example, one or more transposon-based vectors can be administered to a human or animal for the treatment of a single gene disorder including, but not limited to, Huntington's disease, alpha-1-antitrypsin deficiency Alzheimer's disease, various forms or breast cancer, cystic fibrosis, galactosemia, congenital hypothyroidism, maple syrup urine disease, neurofibromatosis 1, phenylketonuria, sickle cell disease, and Smith-Lemli-Opitz (SLO/RSH) Syndrome. Other diseases caused by single gene disorders that may be treated with the present invention include, autoimmune diseases, shipping fever in cattle, mastitis, bacterial or viral diseases, alteration of skin pigment in animals. In these embodiments, the transposon-based vector contains a non-mutated, or non-disease causing form of the gene known to cause such disorder. Preferably, the transposase contained within the transposase-based vector is operably linked to an inducible promoter such as a tissue-specific promoter such that the non-mutated gene of interest is inserted into a specific tissue wherein the mutated gene is expressed in vivo.

In one embodiment of the present invention, a transposon-based vector comprising a gene encoding proinsulin is administered to diabetic animals or humans for incorporation into liver cells in order to treat or cure diabetes. The specific incorporation of the proinsulin gene into the liver is accomplished by placing the transposase gene under the control of liver-specific promoter, such as G6P. This approach is useful for treatment of both Type I and Type II diabetes. The G6P promoter has been shown to be glucose responsive (Arguad, D., et al. 1996. Diabetes 45:1563-1571), and thus, glucose-regulated insulin production is achieved using DNA constructs of the present invention. Integrating a proinsulin gene into liver cells circumvents the problem of destruction of pancreatic islet cells in the course of Type I diabetes.

In another embodiment, shortly after diagnosis of Type I diabetes, the cells of the immune system destroying pancreatic β-cells are selectively removed using the transposon-based vectors of the present invention, thus allowing normal β-cells to repopulate the pancreas.

For treatment of Type II diabetes, a transposon-based vector containing a proinsulin gene is specifically incorporated into the pancreas by placing the transposase gene under the control of a pancreas-specific promoter, such as an insulin promoter. In this embodiment, the vector is delivered to a diabetic animal or human via injection into an artery feeding the pancreas. For delivery, the vector is complexed with a transfection agent. The artery distributes the complex throughout the pancreas, where individual cells receive the vector DNA. Following uptake into the target cell, the insulin promoter is recognized by transcriptional machinery of the cell, the transposase encoded by the vector is expressed, and stable integration of the proinsulin gene occurs. It is expected that a small percentage of the transposon-based vector is transported to other tissues, and that these tissues are transfected. However, these tissues are not stably transfected and the proinsulin gene is not incorporated into the cells' DNA due to failure of these cells to activate the insulin promoter. The vector DNA is likely lost when the cell dies or degraded over time.

In other embodiments, one or more transposon-based vectors are administered to an avian for the treatment of a viral or bacterial infection/disease including, but not limited to, Colibacillosis (Coliform infections), Mycoplasmosis (CRD, Air sac, Sinusitis), Fowl Cholera, Necrotic Enteritis, Ulcerative Enteritis (Quail disease), Pullorum Disease, Fowl Typhoid, Botulism, Infectious Coryza, Erysipelas, Avian Pox, Newcastle Disease, Infectious Bronchitis, Quail Bronchitis, Lymphoid Leukosis, Marek's Disease (Visceral Leukosis), Infectious Bursal Disease (Gumboro). In these embodiments, the transposon-based vectors may be used in a manner similar to traditional vaccines.

In still other embodiments, one or more transposon-based vectors are administered to an animal for the production of an animal with enhanced growth characteristics and nutrient utilization.

The transposon-based vectors of the present invention can be used to transform any animal cell, including but not limited to: cells producing hormones, cytokines, growth factors, or any other biologically active substance; cells of the immune system; cells of the nervous system; muscle (striatal, cardiac, smooth) cells; vascular system cells; endothelial cells; skin cells; mammary cells; and lung cells, including bronchial and alveolar cells. Transformation of any endocrine cell by a transposon-based vector is contemplated as a part of a present invention. In one aspect of the present invention, cells of the immune system may be the target for incorporation of a desired gene or genes encoding for production of antibodies. Accordingly, the thymus, bone marrow, beta lymphocytes (or B cells), gastrointestinal associated lymphatic tissue (GALT), Peyer's patches, bursa Fabricius, lymph nodes, spleen, and tonsil, and any other lymphatic tissue, may all be targets for administration of the compositions of the present invention.

The transposon-based vectors of the present invention can be used to modulate (stimulate or inhibit) production of any substance, including but not limited to a hormone, a cytokine, or a growth factor, by an animal or a human cell. Modulation of a regulated signal within a cell or a tissue, such as production of a second messenger, is also contemplated as a part of the present invention. Use of the transposon-based vectors of the present invention is contemplated for treatment of any animal or human disease or condition that results from underproduction (such as diabetes) or overproduction (such as hyperthyroidism) of a hormone or other endogenous biologically active substance. Use of the transposon-based vectors of the present invention to integrate nucleotide sequences encoding RNA molecules, such as antisense RNA or short interfering RNA, is also contemplated as a part of the present invention.

Additionally, the transposon-based vectors of the present invention may be used to provide cells or tissues with "beacons", such as receptor molecules, for binding of therapeutic agents in order to provide tissue and cell specificity for the therapeutic agents. Several promoters and exogenous genes can be combined in one vector to produce progressive, controlled treatments from a single vector delivery.

The following examples will serve to further illustrate the present invention without, at the same time, however, constituting any limitation thereof. On the contrary, it is to be clearly understood that resort may be had to various embodiments, modifications and equivalents thereof which, after reading the description herein, may suggest themselves to those skilled in the art without departing from the spirit of the invention.

### EXAMPLE 1

### Preparation of Transposon-Based Vector pTnMod

A vector was designed for inserting a desired coding sequence into the genome of eukaryotic cells, given below as SEQ ID NO:1. The vector of SEQ ID NO:1, termed pTnMod, was constructed and its sequence verified.

This vector employed a cytomegalovirus (CMV) promoter. A modified Kozak sequence (ACCATG) (SEQ ID NO: 13) was added to the promoter. The nucleotide in the wobble position in nucleotide triplet codons encoding the first 10 amino acids of transposase was changed to an adenine (A) or thymine (T), which did not alter the amino acid encoded by this codon. Two stop codons were added and a synthetic polyA was used to provide a strong termination sequence. This vector uses a promoter designed to be active soon after entering the cell (without any induction) to increase the likelihood of stable integration. The additional stop codons and synthetic polyA insures proper termination without read through to potential genes downstream.

The first step in constructing this vector was to modify the transposase to have the desired changes. Modifications to the transposase were accomplished with the primers High Efficiency forward primer (Hef) Altered transposase (ATS)-Hef 5' ATCTCGAGACCATGTGTGAACTTGATATTTTACATGATTCTCTTTACC 3' (SEQ ID NO:10) and Altered transposase- High efficiency reverse primer (Her) 5' GATTGATCATTATCATAATTTCCCCAAAGCGTAACC 3' (SEQ ID NO:11, a reverse complement primer). In the 5' forward primer ATS-Hef, the sequence CTCGAG (SEQ ID NO: 12) is the recognition site for the restriction enzyme Xho I, which permits directional cloning of the amplified gene. The sequence ACCATG (SEQ ID NO:13) contains the Kozak sequence and start codon for the transposase and the underlined bases represent changes in the wobble position to an A or T of codons for the first 10 amino acids (without changing the amino acid coded by the codon). Primer ATS-Her (SEQ ID NO:11) contains an additional stop codon TAA in addition to native stop codon TGA and adds a Bcl I restriction site, TGATCA (SEQ ID NO:14), to allow directional cloning. These primers were used in a PCR reaction with pTnLac (p defines plasmid, tn defines transposon, and lac defines the beta fragment of the lactose gene, which contains a multiple cloning site) as the template for the transposase and a FailSafe^{™} PCR System (which includes enzyme, buffers, dNTP's, MgCl₂ and PCR Enhancer; Epicentre Technologies, Madison, WI). Amplified PCR product was electrophoresed on a 1% agarose gel, stained with ethidium bromide, and visualized on an ultraviolet transilluminator. A band corresponding to the expected size was excised from the gel and purified from the agarose using a Zymo Clean Gel Recovery Kit (Zymo Research, Orange, CA). Purified DNA was digested with restriction enzymes Xho I (5') and Bcl I (3') (New England Biolabs, Beverly, MA) according to the manufacturer's protocol. Digested DNA was purified from restriction enzymes using a Zymo DNA Clean and Concentrator kit (Zymo Research).

Plasmid gWhiz (Gene Therapy Systems, San Diego, CA) was digested with restriction enzymes Sal I and BamH I (New England Biolabs), which are compatible with Xho I and Bel I, but destroy the restriction sites. Digested gWhiz was separated on an agarose gel, the desired band excised and purified as described above. Cutting the vector in this manner facilitated directional cloning of the modified transposase (mATS) between the CMV promoter and synthetic polyA.

To insert the mATS between the CMV promoter and synthetic polyA in gWhiz, a Stratagene T4 Ligase Kit (Stratagene, Inc. La Jolla, CA) was used and the ligation set up according to the manufacturer's protocol. Ligated product was transformed into *E. coli* Top10 competent cells (Invitrogen Life Technologies, Carlsbad, CA) using chemical transformation according to Invitrogen's protocol. Transformed bacteria were incubated in 1 ml of SOC (GIBCO BRL, CAT# 15544-042) medium for 1 hour at 37° C before being spread to LB (Luria-Bertani media (broth or agar)) plates supplemented with 100 µg/ml ampicillin (LB/amp plates). These plates were incubated overnight at 37° C and resulting colonies picked to LB/amp broth for overnight growth at 37° C. Plasmid DNA was isolated using a modified alkaline lysis protocol (Sambrook et al., 1989), electrophoresed on a 1% agarose gel, and visualized on a U.V. transilluminator after ethidium bromide staining. Colonies producing a plasmid of the expected size (approximately 6.4 kbp) were cultured in at least 250 ml of LB/amp broth and plasmid DNA harvested using a Qiagen Maxi-Prep Kit (column purification) according to the manufacturer's protocol (Qiagen, Inc., Chatsworth, CA). Column purified DNA was used as template for sequencing to verify the changes made in the transposase were the desired changes and no further changes or mutations occurred due to PCR amplification. For sequencing, Perkin-Elmer's Big Dye Sequencing Kit was used. All samples were sent to the Gene Probes and Expression Laboratory (LSU School of Veterinary Medicine) for sequencing on a Perkin-Elmer Model 377 Automated Sequencer.

Once a clone was identified that contained the desired mATS in the correct orientation, primers CMVf-NgoM IV (5' TTGCCGGCATCAGATTGGCTAT (SEQ ID NO:15); underlined bases denote NgoM IV recognition site) and Syn-polyA-BstE II (5' AGAGGTCACCGGGTCAATTCTTCAGCACCTGGTA (SEQ ID NO:16); underlined bases denote BstE II recognition site) were used to PCR amplify the entire CMV promoter, mATS, and synthetic polyA for cloning upstream of the transposon in pTnLac. The PCR was conducted with FailSafe^{™} as described above, purified using the Zymo Clean and Concentrator kit, the ends digested with NgoM IV and BstE II (New England Biolabs), purified with the Zymo kit again and cloned upstream of the transposon in pTnLac as described below.

Plasmid pTnLac was digested with NgoM IV and BstE II to remove the ptac promoter and transposase and the fragments separated on an agarose gel. The band corresponding to the vector and transposon was excised, purified from the agarose, and dephosphorylated with calf intestinal alkaline phosphatase (New England Biolabs) to prevent self-annealing. The enzyme was removed from the vector using a Zymo DNA Clean and Concentrator-5. The purified vector and CMVp/mATS/polyA were ligated together using a Stratagene T4 Ligase Kit and transformed into *E. coli* as described above.

Colonies resulting from this transformation were screened (mini-preps) as describe above and clones that were the correct size were verified by DNA sequence analysis as described above. The vector was given the name pTnMod (SEQ ID NO: 1) and includes the following components:
Base pairs 1-130 are a remainder of F1(-) on from pBluescriptll sk(-) (Stratagene), corresponding to base pairs 1-130 of pBluescriptll sk(-).
Base pairs 131 - 132 are a residue from ligation of restriction enzyme sites used in constructing the vector.
Base pairs 133 -1777 are the CMV promoter/enhancer taken from vector pGWiz (Gene Therapy Systems), corresponding to bp 229-1873 of pGWiz. The CMV promoter was modified by the addition of an ACC sequence upstream of ATG.
Base pairs 1778-1779 are a residue from ligation of restriction enzyme sites used in constructing the vector.
Base pairs 1780 - 2987 are the coding sequence for the transposase, modified from Tn10 (GenBank accession J01829) by optimizing codons for stability of the transposase mRNA and for the expression of protein. More specifically, in each of the codons for the first ten amino acids of the transposase, G or C was changed to A or T when such a substitution would not alter the amino acid that was encoded.
Base pairs 2988-2993 are two engineered stop codons.
Base pair 2994 is a residue from ligation of restriction enzyme sites used in constructing the vector.
Base pairs 2995 - 3410 are a synthetic polyA sequence taken from the pGWiz vector (Gene Therapy Systems), corresponding to bp 1922-2337 of 10 pGWiz.
Base pairs 3415 - 3718 are non-coding DNA that is residual from vector pNK2859.
Base pairs 3719 - 3761 are non-coding λ DNA that is residual from pNK2859.
Base pairs 3762 - 3831 are the 70 bp of the left insertion sequence recognized by the transposon Tn10.
Base pairs 3832-3837 are a residue from ligation of restriction enzyme sites used in constructing the vector.
Base pairs 3838 - 4527 are the multiple cloning site from pBluescriptll sk(20), corresponding to bp 924-235 of pBluescriptll sk(-). This multiple cloning site may be used to insert any coding sequence of interest into the vector.
Base pairs 4528-4532 are a residue from ligation of restriction enzyme sites used in constructing the vector.
Base pairs 4533 - 4602 are the 70 bp of the right insertion sequence recognized by the transposon Tn10.
Base pairs 4603 - 4644 are non-coding λ DNA that is residual from pNK2859.
Base pairs 4645 - 5488 are non-coding DNA that is residual from pNK2859.
Base pairs 5489 - 7689 are from the pBluescriptll sk(-) base vector - (Stratagene, Inc.), corresponding to bp 761-2961 of pBluescriptll sk(-).
Completing pTnMod is a pBlueScript backbone that contains a colE I origin of replication and an antibiotic resistance marker (ampicillin).
It should be noted that all non-coding DNA sequences described above can be replaced with any other non-coding DNA sequence(s). Missing nucleotide sequences in the above construct represent restriction site remnants.
All plasmid DNA was isolated by standard procedures. Briefly, *Escherichia coli* containing the plasmid was grown in 500 mL aliquots of LB broth (supplemented with an appropriate antibiotic) at 37°C overnight with shaking. Plasmid DNA was recovered from the bacteria using a Qiagen Maxi-Prep kit (Qiagen, Inc., Chatsworth, CA) according to the manufacturer's protocol. Plasmid DNA was resuspended in 500 µL of PCR-grade water and stored at -20°C until used.

### EXAMPLE 2

### Preparation of Transposon-Based Vector pTnMod (CMV/Red)

A vector was designed for inserting a reporter gene (DsRed) under the control of the CMV promoter into the genome of vertebrate cells given below as SEQ ID NO:2. The reporter gene chosen was the DsRed gene, driven by the immediate early cytomegalovirus promoter, to produce a plasmid called pTnCMV/DsRed. The DsRed gene product is a red fluorescent protein from an IndoPacific sea anemone, Discosoma sp., which fluoresces bright red at 558 nm. It is to be understood that the reporter gene, i.e., the DsRed gene, is only one embodiment of the present invention and that any gene of interest may be inserted into the plasmid in place of the DsRed reporter gene in any Experiment described herein.

The vector of SEQ ID NO:2, named pTnMod (CMV/Red), was constructed, and its sequence verified by re-sequencing. SEQ ID NO:2, pTnMod (CMV/Red), includes the following components:
Base pairs 1-130 are a remainder of F1(-) on from pBluescriptll sk(-) (Stratagene), corresponding to bp 1-130 of pBluescriptll sk(-).
Base pairs 131 - 132 are a residue from ligation of restriction enzyme sites used in constructing the vector.
Base pairs 133 -1777 are the CMV promoter/enhancer taken from vector pGWiz (Gene Therapy Systems, corresponding to bp 229-1873 of pGWiz.
Base pairs 1778-1779 are a residue from ligation of restriction enzyme sites used in constructing the vector.
Base pairs 1780 - 2987 are the coding sequence for the transposase, modified from Tn10 (GenBank accession J01829) by optimizing codons as discussed above.
Base pairs 2988-2993 are two engineered stop codons.
Base pair 2994 is a residue from ligation of restriction enzyme sites used in constructing the vector.
Base pairs 2995 - 3410 are a synthetic polyA sequence taken from the pGWiz vector (Gene Therapy Systems), corresponding to bp 1922-2337 of pGWiz.
Base pairs 3415 - 3718 are non-coding DNA that is residual from vector pNK2859.
Base pairs 3719 - 3761 are non-coding λ DNA that is residual from pNK2859.
Base pairs 3762 - 3831 are the 70 bp of the left insertion sequence recognized by the transposon Tun10.
Base pairs 3832-3837 are a residue from ligation of restriction enzyme sites used in constructing the vector.
Base pairs 3838 - 4044 are part of the multiple cloning site from pBluescriptll sk(-), corresponding to bp 924-718 of pBluescriptll sk(-).
Base pairs 4045-4048 are a residue from ligation of restriction enzyme sites used in constructing the vector.
Base pairs 4049-5693 are the CMV promoter/enhancer, taken from vector pGWiz (Gene Therapy Systems), corresponding to bp 229-1873 of pGWiz.
Base pairs 5694-5701 are a residue from ligation of restriction enzyme sites used in constructing the vector.
Base pairs 5702 - 6617 are the DsRed reporter coding sequence, including polyA sequence, from pDsRedl.1 (Clontech), corresponding to bp 77 - 992 of pDsRed1.1.
Base pairs 6618-7101 are part of the multiple cloning site from pBluescriptll sk(-), corresponding to bp 718-235 of pBluescriptll sk(-).
Base pairs 7102-7106 are a residue from ligation of restriction enzyme sites used in constructing the vector.
Base pairs 7107 - 7176 are the 70 bp of the right insertion sequence recognized by the transposon Tn10.
Base pairs 7177 - 7218 are non-coding λ DNA that is residual from pNK2859.
Base pairs 7219 - 8062 are non-coding DNA that is residual from pNK2859.
Base pairs 8063 - 10263 are from the pBluescriptll sk(-) base vector (Stratagene, Inc.), corresponding to bp 761-2961 of pBluescriptll sk(-).
It should be noted that all non-coding DNA sequences described above can be replaced with any other non-coding DNA sequence(s).

### EXAMPLE 3

### Preparation of Transposon-Based Vector pTnMod (Oval/Red) - Chicken

A vector was designed for inserting a reporter gene (DsRed) under the control of the ovalbumin promoter, and including the ovalbumin signal sequence, into the genome of a bird. One version of this vector is given below as SEQ ID NO:3. The vector of SEQ ID NO:3, named pTnMod (Oval/Red) - Chicken, includes chicken ovalbumin promoter and signal sequences.

SEQ ID NO:3, pTnMod (Oval/Red) - Chicken, includes the following components:
Base pairs 1-130 are a remainder of F1(-) on from pBluescriptll sk(-) (Stratagene), corresponding to bp 1-130 of pBluescriptll sk(-).
Base pairs 131 - 132 are a residue from ligation of restriction enzyme sites used in constructing the vector.
Base pairs 133 -1777 are the CMV promoter/enhancer taken from vector pGWiz (Gene Therapy Systems, corresponding to bp 229-1873 of pGWiz.
Base pairs 1778-1779 are a residue from ligation of restriction enzyme sites used in constructing the vector.
Base pairs 1780 - 2987 are the coding sequence for the transposase, modified from Tn10 (GenBank accession J01829) by optimizing codons as discussed above.
Base pairs 2988-2993 are two engineered stop codons.
Base pair 2994 is a residue from ligation of restriction enzyme sites used in constructing the vector.
Base pairs 2995 - 3410 are a synthetic polyA sequence taken from the pGWiz vector (Gene Therapy Systems), corresponding to bp 1922-2337 of pGWiz.
Base pairs 3415 --3718 are non-coding DNA that is residual from vector pNK2859.
Base pairs 3719 - 3761 are non-coding λ DNA that is residual from 10 pNK2859.
Base pairs 3762 - 3831 are the 70 bp of the left insertion sequence recognized by the transposon Tn10.
Base pairs 3832-3837 are a residue from ligation of restriction enzyme sites used in constructing the vector.
Base pairs 3838 - 4044 are part of the multiple cloning site from pBluescriptll sk(-), corresponding to bp 924-718 of pBluescriptll sk(-).
Base pairs 4045-4049 are a residue from ligation of restriction enzyme sites used in constructing the vector.
Base pairs 4050 - 4951 contain upstream elements of the (including SDRE, steroid-dependent response element). See GenBank accession number J00895 M24999, bp 431-1332. Base pairs 4952-4959 are a residue from ligation of restriction enzyme sites used in constructing the vector.
Base pairs 4960 - 5112 are the chicken ovalbumin signal sequence (GenBank accession number J00895 M24999, bp 2996-3148).
Base pairs 5113-5118 are a residue from ligation of restriction enzyme sites used in constructing the vector.
Base pairs 5119 - 6011 are the DsRed reporter coding sequence, including polyA sequence, from pDsRed1.1 (Clontech), corresponding to bp 100 - 992 of pDsRed1.1.
Base pairs 6012-6017 are a residue from ligation of restriction enzyme sites used in constructing the vector.
Base pairs 6018 - 6056 are part of the multiple cloning site of the ZeroBlunt Topo cloning vector (Invitrogen), corresponding to bp 337-377 of ZeroBlunt.
Base pairs 6057-6062 are a residue from ligation of restriction enzyme sites used in constructing the vector.
Base pairs 6063 - 6495 are part of the multiple cloning site from pBluescriptll sk(-), corresponding to bp 667-235 of pbluescriptll sk(-).
Base pairs 6496-6500 are a residue from ligation of restriction enzyme sites used in constructing the vector.
Base pairs 6501 - 6570 are the 70 bp of the right insertion sequence recognized by the transposon Tn10.
Base pairs 6571 - 6612 are non-coding λ. DNA that is residual from pNK2859.
Base pairs 6613 - 7477 are non-coding DNA that is residual from pNK2859.
Base pairs 7478 - 9678 are from the pBluescriptll sk(-) base vector (Stratagene, Inc.), corresponding to bp 761-2961 of pbluescriptll sk(-).
It should be noted that all non-coding DNA sequences described above can be replaced with any other non-coding DNA sequence(s).

### EXAMPLE 4

### Preparation of Transposon-Based Vector pTnMod(Oval/Red) - Quail

A vector was designed for inserting a reporter gene (DsRed) under the control of the ovalbumin promoter, and including the ovalbumin signal sequence, into the genome of a bird given below as SEQ ID NO:4. The vector of SEQ ID NO:4, named pTnMod (Oval/Red) - Quail, has been constructed, and selected portions of the sequence have been verified by re-sequencing.

SEQ ID NO:4, pTnMod (Oval/Red) - Quail, includes the following components:
Base pairs 1-130 are a remainder of F1(-) on from pBluescriptll sk(-) (Stratagene), corresponding to bp 1-130 of pBluescriptll sk(-).
Base pairs 131 - 132 are a residue from ligation of restriction enzyme sites used in constructing the vector.
Base pairs 133 - 1777 are the CMV promoter/enhancer taken from vector pGWiz (Gene Therapy Systems), corresponding to bp 229-1873 of pGWiz.
Base pairs 1778-1779 are a residue from ligation of restriction enzyme sites used in constructing the vector.
Base pairs 1780 - 2987 are the coding sequence for the transposase, modified from Tn10 (GenBank accession J01829) by optimizing codons as discussed above.
Base pairs 2988-2993 are two engineered stop codons. Base pair 2994 is a residue from ligation of restriction enzyme sites used in constructing the vector.
Base pairs 2995 - 3410 are a synthetic polyA sequence taken from the pGWiz vector (Gene Therapy Systems), corresponding to bp 1922-2337 of pGWiz.
Base pairs 3415 - 3718 are non-coding DNA that is residual from vector pNK2859.
Base pairs 3719 - 3761 are non-coding λ DNA that is residual from pNK2859.
Base pairs 3762 - 3831 are the 70 base pairs of the left insertion sequence recognized by the transposon Tn10.
Base pairs 3832-3837 are a residue from ligation of restriction enzyme sites used in constructing the vector.
Base pairs 3838 - 4044 are part of the multiple cloning site from pBluescriptll sk(-), corresponding to bp 924-718 of pBluescriptll sk(-).
Base pairs 4045-4049 are a residue from ligation of restriction enzyme sites used in constructing the vector.
Base pairs 4050 - 4934 are the Japanese quail ovalbumin promoter (including SDRE, steroid-dependent response element). The Japanese quail ovalbumin promoter was isolated by its high degree of homology to the chicken ovalbumin promoter (GenBank accession number J00895 M24999, base pairs 431-1332). Some deletions were noted in the quail sequence, as compared to the chicken sequence.
Base pairs 4935-4942 are a residue from ligation of restriction enzyme sites used in constructing the vector.
Base pairs 4943 - 5092 are the Japanese quail ovalbumin signal sequence. The quail signal sequence was isolated by its high degree of homology to the chicken signal sequence (GenBank accession number J00895 M24999, base pairs 2996-3148). Some deletions were noted in the quail sequence, as compared to the chicken sequence.
Base pairs 5093-5098 are a residue from ligation of restriction enzyme sites used in constructing the vector.
Base pairs 5099 - 5991 are the DsRed reporter coding sequence, including polyA sequence, from pDsRed1.1 (Clontech), corresponding to bp 100 - 992 of pDsRed 1.1.
Base pairs 5992-5997 are a residue from ligation of restriction enzyme sites used in constructing the vector.
Base pairs 5998 - 6036 are part of the multiple cloning site of the ZeroBlunt Topo cloning vector (Invitrogen), corresponding to base pairs 337-377 of ZeroBlunt.
Base pairs 6037-6042 are a residue from ligation of restriction enzyme sites used in constructing the vector.
Base pairs 6043 - 6475 are part of the multiple cloning site from pBluescriptll sk(-), corresponding to bp 667-235 of pBluescriptll sk(-).
Base pairs 6476-6480 are a residue from ligation of restriction enzyme sites used in constructing the vector.
Base pairs 6481 - 6550 are the 70 bp of the right insertion sequence recognized by the transposon Tn10.
Base pairs 6551 - 6592 are non-coding λ DNA that is residual from pNK2859.
Base pairs 6593 - 7457 are non-coding DNA that is residual from pNK2859.
Base pairs 7458 - 9658 are from the pBluescriptll sk(-) base vector (Stratagene, Inc.), corresponding to base pairs 761-2961 of pBluescriptll sk(-).
It should be noted that all non-coding DNA sequences described above can be replaced with any other non-coding DNA sequence(s).

### EXAMPLE 5

### Transfection of Stage X Japanese Quail Eggs with pTnMod(oval/Red) - Quail via embryo injection

Transgenic Japanese quail were produced by transfecting Stage X embryos and the heritability of the transgene delivered by embryo transfection was established. More specifically, fertile eggs were collected in the morning and placed at 15° C until enough were collected for injection, but were held no longer than 7 days. Stage X embryos (eggs) were assigned to one of two treatment groups. Before treatment, each egg was incubated on its side at room temperature for about 2 hours to allow the embryo to move to "top dead center" (TDC). Each egg was transfected by drilling a 1 mm hole (directly above the embryo) through the shell without penetrating the underlying shell membrane. A 0.5 ml syringe fitted with a 28 gauge needle was used to deliver DNA complexed to a transfecting reagent, i.e. SUPERFECT®, in a 50 µl volume. An adhesive disc was used to seal the hole and provide a label for treatment identification. After all eggs were transfected, they were set in an incubator with the adhesive disc pointing upward for hatching.

Each bird that hatched was bled at one week of age, DNA was extracted from blood cells, and PCR was conducted using 28s primers as a positive control and primers specific to DsRed. Any bird that was negative was terminated, while positive birds were monitored to determine maintenance of the transgene. Birds consistently positive were maintained until sexual maturity and bred. Positive male and female birds were mated. The eggs of mated hens were hatched and the resulting chicks, the G1 generation, were evaluated to determine if they were transgenic. All G1s resulting from this mating were bled and PCR conducted as described above.

Egg injection: Two treatment groups and one control group were used for this experiment. Vector pTnMod (Oval/Red) in supercoiled form (Treatment 1) and in linear form (Treatment 2) were used to transfect 15 eggs per treatment. To obtain linear DNA for this experiment, pTnMod (Oval/Red) was digested with NgoM IV, column purified, and resuspended in TE buffer.

Each egg was injected with 0.75 µg of DNA complexed with SUPERFECT^{®} in a 1:3 ratio in a total injection volume of 50 µl Hank's Balanced Salt solution (HBSS) was used to bring the volume to 50 µl. The DNA Superfect mixture must be allowed to incubate (for complex formation) at room temperature for 10 minutes prior to injection and must be used within 40 minutes post initial mixing. Eggs were incubated as described above after injection.

Results: In the supercoiled injection group, 2 females and 1 male were identified as PCR positive using primers specific to the DsRed coding sequence. These birds were mated as described above. Blood was taken from the G1 chicks and PCR was conducted. The results showed that the transgene was incorporated into the gametes of these birds. The G1 chicks from these birds were examined on a weekly basis until it was verified that the gene was not present or enough transgenic G1s were obtained to initiate a breeding flock of fully transgenic birds. Eggs from these G1 chicks expressed DsRed protein in the albumin portion of their eggs.

### EXAMPLE 6

### Intratesticular Injection of Chickens with pTnMod(CMV/Red) (SEQ ID NO: 2)

Immature birds of different ages (4, 6, 8, 10, 12, and 14 weeks) were placed under anesthesia and injected in the testes with the construct pTnMod(CMV/Red). A saline solution containing 1-5 µg of purified DNA vector, mixed with SUPERFECT^{®} transfecting reagent (Qiagen, Valencia, CA) in a 1:6 (wt:vol) ratio. The volume of saline was adjusted so that the total volume injected into each testis was 150-200 µl, depending on the age and size of the bird. For the 4- and 6-week-old chickens, 1 µg DNA in 150 µl was injected in each testis, divided into three doses of 50 µl each. For the older birds, 200 µl total volume was injected, containing either 3 µg DNA (for 8-week-old birds) or 5 µg DNA (for older birds) per testis. First, one testis was surgically exposed prior to injection. After injection, the incision was sutured, and the sequence was repeated for the alternate testis.

From six to nine months post-surgery, weekly sperm samples were taken from each injected bird, as well as from control birds. Each sperm sample was evaluated for uptake and expression of the injected gene. Samples were evaluated by PCR on whole sperm, within one week after collection.

Approximately 100 male white leghorn chickens, in groups of 5-26, at ages 4, 6, 8, 10, 12, and 14 weeks, were used as this is the age range in which it is expected that the testes are likely to be most "receptive." In this age range, the blood/testis barrier has not yet formed, and there is a relatively high number of spermatogonia relative to the numbers of other cell types, e.g., spermatids, etc. See J. Kumaran et al., 1949. Poultry Sci., vol. 29, pp. 511-520. See also E. Oakberg, 1956. Am. J. Anatomy, vol. 99, pp. 507-515; and P. Kluin et al., 1984. Anat. EmbryoL, vol. 169, pp. 73-78.

The experimental and control males were obtained from commercial sources at one day of age, and maintained in brooders until used. The male birds were housed in temperature-controlled spaces in individual standard caging as they approached maturity. They were given water and standard commercial feed ad lib. They were kept initially in a 23:1 hour light/dark cycle, stepped down at approximately weekly intervals to a 15:8 hour light/dark cycle, as this regimen has been reported to optimize sexual maturity and fertility.

### Surgical and DNA Injection Procedures

At the appropriate ages, groups of individual males were starved overnight and then subjected to transgene delivery by direct intratesticular injection of DNA by experienced animal surgeons. Each male was anesthetized with isoflurane via a simplified gas machine.

Various devices and anesthesia machines have previously been described for administering isoflurane (and other gaseous anesthetics) to birds. See Alsage et al., Poultry Sci., 50:1876-1878 (1971); Greenlees et al., Am. J. Vet. Res., vol. 51, pp. 757-758 (1990). However, these prior techniques are somewhat cumbersome and complex to implement. A novel and much simpler system to administer isoflurane (or other gaseous) anesthesia was developed due to the deficiencies in the prior art, a system that we found worked well on all ages of chicks. A standard nose cone was placed over the chick's head, similar to the system that has been used for decades to administer ether to mice. A plastic tube approximately 3.5 cm in diameter and 12 cm long was filled with cotton, into which was poured approximately 2 mL isoflurane (Abbott Laboratories, Chicago). The chick's head was placed partially into the cylinder, and was held in place there intermittently throughout the surgery as required to maintain the proper plane of anesthesia, without overdosing.

Each anesthetized bird was positioned on its side on an animal board with cords tractioning the wings and feet to allow access to the testes area. The area was swabbed with 0.5% chlorhexidine, and a 2 cm dorsolateral incision was made in the skin over the testis (similar to the procedure commonly used for caponization). A small-animal retractor was used to spread the last two ribs, exposing the testis. The DNA solution was then mixed with SUPERFECT^{®} (Qiagen) according to the manufacturer's protocol, approximately a 1:6 wt/vol ratio, to a final concentration of 0.01 - 0.05 µg/µl. This resulted in 1 - 5 µg total DNA (in a 150-200 µl volume) being injected into each testis, spread over three injection sites: one at each end of the testis, and one in the middle.

The injection device was a standard 25 gauge, 1/2 inch (1.27 cm) hypodermic needle, attached to a 50, 100, or 200 µl syringe. Approximately 5 mm of the needle tip was bent at a 90 degree angle, to facilitate insertion into the testes. Approximately 50 - 70 µl of the DNA-SUPERFECT^{®} solution was injected into each of three sites per testis. The multiple injections were calculated to suffuse the DNA throughout the whole testis, the idea being to promote contact between DNA and spermatogonia as much as feasible. We estimated that our procedure resulted in the injection of about 100,000 DNA molecules per spermatogonium. The construct used in these tests was a highly potent constitutive modified CMV promoter, operatively linked to the dsRed gene as shown in SEQ ID NO:2.

Following injection, the incision was closed in two layers with 4-0 absorbable suture, and then the contralateral testis was similarly exposed and injected. Following surgery, each bird was returned to its cage to recover. One hundred thirteen males were ultimately used in the experimental regimen to increase the overall likelihood of success, along with 4 control birds (16 weeks 20 old) subjected to sham surgery (with injections containing only the transfection reagent.

### Evaluation of Birds

Thus, a total of 113 white leghorn chickens were injected with the DNA vector in groups of 5-26 at varying ages. Fourteen birds were transformed at 4 weeks, 23 birds at 6 weeks; 26 birds at 8 weeks; 23 birds at 10 weeks; 5 birds at 12 weeks; and 22 birds at 14 weeks. Sixteen birds died before they could be sampled, so to date, 97 roosters have been sampled, plus the four controls. Birds were evaluated at 18-24 weeks of age for (a) potential transformation in the sperm, and (b) successful testis transfection. Sperm samples were obtained from each rooster by manual manipulation using standard techniques. The sperm were washed, and their DNA was extracted following the techniques of G. Mann et al., 1993. J. Reprod. Fert., 99:505-12. The samples were then frozen until analyzed. Evaluation was conducted by PCR analysis to detect DNA integration into the sperm, or into any of the testicular cells. Additionally, selected testes were harvested at the end of the sperm sampling period.

Of 97 birds tested, at least 22 showed probable positive results. Positive results were observed at all transformation ages, except for 4 weeks, which was not tested. At least two birds were confirmed positive by PCR of sperm, conducted four months after the initial injection. These results were transient in many cases, however since it was believed that the DsRed gene product used in these initial proof of concept experiments was toxic. Nevertheless, the positive PCR results presumptively demonstrated that the transgene was incorporated into spermatogonia (before puberty), and that it was carried in transgenic sperm. Such sperm could then transmit the gene to subsequent generations, resulting in the production of true, germ-line transgenic "founder" birds.

To further confirm that the DNA had been incorporated into the sperm, and that contaminating vector was not being detected from other sources, it was confirmed through PCR on sperm of experimental birds, and on positive and negative controls that the sperm of the experimental birds lacked DNA encoding the transposase. The design of the preferred transposon-based vector is such that the sequence encoding the transposase is contained in the vector, but is not incorporated into the transformed chromosome. Thus, presence of the exogenous coding sequence, coupled with absence of the transposase gene, is strong evidence for incorporation of the exogenous coding sequence, or transgene.

These results demonstrated proof of concept, as positive PCR results were obtained from the sperm of treated birds. Interpretation of these preliminary results was made more difficult by the fact that the modified CMV promoter used in the experiment was probably too "hot." As the DsRed product is not secreted from the cells, the product built up intracellularly to levels that were toxic, frequently killing the cells. Even this result, of course, means that the transformation was successful. The transgene could not have killed the cells otherwise.

In order to resolve to the problem with toxicity of the DsRed gene product, experiments were conducted using a different reporter gene operably linked to the ovalbumin promoter, so that the transgene was expressed in the egg white. These experiments are provided in Examples12-15 below.

### EXAMPLE 7

### Transfection of Male White Leghorn Chickens Using the Vector pTnMod(Oval/Red) - Quail (SEQ ID NO: 4) via Testicular Injections

In further experiments conducted on leghorn chickens, it was demonstrated that chickens injected intratesticularly at 8, 10, 12, or 14 weeks of age, had, on average, approximately 40% positive sperm between 6 and 8 months after injection. In other experiments, successful transfection was achieved with chickens injected at 13 weeks of age.

Forty-nine white leghorn roosters approximately 8, 10, 12, or 14 weeks of age were obtained and housed. Birds were identified, wing banded, and assigned to a treatment group. If appropriate (based on testes size and vascularization), one testis was caponized and the entire DNA injection volume was delivered to the remaining testis. Thirty-two males received DNA injections of 5µg DNA/testis at a 1:3 ratio of DNA to SUPERFECT^{®}. The remaining birds were used as controls. After injection, all birds were mated with at least 5 females and observed until sexual maturity and egg-laying began. All eggs collected prior to peak egg production (approximately 24 weeks of age for the hens) were incubated and candled to determine embryo presence. Any embryos identified were incubated to hatch to extract DNA, PCR was conducted, and transgene presence was determined.

Roosters positive for the pTnMod(Oval/Red) - Quail construct were kept to produce F1 offspring (eggs collected at peak production). Offspring from this hatch were bled, DNA extracted from the blood, and PCR conducted using primers specific for the DsRed gene. It was determined that 77% of the offspring were transgenic.

### EXAMPLE 8

### Transfection of Mature Male Japanese Quail using the vector pTnMod(Oval/Red) - Quail (SEQ ID NO:4) via Testicular Injections

Twelve sexually mature males (at approximately 13 weeks of age) underwent surgery for testicular injection as described above for chickens. At 21-28 days of age, the birds were identified, leg banded, debeaked, and separated based on sex. Injections comprised 5 µg/testes of the vector in concentrations 1:3 or 1:10 for SUPERFECT^{®} or a 1:1 ratio with Mirrus. The study consisted of 3 treatment groups with 5 males in the 1:3 DNA:SUPERFECT^{®} group, 3 males in the 1:10 DNA:SUPERFECT^{®} group, and 4 males in the 1:1 Mirrus group. All surgeries were conducted in one day.

Any unincorporated DNA was allowed to clear from the testes by holding the birds for 19 days before mating with females. At 15 weeks of age, 2 age-matched females were housed with each treated male. The presence of the transfected DNA was determined in the fertilized eggs during the second week of egg lay. The subsequent eggs collected from parents producing positively identified transgenic eggs were collected and stored until taken to hatch.

PCR performed on the sperm of quail injected at three months of age indicated successful incorporation of the DsRed transgene into the quail sperm.

### EXAMPLE 9

### Transfection of Immature Male Japanese Quail using the vector pTnMod(oval/Red) - Quail (SEQ ID NO:4) via Testicular Injections

Approximately 450 quail eggs were set and hatched. At 21-28 days of age, the birds were identified, wingbanded, debeaked, and separated based on sex. At 4 weeks of age, 65 male birds underwent surgery and testicular injections as described above. Injections comprised a control and 2 µg/testes of the vector in varying concentrations (0, 1/3, 1/5, and 1/10) of three different transfection reagents: 1) SUPERFECT^{®}, 2) Mirus/Panvera and 3) Dosper. The study comprised 13 treatment groups with 5 males per group. One transfection reagent was administered per day.

At 7 weeks of age, 2 age-matched females were housed with each treated male. The presence of the transfected DNA was determined in the fertilized eggs during the second week of egg lay. The subsequent eggs collected from parents producing positively identified transgenic eggs were collected and stored until taken to hatch. PCR performed on the sperm of quail injected at four and five weeks of age indicated successful incorporation of the DsRed transgene into the quail sperm.

### EXAMPLE 10

### Preparation of Transposon-Based Vector pTnMod(oval/ENT TAG/p146/PA) - Chicken

A vector is designed for inserting a p146 gene under the control of a chicken ovalbumin promoter, and a ovalbumin gene including an ovalbumin signal sequence, into the genome of a bird given below as SEQ ID NO:29.

Base pairs 1 - 130 are a remainder of F1(-) ori of pBlueScriptII sk(-) (Stratagene) corresponding to base pairs 1-130 of pBluescriptll sk(-).

Base pairs 133 - 1777 are a CMV promoter/enhancer taken from vector pGWiz (Gene Therapy Systems) corresponding to base pairs 229-1873 of pGWiz.

Base pairs 1780 - 2987 are a transposase, modified from Tn10 (GenBank accession number J01829).

Base pairs 2988-2993 are an engineered stop codon.

Base pairs 2995 - 3410 are a synthetic polyA from pGWiz (Gene Therapy Systems) corresponding to base pairs 1922- 2337 of pGWiz.

Base pairs 3415 - 3718 are non coding DNA that is residual from vector pNK2859.

Base pairs 3719 - 3761 are λ, DNA that is residual from pNK2859.

Base pairs 3762 - 3831 are the 70 base pairs of the left insertion sequence (IS10) recognized by the transposon Tn10.

Base pairs 3838 - 4044 are a multiple cloning site from pBlueScriptII sk(-) corresponding to base pairs 924-718 of pBluescriptll sk(-).

Base pairs 4050 - 4951 are a chicken ovalbumin promoter (including SDRE) that corresponds to base pairs 431-1332 of the chicken ovalbumin promoter in GenBank Accession Number J00895 M24999.

Base pairs 4958 - 6115 are a chicken ovalbumin signal sequence and Ovalbumin gene that correspond to base pairs 66-1223 of GenBank Accession Number V00383.1 (The STOP codon being omitted).

Base pairs 6122 - 6271 are a TAG sequence containing a gp41 hairpin loop from HIV I, an enterokinase cleavage site and a spacer (synthetic).

Base pairs 6272 - 6316 are a p146 sequence (synthetic) with 2 added stop codons.

Base pairs 6324 - 6676 are a synthetic polyadenylation sequence from pGWiz (Gene Therapy Systems) corresponding to base pairs 1920 - 2272of pGWiz.

Base pairs 6682 - 7114 are a multiple cloning site from pBlueScriptII sk(-) corresponding to base pairs 667-235 of pBluescriptll sk(-).

Base pairs 7120- 7189 are the 70 base pairs of the right insertion sequence (IS 10) recognized by the transposon Tn10.

Base pairs 7190-7231 are λ DNA that is residual from pNK2859.

Base pairs 7232 - 8096 are non coding DNA that is residual from pNK2859.

Base pairs 8097 - 10297 are pBlueScript sk(-) base vector (Stratagene, Inc.) corresponding to base pairs 761-2961of pBluescriptll sk(-).

It should be noted that all non-coding DNA sequences described above can be replaced with any other non-coding DNA sequence(s). Missing nucleotide sequences in the above construct represent restriction site remnants.

### EXAMPLE 11

### Preparation of Transposon-Based Vector pTnMod(Oval/ENT TAG/p146/PA) - Quail

A vector is designed for inserting a p146 gene under the control of a quail ovalbumin promoter, and a ovalbumin gene including an ovalbumin signal sequence, into the genome of a bird given below as SEQ ID NO:30.

Base pairs 1 - 130 are a remainder of F1(-) ori of pBluescriptII sk(-) (Stratagene) corresponding to base pairs 1-130 of pBluescriptll sk(-).

Base pairs 133 - 1777 are a CMV promoter/enhancer taken from vector pGWiz (Gene Therapy Systems) corresponding to base pairs 229-1873 of pGWiz.

Base pairs 1780 - 2987 are a transposase, modified from Tn10 (GenBank accession number J01829).

Base pairs 2988-2993 are an engineered stop codon.

Base pairs 2995 - 3410 are a synthetic polyA from pGWiz (Gene Therapy Systems) corresponding to base pairs 1922-2337 of pGWiz.

Base pairs 3415 - 3718 are non coding DNA that is residual from vector pNK2859.

Base pairs 3719 - 3761 are λ DNA that is residual from pNK2859.

Base pairs 3762 - 3831 are the 70 base pairs of the left insertion sequence (IS 10) recognized by the transposon Tn10.

Base pairs 3838 - 4044 are a multiple cloning site from pBlueScriptII sk(-) corresponding to base pairs 924-718 of pBluescriptll sk(-).

Base pairs 4050 - 4938 are the Japanese quail ovalbumin promoter (including SDRE, steroid-dependent response element). The Japanese quail ovalbumin promoter was isolated by its high degree of homology to the chicken ovalbumin promoter (GenBank accession number J00895 M24999, base pairs 431-1332).

Bp 4945 - 6092 are a quail ovalbumin signal sequence and ovalbumin gene that corresponds to base pairs 54 - 1201 of GenBank accession number X53964.1. (The STOP codon being omitted).

Base pairs 6097 - 6246 are a TAG sequence containing a gp41 hairpin loop from HIV I, an enterokinase cleavage site and a spacer (synthetic).

Base pairs 6247 - 6291 are a p146 sequence (synthetic) with 2 added stop codons.

Base pairs 6299 - 6651 are a synthetic polyadenylation sequence from pGWiz (Gene Therapy Systems) corresponding to base pairs 1920 - 2272of pGWiz.

Base pairs 6657 - 7089 are a multiple cloning site from pBlueScriptII sk(-) corresponding to base pairs 667-235 of pBluescriptll sk(-).

Base pairs 7095- 7164 are the 70 base pairs of the right insertion sequence (IS10) recognized by the transposon Tn10.

Base pairs 7165 - 7206 are λ DNA that is residual from pNK2859.

Base pairs 7207 - 8071 are non coding DNA that is residual from pNK2859.

Base pairs 8072 - 10272 are pBlueScript sk(-) base vector (Stratagene, Inc.) corresponding to base pairs 761-2961 of pBluescriptll sk(-).

It should be noted that all non-coding DNA sequences described above can be replaced with any other non-coding DNA sequence(s). Missing nucleotide sequences in the above construct represent restriction site remnants.

### EXAMPLE 12

### Preparation of Transposon-Based Vector pTnMod(Oval/ENT TAG/ProIns/PA) - Chicken

A vector is designed for inserting a proinsulin gene under the control of a chicken ovalbumin promoter, and a ovalbumin gene including an ovalbumin, signal sequence, into the genome of a bird given below as SEQ ID NO:31.

Base pairs 1 - 130 are a remainder of F1(-) ori of pBluescriptII sk(-) (Stratagene) corresponding to base pairs 1-130 of pBluescriptll sk(-).

Base pairs 133 - 1777 are a CMV promoter/enhancer taken from vector pGWiz (Gene Therapy Systems) corresponding to base pairs 229-1873 of pGWiz.

Base pairs 1780 - 2987 are a transposase, modified from Tn10 (GenBank accession number J01829).

Base pairs 2988-2993 are an engineered stop codon.

Base pairs 2995 - 3410 are a synthetic polyA from pGWiz (Gene Therapy Systems) corresponding to base pairs 1922- 2337 of pGWiz.

Base pairs 3415 - 3718 are non coding DNA that is residual from vector pNK2859.

Base pairs 3719 - 3761 are λ DNA that is residual from pNK2859.

Base pairs 3762 - 3831 are the 70 base pairs of the left insertion sequence (IS 10) recognized by the transposon Tn10.

Base pairs 3838 - 4044 are a multiple cloning site from pBlueScriptII sk(-) corresponding to base pairs 924-718 of pBluescriptll sk(-).

Base pairs 4050 - 4951 are a chicken ovalbumin promoter (including SDRE) that corresponds to base pairs 431-1332 of the chicken ovalbumin promoter in GenBank Accession Number J00895 M24999.

Base pairs 4958 - 6115 are a chicken ovalbumin signal sequence and ovalbumin gene that correspond to base pairs 66-1223 of GenBank Accession Number V00383.1. (The STOP codon being omitted).

Base pairs 6122 - 6271 are a TAG sequence containing a gp41 hairpin loop from HIV I, an enterokinase cleavage site and a spacer (synthetic).

Base pairs 6272 - 6531 are a proinsulin gene.

Base pairs 6539 - 6891 are a synthetic polyadenylation sequence from pGWiz (Gene Therapy Systems) corresponding to base pairs 1920 - 2272of pGWiz.

Base pairs 6897 - 7329 are a multiple cloning site from pBlueScriptII sk(-) corresponding to base pairs 667-235 of pBluescriptll sk(-).

Base pairs 7335- 7404 are the 70 base pairs of the right insertion sequence (IS 10) recognized by the transposon Tn10.

Base pairs 7405 - 7446 are λ DNA that is residual from pNK2859.

Base pairs 7447 - 8311 are non coding DNA that is residual from pNK2859.

Base pairs 8312 - 10512 are pBlueScript sk(-) base vector (Stratagene, Inc.) corresponding to base pairs 761-2961 of pBluescriptll sk(-).

It should be noted that all non-coding DNA sequences described above can be replaced with any other non-coding DNA sequence(s). Missing nucleotide sequences in the above construct represent restriction site remnants.

### EXAMPLE 13

### Preparation of Transposon-Based Vector pTnMod(Oval/ENT TAGIProIns/PA) - Quail

A vector is designed for inserting a proinsulin gene under the control of a chicken ovalbumin promoter, and a ovalbumin gene including an ovalbumin signal sequence, into the genome of a bird given below as SEQ ID NO:32.

Base pairs 1 -130 are a remainder of F1(-) ori of pBluescriptII sk(-) (Stratagene) corresponding to base pairs 1-130 of pBluescriptll sk(-).

Base pairs 133 - 1777 are a CMV promoter/enhancer taken from vector pGWiz (Gene Therapy Systems) corresponding to base pairs 229-1873 of pGWiz.

Base pairs 1780 - 2987 are a transposase, modified from Tn10 (GenBank accession number J01829).

Base pairs 2988-2993 are an engineered stop codon.

Base pairs 2995 - 3410 are a synthetic polyA from pGWiz (Gene Therapy Systems) corresponding to base pairs 1922- 2337 of pGWiz.

Base pairs 3415 - 3718 are non coding DNA that is residual from vector pNK2859.

Base pairs 3719-3761 are λ DNA that is residual from pNK2859.

Base pairs 3762 - 3831 are the 70 base pairs of the left insertion sequence (IS 10) recognized by the transposon Tn10.

Base pairs 3838 - 4044 are a multiple cloning site from pBlueScriptII sk(-) corresponding to base pairs 924-718 of pBluescriptll sk(-).

Base pairs 4050 - 4938 are the Japanese quail ovalbumin promoter (including SDRE, steroid-dependent response element). The Japanese quail ovalbumin promoter was isolated by its high degree of homology to the chicken ovalbumin promoter (GenBank accession number J00895 M24999, base pairs 431-1332). Some deletions were noted in the quail sequence, as compared to the chicken sequence.

Base pairs 4945 - 6092 are a quail ovalbumin signal sequence and ovalbumin gene that corresponds to base pairs 54 - 1201 of GenBank accession number X53964.1. (The STOP codon being omitted).

Base pairs 6093 - 6246 are a TAG sequence containing a gp41 hairpin loop from HIV I an enterokinase cleavage site and a spacer (synthetic).

Base pairs 6247 - 6507 are a proinsulin gene.

Base pairs 6514 - 6866 are a synthetic polyadenylation sequence from pGWiz (Gene Therapy Systems) corresponding to base pairs 1920 - 2272of pGWiz.

Base pairs 6867 - 7303 are a multiple cloning site from pBlueScriptII sk(-) corresponding to base pairs 667-235 of pBluescriptII sk(-).

Base pairs 7304- 7379 are the 70 base pairs of the right insertion sequence (IS 10) recognized by the transposon Tn10.

Base pairs 7380 - 7421 are λ DNA that is residual from pNK2859.

Base pairs 7422 - 8286 are non coding DNA that is residual from pNK2859.

Base pairs 8287 - 10487 are pBlueScript sk(-) base vector (Stratagene, Inc.) corresponding to base pairs 761-2961 of pBluescriptII sk(-).

It should be noted that all non-coding DNA sequences described above can be replaced with any other non-coding DNA sequence(s). Missing nucleotide sequences in the above construct represent restriction site remnants.

### EXAMPLE 14

### Transfection of Immature Leghorn Roosters using a Transpson-based Vector containing a Proinsulin Gene via Testicular Injections

Vectors containing the elements Oval promoter/Oval gene/GP41 Enterokinase TAG/Proinsulin/Poly A (SEQ ID NO:31) and CMV promoter/Oval gene/GP41 Enterokinase TAG/Proinsulin/Poly A (SEQ ID NO:42) were each injected into the testes of 11 week old white leghorn roosters. These birds were held under normal conditions until sexual maturity was reached.

At the time of sexual maturity, each bird was handled and manipulated to obtain sperm. Sperm samples were collected in Hank's Buffered Salt Solution (HBSS) and stored at either -20° C or 4° C until needed. DNA was extracted from sperm using a MoBio Ultra Clean DNA Bloodspin Kit (MoBio laboratories, Solana Beach CA). Fifty microliters of sperm was used in the DNA extraction protocol and the purified genomic DNA eluted in 100 µl of water. In each PCR reaction, approximately 0.5 - 0.75 µg of genomic DNA was used with primers anchored in the entag-1 (5') and the synthetic polyA-2 (3'), which amplify a 685 bp fragment. Five of nine birds gave positive reactions for the presence of the appropriate vector construct. These birds were then mated with normal females.

Birds that did not yield positive results with PCR on the sperm were sacrificed, their testes removed, and DNA extracted using an approximately 25 mg piece of tissue in a Qiagen DNEasy Tissue Kit; purified DNA was eluted in 200 µl water and PCR conducted as described above. Two of these birds gave a very strong, positive PCR reaction.

### EXAMPLE 15

### Transfection of Japanese Quail using a Transposon-based Vector containing a Proinsulin Gene via Oviduct Injections

Two experiments were conducted in Japanese quail using transpson-based vectors containing either Oval promoter/Oval gene/GP41 Enterokinase TAG/Proinsulin/Poly A (SEQ ID NO:31) or CMV promoter/Oval gene/GP41 Enterokinase TAG/Proinsulin/Poly A (SEQ ID NO:42).

In the first experiment, the Oval promoter/Oval gene/GP41 Enterokinase TAG/Proinsulin/Poly A containing construct was injected into the oviduct of sexually mature quail; three hens received 5 µg at a 1:3 Superfect ratio and three received 10 µg at a 1:3 Superfect ratio. As of the writing of the present application, at least one bird that received 10 µg of DNA was producing human proinsulin in egg white (other birds remain to be tested). This experiment indicates that 1) the DNA has been stable for at least 3 months; 2) protein levels are comparable to those observed with a constitutive promoter such as the CMV promoter; and 3) sexually mature birds can be injected and results obtained without the need for cell culture.

In the second experiment, the transposon-based vector containing CMV promoter/Oval gene/GP41 Enterokinase TAG/Proinsulin/Poly A was injected into the oviduct of sexually immature Japanese quail. A total of 9 birds were injected. Of the 8 survivors, 3 produced human proinsulin in the white of their eggs for over 6 weeks. An ELISA assay described in detail below was developed to detect GP41 in the fusion peptide (Oval gene/GP41 Enterokinase TAG/Proinsulin) since the GP41 peptide sequence is unique and not found as part of normal egg white protein. In all ELISA assays, the same birds produced positive results and all controls worked as expected.

ELISA Procedure: Individual egg white samples were diluted in sodium carbonate buffer, pH 9.6, and added to individual wells of 96 well microtiter ELISA plates at a total volume of 0.1 ml. These plates were then allowed to coat overnight at 4°C. Prior to ELISA development, the plates were allowed warm to room temperature. Upon decanting the coating solutions and blotting away any excess, non-specific binding of antibodies was blocked by adding a solution of phosphate buffered saline (PBS), 1% (w/v) BSA, and 0.05% (v/v) Tween 20 and allowing it to incubate with shaking for a minimum of 45 minutes. This blocking solution was subsequently decanted and replaced with a solution of the primary antibody (Goat Anti-GP41 TAG) diluted in fresh PBSBSA/Tween 20. After a two hour period of incubation with the primary antibody, each plate was washed with a solution of PBS and 0.05% Tween 20 in an automated plate washer to remove unbound antibody. Next, the secondary antibody, Rabbit anti-Goat Alkaline Phosphatase-conjugated, was diluted in PBSBSA/Tween 20 and allowed to incubate 1 hour. The plates were then subjected to a second wash with PBS/Tween 20. Antigen was detected using a solution of *p*-Nitrophenyl Phosphate in Diethanolamine Substrate Buffer for Alkaline Phosphatase and measuring the absorbance at 30 minutes and 1 hour.

### EXAMPLE 16

### Optimization of Intra-oviduct and Intra-ovarian Arterial Injections

Overall transfection rates of oviduct cells in a flock of chicken or quail hens are enhanced by synchronizing the development of the oviduct and ovary within the flock. When the development of the oviducts and ovaries are uniform across a group of hens and when the stage of oviduct and ovarian development can be determined or predicted, timing of injections is optimized to transfect the greatest number of cells. Accordingly, oviduct development is synchronized as described below to ensure that a large and uniform proportion of oviduct secretory cells are transfected with the gene of interest.

Hens are treated with estradiol to stimulate oviduct maturation as described in Oka and Schimke (T. Oka and RT Schimke, J. Cell Biol., 41, 816 (1969)), Palmiter, Christensen and Schimke (J Biol. Chem. 245(4):833-845, 1970). Specifically, repeated daily injections of 1 mg estradiol benzoate are performed sometime before the onset of sexual maturation, a period ranging from 1-14 weeks of age. After a stimulation period sufficient to maximize development of the oviduct, hormone treatment is withdrawn thereby causing regression in oviduct secretory cell size but not cell number. At an optimum time after hormone withdrawal, the oviducts of treated hens are injected with the transposon-based vector. Hens are subjected to additional estrogen stimulation after an optimized time during which the transposon-based vector is taken up into oviduct secretory cells. Re-stimulation by estrogen activates the transposon mechanism of the transposon-based vector, causing the integration of the gene of interest into the host genome. Estrogen stimulation is then withdrawn and hens continue normal sexual development. If a developmentally regulated promoter such as the ovalbumin promoter is used, expression of the transposon-based vector initiates in the oviduct at the time of sexual maturation. Intra-ovarian artery injection during this window allows for high and uniform transfection efficiencies of ovarian follicles to produce germ-line transfections and possibly oviduct expression.

Other means are also used to synchronize the development, or regression, of the oviduct and ovary to allow high and uniform transfection efficiencies. Alterations of lighting and/or feed regimens, for example, cause hens to 'molt' during which time the oviduct and ovary regress. Molting is used to synchronize hens for transfection, and may be used in conjunction with other hormonal methods to control regression and/or development of the oviduct and ovary.

### EXAMPLE 17

### Isolation ofHuman Proinsulin Using Anti-TAG Column Chromotography

A HiTrap NHS-activated 1 mL column (Amersham) was charged with a 30 amino acid peptide that contained the gp-41 epitope containing gp-41's native disulfide bond that stabilizes the formation of the gp-41 hairpin loop. The 30 amino acid gp41 peptide is provided as SEQ ID NO:23. Approximately 10 mg of the peptide was dissolved in coupling buffer (0.2 M NaHCO3, 0.5 M NaCl, pH 8.3 and the ligand was circulated on the column for 2 hours at room temperature at 0.5 mL/minute. Excess active groups were then deactivated using 6 column volumes of 0.5 M ethanolamine, 0.5 M NaCl, pH 8.3 and the column was washed alternately with 6 column volumes of acetate buffer (0.1 M acetate, 0.5 M NaCl, pH 4.0) and ethanolamine (above). The column was neutralized using 1 X PBS. The column was then washed with buffers to be used in affinity purification: 75 mM Tris, pH 8.0 and elution buffer, 100 mM glycine-HCl, 0.5 M NaCl, pH 2.7. Finally, the column was equilibrated in 75 mM Tris buffer, pH 8.0.

Antibodies to gp-41 were raised in goats by inoculation with the gp-41 peptide described above. More specifically, goats were inoculated, given a booster injection of the gp-41 peptide and then bled. Serum was harvested by centrifugation. Approximately 30 mL of goat serum was filtered to 0.45 uM and passed over a TAG column at a rate of 0.5 mL/min. The column was washed with 75 mM Tris, pH 8.0 until absorbance at 280 nm reached a baseline. Three column volumes (3 mL) of elution buffer (100 mM glycine, 0.5 M NaCl, pH 2.7) was applied, followed by 75 mM Tris buffer, pH 8.0, all at a rate of 0.5 mL/min. One milliliter fractions were collected. Fractions were collected into 200 uL 1 M Tris, pH 9.0 to neutralize acidic factions as rapidly as possible. A large peak eluted from the column, coincident with the application the elution buffer. Fractions were pooled. Analysis by SDS-PAGE showed a high molecular weight species that separated into two fragments under reducing condition, in keeping with the heavy and light chain structure of IgG.

Pooled antibody fractions were used to charge two 1 mL HiTrap NHS-activated columns, attached in series. Coupling was carried out in the same manner as that used for charging the TAG column.

### Isolation of Ovalbumin-TAG-Proinsulin from Egg White

Egg white from quail and chickens treated by intra-oviduct injection of the CMV-ovalbumin-TAG-proinsulin construct were pooled. Viscosity was lowered by subjecting the allantoid fluid to successively finer pore sizes using negative pressure filtration, finishing with a 0.22 µM pore size. Through the process, egg white was diluted approximately 1:16. The clarified sample was loaded on the Anti-TAG column and eluted in the same manner as described for the purification of the anti-TAG antibodies. A peak of absorbance at 280 nm, coincident with the application of the elution buffer, indicated that protein had been specifically eluted from the Anti-TAG column. Fractions containing the eluted peak were pooled for analysis.

The pooled fractions from the Anti-TAG affinity column were characterized by SDS-PAGE and western blot analysis. SDS-PAGE of the pooled fractions revealed a 60 kDal molecular weight band not present in control egg white fluid, consistent with the predicted molecular weight of the transgenic protein. Although some contaminating bands were observed, the 60 kDal species was greatly enriched compared to the other proteins. An aliquot of the pooled fractions was cleaved overnight at room temperature with the protease, enterokinase. SDS-PAGE analysis of the cleavage product, revealed a band not present in the uncut material that co-migrated with a commercial human proinsulin positive control. Western blot analysis showed specific binding to the 60 kDal species under non-reducing condition (which preserve the hairpin epitope of gp-41 by retaining the disulfide bond). Western analysis of the low molecular weight species that appeared upon cleavage with an anti-human proinsulin antibody, conclusively identified the cleaved fragment as human proinsulin.

### EXAMPLE 18

### Construction of a Transposon-based Transgene for the Expression of a Monoclonal Antibody

Production of a monoclonal antibody using transposon-based transgenic methodology is accomplished in a variety of ways.
1) two vectors are constructed: one that encodes the light chain and a second vector that encodes the heavy chain of the monoclonal antibody. These vectors are then incorporated into the genome of the target animal by at least one of two methods: a) direct transfection of a single animal with both vectors (simultaneously or as separate events); or, b) a male and a female of the species carry in their germline one of the vectors and then they are mated to produce progeny that inherit a copy of each.
2) the light and heavy chains are included on a single DNA construct, either separated by insulators and expression is governed by the same (or different) promoters, or by using a single promoter governing expression of both transgenes with the inclusion of elements that permit separate transcription of both transgenes, such as an internal ribosome entry site.

The following example describes the production of a transposon-based DNA construct that contains both the coding region for a monoclonal light chain and a heavy chain on a single construct. Beginning with the vector pTnMod, the coding sequences for the heavy and light chains are added, each preceded by an appropriate promoter and signal sequence. Using methods known to one skilled in the art, approximately 1 Kb of the proximal elements of the ovalbumin promoter are linked to the signal sequence of ovalbumin or some other protein secreted from the target tissue. Two copies of the promoter and signal sequence are added to the multiple cloning site of pTnMod, leaving space and key restriction sites between them to allow the subsequent addition of the coding sequences of the light and heavy chains of the monoclonal antibody. Methods known to one skilled in the art allow the coding sequences of the light and heavy chains to be inserted in-frame for appropriate expression. For example, the coding sequence of light and heavy chains of a murine monoclonal antibody that show specificity for human seminoprotein have recently been disclosed (GenBank Accession numbers AY 129006 and AY 129304 for the light and heavy chains, respectively). The light chain cDNA sequence is provided in SEQ ID NO:34, whereas the cDNA of the heavy chain is reported as provided in SEQ ID NO:35.

Thus one skilled in the art can produce both the heavy and light chains of a monoclonal antibody in a single cell within a target tissue and species. If the modified cell contained normal posttranslational modification capabilities, the two chains would form their native configuration and disulfide attachments and be substrates for glycosylation. Upon secretion, then, the monoclonal antibody is accumulated, for example, in the egg white of a chicken egg, if the transgenes are expressed in the magnum of the oviduct.

It should also be noted that, although this example details production of a full-length murine monoclonal antibody, the method is quite capable of producing hybrid antibodies (e.g. a combination of human and murine sequences; 'humanized' monoclonal antibodies), as well as useful antibody fragments, known to one skilled in the art, such as Fab, Fc, F(ab) and Fv fragments. This method can be used to produce molecules containing the specific areas thought to be the antigen recognition sequences of antibodies (complementarity determining regions), linked, modified or incorporated into other proteins as desired.

### EXAMPLE 19

### Treatment of rats with a transposon-based vector for tissue-specific insulin gene incorporation

Rats are made diabetic by administering the drug streptozotocin (Zanosar; Upjohn, Kalamazoo, MI) at approximately 200 mg/kg. The rats are bred and maintained according to standard procedures. A transposon-based vector containing a proinsulin gene, an appropriate carrier, and, optionally, a transfection agent, are injected into rats' singhepatic (if using G6P) artery with the purpose of stable transformation. Incorporation of the insulin gene into the rat genome and levels of insulin expression are ascertained by a variety of methods known in the art. Blood and tissue samples from live or sacrificed animals are tested. A combination of PCR, Southern and Northern blots, *in-situ* hybridization and related nucleic acid analysis methods are used to determine incorporation of the vector-derived proinsulin DNA and levels of transcription of the corresponding mRNA in various organs and tissues of the rats. A combination of SDS-PAGE gels, Western Blot analysis, radioimmunoassay, and ELISA and other methods known to one of ordinary skill in the art are used to determine the presence of insulin and the amount produced. Additional transfections of the vector are used to increase protein expression if the initial amounts of the expressed insulin are not satisfactory, or if the level of expression tapers off. The physiological condition of the rats is closely examined post-transfection to register positive or any negative effects of the gene therapy. Animals are examined over extended periods of time post-transfection in order to monitor the stability of gene incorporation and protein expression.

### EXAMPLE 20

### Exemplary Transposon-Based Vectors

The following example provides a description of various transposon-based vectors of the present invention and several constructs for insertion into the transposon-based vectors of the present invention. These examples are not meant to be limiting in any way. The constructs for insertion into a transposon-based vector are provided in a cloning vector labeled pTnMCS.

### pTnMCS (base vector)

Bp 1 - 130 Remainder of F1 (-) ori of pBluescriptII sk(-) (Stratagene) bp1-130
Bp 133 - 1777 CMV promoter/enhancer taken from vector pGWIZ (Gene Therapy Systems) bp2 29-1873
Bp 1783 - 2991 Transposase, from Tn10 (GenBank accession #J01829) bp 108-1316
Bp 2992 - 3344 Non coding DNA from vector pNK2859
Bp 3345 - 3387 Lambda DNA from pNK2859
Bp 3388 - 3457 70 bp of IS 10 left from Tn10
Bp 3464 - 3670 Multiple cloning site from pBluescriptII sk(-), thru the XmaI site bp924-718
Bp 3671 - 3715 Multiple cloning site from pBluescriptII sk(-), from the XmaI site thru the XhoI site. These base pairs are usually lost when cloning into pTnMCS.bp 717-673
Bp 3716 - 4153 Multiple cloning site from pBluescriptII sk(-), from the XhoI site bp672-235
Bp 4159 - 4228 70 bp of IS10 left from Tn10
Bp 4229 - 4270 Lambda DNA from pNK2859
Bp 4271 - 5114 Non-coding DNA from pNK2859
Bp 5115 - 7315 pBluescript sk (-) base vector (Stratagene, Inc.) bp 761-2961

### pTnMCS (CMV-preoro-ent-hGH-CPA)

Bp 1 - 3670 from vector PTnMCS, bp 1 - 3670
Bp 3676 - 5320 CMV promoter/enhancer taken from vector pGWIZ (Gene Therapy Systems), bp 230-1864
Bp 5326 - 5496 Capsite/Prepro taken from GenBank accession # X07404, bp 563 - 733
Bp 5504 - 5652 Synthetic spacer sequence and hairpin loop of HIV gp41 with an added enterokinase cleavage site
Bp 5653 - 6306 Human growth hormone taken from GenBank accession # V00519, bp 1-654
Bp 6313 - 6720 Conalbumin polyA taken from GenBank accession # Y00407, bp 10651-11058
Bp 6722 -10321 from cloning vector pTnMCS, bp 3716-7315

### pTnMCS (CMV-CHOVg-ent-ProInsulin-svnPA) (SEO ID NO:41)

Bp 1 - 3670 from vector PTnMCS, bp 1 - 3670
Bp 3676 - 5320 CMV promoter/enhancer taken from vector pGWIZ (Gene Therapy Systems), bp 230-1864
Bp 5327 -6480 Chicken ovalbumin gene taken from GenBank accession # V00383, bp 66-1219
Bp 6487 - 6636 Synthetic spacer sequence and hairpin loop of HIV gp41 with an added enterokinase cleavage site
Bp 6637 - 6897 Human Proinsulin taken from GenBank accession # NM000207, bp 117-377
Bp 6898 - 6942 Spacer DNA, derived as an artifact from the cloning vectors pTOPO Blunt II (Invitrogen) and pGWIZ (Gene Therapy Systems)
Bp 6943 - 7295 Synthetic polyA from the cloning vector pGWIZ (Gene Therapy Systems), bp 1920-2271
Bp 7296 -10895 from cloning vector pTnMCS, bp 3716-7315
pTnMCS (CMV-prepro-ent-ProInsulin-synPA)
Bp 1 - 3670 from vector PTnMCS, bp 1 - 3670
Bp 3676 - 5320 CMV promoter/enhancer taken from vector pGWIZ (Gene Therapy Systems), bp 230-1864
Bp 5326 - 5496 Capsite/Prepro taken fron GenBank accession # X07404, bp 563 - 733
Bp 5504 - 5652 Synthetic spacer sequence and hairpin loop of HIV gp41 with an added enterokinase cleavage site
Bp 5653 - 5913 Human Proinsulin taken from GenBank accession # NM000207, bp 117-377
Bp 5914 - 5958 Spacer DNA, derived as an artifact from the cloning vectors pTOPO Blunt II (Invitrogen) and pGWIZ (Gene Therapy Systems)
Bp 5959 - 6310 Synthetic polyA from the cloning vector pGWIZ (Gene Therapy Systems), bp 1920-2271
Bp 6313 - 9912 from cloning vector pTnMCS, bp 3716-7315

### pTnMCS(Chicken OVep+OVg'+ENT+proins+syn polyA)

Bp 1 - 3670 from vector pTnMCS, bp 1 - 3670
Bp 3676 - 4350 Chicken Ovalbumin enhancer taken from GenBank accession #S82527.1 bp 1-675
Bp 4357 - 5692 Chicken Ovalbumin promoter taken from GenBank accession # J00895M24999 bp 1-1336
Bp 5699 - 6917 Chicken Ovalbumin gene from GenBank Accession # V00383.1 bp 2-1220. (This sequence includes the 5'UTR, containing putative cap site, bp 5699-5762.)
Bp 6924 - 7073 Synthetic spacer sequence and hairpin loop of HIV gp41 with an added enterokinase cleavage site
Bp 7074 - 7334 Human proinsulin GenBank Accession # NM000207 bp 117-377
Bp 7335 - 7379 Spacer DNA, derived as an artifact from the cloning vectors pTOPO Blunt II (Invitrogen) and gWIZ (Gene Therapy Systems)
Bp 7380 - 7731 Synthetic polyA from the cloning vector gWIZ (Gene Therapy Systems) bp 1920 - 2271
Bp 7733 -11332 from vector pTnMCS, bp 3716 - 7315

### pTnMCS(Chicken OVep+prepro+ENT+proins+syn polyA)

Bp1 - 3670 from cloning vector pTnMCS, bp 1 - 3670
Bp 3676 - 4350 Chicken Ovalbumin enhancer taken from GenBank accession # S82527.1 bp 1-675
Bp 4357 - 5692 Chicken Ovalbumin promoter taken from GenBank accession # J00895-M24999 bp 1-1336
Bp 5699 - 5869 Cecropin cap site and Prepro, Genbank accession # X07404 bp 563-733
Bp 5876 - 6025 Synthetic spacer sequence and hairpin loop of HIV gp41 with an added enterokinase cleavage site
Bp 6026 - 6286 Human proinsulin GenBank Accession # NM000207 bp 117-377
Bp 6287 - 6331 Spacer DNA, derived as an artifact from the cloning vectors pTOPO Blunt II (Invitrogen) and gWIZ (Gene Therapy Systems)
Bp 6332 - 6683 Synthetic polyA from the cloning vector gWIZ (Gene Therapy Systems) bp 1920-2271
Bp 6685 -10284 from cloning vector pTnMCS, bp 3716 - 7315

### pTnMCS(Ouail OVep+OVg'+ENT+proins+syn polyA)

Bp 1 - 3670 from cloning vector pTnMCS, bp 1 - 3670
Bp 3676 - 4333 Quail Ovalbumin enhancer: 658 bp sequence, amplified in-house from quail genomic DNA, roughly equivalent to the far-upstream chicken ovalbumin enhancer, GenBank accession # S82527.1, bp 1-675. (There are multiple base pair substitutions and deletions in the quail sequence, relative tochicken, so the number of bases does not correspond exactly.)
Bp 4340 - 5705 Quail Ovalbumin promoter: 1366 bp sequence, amplified in-house from quail genomic DNA, roughly corresponding to chicken ovalbumin promoter, GenBank accession # J00895-M24999 bp 1-1336. (There are multiple base pair substitutions and deletions between the quail and chicken sequences, so the number of bases does not correspond exactly.)
Bp 5712 - 6910 Quail Ovalbumin gene, EMBL accession # X53964, bp 1-1199. (This sequence includes the 5'UTR, containing putative cap site bp 5712-5764.)
Bp 6917 - 7066 Synthetic spacer sequence and hairpin loop of HIV gp41 with an added enterokinase cleavage site
Bp 7067 - 7327 Human proinsulin GenBank Accession # NM000207 bp 117-377
Bp 7328 - 7372 Spacer DNA, derived as an artifact from the cloning vectors pTOPO Blunt II (Invitrogen) and gWIZ (Gene Therapy Systems)
Bp 7373 - 7724 Synthetic polyA from the cloning vector gWIZ (Gene Therapy Systems) bp 1920-2271
Bp 7726 - 11325 from cloning vector pTnMCS, bp 3716 - 7315

### pTnMCS (CHOVep-prepro-ent-hGH-CPA)

Bp 1 - 3670 from vector PTnMCS, bp 1-3670
Bp 3676 - 4350 Chicken Ovalbumin enhancer taken from GenBank accession # S82527.1, bp 1-675
Bp 4357 - 5692 Chicken Ovalbumin promoter taken from GenBank accession # J00899-M24999, bp 1-1336
Bp 5699 - 5869 Capsite/Prepro taken fron GenBank accession # X07404, bp 563-733
Bp 5877 - 6025 Synthetic spacer sequence and hairpin loop of HIV gp41 with an added enterokinase cleavage site
Bp 6026 - 6679 Human growth hormone taken from GenBank accession # V00519, bp 1-654
Bp 6686 - 7093 Conalbumin polyA taken from GenBank accession # Y00407, bp 10651-11058
Bp 7095 - 10694 from cloning vector pTnMCS, bp 3716-7315

### pTnMCS(Ouail OVep+prepro+ENT+proins+syn polyA)

Bp 1 - 3670 from cloning vector pTnMCS, bp 1 - 3670
Bp 3676 - 4333 Quail Ovalbumin enhancer: 658 bp sequence, amplified in-house from quail genomic DNA, roughly equivalent to the far- upstream chicken ovalbumin enhancer, GenBank accession #S82527.1, bp 1-675. (There are multiple base pair substitutions and deletions in the quail sequence, relative to chicken, so the number of bases does not correspond exactly.)
Bp 4340 - 5705 Quail Ovalbumin promoter: 1366 bp sequence, amplified in-house from quail genomic DNA, roughly corresponding to chicken ovalbumin promoter, GenBank accession # J00895-M24999 bp 1-1336. (There are multiple base pair substitutions and deletions between the quail and chicken sequences, so the number of bases does not correspond exactly.)
Bp 5712 - 5882 Cecropin cap site and Prepro, Genbank accession # X07404 bp 563-733
Bp 5889 - 6038 Synthetic spacer sequence and hairpin loop of HIV gp41 with an added enterokinase cleavage site
Bp 6039 - 6299 Human proinsulin GenBank Accession # NM000207 bp 117-377
Bp 6300 - 6344 Spacer DNA, derived as an artifact from the cloning vectors pTOPO Blunt II (Invitrogen) and gWIZ (Gene Therapy Systems)
Bp 6345 - 6696 Synthetic polyA from the cloning vector gWIZ (Gene Therapy Systems) bp 1920 - 2271
Bp 6698 - 10297 from cloning vector pTnMCS, bp 3716 - 7315

### PTnMOD

Bp 1 - 130 remainder of F1 (-) ori of pBluescriptII sk(-) (Stratagene) bp1-130
Bp 133 - 1777 CMV promoter/enhancer taken from vector pGWIZ (Gene Therapy Systems) bp229-1873
Bp 1783 - 2991 Transposase, modified from Tn10 (GenBank accession #J01829) bp 108-1316
Bp 2992 - 2994 Engineered stop codon
Bp 2996 - 3411 Synthetic polyA from gWIZ (Gene Therapy Systems) bp 1922 - 2337
Bp 3412 - 3719 Non-coding DNA from vector pNK2859
Bp 3720 - 3762 Lambda DNA from pNK2859
Bp 3763 - 3832 70 bp of IS10 left from Tn10
Bp 3839 - 4045 Multiple cloning site from pBluescriptII sk(-), thru the XmaI site bp 924-718
Bp 4046 - 4090 Multiple cloning site from pBluescriptII sk(-), from the XmaI site thru the XhoI site. These base pairs are usually lost when cloning into pTnMCS. bp 717-673
Bp 4091 - 4528 Multiple cloning site from pBluescriptII sk(-), from the XhoI site bp 672-235
Bp 4534 - 4603 70 bp of IS10 left from Tn10
Bp 4604 - 4645 Lambda DNA from pNK2859
Bp 4646 - 5489 Non-coding DNA from pNK2859
Bp 5490 - 7690 pBluescript sk (-) base vector (Stratagene, INC) bp 761-2961

### pTnMOD (CHOVep-prepro-ent-hGH-CPA)

Bp 1 - 4045 from vector PTnMCS, bp 1 - 4045
Bp 4051 - 4725 Chicken Ovalbumin enhancer taken from GenBank accession # 582527.1, bp 1 - 675
Bp 4732 - 6067 Chicken Ovalbumin promoter taken from GenBank accession # J00899-M24999, bp 1-1336
Bp 6074 - 6245 Capsite/Prepro taken fron GenBank accession # X07404, bp 563 - 733
Bp 6252 - 6400 Synthetic spacer sequence and hairpin loop of HIV gp41 with an added enterokinase cleavage site
Bp 6401 - 7054 Human growth hormone taken from GenBank accession # V00519, bp 1-654
Bp 7061 - 7468 Conalbumin polyA taken from GenBank accession # Y00407, bp 10651-11058
Bp 7470 - 11069 from cloning vector pTnMCS, bp 3716-7315

### pTnMOD (CMV-CHOVg-ent-ProInsulin-synPA) (SEQ ID NO:42)

Bp 1 - 4045 from vector PTnMCS, bp 1 - 4045
Bp 4051 - 5695 CMV promoter/enhancer taken from vector pGWIZ (Gene therapy systems), bp 230-1864
Bp 5702 -6855 Chicken ovalbumin gene taken from GenBank accession # V00383, bp 66-1219
Bp 6862 - 7011 Synthetic spacer sequence and hairpin loop of HIV gp41 with an added enterokinase cleavage site
Bp 7012 - 7272 Human Proinsulin taken from GenBank accession # NM000207, bp 117-377
Bp 7273 - 7317 Spacer DNA, derived as an artifact from the cloning vectors pTOPO Blunt II (Invitrogen) and pGWIZ (Gene Therapy Systems)
Bp 7318 - 7670 Synthetic polyA from the cloning vector pGWIZ (Gene Therapy Systems), bp 1920-2271
Bp 7672 -11271 from cloning vector pTnMCS, bp 3716-7315

### pTnMOD (CMV-prepro-ent-hGH-CPA)

Bp 1 - 4045 from vector PTnMCS, bp 1 - 4045
Bp 4051 - 5695 CMV promoter/enhancer taken from vector pGWIZ (Gene therapy systems), bp 230-1864
Bp 5701 - 5871 Capsite/Prepro taken fron GenBank accession # X07404, bp 563 - 733
Bp 5879 - 6027 Synthetic spacer sequence and hairpin loop of HIV gp41 with an added enterokinase cleavage site
Bp 6028 - 6681 Human growth hormone taken from GenBank accession # V00519, bp 1-654
Bp 6688 - 7095 Conalbumin polyA taken from GenBank accession # Y00407, bp 10651-11058
Bp 7097-10696 from cloning vector pTnMCS, bp 3716-7315

### pTnMOD (CMV-prepro-ent-ProInsulin-smPA)

Bp 1 - 4045 from vector PTnMCS, bp 1 - 4045
Bp 4051 - 5695 CMV promoter/enhancer taken from vector pGWIZ (Gene therapy systems), bp 230-1864
Bp 5701 - 5871 Capsite/Prepro taken from GenBank accession # X07404, bp 563 - 733
Bp 5879 - 6027 Synthetic spacer sequence and hairpin loop of HIV gp41 with an added enterokinase cleavage site
Bp 6028 - 6288 Human Proinsulin taken from GenBank accession # NM000207, bp 117-377
Bp 6289 - 6333 Spacer DNA, derived as an artifact from the cloning vectors pTOPO Blunt II (Invitrogen) and pGWIZ (Gene Therapy Systems)
Bp 6334 - 6685 Synthetic polyA from the cloning vector pGWIZ (Gene Therapy Systems), bp 1920-2271
Bp 6687 -10286 from cloning vector pTnMCS, bp 3716-7315

### pTnMOD(Chicken OVep+OVg'+ENT+proins+syn polyA) (SEQ ID NO:43)

Bp 1 - 4045 from cloning vector pTnMOD, bp 1 - 4045
Bp 4051 - 4725 Chicken Ovalbumin enhancer taken from GenBank accession # S82527.1 bp 1-675
Bp 4732 - 6067 Chicken Ovalbumin promoter taken from GenBank accession # J00895-M24999 bp 1-1336
Bp 6074 - 7292 Chicken Ovalbumin gene from GenBank Accession # V00383.1 bp 2-1220. (This sequence includes the 5'UTR, containing putative cap site bp 6074-6137.)
Bp 7299 - 7448 Synthetic spacer sequence and hairpin loop of HIV gp41 with an added enterokinase cleavage site
Bp 7449 - 7709 Human proinsulin GenBank Accession # NM000207 bp 117-377
Bp 7710 - 7754 Spacer DNA, derived as an artifact from the cloning vectors pTOPO Blunt II (Invitrogen) and gWIZ (Gene Therapy Systems)
Bp 7755 - 8106 Synthetic polyA from the cloning vector gWIZ (Gene Therapy Systems) bp 1920-2271
Bp 8108 - 11707 from cloning vector pTnMCS, bp 3716 - 7315

### pTnMOD(Chicken OVep+prepro+ENT+proins+syn polyA)

Bp 1 - 4045 from cloning vector pTnMCS, bp 1 - 4045
Bp 4051 - 4725 Chicken Ovalbumin enhancer taken from GenBank accession # S82527.1 bp 1-675
Bp 4732 - 6067 Chicken Ovalbumin promoter taken from GenBank accession # J00895-M24999 bp 1-1336
Bp 6074 - 6244 Cecropin cap site and Prepro, Genbank accession # X07404 bp 563-733
Bp 6251 - 6400 Synthetic spacer sequence and hairpin loop of HIV gp41 with an added enterokinase cleavage site
Bp 6401 - 6661 Human proinsulin GenBank Accession # NM000207 bp 117-377
Bp 6662 - 6706 Spacer DNA, derived as an artifact from the cloning vectors pTOPO Blunt II (Invitrogen) and gWIZ (Gene Therapy Systems)
Bp 6707 - 7058 Synthetic polyA from the cloning vector gWIZ (Gene Therapy Systems) bp 1920 - 2271
Bp 7060 - 10659 from cloning vector pTnMCS, bp 3716 - 7315

### pTnMOD(Ouail OVep+OVg'+ENT+proins+syn polyA)

Bp 1 - 4045 from cloning vector pTnMCS, bp 1 - 4045
Bp 4051 - 4708 Quail Ovalbumin enhancer: 658 bp sequence, amplified in-house from quail genomic DNA, roughly equivalent to the far-upstream chicken ovalbumin enhancer, GenBank accession # S82527.1, bp 1-675. (There are multiple base pair substitutions and deletions in the quail sequence, relative to chicken, so the number of bases does not correspond exactly.)
Bp 4715 - 6080 Quail Ovalbumin promoter: 1366 bp sequence, amplified in-house from quail genomic DNA, roughly corresponding to chicken ovalbumin promoter, GenBank accession # J00895-M24999 bp 1-1336. (There are multiple base pair substitutions and deletions between the quail and chicken sequences, so the number of bases does not correspond exactly.)
Bp 6087 - 7285 Quail Ovalbumin gene, EMBL accession # X53964, bp 1-1199. (This sequence includes the 5'UTR, containing putative cap site bp 6087-6139.)
Bp 7292 - 7441 Synthetic spacer sequence and hairpin loop of HIV gp41 with an added enterokinase cleavage site
Bp 7442 - 7702 Human proinsulin GenBank Accession # NM000207 bp 117-377
Bp 7703 - 7747 Spacer DNA, derived as an artifact from the cloning vectors pTOPO Blunt II (Invitrogen) and gWIZ (Gene Therapy Systems)
Bp 7748 - 8099 Synthetic polyA from the cloning vector gWIZ (Gene Therapy Systems) bp 1920 - 2271
Bp 8101 - 11700 from cloning vector pTnMCS, bp 3716 - 7315

### pTnMOD(Ouail OVep+prepro+ENT+proins+syn polyA)

Bp 1 -4045 from cloning vector pTnMCS, bp 1 - 4045
Bp 4051 - 4708 Quail Ovalbumin enhancer: 658 bp sequence, amplified in-housefrom quail genomic DNA, roughly equivalent to the far-upstream chicken ovalbumin enhancer, GenBank accession #S82527.1, bp 1-675. (There are multiple base pair substitutions and deletions in the quail sequence, relative to chicken, so the number of bases does not correspond exactly.)
Bp 4715 - 6080 Quail Ovalbumin promoter: 1366 bp sequence, amplified in-house from quail genomic DNA, roughly corresponding to chicken ovalbumin promoter, GenBank accession # J00895-M24999 bp 1-1336. (There are multiple base pair substitutions and deletions between the quail and chicken sequences, so the number of bases does not correspond exactly.)
Bp 6087 - 6257 Cecropin cap site and Prepro, Genbank accession # X07404 bp 563-733
Bp 6264 - 6413 Synthetic spacer sequence and hairpin loop of HIV gp41 with an added enterokinase cleavage site
Bp 6414 - 6674 Human proinsulin GenBank Accession # NM000207 bp 117-377
Bp 6675 - 6719 Spacer DNA, derived as an artifact from the cloning vectors pTOPO Blunt II (Invitrogen) and gWIZ (Gene Therapy Systems)
Bp 6720 - 7071 Synthetic polyA from the cloning vector gWIZ (Gene Therapy Systems) bp 1920 - 2271
Bp 7073 - 10672 from cloning vector pTnMCS, bp 3716 - 7315

### PTnMod(CNV/Transposase/ChickOvep/prepro/ProteinA/ConpolyA)

BP 1-130 remainder of F1 (-) ori of pBluescriptII sk(-) (Stragagene) bp 1-130.
BP 133-1777 CMV promoter/enhancer taken from vector pGWIZ (Gene Therapy Systems) bp 229-1873.
BP 1780-2987 Transposase, modified from Tn10 (GenBank #J01829).
BP 2988-2990 Engineered stop codon.
BP 2991-3343 non coding DNA from vector pNK2859.
BP 3344-3386 Lambda DNA from pNK2859.
BP 3387-3456 70bp of IS10 left from Tn10.
BP 3457-3674 multiple cloning site from pBluescriptII sk(-) bp 924-707.
BP 3675-5691 Chicken Ovalbumin enhancer plus promoter from a Topo Clone 10 maxi 040303 (5' XmaI, 3' BamHI)
BP 5698-5865 prepro with Cap site amplified from cecropin of pMON200
GenBank # X07404 (5'BamHI, 3'KpnI)
BP 5872-7338 Protein A gene from GenBank# J01786, mature peptide bp 292-1755 (5'KpnI, 3'SacII)
BP 7345-7752 ConPolyA from Chicken conalbumin polyA from GenBank # Y00407 bp 10651-11058. (5'SacII, 3'XhoI)
BP 7753-8195 multiple cloning site from pBluescriptII sk(-) bp 677-235.
BP 8196-8265 70 bp of IS10 left from Tn10.
BP 8266-8307 Lamda DNA from pNK2859
BP 8308-9151 noncoding DNA from pNK2859
BP 9152-11352 pBluescriptII sk(-) base vector (Stratagene, INC.) bp 761-2961

All patents, publications and abstracts cited above are incorporated herein by reference in their entirety. It should be understood that the foregoing relates only to preferred embodiments of the present invention and that numerous modifications or alterations may be made therein without departing from the spirit and the scope of the present invention as defined in the following claims.

### Appendix A

SEQ ID NO:1 (pTnMod)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850

ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGC 4050
TGCAGGAATT CGATATCAAG CTTATCGATA CCGCTGACCT CGAGGGGGGG 4100
CCCGGTACCC AATTCGCCCT ATAGTGAGTC GTATTACGCG CGCTCACTGG 4150
CCGTCGTTTT ACAACGTCGT GACTGGGAAA ACCCTGGCGT TACCCAACTT 4200
AATCGCCTTG CAGCACATCC CCCTTTCGCC AGCTGGCGTA ATAGCGAAGA 4250
GGCCCGCACC GATCGCCCTT CCCAACAGTT GCGCAGCCTG AATGGCGAAT 4300
GGAAATTGTA AGCGTTAATA TTTTGTTAAA ATTCGCGTTA AATTTTTGTT 4350
AAATCAGCTC ATTTTTTAAC CAATAGGCCG AAATCGGCAA AATCCCTTAT 4400
AAATCAAAAG AATAGACCGA GATAGGGTTG AGTGTTGTTC CAGTTTGGAA 4450
CAAGAGTCCA CTATTAAAGA ACGTGGACTC CAACGTCAAA GGGCGAAAAA 4500
CCGTCTATCA GGGCGATGGC CCACTACTCC GGGATCATAT GACAAGATGT 4550
GTATCCACCT TAACTTAATG ATTTTTACCA AAATCATTAG GGGATTCATC 4600
AGTGCTCAGG GTCAACGAGA ATTAACATTC CGTCAGGAAA GCTTATGATG 4650
ATGATGTGCT TAAAAACTTA CTCAATGGCT GGTTATGCAT ATCGCAATAC 4700
ATGCGAAAAA CCTAAAAGAG CTTGCCGATA AAAAAGGCCA ATTTATTGCT 4750
ATTTACCGCG GCTTTTTATT GAGCTTGAAA GATAAATAAA ATAGATAGGT 4800
TTTATTTGAA GCTAAATCTT CTTTATCGTA AAAAATGCCC TCTTGGGTTA 4850
TCAAGAGGGT CATTATATTT CGCGGAATAA CATCATTTGG TGACGAAATA 4900
ACTAAGCACT TGTCTCCTGT TTACTCCCCT GAGCTTGAGG GGTTAACATG 4950
AAGGTCATCG ATAGCAGGAT AATAATACAG TAAAACGCTA AACCAATAAT 5000
CCAAATCCAG CCATCCCAAA TTGGTAGTGA ATGATTATAA ATAACAGCAA 5050
ACAGTAATGG GCCAATAACA CCGGTTGCAT TGGTAAGGCT CACCAATAAT 5100
CCCTGTAAAG CACCTTGCTG ATGACTCTTT GTTTGGATAG ACATCACTCC 5150
CTGTAATGCA GGTAAAGCGA TCCCACCACC AGCCAATAAA ATTAAAACAG 5200
GGAAAACTAA CCAACCTTCA GATATAAACG CTAAAAAGGC AAATGCACTA 5250
CTATCTGCAA TAAATCCGAG CAGTACTGCC GTTTTTTCGC CCATTTAGTG 5300
GCTATTCTTC CTGCCACAAA GGCTTGGAAT ACTGAGTGTA AAAGACCAAG 5350
ACCCGTAATG AAAAGCCAAC CATCATGCTA TTCATCATCA CGATTTCTGT 5400
AATAGCACCA CACCGTGCTG GATTGGCTAT CAATGCGCTG AAATAATAAT 5450
CAACAAATGG CATCGTTAAA TAAGTGATGT ATACCGATCA GCTTTTGTTC 5500
CCTTTAGTGA GGGTTAATTG CGCGCTTGGC GTAATCATGG TCATAGCTGT 5550
TTCCTGTGTG AAATTGTTAT CCGCTCACAA TTCCACACAA CATACGAGCC 5600
GGAAGCATAA AGTGTAAAGC CTGGGGTGCC TAATGAGTGA GCTAACTCAC 5650
ATTAATTGCG TTGCGCTCAC TGCCCGCTTT CCAGTCGGGA AACCTGTCGT 5700
GCCAGCTGCA TTAATGAATC GGCCAACGCG CGGGGAGAGG CGGTTTGCGT 5750
ATTGGGCGCT CTTCCGCTTC CTCGCTCACT GACTCGCTGC GCTCGGTCGT 5800
TCGGCTGCGG CGAGCGGTAT CAGCTCACTC AAAGGCGGTA ATACGGTTAT 5850
CCACAGAATC AGGGGATAAC GCAGGAAAGA ACATGTGAGC AAAAGGCCAG 5900

CAAAAGGCCA GGAACCGTAA AAAGGCCGCG TTGCTGGCGT TTTTCCATAG 5950
GCTCCGCCCC CCTGACGAGC ATCACAAAAA TCGACGCTCA AGTCAGAGGT 6000
GGCGAAACCC GACAGGACTA TAAAGATACC AGGCGTTTCC CCCTGGAAGC 6050
TCCCTCGTGC GCTCTCCTGT TCCGACCCTG CCGCTTACCG GATACCTGTC 6100
CGCCTTTCTC CCTTCGGGAA GCGTGGCGCT TTCTCATAGC TCACGCTGTA 6150
GGTATCTCAG TTCGGTGTAG GTCGTTCGCT CCAAGCTGGG CTGTGTGCAC 6200
GAACCCCCCG TTCAGCCCGA CCGCTGCGCC TTATCCGGTA ACTATCGTCT 6250
TGAGTCCAAC CCGGTAAGAC ACGACTTATC GCCACTGGCA GCAGCCACTG 6300
GTAACAGGAT TAGCAGAGCG AGGTATGTAG GCGGTGCTAC AGAGTTCTTG 6350
AAGTGGTGGC CTAACTACGG CTACACTAGA AGGACAGTAT TTGGTATCTG 6400
CGCTCTGCTG AAGCCAGTTA CCTTCGGAAA AAGAGTTGGT AGCTCTTGAT 6450
CCGGCAAACA AACCACCGCT GGTAGCGGTG GTTTTTTTGT TTGCAAGCAG 6500
CAGATTACGC GCAGAAAAAA AGGATCTCAA GAAGATCCTT TGATCTTTTC 6550
TACGGGGTCT GACGCTCAGT GGAACGAAAA CTCACGTTAA GGGATTTTGG 6600
TCATGAGATT ATCAAAAAGG ATCTTCACCT AGATCCTTTT AAATTAAAAA 6650
TGAAGTTTTA AATCAATCTA AAGTATATAT GAGTAAACTT GGTCTGACAG 6700
TTACCAATGC TTAATCAGTG AGGCACCTAT CTCAGCGATC TGTCTATTTC 6750
GTTCATCCAT AGTTGCCTGA CTCCCCGTCG TGTAGATAAC TACGATACGG 6800
GAGGGCTTAC CATCTGGCCC CAGTGCTGCA ATGATACCGC GAGACCCACG 6850
CTCACCGGCT CCAGATTTAT CAGCAATAAA CCAGCCAGCC GGAAGGGCCG 6900
AGCGCAGAAG TGGTCCTGCA ACTTTATCCG CCTCCATCCA GTCTATTAAT 6950
TGTTGCCGGG AAGCTAGAGT AAGTAGTTCG CCAGTTAATA GTTTGCGCAA 7000
CGTTGTTGCC ATTGCTACAG GCATCGTGGT GTCACGCTCG TCGTTTGGTA 7050
TGGCTTCATT CAGCTCCGGT TCCCAACGAT CAAGGCGAGT TACATGATCC 7100
CCCATGTTGT GCAAAAAAGC GGTTAGCTCC TTCGGTCCTC CGATCGTTGT 7150
CAGAAGTAAG TTGGCCGCAG TGTTATCACT CATGGTTATG GCAGCACTGC 7200
ATAATTCTCT TACTGTCATG CCATCCGTAA GATGCTTTTC TGTGACTGGT 7250
GAGTACTCAA CCAAGTCATT CTGAGAATAG TGTATGCGGC GACCGAGTTG 7300
CTCTTGCCCG GCGTCAATAC GGGATAATAC CGCGCCACAT AGCAGAACTT 7350
TAAAAGTGCT CATCATTGGA AAACGTTCTT CGGGGCGAAA ACTCTCAAGG 7400
ATCTTACCGC TGTTGAGATC CAGTTCGATG TAACCCACTC GTGCACCCAA 7450
CTGATCTTCA GCATCTTTTA CTTTCACCAG CGTTTCTGGG TGAGCAAAAA 7500
CAGGAAGGCA AAATGCCGCA AAAAAGGGAA TAAGGGCGAC ACGGAAATGT 7550
TGAATACTCA TACTCTTCCT TTTTCAATAT TATTGAAGCA TTTATCAGGG 7600
TTATTGTCTC ATGAGCGGAT ACATATTTGA ATGTATTTAG AAAAATAAAC 7650
AAATAGGGGT TCCGCGCACA TTTCCCCGAA AAGTGCCAC 7689

SEQ ID NO:2 (PTnMod (CMV/Red))

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050

ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGC 4050
ATCAGATTGG CTATTGGCCA TTGCATACGT TGTATCCATA TCATAATATG 4100

TACATTTATA TTGGCTCATG TCCAACATTA CCGCCATGTT GACATTGATT 4150
ATTGACTAGT TATTAATAGT AATCAATTAC GGGGTCATTA GTTCATAGCC 4200
CATATATGGA GTTCCGCGTT ACATAACTTA CGGTAAATGG CCCGCCTGGC 4250
TGACCGCCCA ACGACCCCCG CCCATTGACG TCAATAATGA CGTATGTTCC 4300
CATAGTAACG CCAATAGGGA CTTTCCATTG ACGTCAATGG GTGGAGTATT 4350
TACGGTAAAC TGCCCACTTG GCAGTACATC AAGTGTATCA TATGCCAAGT 4400
ACGCCCCCTA TTGACGTCAA TGACGGTAAA TGGCCCGCCT GGCATTATGC 4450
CCAGTACATG ACCTTATGGG ACTTTCCTAC TTGGCAGTAC ATCTACGTAT 4500
TAGTCATCGC TATTACCATG GTGATGCGGT TTTGGCAGTA CATCAATGGG 4550
CGTGGATAGC GGTTTGACTC ACGGGGATTT CCAAGTCTCC ACCCCATTGA 4600
CGTCAATGGG AGTTTGTTTT GGCACCAAAA TCAACGGGAC TTTCCAAAAT 4650
GTCGTAACAA CTCCGCCCCA TTGACGCAAA TGGGCGGTAG GCGTGTACGG 4700
TGGGAGGTCT ATATAAGCAG AGCTCGTTTA GTGAACCGTC AGATCGCCTG 4750
GAGACGCCAT CCACGCTGTT TTGACCTCCA TAGAAGACAC CGGGACCGAT 4800
CCAGCCTCCG CGGCCGGGAA CGGTGCATTG GAACGCGGAT TCCCCGTGCC 4850
AAGAGTGACG TAAGTACCGC CTATAGACTC TATAGGCACA CCCCTTTGGC 4900
TCTTATGCAT GCTATACTGT TTTTGGCTTG GGGCCTATAC ACCCCCGCTT 4950
CCTTATGCTA TAGGTGATGG TATAGCTTAG CCTATAGGTG TGGGTTATTG 5000
ACCATTATTG ACCACTCCCC TATTGGTGAC GATACTTTCC ATTACTAATC 5050
CATAACATGG CTCTTTGCCA CAACTATCTC TATTGGCTAT ATGCCAATAC 5100
TCTGTCCTTC AGAGACTGAC ACGGACTCTG TATTTTTACA GGATGGGGTC 5150
CCATTTATTA TTTACAAATT CACATATACA ACAACGCCGT CCCCCGTGCC 5200
CGCAGTTTTT ATTAAACATA GCGTGGGATC TCCACGCGAA TCTCGGGTAC 5250
GTGTTCCGGA CATGGGCTCT TCTCCGGTAG CGGCGGAGCT TCCACATCCG 5300
AGCCCTGGTC CCATGCCTCC AGCGGCTCAT GGTCGCTCGG CAGCTCCTTG 5350
CTCCTAACAG TGGAGGCCAG ACTTAGGCAC AGCACAATGC CCACCACCAC 5400
CAGTGTGCCG CACAAGGCCG TGGCGGTAGG GTATGTGTCT GAAAATGAGC 5450
GTGGAGATTG GGCTCGCACG GCTGACGCAG ATGGAAGACT TAAGGCAGCG 5500
GCAGAAGAAG ATGCAGGCAG CTGAGTTGTT GTATTCTGAT AAGAGTCAGA 5550
GGTAACTCCC GTTGCGGTGC TGTTAACGGT GGAGGGCAGT GTAGTCTGAG 5600
CAGTACTCGT TGCTGCCGCG CGCGCCACCA GACATAATAG CTGACAGACT 5650
AACAGACTGT TCCTTTCCAT GGGTCTTTTC TGCAGTCACC GTCTCGCGAC 5700
AGGGATCCAC CGGTCGCCAC CATGGTGCGC TCCTCCAAGA ACGTCATCAA 5750
GGAGTTCATG CGCTTCAAGG TGCGCATGGA GGGCACCGTG AACGGCCACG 5800
AGTTCGAGAT CGAGGGCGAG GGCGAGGGCC GCCCCTACGA GGGCCACAAC 5850
ACCGTGAAGC TGAAGGTGAC CAAGGGCGGC CCCCTGCCCT TCGCCTGGGA 5900
CATCCTGTCC CCCCAGTTCC AGTACGGCTC CAAGGTGTAC GTGAAGCACC 5950
CCGCCGACAT CCCCGACTAC AAGAAGCTGT CCTTCCCCGA GGGCTTCAAG 6000
TGGGAGCGCG TGATGAACTT CGAGGACGGC GGCGTGGTGA CCGTGACCCA 6050
GGACTCCTCC CTGCAGGACG GCTGCTTCAT CTACAAGGTG AAGTTCATCG 6100
GCGTGAACTT CCCCTCCGAC GGCCCCGTAA TGCAGAAGAA GACCATGGGC 6150
TGGGAGGCCT CCACCGAGCG CCTGTACCCC CGCGACGGCG TGCTGAAGGG 6200
CGAGATCCAC AAGGCCCTGA AGCTGAAGGA CGGCGGCCAC TACCTGGTGG 6250
AGTTCAAGTC CATCTACATG GCCAAGAAGC CCGTGCAGCT GCCCGGCTAC 6300
TACTACGTGG ACTCCAAGCT GGACATCACC TCCCACAACG AGGACTACAC 6350
CATCGTGGAG CAGTACGAGC GCACCGAGGG CCGCCACCAC CTGTTCCTGT 6400
AGCGGCCGCG ACTCTAGATC ATAATCAGCC ATACCACATT TGTAGAGGTT 6450
TTACTTGCTT TAAAAAACCT CCCACACCTC CCCCTGAACC TGAAACATAA 6500
AATGAATGCA ATTGTTGTTG TTAACTTGTT TATTGCAGCT TATAATGGTT 6550
ACAAATAAAG CAATAGCATC ACAAATTTCA CAAATAAAGC ATTTTTTTCA 6600
CTGCATTCTA GTTGTGGCCC GGGCTGCAGG AATTCGATAT CAAGCTTATC 6650
GATACCGCTG ACCTCGAGGG GGGGCCCGGT ACCCAATTCG CCCTATAGTG 6700
AGTCGTATTA CGCGCGCTCA CTGGCCGTCG TTTTACAACG TCGTGACTGG 6750
GAAAACCCTG GCGTTACCCA ACTTAATCGC CTTGCAGCAC ATCCCCCTTT 6800
CGCCAGCTGG CGTAATAGCG AAGAGGCCCG CACCGATCGC CCTTCCCAAC 6850
AGTTGCGCAG CCTGAATGGC GAATGGAAAT TGTAAGCGTT AATATTTTGT 6900
TAAAATTCGC GTTAAATTTT TGTTAAATCA GCTCATTTTT TAACCAATAG 6950
GCCGAAATCG GCAAAATCCC TTATAAATCA AAAGAATAGA CCGAGATAGG 7000
GTTGAGTGTT GTTCCAGTTT GGAACAAGAG TCCACTATTA AAGAACGTGG 7050
ACTCCAACGT CAAAGGGCGA AAAACCGTCT ATCAGGGCGA TGGCCCACTA 7100
CTCCGGGATC ATATGACAAG ATGTGTATCC ACCTTAACTT AATGATTTTT 7150

ACCAAAATCA TTAGGGGATT CATCAGTGCT CAGGGTCAAC GAGAATTAAC 7200
ATTCCGTCAG GAAAGCTTAT GATGATGATG TGCTTAAAAA CTTACTCAAT 7250
GGCTGGTTAT GCATATCGCA ATACATGCGA AAAACCTAAA AGAGCTTGCC 7300
GATAAAAAAG GCCAATTTAT TGCTATTTAC CGCGGCTTTT TATTGAGCTT 7350
GAAAGATAAA TAAAATAGAT AGGTTTTATT TGAAGCTAAA TCTTCTTTAT 7400
CGTAAAAAAT GCCCTCTTGG GTTATCAAGA GGGTCATTAT ATTTCGCGGA 7450
ATAACATCAT TTGGTGACGA AATAACTAAG CACTTGTCTC CTGTTTACTC 7500
CCCTGAGCTT GAGGGGTTAA CATGAAGGTC ATCGATAGCA GGATAATAAT 7550
ACAGTAAAAC GCTAAACCAA TAATCCAAAT CCAGCCATCC CAAATTGGTA 7600
GTGAATGATT ATAAATAACA GCAAACAGTA ATGGGCCAAT AACACCGGTT 7650
GCATTGGTAA GGCTCACCAA TAATCCCTGT AAAGCACCTT GCTGATGACT 7700
CTTTGTTTGG ATAGACATCA CTCCCTGTAA TGCAGGTAAA GCGATCCCAC 7750
CACCAGCCAA TAAAATTAAA ACAGGGAAAA CTAACCAACC TTCAGATATA 7800
AACGCTAAAA AGGCAAATGC ACTACTATCT GCAATAAATC CGAGCAGTAC 7850
TGCCGTTTTT TCGCCCATTT AGTGGCTATT CTTCCTGCCA CAAAGGCTTG 7900
GAATACTGAG TGTAAAAGAC CAAGACCCGT AATGAAAAGC CAACCATCAT 7950
GCTATTCATC ATCACGATTT CTGTAATAGC ACCACACCGT GCTGGATTGG 8000
CTATCAATGC GCTGAAATAA TAATCAACAA ATGGCATCGT TAAATAAGTG 8050
ATGTATACCG ATCAGCTTTT GTTCCCTTTA GTGAGGGTTA ATTGCGCGCT 8100
TGGCGTAATC ATGGTCATAG CTGTTTCCTG TGTGAAATTG TTATCCGCTC 8150
ACAATTCCAC ACAACATACG AGCCGGAAGC ATAAAGTGTA AAGCCTGGGG 8200
TGCCTAATGA GTGAGCTAAC TCACATTAAT TGCGTTGCGC TCACTGCCCG 8250
CTTTCCAGTC GGGAAACCTG TCGTGCCAGC TGCATTAATG AATCGGCCAA 8300
CGCGCGGGGA GAGGCGGTTT GCGTATTGGG CGCTCTTCCG CTTCCTCGCT 8350
CACTGACTCG CTGCGCTCGG TCGTTCGGCT GCGGCGAGCG GTATCAGCTC 8400
ACTCAAAGGC GGTAATACGG TTATCCACAG AATCAGGGGA TAACGCAGGA 8450
AAGAACATGT GAGCAAAAGG CCAGCAAAAG GCCAGGAACC GTAAAAAGGC 8500
CGCGTTGCTG GCGTTTTTCC ATAGGCTCCG CCCCCCTGAC GAGCATCACA 8550
AAAATCGACG CTCAAGTCAG AGGTGGCGAA ACCCGACAGG ACTATAAAGA 8600
TACCAGGCGT TTCCCCCTGG AAGCTCCCTC GTGCGCTCTC CTGTTCCGAC 8650
CCTGCCGCTT ACCGGATACC TGTCCGCCTT TCTCCCTTCG GGAAGCGTGG 8700
CGCTTTCTCA TAGCTCACGC TGTAGGTATC TCAGTTCGGT GTAGGTCGTT 8750
CGCTCCAAGC TGGGCTGTGT GCACGAACCC CCCGTTCAGC CCGACCGCTG 8800
CGCCTTATCC GGTAACTATC GTCTTGAGTC CAACCCGGTA AGACACGACT 8850
TATCGCCACT GGCAGCAGCC ACTGGTAACA GGATTAGCAG AGCGAGGTAT 8900
GTAGGCGGTG CTACAGAGTT CTTGAAGTGG TGGCCTAACT ACGGCTACAC 8950
TAGAAGGACA GTATTTGGTA TCTGCGCTCT GCTGAAGCCA GTTACCTTCG 9000
GAAAAAGAGT TGGTAGCTCT TGATCCGGCA AACAAACCAC CGCTGGTAGC 9050
GGTGGTTTTT TTGTTTGCAA GCAGCAGATT ACGCGCAGAA AAAAAGGATC 9100
TCAAGAAGAT CCTTTGATCT TTTCTACGGG GTCTGACGCT CAGTGGAACG 9150
AAAACTCACG TTAAGGGATT TTGGTCATGA GATTATCAAA AAGGATCTTC 9200
ACCTAGATCC TTTTAAATTA AAAATGAAGT TTTAAATCAA TCTAAAGTAT 9250
ATATGAGTAA ACTTGGTCTG ACAGTTACCA ATGCTTAATC AGTGAGGCAC 9300
CTATCTCAGC GATCTGTCTA TTTCGTTCAT CCATAGTTGC CTGACTCCCC 9350
GTCGTGTAGA TAACTACGAT ACGGGAGGGC TTACCATCTG GCCCCAGTGC 9400
TGCAATGATA CCGCGAGACC CACGCTCACC GGCTCCAGAT TTATCAGCAA 9450
TAAACCAGCC AGCCGGAAGG GCCGAGCGCA GAAGTGGTCC TGCAACTTTA 9500
TCCGCCTCCA TCCAGTCTAT TAATTGTTGC CGGGAAGCTA GAGTAAGTAG 9550
TTCGCCAGTT AATAGTTTGC GCAACGTTGT TGCCATTGCT ACAGGCATCG 9600
TGGTGTCACG CTCGTCGTTT GGTATGGCTT CATTCAGCTC CGGTTCCCAA 9650
CGATCAAGGC GAGTTACATG ATCCCCCATG TTGTGCAAAA AAGCGGTTAG 9700
CTCCTTCGGT CCTCCGATCG TTGTCAGAAG TAAGTTGGCC GCAGTGTTAT 9750
CACTCATGGT TATGGCAGCA CTGCATAATT CTCTTACTGT CATGCCATCC 9800
GTAAGATGCT TTTCTGTGAC TGGTGAGTAC TCAACCAAGT CATTCTGAGA 9850
ATAGTGTATG CGGCGACCGA GTTGCTCTTG CCCGGCGTCA ATACGGGATA 9900
ATACCGCGCC ACATAGCAGA ACTTTAAAAG TGCTCATCAT TGGAAAACGT 9950
TCTTCGGGGC GAAAACTCTC AAGGATCTTA CCGCTGTTGA GATCCAGTTC 10000
GATGTAACCC ACTCGTGCAC CCAACTGATC TTCAGCATCT TTTACTTTCA 10050
CCAGCGTTTC TGGGTGAGCA AAAACAGGAA GGCAAAATGC CGCAAAAAAG 10100
GGAATAAGGG CGACACGGAA ATGTTGAATA CTCATACTCT TCCTTTTTCA 10150
ATATTATTGA AGCATTTATC AGGGTTATTG TCTCATGAGC GGATACATAT 10200

TTGAATGTAT TTAGAAAAAT AAACAAATAG GGGTTCCGCG CACATTTCCC 10250
CGAAAAGTGC CAC 10263

SEQ ID NO:3 (PTnMod (Oval/Red) Chicken)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750

ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGG 4050
AGGTCAGAAT GGTTTCTTTA CTGTTTGTCA ATTCTATTAT TTCAATACAG 4100
AACAATAGCT TCTATAACTG AAATATATTT GCTATTGTAT ATTATGATTG 4150
TCCCTCGAAC CATGAACACT CCTCCAGCTG AATTTCACAA TTCCTCTGTC 4200
ATCTGCCAGG CCATTAAGTT ATTCATGGAA GATCTTTGAG GAACACTGCA 4250
AGTTCATATC ATAAACACAT TTGAAATTGA GTATTGTTTT GCATTGTATG 4300
GAGCTATGTT TTGCTGTATC CTCAGAAAAA AAGTTTGTTA TAAAGCATTC 4350
ACACCCATAA AAAGATAGAT TTAAATATTC CAGCTATAGG AAAGAAAGTG 4400
CGTCTGCTCT TCACTCTAGT CTCAGTTGGC TCCTTCACAT GCATGCTTCT 4450
TTATTTCTCC TATTTTGTCA AGAAAATAAT AGGTCACGTC TTGTTCTCAC 4500
TTATGTCCTG CCTAGCATGG CTCAGATGCA CGTTGTAGAT ACAAGAAGGA 4550
TCAAATGAAA CAGACTTCTG GTCTGTTACT ACAACCATAG TAATAAGCAC 4600
ACTAACTAAT AATTGCTAAT TATGTTTTCC ATCTCTAAGG TTCCCACATT 4650
TTTCTGTTTT CTTAAAGATC CCATTATCTG GTTGTAACTG AAGCTCAATG 4700
GAACATGAGC AATATTTCCC AGTCTTCTCT CCCATCCAAC AGTCCTGATG 4750
GATTAGCAGA ACAGGCAGAA AACACATTGT TACCCAGAAT TAAAAACTAA 4800
TATTTGCTCT CCATTCAATC CAAAATGGAC CTATTGAAAC TAAAATCTAA 4850
CCCAATCCCA TTAAATGATT TCTATGGCGT CAAAGGTCAA ACTTCTGAAG 4900
GGAACCTGTG GGTGGGTCAC AATTCAGGCT ATATATTCCC CAGGGCTCAG 4950
CGGATCTCCA TGGGCTCCAT CGGTGCAGCA AGCATGGAAT TTTGTTTTGA 5000
TGTATTCAAG GAGCTCAAAG TCCACCATGC CAATGAGAAC ATCTTCTACT 5050
GCCCCATTGC CATCATGTCA GCTCTAGCCA TGGTATACCT GGGTGCAAAA 5100
GACAGCACCA GGGAATTCGT GCGCTCCTCC AAGAACGTCA TCAAGGAGTT 5150
CATGCGCTTC AAGGTGCGCA TGGAGGGCAC CGTGAACGGC CACGAGTTCG 5200
AGATCGAGGG CGAGGGCGAG GGCCGCCCCT ACGAGGGCCA CAACACCGTG 5250
AAGCTGAAGG TGACCAAGGG CGGCCCCCTG CCCTTCGCCT GGGACATCCT 5300
GTCCCCCCAG TTCCAGTACG GCTCCAAGGT GTACGTGAAG CACCCCGCCG 5350
ACATCCCCGA CTACAAGAAG CTGTCCTTCC CCGAGGGCTT CAAGTGGGAG 5400
CGCGTGATGA ACTTCGAGGA CGGCGGCGTG GTGACCGTGA CCCAGGACTC 5450
CTCCCTGCAG GACGGCTGCT TCATCTACAA GGTGAAGTTC ATCGGCGTGA 5500
ACTTCCCCTC CGACGGCCCC GTAATGCAGA AGAAGACCAT GGGCTGGGAG 5550
GCCTCCACCG AGCGCCTGTA CCCCCGCGAC GGCGTGCTGA AGGGCGAGAT 5600
CCACAAGGCC CTGAAGCTGA AGGACGGCGG CCACTACCTG GTGGAGTTCA 5650
AGTCCATCTA CATGGCCAAG AAGCCCGTGC AGCTGCCCGG CTACTACTAC 5700
GTGGACTCCA AGCTGGACAT CACCTCCCAC AACGAGGACT ACACCATCGT 5750
GGAGCAGTAC GAGCGCACCG AGGGCCGCCA CCACCTGTTC CTGTAGCGGC 5800

CGCGACTCTA GATCATAATC AGCCATACCA CATTTGTAGA GGTTTTACTT 5850
GCTTTAAAAA ACCTCCCACA CCTCCCCCTG AACCTGAAAC ATAAAATGAA 5900
TGCAATTGTT GTTGTTAACT TGTTTATTGC AGCTTATAAT GGTTACAAAT 5950
AAAGCAATAG CATCACAAAT TTCACAAATA AAGCATTTTT TTCACTGCAT 6000
TCTAGTTGTG GCTCGAGAAG GGCGAATTCT GCAGATATCC ATCACACTGG 6050
CGGCCGCTCG AGGGGGGGCC CGGTACCCAA TTCGCCCTAT AGTGAGTCGT 6100
ATTACGCGCG CTCACTGGCC GTCGTTTTAC AACGTCGTGA CTGGGAAAAC 6150
CCTGGCGTTA CCCAACTTAA TCGCCTTGCA GCACATCCCC CTTTCGCCAG 6200
CTGGCGTAAT AGCGAAGAGG CCCGCACCGA TCGCCCTTCC CAACAGTTGC 6250
GCAGCCTGAA TGGCGAATGG AAATTGTAAG CGTTAATATT TTGTTAAAAT 6300
TCGCGTTAAA TTTTTGTTAA ATCAGCTCAT TTTTTAACCA ATAGGCCGAA 6350
ATCGGCAAAA TCCCTTATAA ATCAAAAGAA TAGACCGAGA TAGGGTTGAG 6400
TGTTGTTCCA GTTTGGAACA AGAGTCCACT ATTAAAGAAC GTGGACTCCA 6450
ACGTCAAAGG GCGAAAAACC GTCTATCAGG GCGATGGCCC ACTACTCCGG 6500
GATCATATGA CAAGATGTGT ATCCACCTTA ACTTAATGAT TTTTACCAAA 6550
ATCATTAGGG GATTCATCAG TGCTCAGGGT CAACGAGAAT TAACATTCCG 6600
TCAGGAAAGC TTATGATGAT GATGTGCTTA AAAACTTACT CAATGGCTGG 6650
TTATGCATAT CGCAATACAT GCGAAAAACC TAAAAGAGCT TGCCGATAAA 6700
AAAGGCCAAT TTATTGCTAT TTACCGCGGC TTTTTATTGA GCTTGAAAGA 6750
TAAATAAAAT AGATAGGTTT TATTTGAAGC TAAATCTTCT TTATCGTAAA 6800
AAATGCCCTC TTGGGTTATC AAGAGGGTCA TTATATTTCG CGGAATAACA 6850
TCATTTGGTG ACGAAATAAC TAAGCACTTG TCTCCTGTTT ACTCCCCTGA 6900
GCTTGAGGGG TTAACATGAA GGTCATCGAT AGCAGGATAA TAATACAGTA 6950
AAACGCTAAA CCAATAATCC AAATCCAGCC ATCCCAAATT GGTAGTGAAT 7000
GATTATAAAT AACAGCAAAC AGTAATGGGC CAATAACACC GGTTGCATTG 7050
GTAAGGCTCA CCAATAATCC CTGTAAAGCA CCTTGCTGAT GACTCTTTGT 7100
TTGGATAGAC ATCACTCCCT GTAATGCAGG TAAAGCGATC CCACCACCAG 7150
CCAATAAAAT TAAAACAGGG AAAACTAACC AACCTTCAGA TATAAACGCT 7200
AAAAAGGCAA ATGCACTACT ATCTGCAATA AATCCGAGCA GTACTGCCGT 7250
TTTTTCGCCC CATTTAGTGG CTATTCTTCC TGCCACAAAG GCTTGGAATA 7300
CTGAGTGTAA AAGACCAAGA CCCGCTAATG AAAAGCCAAC CATCATGCTA 7350
TTCCATCCAA AACGATTTTC GGTAAATAGC ACCCACACCG TTGCGGGAAT 7400
TTGGCCTATC AATTGCGCTG AAAAATAAAT AATCAACAAA ATGGCATCGT 7450
TTTAAATAAA GTGATGTATA CCGAATTCAG CTTTTGTTCC CTTTAGTGAG 7500
GGTTAATTGC GCGCTTGGCG TAATCATGGT CATAGCTGTT TCCTGTGTGA 7550
AATTGTTATC CGCTCACAAT TCCACACAAC ATACGAGCCG GAAGCATAAA 7600
GTGTAAAGCC TGGGGTGCCT AATGAGTGAG CTAACTCACA TTAATTGCGT 7650
TGCGCTCACT GCCCGCTTTC CAGTCGGGAA ACCTGTCGTG CCAGCTGCAT 7700
TAATGAATCG GCCAACGCGC GGGGAGAGGC GGTTTGCGTA TTGGGCGCTC 7750
TTCCGCTTCC TCGCTCACTG ACTCGCTGCG CTCGGTCGTT CGGCTGCGGC 7800
GAGCGGTATC AGCTCACTCA AAGGCGGTAA TACGGTTATC CACAGAATCA 7850
GGGGATAACG CAGGAAAGAA CATGTGAGCA AAAGGCCAGC AAAAGGCCAG 7900
GAACCGTAAA AAGGCCGCGT TGCTGGCGTT TTTCCATAGG CTCCGCCCCC 7950
CTGACGAGCA TCACAAAAAT CGACGCTCAA GTCAGAGGTG GCGAAACCCG 8000
ACAGGACTAT AAAGATACCA GGCGTTTCCC CCTGGAAGCT CCCTCGTGCG 8050
CTCTCCTGTT CCGACCCTGC CGCTTACCGG ATACCTGTCC GCCTTTCTCC 8100
CTTCGGGAAG CGTGGCGCTT TCTCATAGCT CACGCTGTAG GTATCTCAGT 8150
TCGGTGTAGG TCGTTCGCTC CAAGCTGGGC TGTGTGCACG AACCCCCCGT 8200
TCAGCCCGAC CGCTGCGCCT TATCCGGTAA CTATCGTCTT GAGTCCAACC 8250
CGGTAAGACA CGACTTATCG CCACTGGCAG CAGCCACTGG TAACAGGATT 8300
AGCAGAGCGA GGTATGTAGG CGGTGCTACA GAGTTCTTGA AGTGGTGGCC 8350
TAACTACGGC TACACTAGAA GGACAGTATT TGGTATCTGC GCTCTGCTGA 8400
AGCCAGTTAC CTTCGGAAAA AGAGTTGGTA GCTCTTGATC CGGCAAACAA 8450
ACCACCGCTG GTAGCGGTGG TTTTTTTGTT TGCAAGCAGC AGATTACGCG 8500
CAGAAAAAAA GGATCTCAAG AAGATCCTTT GATCTTTTCT ACGGGGTCTG 8550
ACGCTCAGTG GAACGAAAAC TCACGTTAAG GGATTTTGGT CATGAGATTA 8600
TCAAAAAGGA TCTTCACCTA GATCCTTTTA AATTAAAAAT GAAGTTTTAA 8650
ATCAATCTAA AGTATATATG AGTAAACTTG GTCTGACAGT TACCAATGCT 8700
TAATCAGTGA GGCACCTATC TCAGCGATCT GTCTATTTCG TTCATCCATA 8750
GTTGCCTGAC TCCCCGTCGT GTAGATAACT ACGATACGGG AGGGCTTACC 8800
ATCTGGCCCC AGTGCTGCAA TGATACCGCG AGACCCACGC TCACCGGCTC 8850

CAGATTTATC AGCAATAAAC CAGCCAGCCG GAAGGGCCGA GCGCAGAAGT 8900
GGTCCTGCAA CTTTATCCGC CTCCATCCAG TCTATTAATT GTTGCCGGGA 8950
AGCTAGAGTA AGTAGTTCGC CAGTTAATAG TTTGCGCAAC GTTGTTGCCA 9000
TTGCTACAGG CATCGTGGTG TCACGCTCGT CGTTTGGTAT GGCTTCATTC 9050
AGCTCCGGTT CCCAACGATC AAGGCGAGTT ACATGATCCC CCATGTTGTG 9100
CAAAAAAGCG GTTAGCTCCT TCGGTCCTCC GATCGTTGTC AGAAGTAAGT 9150
TGGCCGCAGT GTTATCACTC ATGGTTATGG CAGCACTGCA TAATTCTCTT 9200
ACTGTCATGC CATCCGTAAG ATGCTTTTCT GTGACTGGTG AGTACTCAAC 9250
CAAGTCATTC TGAGAATAGT GTATGCGGCG ACCGAGTTGC TCTTGCCCGG 9300
CGTCAATACG GGATAATACC GCGCCACATA GCAGAACTTT AAAAGTGCTC 9350
ATCATTGGAA AACGTTCTTC GGGGCGAAAA CTCTCAAGGA TCTTACCGCT 9400
GTTGAGATCC AGTTCGATGT AACCCACTCG TGCACCCAAC TGATCTTCAG 9450
CATCTTTTAC TTTCACCAGC GTTTCTGGGT GAGCAAAAAC AGGAAGGCAA 9500
AATGCCGCAA AAAAGGGAAT AAGGGCGACA CGGAAATGTT GAATACTCAT 9550
ACTCTTCCTT TTTCAATATT ATTGAAGCAT TTATCAGGGT TATTGTCTCA 9600
TGAGCGGATA CATATTTGAA TGTATTTAGA AAAATAAACA AATAGGGGTT 9650
CCGCGCACAT TTCCCCGAAA AGTGCCAC 9678

SEQ ID NO:4 (PTnMod (Oval/Red) Quail)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000

GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGG 4050
AGGTCAGAAT GGTTTCTTTA CTGTTTGTCA ATTCTATTAT TTCAATACAG 4100
AACAAAAGCT TCTATAACTG AAATATATTT GCTATTGTAT ATTATGATTG 4150
TCCCTCGAAC CATGAACACT CCTCCAGCTG AATTTCACAA TTCCTCTGTC 4200
ATCTGCCAGG CTGGAAGATC ATGGAAGATC TCTGAGGAAC ATTGCAAGTT 4250
CATACCATAA ACTCATTTGG AATTGAGTAT TATTTTGCTT TGAATGGAGC 4300
TATGTTTTGC AGTTCCCTCA GAAGAAAAGC TTGTTATAAA GCGTCTACAC 4350
CCATCAAAAG ATATATTTAA ATATTCCAAC TACAGAAAGA TTTTGTCTGC 4400
TCTTCACTCT GATCTCAGTT GGTTTCTTCA CGTACATGCT TCTTTATTTG 4450
CCTATTTTGT CAAGAAAATA ATAGGTCAAG TCCTGTTCTC ACTTATCTCC 4500
TGCCTAGCAT GGCTTAGATG CACGTTGTAC ATTCAAGAAG GATCAAATGA 4550
AACAGACTTC TGGTCTGTTA CAACAACCAT AGTAATAAAC AGACTAACTA 4600
ATAATTGCTA ATTATGTTTT CCATCTCTAA GGTTCCCACA TTTTTCTGTT 4650
TTAAGATCCC ATTATCTGGT TGTAACTGAA GCTCAATGGA ACATGAACAG 4700
TATTTCTCAG TCTTTTCTCC AGCAATCCTG ACGGATTAGA AGAACTGGCA 4750
GAAAACACTT TGTTACCCAG AATTAAAAAC TAATATTTGC TCTCCCTTCA 4800
ATCCAAAATG GACCTATTGA AACTAAAATC TGACCCAATC CCATTAAATT 4850
ATTTCTATGG CGTCAAAGGT CAAACTTTTG AAGGGAACCT GTGGGTGGGT 4900
CCCAATTCAG GCTATATATT CCCCAGGGCT CAGCGGATCT CCATGGGCTC 4950
CTCGTGCAGC AAGCATGGAA TTTTGCCTTG ATGTATTCAA GGAGCTCAAA 5000
GTCCACCATG CCAATGACAA CATGCTCTAC TCCCCCTTTG CCATCTGTCA 5050

ACTCTGGCCA TGGTCTCCCT GGGTGCAAAA GACAGCACCA GGGAATTCGT 5100
GCGCTCCTCC AAGAACGTCA TCAAGGAGTT CATGCGCTTC AAGGTGCGCA 5150
TGGAGGGCAC CGTGAACGGC CACGAGTTCG AGATCGAGGG CGAGGGCGAG 5200
GGCCGCCCCT ACGAGGGCCA CAACACCGTG AAGCTGAAGG TGACCAAGGG 5250
CGGCCCCCTG CCCTTCGCCT GGGACATCCT GTCCCCCCAG TTCCAGTACG 5300
GCTCCAAGGT GTACGTGAAG CACCCCGCCG ACATCCCCGA CTACAAGAAG 5350
CTGTCCTTCC CCGAGGGCTT CAAGTGGGAG CGCGTGATGA ACTTCGAGGA 5400
CGGCGGCGTG GTGACCGTGA CCCAGGACTC CTCCCTGCAG GACGGCTGCT 5450
TCATCTACAA GGTGAAGTTC ATCGGCGTGA ACTTCCCCTC CGACGGCCCC 5500
GTAATGCAGA AGAAGACCAT GGGCTGGGAG GCCTCCACCG AGCGCCTGTA 5550
CCCCCGCGAC GGCGTGCTGA AGGGCGAGAT CCACAAGGCC CTGAAGCTGA 5600
AGGACGGCGG CCACTACCTG GTGGAGTTCA AGTCCATCTA CATGGCCAAG 5650
AAGCCCGTGC AGCTGCCCGG CTACTACTAC GTGGACTCCA AGCTGGACAT 5700
CACCTCCCAC AACGAGGACT ACACCATCGT GGAGCAGTAC GAGCGCACCG 5750
AGGGCCGCCA CCACCTGTTC CTGTAGCGGC CGCGACTCTA GATCATAATC 5800
AGCCATACCA CATTTGTAGA GGTTTTACTT GCTTTAAAAA ACCTCCCACA 5850
CCTCCCCCTG AACCTGAAAC ATAAAATGAA TGCAATTGTT GTTGTTAACT 5900
TGTTTATTGC AGCTTATAAT GGTTACAAAT AAAGCAATAG CATCACAAAT 5950
TTCACAAATA AAGCATTTTT TTCACTGCAT TCTAGTTGTG GCTCGAGAAG 6000
GGCGAATTCT GCAGATATCC ATCACACTGG CGGCCGCTCG AGGGGGGGCC 6050
CGGTACCCAA TTCGCCCTAT AGTGAGTCGT ATTACGCGCG CTCACTGGCC 6100
GTCGTTTTAC AACGTCGTGA CTGGGAAAAC CCTGGCGTTA CCCAACTTAA 6150
TCGCCTTGCA GCACATCCCC CTTTCGCCAG CTGGCGTAAT AGCGAAGAGG 6200
CCCGCACCGA TCGCCCTTCC CAACAGTTGC GCAGCCTGAA TGGCGAATGG 6250
AAATTGTAAG CGTTAATATT TTGTTAAAAT TCGCGTTAAA TTTTTGTTAA 6300
ATCAGCTCAT TTTTTAACCA ATAGGCCGAA ATCGGCAAAA TCCCTTATAA 6350
ATCAAAAGAA TAGACCGAGA TAGGGTTGAG TGTTGTTCCA GTTTGGAACA 6400
AGAGTCCACT ATTAAAGAAC GTGGACTCCA ACGTCAAAGG GCGAAAAACC 6450
GTCTATCAGG GCGATGGCCC ACTACTCCGG GATCATATGA CAAGATGTGT 6500
ATCCACCTTA ACTTAATGAT TTTTACCAAA ATCATTAGGG GATTCATCAG 6550
TGCTCAGGGT CAACGAGAAT TAACATTCCG TCAGGAAAGC TTATGATGAT 6600
GATGTGCTTA AAAACTTACT CAATGGCTGG TTATGCATAT CGCAATACAT 6650
GCGAAAAACC TAAAAGAGCT TGCCGATAAA AAAGGCCAAT TTATTGCTAT 6700
TTACCGCGGC TTTTTATTGA GCTTGAAAGA TAAATAAAAT AGATAGGTTT 6750
TATTTGAAGC TAAATCTTCT TTATCGTAAA AAATGCCCTC TTGGGTTATC 6800
AAGAGGGTCA TTATATTTCG CGGAATAACA TCATTTGGTG ACGAAATAAC 6850
TAAGCACTTG TCTCCTGTTT ACTCCCCTGA GCTTGAGGGG TTAACATGAA 6900
GGTCATCGAT AGCAGGATAA TAATACAGTA AAACGCTAAA CCAATAATCC 6950
AAATCCAGCC ATCCCAAATT GGTAGTGAAT GATTATAAAT AACAGCAAAC 7000
AGTAATGGGC CAATAACACC GGTTGCATTG GTAAGGCTCA CCAATAATCC 7050
CTGTAAAGCA CCTTGCTGAT GACTCTTTGT TTGGATAGAC ATCACTCCCT 7100
GTAATGCAGG TAAAGCGATC CCACCACCAG CCAATAAAAT TAAAACAGGG 7150
AAAACTAACC AACCTTCAGA TATAAACGCT AAAAAGGCAA ATGCACTACT 7200
ATCTGCAATA AATCCGAGCA GTACTGCCGT TTTTTCGCCC CATTTAGTGG 7250
CTATTCTTCC TGCCACAAAG GCTTGGAATA CTGAGTGTAA AAGACCAAGA 7300
CCCGCTAATG AAAAGCCAAC CATCATGCTA TTCCATCCAA AACGATTTTC 7350
GGTAAATAGC ACCCACACCG TTGCGGGAAT TTGGCCTATC AATTGCGCTG 7400
AAAAATAAAT AATCAACAAA ATGGCATCGT TTTAAATAAA GTGATGTATA 7450
CCGAATTCAG CTTTTGTTCC CTTTAGTGAG GGTTAATTGC GCGCTTGGCG 7500
TAATCATGGT CATAGCTGTT TCCTGTGTGA AATTGTTATC CGCTCACAAT 7550
TCCACACAAC ATACGAGCCG GAAGCATAAA GTGTAAAGCC TGGGGTGCCT 7600
AATGAGTGAG CTAACTCACA TTAATTGCGT TGCGCTCACT GCCCGCTTTC 7650
CAGTCGGGAA ACCTGTCGTG CCAGCTGCAT TAATGAATCG GCCAACGCGC 7700
GGGGAGAGGC GGTTTGCGTA TTGGGCGCTC TTCCGCTTCC TCGCTCACTG 7750
ACTCGCTGCG CTCGGTCGTT CGGCTGCGGC GAGCGGTATC AGCTCACTCA 7800
AAGGCGGTAA TACGGTTATC CACAGAATCA GGGGATAACG CAGGAAAGAA 7850
CATGTGAGCA AAAGGCCAGC AAAAGGCCAG GAACCGTAAA AAGGCCGCGT 7900
TGCTGGCGTT TTTCCATAGG CTCCGCCCCC CTGACGAGCA TCACAAAAAT 7950
CGACGCTCAA GTCAGAGGTG GCGAAACCCG ACAGGACTAT AAAGATACCA 8000
GGCGTTTCCC CCTGGAAGCT CCCTCGTGCG CTCTCCTGTT CCGACCCTGC 8050
CGCTTACCGG ATACCTGTCC GCCTTTCTCC CTTCGGGAAG CGTGGCGCTT 8100

TCTCATAGCT CACGCTGTAG GTATCTCAGT TCGGTGTAGG TCGTTCGCTC 8150
CAAGCTGGGC TGTGTGCACG AACCCCCCGT TCAGCCCGAC CGCTGCGCCT 8200
TATCCGGTAA CTATCGTCTT GAGTCCAACC CGGTAAGACA CGACTTATCG 8250
CCACTGGCAG CAGCCACTGG TAACAGGATT AGCAGAGCGA GGTATGTAGG 8300
CGGTGCTACA GAGTTCTTGA AGTGGTGGCC TAACTACGGC TACACTAGAA 8350
GGACAGTATT TGGTATCTGC GCTCTGCTGA AGCCAGTTAC CTTCGGAAAA 8400
AGAGTTGGTA GCTCTTGATC CGGCAAACAA ACCACCGCTG GTAGCGGTGG 8450
TTTTTTTGTT TGCAAGCAGC AGATTACGCG CAGAAAAAAA GGATCTCAAG 8500
AAGATCCTTT GATCTTTTCT ACGGGGTCTG ACGCTCAGTG GAACGAAAAC 8550
TCACGTTAAG GGATTTTGGT CATGAGATTA TCAAAAAGGA TCTTCACCTA 8600
GATCCTTTTA AATTAAAAAT GAAGTTTTAA ATCAATCTAA AGTATATATG 8650
AGTAAACTTG GTCTGACAGT TACCAATGCT TAATCAGTGA GGCACCTATC 8700
TCAGCGATCT GTCTATTTCG TTCATCCATA GTTGCCTGAC TCCCCGTCGT 8750
GTAGATAACT ACGATACGGG AGGGCTTACC ATCTGGCCCC AGTGCTGCAA 8800
TGATACCGCG AGACCCACGC TCACCGGCTC CAGATTTATC AGCAATAAAC 8850
CAGCCAGCCG GAAGGGCCGA GCGCAGAAGT GGTCCTGCAA CTTTATCCGC 8900
CTCCATCCAG TCTATTAATT GTTGCCGGGA AGCTAGAGTA AGTAGTTCGC 8950
CAGTTAATAG TTTGCGCAAC GTTGTTGCCA TTGCTACAGG CATCGTGGTG 9000
TCACGCTCGT CGTTTGGTAT GGCTTCATTC AGCTCCGGTT CCCAACGATC 9050
AAGGCGAGTT ACATGATCCC CCATGTTGTG CAAAAAAGCG GTTAGCTCCT 9100
TCGGTCCTCC GATCGTTGTC AGAAGTAAGT TGGCCGCAGT GTTATCACTC 9150
ATGGTTATGG CAGCACTGCA TAATTCTCTT ACTGTCATGC CATCCGTAAG 9200
ATGCTTTTCT GTGACTGGTG AGTACTCAAC CAAGTCATTC TGAGAATAGT 9250
GTATGCGGCG ACCGAGTTGC TCTTGCCCGG CGTCAATACG GGATAATACC 9300
GCGCCACATA GCAGAACTTT AAAAGTGCTC ATCATTGGAA AACGTTCTTC 9350
GGGGCGAAAA CTCTCAAGGA TCTTACCGCT GTTGAGATCC AGTTCGATGT 9400
AACCCACTCG TGCACCCAAC TGATCTTCAG CATCTTTTAC TTTCACCAGC 9450
GTTTCTGGGT GAGCAAAAAC AGGAAGGCAA AATGCCGCAA AAAAGGGAAT 9500
AAGGGCGACA CGGAAATGTT GAATACTCAT ACTCTTCCTT TTTCAATATT 9550
ATTGAAGCAT TTATCAGGGT TATTGTCTCA TGAGCGGATA CATATTTGAA 9600
TGTATTTAGA AAAATAAACA AATAGGGGTT CCGCGCACAT TTCCCCGAAA 9650
AGTGCCAC 9658

SEQ ID NO:5 (spacer)
   (GPGG)ₓ
SEQ ID NO:6 (spacer)
   GPGGGPGGGPGG
SEQ ID NO:7 (spacer)
   GGGGSGGGGSGGGGS
SEQ ID NO:8 (spacer)
   GGGGSGGGGSGGGGSGGGGS
SEQ ID NO:9 (enterokinase cleavage site)
   DDDDK
SEQ ID NO:10 (altered transposase Hef forward primer)
   ATCTCGAGACCATGTGTGAACTTGATATTTTACATGATTCTCTTTACC
SEQ ID NO:11 (altered transposase Her reverse primer)
   GATTGATCATTATCATAATTTCCCCAAAGCGTAACC
SEQ ID NO:12 (Xho I restriction site)
   CTCGAG
SEQ ID NO:13 (modified Kozak sequence)
   ACCATG
SEQ ID NO:14 (Bcl I restriction site)
   TGATCA
SEQ ID NO:15 (CMVf-NgoM IV primer)
   TTGCCGGCATCAGATTGGCTAT
SEQ ID NO:16 (Syn-polyAr-BstE II primer)
   AGAGGTCACCGGGTCAATTCTTCAGCACCTGGTA 
SEQ ID NO:17 (vitellogenin promoter)

TGAATGTGTT CTTGTGTTAT CAATATAAAT CACAGTTAGT GATGAAGTTG GCTGCAAGCC
TGCATCAGTT CAGCTACTTG GCTGCATTTT GTATTTGGTT CTGTAGGAAA TGCAAAAGGT
TCTAGGCTGA CCTGCACTTC TATCCCTCTT GCCTTACTGC TGAGAATCTC TGCAGGTTTT
AATTGTTCAC ATTTTGCTCC CATTTACTTT GGAAGATAAA ATATTTACAG AATGCTTATG
AAACCTTTGT TCATTTAAAA ATATTCCTGG TCAGCGTGAC CGGAGCTGAA AGAACACATT
GATCCCGTGA TTTCAATAAA TACATATGTT CCATATATTG TTTCTCAGTA GCCTCTTAAA
TCATGTGCGT TGGTGCACAT ATGAATACAT GAATAGCAAA GGTTTATCTG GATTACGCTC
TGGCCTGCAG GAATGGCCAT AAACCAAAGC TGAGGGAAGA GGGAGAGTAT AGTCAATGTA
GATTATACTG ATTGCTGATT GGGTTATTAT CAGCTAGATA ACAACTTGGG TCAGGTGCCA
GGTCAACATA ACCTGGGCAA AACCAGTCTC ATCTGTGGCA GGACCATGTA CCAGCAGCCA
GCCGTGACCC AATCTAGGAA AGCAAGTAGC ACATCAATTT TAAATTTATT GTAAATGCCG
TAGTAGAAGT GTTTTACTGT GATACATTGA AACTTCTGGT CAATCAGAAA AAGGTTTTTT
ATCAGAGATG CCAAGGTATT ATTTGATTTT CTTTATTCGC CGTGAAGAGA ATTTATGATT
GCAAAAAGAG GAGTGTTTAC ATAAACTGAT AAAAAACTTG AGGAATTCAG CAGAAAACAG
CCACGTGTTC CTGAACATTC TTCCATAAAA GTCTCACCAT GCCTGGCAGA GCCCTATTCA
CCTTCGCT
SEQ ID NO:18 (vitellogenin targeting sequence)
   ATGAGGGGGATCATACTGGCATTAGTGCTCACCCTTGTAGGCAGCCAGAAGTTTGACATTGGT
SEQ ID NO:19 (p146 protein)
   KYKKALKKLAKLL
SEQ ID NO:20 (p146 coding sequence)
   AAATACAAAAAAGCACTGAAAAAACTGGCAAAACTGCTG 
SEQ ID NO:21 (pro-insulin sequence)
TTTGTGAACCAACACCTGTGCGGCTCACACCTGGTGGAAGCTCTCTACCTAGTGTGCGGGGAACGAGGC
TTCTTCTACACACCCAAGACCCGCCGGGAGGCAGAGGACCTGCAGGTGGGGCAGGTGGAGCTGGGCGGG
GGCCCTGGTGCAGGCAGCCTGCAGCCCTTGGCCCTGGAGGGGTCCCTGCAGAAGCGTGGCATTGTGGAA
CAATGCTGTACCAGCATCTGCTCCCTCTACCAGCTGGAGAACTCTGCAACTAG
 SEQ ID NO:22 (TAG sequence) 
Pro Ala Asp Asp Ala Pro Ala Asp Asp Ala Pro Ala Asp Asp Ala Pro Ala Asp Asp
Ala Pro Ala Asp Asp Ala Pro Ala Asp Asp Ala Thr Thr Cys Ile Leu Lys Gly Ser Cys
Gly Trp Ile Gly Leu Leu Asp Asp Asp Asp Lys
SEQ ID NO:23 (gp41 epitope)
   Ala Thr Thr Cys Ile Leu Lys Gly Ser Cys Gly Trp Ile Gly Leu Leu 
SEQ ID NO:24 (polynucleotide sequence encoding gp41 epitope)
Pro Ala Asp Asp Ala Pro Ala Asp Asp Ala Thr Thr Cys Ile Leu Lys Gly
Ser Cys Gly Trp Ile Gly Leu Leu Asp Asp Asp Asp Lys
SEQ ID NO:25 (repeat domain in TAG spacer sequence)
   Pro Ala Asp Asp Ala 
SEQ ID NO:26 (TAG spacer sequence)
Pro Ala Asp Asp Ala Pro Ala Asp Asp Ala Pro Ala Asp Asp Ala Pro Ala Asp Asp
Ala Pro Ala Asp Asp Ala Pro Ala Asp Asp
 
SEQ ID NO:27 (Vit pro/Vit targ/TAG/pro-insulin/synthetic polyA)
TGAATGTGTT CTTGTGTTAT CAATATAAAT CACAGTTAGT GATGAAGTTG GCTGCAAGCC
TGCATCAGTT CAGCTACTTG GCTGCATTTT GTATTTGGTT CTGTAGGAAA TGCAAAAGGT
TCTAGGCTGA CCTGCACTTC TATCCCTCTT GCCTTACTGC TGAGAATCTC TGCAGGTTTT
AATTGTTCAC ATTTTGCTCC CATTTACTTT GGAAGATAAA ATATTTACAG AATGCTTATG
AAACCTTTGT TCATTTAAAA ATATTCCTGG TCAGCGTGAC CGGAGCTGAA AGAACACATT
GATCCCGTGA TTTCAATAAA TACATATGTT CCATATATTG TTTCTCAGTA GCCTCTTAAA
TCATGTGCGT TGGTGCACAT ATGAATACAT GAATAGCAAA GGTTTATCTG GATTACGCTC
TGGCCTGCAG GAATGGCCAT AAACCAAAGC TGAGGGAAGA GGGAGAGTAT AGTCAATGTA
GATTATACTG ATTGCTGATT GGGTTATTAT CAGCTAGATA ACAACTTGGG TCAGGTGCCA
GGTCAACATA ACCTGGGCAA AACCAGTCTC ATCTGTGGCA GGACCATGTA CCAGCAGCCA
GCCGTGACCC AATCTAGGAA AGCAAGTAGC ACATCAATTT TAAATTTATT GTAAATGCCG
TAGTAGAAGT GTTTTACTGT GATACATTGA AACTTCTGGT CAATCAGAAA AAGGTTTTTT
ATCAGAGATG CCAAGGTATT ATTTGATTTT CTTTATTCGC CGTGAAGAGA ATTTATGATT
GCAAAAAGAG GAGTGTTTAC ATAAACTGAT AAAAAACTTG AGGAATTCAG CAGAAAACAG
CCACGTGTTC CTGAACATTC TTCCATAAAA GTCTCACCAT GCCTGGCAGA GCCCTATTCA
CCTTCGCTAT GAGGGGGATC ATACTGGCAT TAGTGCTCAC CCTTGTAGGC AGCCAGAAGT
TTGACATTGG TAGACTGAGA ATGGCAAGAA GAATGAGAAGA TGGTTTGTG AACCAACACC
TGTGCGGCTCA CACCTGGTGG AAGCTCTCTA CCTAGTGTGCG GGGAACGAGG CTTCTTCTAC
ACACCCAAGA CCCGCCGGGA GGCAGAGGAC CTGCAGGTGGG GCAGGTGGAG CTGGGCGGGG
GCCCTGGTGC AGGCAGCCTG CAGCCCTTGG CCCTGGAGGGG TCCCTGCAGA AGCGTGGCAT
TGTGGAACAA TGCTGTACCA GCATCTGCTC CCTCTACCAGC TGGAGAACTA CTGCAACTAG
GGCGCCTGGATCCAGATCACTTCTGGCTAATAAAAGATCAGAGCTCTAGAGATCTGTGTGTTGGTTTTT
TGTGGATCTGCTGTGCCTTCTAGTTGCCAGCCATCTGTTGTTTGCCCCTCCCCCGTGCCTTCCTTGACC
CTGGAAGGTGCCACTCCCACTGTCCTTTCCTAATAAAATGAGGAAATTGCATCGCATTGTCTGAGTAGG
TGTCATTCTATTCTGGGGGGTGGGGTGGGGCAGCACAGCAAGGGGGAGGATTGGGAAGACAATAGCAGG
CATGCTGGGGATGCGGTGGGCTCTATGGGTACCTCTCTCTCTCTCTCTCTCTCTCTCTCTCTCTCTCTC
TCTCGGTACCTCTCTC
 
SEQ ID NO:28 (synthetic polyA sequence)
GGCGCCTGGATCCAGATCACTTCTGGCTAATAAAAGATCAGAGCTCTAGAGATCTGTGTGTTGGTTTTT
TGTGGATCTGCTGTGCCTTCTAGTTGCCAGCCATCTGTTGTTTGCCCCTCCCCCGTGCCTTCCTTGACC
CTGGAAGGTGCCACTCCCACTGTCCTTTCCTAATAAAATGAGGAAATTGCATCGCATTGTCTGAGTAGG
TGTCATTCTATTCTGGGGGGTGGGGTGGGGCAGCACAGCAAGGGGGAGGATTGGGAAGACAATAGCAGG
CATGCTGGGGATGCGGTGGGCTCTATGGGTACCTCTCTCTCTCTCTCTCTCTCTCTCTCTCTCTCTCTC
TCTCGGTACCTCTCTC
 
SEQ ID NO:29 (pTnMod(Oval/ENT tag/P146/PA)- Chicken)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
 
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGG 4050
AGGTCAGAAT GGTTTCTTTA CTGTTTGTCA ATTCTATTAT TTCAATACAG 4100
AACAATAGCT TCTATAACTG AAATATATTT GCTATTGTAT ATTATGATTG 4150
TCCCTCGAAC CATGAACACT CCTCCAGCTG AATTTCACAA TTCCTCTGTC 4200
ATCTGCCAGG CCATTAAGTT ATTCATGGAA GATCTTTGAG GAACACTGCA 4250
AGTTCATATC ATAAACACAT TTGAAATTGA GTATTGTTTT GCATTGTATG 4300
GAGCTATGTT TTGCTGTATC CTCAGAAAAA AAGTTTGTTA TAAAGCATTC 4350
ACACCCATAA AAAGATAGAT TTAAATATTC CAGCTATAGG AAAGAAAGTG 4400
CGTCTGCTCT TCACTCTAGT CTCAGTTGGC TCCTTCACAT GCATGCTTCT 4450
TTATTTCTCC TATTTTGTCA AGAAAATAAT AGGTCACGTC TTGTTCTCAC 4500
TTATGTCCTG CCTAGCATGG CTCAGATGCA CGTTGTAGAT ACAAGAAGGA 4550
TCAAATGAAA CAGACTTCTG GTCTGTTACT ACAACCATAG TAATAAGCAC 4600
ACTAACTAAT AATTGCTAAT TATGTTTTCC ATCTCTAAGG TTCCCACATT 4650
TTTCTGTTTT CTTAAAGATC CCATTATCTG GTTGTAACTG AAGCTCAATG 4700
GAACATGAGC AATATTTCCC AGTCTTCTCT CCCATCCAAC AGTCCTGATG 4750
GATTAGCAGA ACAGGCAGAA AACACATTGT TACCCAGAAT TAAAAACTAA 4800
TATTTGCTCT CCATTCAATC CAAAATGGAC CTATTGAAAC TAAAATCTAA 4850
CCCAATCCCA TTAAATGATT TCTATGGCGT CAAAGGTCAA ACTTCTGAAG 4900
GGAACCTGTG GGTGGGTCAC AATTCAGGCT ATATATTCCC CAGGGCTCAG 4950
CGGATCCATG GGCTCCATCG GCGCAGCAAG CATGGAATTT TGTTTTGATG 5000
TATTCAAGGA GCTCAAAGTC CACCATGCCA ATGAGAACAT CTTCTACTGC 5050
CCCATTGCCA TCATGTCAGC TCTAGCCATG GTATACCTGG GTGCAAAAGA 5100
CAGCACCAGG ACACAGATAA ATAAGGTTGT TCGCTTTGAT AAACTTCCAG 5150
GATTCGGAGA CAGTATTGAA GCTCAGTGTG GCACATCTGT AAACGTTCAC 5200
TCTTCACTTA GAGACATCCT CAACCAAATC ACCAAACCAA ATGATGTTTA 5250
TTCGTTCAGC CTTGCCAGTA GACTTTATGC TGAAGAGAGA TACCCAATCC 5300
TGCCAGAATA CTTGCAGTGT GTGAAGGAAC TGTATAGAGG AGGCTTGGAA 5350
CCTATCAACT TTCAAACAGC TGCAGATCAA GCCAGAGAGC TCATCAATTC 5400
CTGGGTAGAA AGTCAGACAA ATGGAATTAT CAGAAATGTC CTTCAGCCAA 5450
GCTCCGTGGA TTCTCAAACT GCAATGGTTC TGGTTAATGC CATTGTCTTC 5500
AAAGGACTGT GGGAGAAAAC ATTTAAGGAT GAAGACACAC AAGCAATGCC 5550
TTTCAGAGTG ACTGAGCAAG AAAGCAAACC TGTGCAGATG ATGTACCAGA 5600
TTGGTTTATT TAGAGTGGCA TCAATGGCTT CTGAGAAAAT GAAGATCCTG 5650
GAGCTTCCAT TTGCCAGTGG GACAATGAGC ATGTTGGTGC TGTTGCCTGA 5700
TGAAGTCTCA GGCCTTGAGC AGCTTGAGAG TATAATCAAC TTTGAAAAAC 5750
TGACTGAATG GACCAGTTCT AATGTTATGG AAGAGAGGAA GATCAAAGTG 5800
TACTTACCTC GCATGAAGAT GGAGGAAAAA TACAACCTCA CATCTGTCTT 5850
AATGGCTATG GGCATTACTG ACGTGTTTAG CTCTTCAGCC AATCTGTCTG 5900
GCATCTCCTC AGCAGAGAGC CTGAAGATAT CTCAAGCTGT CCATGCAGCA 5950
 
CATGCAGAAA TCAATGAAGC AGGCAGAGAG GTGGTAGGGT CAGCAGAGGC 6000
TGGAGTGGAT GCTGCAAGCG TCTCTGAAGA ATTTAGGGCT GACCATCCAT 6050
TCCTCTTCTG TATCAAGCAC ATCGCAACCA ACGCCGTTCT CTTCTTTGGC 6100
AGATGTGTTT CCCCTCCGCG GCCAGCAGAT GACGCACCAG CAGATGACGC 6150
ACCAGCAGAT GACGCACCAG CAGATGACGC ACCAGCAGAT GACGCACCAG 6200
CAGATGACGC AACAACATGT ATCCTGAAAG GCTCTTGTGG CTGGATCGGC 6250
CTGCTGGATG ACGATGACAA AAAATACAAA AAAGCACTGA AAAAACTGGC 6300
AAAACTGCTG TAATGAGGGC GCCTGGATCC AGATCACTTC TGGCTAATAA 6350
AAGATCAGAG CTCTAGAGAT CTGTGTGTTG GTTTTTTGTG GATCTGCTGT 6400
GCCTTCTAGT TGCCAGCCAT CTGTTGTTTG CCCCTCCCCC GTGCCTTCCT 6450
TGACCCTGGA AGGTGCCACT CCCACTGTCC TTTCCTAATA AAATGAGGAA 6500
ATTGCATCGC ATTGTCTGAG TAGGTGTCAT TCTATTCTGG GGGGTGGGGT 6550
GGGGCAGCAC AGCAAGGGGG AGGATTGGGA AGACAATAGC AGGCATGCTG 6600
GGGATGCGGT GGGCTCTATG GGTACCTCTC TCTCTCTCTC TCTCTCTCTC 6650
TCTCTCTCTC TCTCTCGGTA CCTCTCTCGA GGGGGGGCCC GGTACCCAAT 6700
TCGCCCTATA GTGAGTCGTA TTACGCGCGC TCACTGGCCG TCGTTTTACA 6750
ACGTCGTGAC TGGGAAAACC CTGGCGTTAC CCAACTTAAT CGCCTTGCAG 6800
CACATCCCCC TTTCGCCAGC TGGCGTAATA GCGAAGAGGC CCGCACCGAT 6850
CGCCCTTCCC AACAGTTGCG CAGCCTGAAT GGCGAATGGA AATTGTAAGC 6900
GTTAATATTT TGTTAAAATT CGCGTTAAAT TTTTGTTAAA TCAGCTCATT 6950
TTTTAACCAA TAGGCCGAAA TCGGCAAAAT CCCTTATAAA TCAAAAGAAT 7000
AGACCGAGAT AGGGTTGAGT GTTGTTCCAG TTTGGAACAA GAGTCCACTA 7050
TTAAAGAACG TGGACTCCAA CGTCAAAGGG CGAAAAACCG TCTATCAGGG 7100
CGATGGCCCA CTACTCCGGG ATCATATGAC AAGATGTGTA TCCACCTTAA 7150
CTTAATGATT TTTACCAAAA TCATTAGGGG ATTCATCAGT GCTCAGGGTC 7200
AACGAGAATT AACATTCCGT CAGGAAAGCT TATGATGATG ATGTGCTTAA 7250
AAACTTACTC AATGGCTGGT TATGCATATC GCAATACATG CGAAAAACCT 7300
AAAAGAGCTT GCCGATAAAA AAGGCCAATT TATTGCTATT TACCGCGGCT 7350
TTTTATTGAG CTTGAAAGAT AAATAAAATA GATAGGTTTT ATTTGAAGCT 7400
AAATCTTCTT TATCGTAAAA AATGCCCTCT TGGGTTATCA AGAGGGTCAT 7450
TATATTTCGC GGAATAACAT CATTTGGTGA CGAAATAACT AAGCACTTGT 7500
CTCCTGTTTA CTCCCCTGAG CTTGAGGGGT TAACATGAAG GTCATCGATA 7550
GCAGGATAAT AATACAGTAA AACGCTAAAC CAATAATCCA AATCCAGCCA 7600
TCCCAAATTG GTAGTGAATG ATTATAAATA ACAGCAAACA GTAATGGGCC 7650
AATAACACCG GTTGCATTGG TAAGGCTCAC CAATAATCCC TGTAAAGCAC 7700
CTTGCTGATG ACTCTTTGTT TGGATAGACA TCACTCCCTG TAATGCAGGT 7750
AAAGCGATCC CACCACCAGC CAATAAAATT AAAACAGGGA AAACTAACCA 7800
ACCTTCAGAT ATAAACGCTA AAAAGGCAAA TGCACTACTA TCTGCAATAA 7850
ATCCGAGCAG TACTGCCGTT TTTTCGCCCC ATTTAGTGGC TATTCTTCCT 7900
GCCACAAAGG CTTGGAATAC TGAGTGTAAA AGACCAAGAC CCGCTAATGA 7950
AAAGCCAACC ATCATGCTAT TCCATCCAAA ACGATTTTCG GTAAATAGCA 8000
CCCACACCGT TGCGGGAATT TGGCCTATCA ATTGCGCTGA AAAATAAATA 8050
ATCAACAAAA TGGCATCGTT TTAAATAAAG TGATGTATAC CGAATTCAGC 8100
TTTTGTTCCC TTTAGTGAGG GTTAATTGCG CGCTTGGCGT AATCATGGTC 8150
ATAGCTGTTT CCTGTGTGAA ATTGTTATCC GCTCACAATT CCACACAACA 8200
TACGAGCCGG AAGCATAAAG TGTAAAGCCT GGGGTGCCTA ATGAGTGAGC 8250
TAACTCACAT TAATTGCGTT GCGCTCACTG CCCGCTTTCC AGTCGGGAAA 8300
CCTGTCGTGC CAGCTGCATT AATGAATCGG CCAACGCGCG GGGAGAGGCG 8350
GTTTGCGTAT TGGGCGCTCT TCCGCTTCCT CGCTCACTGA CTCGCTGCGC 8400
TCGGTCGTTC GGCTGCGGCG AGCGGTATCA GCTCACTCAA AGGCGGTAAT 8450
ACGGTTATCC ACAGAATCAG GGGATAACGC AGGAAAGAAC ATGTGAGCAA 8500
AAGGCCAGCA AAAGGCCAGG AACCGTAAAA AGGCCGCGTT GCTGGCGTTT 8550
TTCCATAGGC TCCGCCCCCC TGACGAGCAT CACAAAAATC GACGCTCAAG 8600
TCAGAGGTGG CGAAACCCGA CAGGACTATA AAGATACCAG GCGTTTCCCC 8650
CTGGAAGCTC CCTCGTGCGC TCTCCTGTTC CGACCCTGCC GCTTACCGGA 8700
TACCTGTCCG CCTTTCTCCC TTCGGGAAGC GTGGCGCTTT CTCATAGCTC 8750
ACGCTGTAGG TATCTCAGTT CGGTGTAGGT CGTTCGCTCC AAGCTGGGCT 8800
GTGTGCACGA ACCCCCCGTT CAGCCCGACC GCTGCGCCTT ATCCGGTAAC 8850
TATCGTCTTG AGTCCAACCC GGTAAGACAC GACTTATCGC CACTGGCAGC 8900
AGCCACTGGT AACAGGATTA GCAGAGCGAG GTATGTAGGC GGTGCTACAG 8950
AGTTCTTGAA GTGGTGGCCT AACTACGGCT ACACTAGAAG GACAGTATTT 9000
 
GGTATCTGCG CTCTGCTGAA GCCAGTTACC TTCGGAAAAA GAGTTGGTAG 9050
CTCTTGATCC GGCAAACAAA CCACCGCTGG TAGCGGTGGT TTTTTTGTTT 9100
GCAAGCAGCA GATTACGCGC AGAAAAAAAG GATCTCAAGA AGATCCTTTG 9150
ATCTTTTCTA CGGGGTCTGA CGCTCAGTGG AACGAAAACT CACGTTAAGG 9200
GATTTTGGTC ATGAGATTAT CAAAAAGGAT CTTCACCTAG ATCCTTTTAA 9250
ATTAAAAATG AAGTTTTAAA TCAATCTAAA GTATATATGA GTAAACTTGG 9300
TCTGACAGTT ACCAATGCTT AATCAGTGAG GCACCTATCT CAGCGATCTG 9350
TCTATTTCGT TCATCCATAG TTGCCTGACT CCCCGTCGTG TAGATAACTA 9400
CGATACGGGA GGGCTTACCA TCTGGCCCCA GTGCTGCAAT GATACCGCGA 9450
GACCCACGCT CACCGGCTCC AGATTTATCA GCAATAAACC AGCCAGCCGG 9500
AAGGGCCGAG CGCAGAAGTG GTCCTGCAAC TTTATCCGCC TCCATCCAGT 9550
CTATTAATTG TTGCCGGGAA GCTAGAGTAA GTAGTTCGCC AGTTAATAGT 9600
TTGCGCAACG TTGTTGCCAT TGCTACAGGC ATCGTGGTGT CACGCTCGTC 9650
GTTTGGTATG GCTTCATTCA GCTCCGGTTC CCAACGATCA AGGCGAGTTA 9700
CATGATCCCC CATGTTGTGC AAAAAAGCGG TTAGCTCCTT CGGTCCTCCG 9750
ATCGTTGTCA GAAGTAAGTT GGCCGCAGTG TTATCACTCA TGGTTATGGC 9800
AGCACTGCAT AATTCTCTTA CTGTCATGCC ATCCGTAAGA TGCTTTTCTG 9850
TGACTGGTGA GTACTCAACC AAGTCATTCT GAGAATAGTG TATGCGGCGA 9900
CCGAGTTGCT CTTGCCCGGC GTCAATACGG GATAATACCG CGCCACATAG 9950
CAGAACTTTA AAAGTGCTCA TCATTGGAAA ACGTTCTTCG GGGCGAAAAC 10000
TCTCAAGGAT CTTACCGCTG TTGAGATCCA GTTCGATGTA ACCCACTCGT 10050
GCACCCAACT GATCTTCAGC ATCTTTTACT TTCACCAGCG TTTCTGGGTG 10100
AGCAAAAACA GGAAGGCAAA ATGCCGCAAA AAAGGGAATA AGGGCGACAC 10150
GGAAATGTTG AATACTCATA CTCTTCCTTT TTCAATATTA TTGAAGCATT 10200
TATCAGGGTT ATTGTCTCAT GAGCGGATAC ATATTTGAAT GTATTTAGAA 10250
AAATAAACAA ATAGGGGTTC CGCGCACATT TCCCCGAAAA GTGCCAC 10297

SEQ ID NO:30 (pTnMod(Oval/ENT tag/P146/PA)- QUAIL)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
 
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGG 4050
AGGTCAGAAT GGTTTCTTTA CTGTTTGTCA ATTCTATTAT TTCAATACAG 4100
AACAAAAGCT TCTATAACTG AAATATATTT GCTATTGTAT ATTATGATTG 4150
TCCCTCGAAC CATGAACACT CCTCCAGCTG AATTTCACAA TTCCTCTGTC 4200
ATCTGCCAGG CTGGAAGATC ATGGAAGATC TCTGAGGAAC ATTGCAAGTT 4250
CATACCATAA ACTCATTTGG AATTGAGTAT TATTTTGCTT TGAATGGAGC 4300
TATGTTTTGC AGTTCCCTCA GAAGAAAAGC TTGTTATAAA GCGTCTACAC 4350
CCATCAAAAG ATATATTTAA ATATTCCAAC TACAGAAAGA TTTTGTCTGC 4400
TCTTCACTCT GATCTCAGTT GGTTTCTTCA CGTACATGCT TCTTTATTTG 4450
CCTATTTTGT CAAGAAAATA ATAGGTCAAG TCCTGTTCTC ACTTATCTCC 4500
TGCCTAGCAT GGCTTAGATG CACGTTGTAC ATTCAAGAAG GATCAAATGA 4550
AACAGACTTC TGGTCTGTTA CAACAACCAT AGTAATAAAC AGACTAACTA 4600
 
ATAATTGCTA ATTATGTTTT CCATCTCTAA GGTTCCCACA TTTTTCTGTT 4650
TTAAGATCCC ATTATCTGGT TGTAACTGAA GCTCAATGGA ACATGAACAG 4700
TATTTCTCAG TCTTTTCTCC AGCAATCCTG ACGGATTAGA AGAACTGGCA 4750
GAAAACACTT TGTTACCCAG AATTAAAAAC TAATATTTGC TCTCCCTTCA 4800
ATCCAAAATG GACCTATTGA AACTAAAATC TGACCCAATC CCATTAAATT 4850
ATTTCTATGG CGTCAAAGGT CAAACTTTTG AAGGGAACCT GTGGGTGGGT 4900
CCCAATTCAG GCTATATATT CCCCAGGGCT CAGCCAGTGG ATCCATGGGC 4950
TCCATCGGTG CAGCAAGCAT GGAATTTTGT TTTGATGTAT TCAAGGAGCT 5000
CAAAGTCCAC CATGCCAATG ACAACATGCT CTACTCCCCC TTTGCCATCT 5050
TGTCAACTCT GGCCATGGTC TTCCTAGGTG CAAAAGACAG CACCAGGACC 5100
CAGATAAATA AGGTTGTTCA CTTTGATAAA CTTCCAGGAT TCGGAGACAG 5150
TATTGAAGCT CAGTGTGGCA CATCTGTAAA TGTTCACTCT TCACTTAGAG 5200
ACATACTCAA CCAAATCACC AAACAAAATG ATGCTTATTC GTTCAGCCTT 5250
GCCAGTAGAC TTTATGCTCA AGAGACATAC ACAGTCGTGC CGGAATACTT 5300
GCAATGTGTG AAGGAACTGT ATAGAGGAGG CTTAGAATCC GTCAACTTTC 5350
AAACAGCTGC AGATCAAGCC AGAGGCCTCA TCAATGCCTG GGTAGAAAGT 5400
CAGACAAACG GAATTATCAG AAACATCCTT CAGCCAAGCT CCGTGGATTC 5450
TCAAACTGCA ATGGTCCTGG TTAATGCCAT TGCCTTCAAG GGACTGTGGG 5500
AGAAAGCATT TAAGGCTGAA GACACGCAAA CAATACCTTT CAGAGTGACT 5550
GAGCAAGAAA GCAAACCTGT GCAGATGATG TACCAGATTG GTTCATTTAA 5600
AGTGGCATCA ATGGCTTCTG AGAAAATGAA GATCCTGGAG CTTCCATTTG 5650
CCAGTGGAAC AATGAGCATG TTGGTGCTGT TGCCTGATGA TGTCTCAGGC 5700
CTTGAGCAGC TTGAGAGTAT AATCAGCTTT GAAAAACTGA CTGAATGGAC 5750
CAGTTCTAGT ATTATGGAAG AGAGGAAGGT CAAAGTGTAC TTACCTCGCA 5800
TGAAGATGGA GGAGAAATAC AACCTCACAT CTCTCTTAAT GGCTATGGGA 5850
ATTACTGACC TGTTCAGCTC TTCAGCCAAT CTGTCTGGCA TCTCCTCAGT 5900
AGGGAGCCTG AAGATATCTC AAGCTGTCCA TGCAGCACAT GCAGAAATCA 5950
ATGAAGCGGG CAGAGATGTG GTAGGCTCAG CAGAGGCTGG AGTGGATGCT 6000
ACTGAAGAAT TTAGGGCTGA CCATCCATTC CTCTTCTGTG TCAAGCACAT 6050
CGAAACCAAC GCCATTCTCC TCTTTGGCAG ATGTGTTTCT CCGCGGCCAG 6100
CAGATGACGC ACCAGCAGAT GACGCACCAG CAGATGACGC ACCAGCAGAT 6150
GACGCACCAG CAGATGACGC ACCAGCAGAT GACGCAACAA CATGTATCCT 6200
GAAAGGCTCT TGTGGCTGGA TCGGCCTGCT GGATGACGAT GACAAAAAAT 6250
ACAAAAAAGC ACTGAAAAAA CTGGCAAAAC TGCTGTAATG AGGGCGCCTG 6300
GATCCAGATC ACTTCTGGCT AATAAAAGAT CAGAGCTCTA GAGATCTGTG 6350
TGTTGGTTTT TTGTGGATCT GCTGTGCCTT CTAGTTGCCA GCCATCTGTT 6400
GTTTGCCCCT CCCCCGTGCC TTCCTTGACC CTGGAAGGTG CCACTCCCAC 6450
TGTCCTTTCC TAATAAAATG AGGAAATTGC ATCGCATTGT CTGAGTAGGT 6500
GTCATTCTAT TCTGGGGGGT GGGGTGGGGC AGCACAGCAA GGGGGAGGAT 6550
TGGGAAGACA ATAGCAGGCA TGCTGGGGAT GCGGTGGGCT CTATGGGTAC 6600
CTCTCTCTCT CTCTCTCTCT CTCTCTCTCT CTCTCTCTCT CGGTACCTCT 6650
CTCGAGGGGG GGCCCGGTAC CCAATTCGCC CTATAGTGAG TCGTATTACG 6700
CGCGCTCACT GGCCGTCGTT TTACAACGTC GTGACTGGGA AAACCCTGGC 6750
GTTACCCAAC TTAATCGCCT TGCAGCACAT CCCCCTtTCG CCAGCTGGCG 6800
TAATAGCGAA GAGGCCCGCA CCGATCGCCC TTCCCAACAG TTGCGCAGCC 6850
TGAATGGCGA ATGGAAATTG TAAGCGTTAA TATTTTGTTA AAATTCGCGT 6900
TAAATTTTTG TTAAATCAGC TCATTTTTTA ACCAATAGGC CGAAATCGGC 6950
AAAATCCCTT ATAAATCAAA AGAATAGACC GAGATAGGGT TGAGTGTTGT 7000
TCCAGTTTGG AACAAGAGTC CACTATTAAA GAACGTGGAC TCCAACGTCA 7050
AAGGGCGAAA AACCGTCTAT CAGGGCGATG GCCCACTACT CCGGGATCAT 7100
ATGACAAGAT GTGTATCCAC CTTAACTTAA TGATTTTTAC CAAAATCATT 7150
AGGGGATTCA TCAGTGCTCA GGGTCAACGA GAATTAACAT TCCGTCAGGA 7200
AAGCTTATGA TGATGATGTG CTTAAAAACT TACTCAATGG CTGGTTATGC 7250
ATATCGCAAT ACATGCGAAA AACCTAAAAG AGCTTGCCGA TAAAAAAGGC 7300
CAATTTATTG CTATTTACCG CGGCTTTTTA TTGAGCTTGA AAGATAAATA 7350
AAATAGATAG GTTTTATTTG AAGCTAAATC TTCTTTATCG TAAAAAATGC 7400
CCTCTTGGGT TATCAAGAGG GTCATTATAT TTCGCGGAAT AACATCATTT 7450
GGTGACGAAA TAACTAAGCA CTTGTCTCCT GTTTACTCCC CTGAGCTTGA 7500
GGGGTTAACA TGAAGGTCAT CGATAGCAGG ATAATAATAC AGTAAAACGC 7550
TAAACCAATA ATCCAAATCC AGCCATCCCA AATTGGTAGT GAATGATTAT 7600
AAATAACAGC AAACAGTAAT GGGCCAATAA CACCGGTTGC ATTGGTAAGG 7650
 
CTCACCAATA ATCCCTGTAA AGCACCTTGC TGATGACTCT TTGTTTGGAT 7700
AGACATCACT CCCTGTAATG CAGGTAAAGC GATCCCACCA CCAGCCAATA 7750
AAATTAAAAC AGGGAAAACT AACCAACCTT CAGATATAAA CGCTAAAAAG 7800
GCAAATGCAC TACTATCTGC AATAAATCCG AGCAGTACTG CCGTTTTTTC 7850
GCCCCATTTA GTGGCTATTC TTCCTGCCAC AAAGGCTTGG AATACTGAGT 7900
GTAAAAGACC AAGACCCGCT AATGAAAAGC CAACCATCAT GCTATTCCAT 7950
CCAAAACGAT TTTCGGTAAA TAGCACCCAC ACCGTTGCGG GAATTTGGCC 8000
TATCAATTGC GCTGAAAAAT AAATAATCAA CAAAATGGCA TCGTTTTAAA 8050
TAAAGTGATG TATACCGAAT TCAGCTTTTG TTCCCTTTAG TGAGGGTTAA 8100
TTGCGCGCTT GGCGTAATCA TGGTCATAGC TGTTTCCTGT GTGAAATTGT 8150
TATCCGCTCA CAATTCCACA CAACATACGA GCCGGAAGCA TAAAGTGTAA 8200
AGCCTGGGGT GCCTAATGAG TGAGCTAACT CACATTAATT GCGTTGCGCT 8250
CACTGCCCGC TTTCCAGTCG GGAAACCTGT CGTGCCAGCT GCATTAATGA 8300
ATCGGCCAAC GCGCGGGGAG AGGCGGTTTG CGTATTGGGC GCTCTTCCGC 8350
TTCCTCGCTC ACTGACTCGC TGCGCTCGGT CGTTCGGCTG CGGCGAGCGG 8400
TATCAGCTCA CTCAAAGGCG GTAATACGGT TATCCACAGA ATCAGGGGAT 8450
AACGCAGGAA AGAACATGTG AGCAAAAGGC CAGCAAAAGG CCAGGAACCG 8500
TAAAAAGGCC GCGTTGCTGG CGTTTTTCCA TAGGCTCCGC CCCCCTGACG 8550
AGCATCACAA AAATCGACGC TCAAGTCAGA GGTGGCGAAA CCCGACAGGA 8600
CTATAAAGAT ACCAGGCGTT TCCCCCTGGA AGCTCCCTCG TGCGCTCTCC 8650
TGTTCCGACC CTGCCGCTTA CCGGATACCT GTCCGCCTTT CTCCCTTCGG 8700
GAAGCGTGGC GCTTTCTCAT AGCTCACGCT GTAGGTATCT CAGTTCGGTG 8750
TAGGTCGTTC GCTCCAAGCT GGGCTGTGTG CACGAACCCC CCGTTCAGCC 8800
CGACCGCTGC GCCTTATCCG GTAACTATCG TCTTGAGTCC AACCCGGTAA 8850
GACACGACTT ATCGCCACTG GCAGCAGCCA CTGGTAACAG GATTAGCAGA 8900
GCGAGGTATG TAGGCGGTGC TACAGAGTTC TTGAAGTGGT GGCCTAACTA 8950
CGGCTACACT AGAAGGACAG TATTTGGTAT CTGCGCTCTG CTGAAGCCAG 9000
TTACCTTCGG AAAAAGAGTT GGTAGCTCTT GATCCGGCAA ACAAACCACC 9050
GCTGGTAGCG GTGGTTTTTT TGTTTGCAAG CAGCAGATTA CGCGCAGAAA 9100
AAAAGGATCT CAAGAAGATC CTTTGATCTT TTCTACGGGG TCTGACGCTC 9150
AGTGGAACGA AAACTCACGT TAAGGGATTT TGGTCATGAG ATTATCAAAA 9200
AGGATCTTCA CCTAGATCCT TTTAAATTAA AAATGAAGTT TTAAATCAAT 9250
CTAAAGTATA TATGAGTAAA CTTGGTCTGA CAGTTACCAA TGCTTAATCA 9300
GTGAGGCACC TATCTCAGCG ATCTGTCTAT TTCGTTCATC CATAGTTGCC 9350
TGACTCCCCG TCGTGTAGAT AACTACGATA CGGGAGGGCT TACCATCTGG 9400
CCCCAGTGCT GCAATGATAC CGCGAGACCC ACGCTCACCG GCTCCAGATT 9450
TATCAGCAAT AAACCAGCCA GCCGGAAGGG CCGAGCGCAG AAGTGGTCCT 9500
GCAACTTTAT CCGCCTCCAT CCAGTCTATT AATTGTTGCC GGGAAGCTAG 9550
AGTAAGTAGT TCGCCAGTTA ATAGTTTGCG CAACGTTGTT GCCATTGCTA 9600
CAGGCATCGT GGTGTCACGC TCGTCGTTTG GTATGGCTTC ATTCAGCTCC 9650
GGTTCCCAAC GATCAAGGCG AGTTACATGA TCCCCCATGT TGTGCAAAAA 9700
AGCGGTTAGC TCCTTCGGTC CTCCGATCGT TGTCAGAAGT AAGTTGGCCG 9750
CAGTGTTATC ACTCATGGTT ATGGCAGCAC TGCATAATTC TCTTACTGTC 9800
ATGCCATCCG TAAGATGCTT TTCTGTGACT GGTGAGTACT CAACCAAGTC 9850
ATTCTGAGAA TAGTGTATGC GGCGACCGAG TTGCTCTTGC CCGGCGTCAA 9900
TACGGGATAA TACCGCGCCA CATAGCAGAA CTTTAAAAGT GCTCATCATT 9950
GGAAAACGTT CTTCGGGGCG AAAACTCTCA AGGATCTTAC CGCTGTTGAG 10000
ATCCAGTTCG ATGTAACCCA CTCGTGCACC CAACTGATCT TCAGCATCTT 10050
TTACTTTCAC CAGCGTTTCT GGGTGAGCAA AAACAGGAAG GCAAAATGCC 10100
GCAAAAAAGG GAATAAGGGC GACACGGAAA TGTTGAATAC TCATACTCTT 10150
CCTTTTTCAA TATTATTGAA GCATTTATCA GGGTTATTGT CTCATGAGCG 10200
GATACATaTT TGAATGTATT TAGAAAAATA AACAAATAGG GGTTCCGCGC 10250
ACATTTCCCC GAAAAGTGCC AC 10272

SEQ ID NO:31 (pTnMod(Oval/ENT tag/Proins/PA)- Chicken)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
 
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
 
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGG 4050
AGGTCAGAAT GGTTTCTTTA CTGTTTGTCA ATTCTATTAT TTCAATACAG 4100
AACAATAGCT TCTATAACTG AAATATATTT GCTATTGTAT ATTATGATTG 4150
TCCCTCGAAC CATGAACACT CCTCCAGCTG AATTTCACAA TTCCTCTGTC 4200
ATCTGCCAGG CCATTAAGTT ATTCATGGAA GATCTTTGAG GAACACTGCA 4250
AGTTCATATC ATAAACACAT TTGAAATTGA GTATTGTTTT GCATTGTATG 4300
GAGCTATGTT TTGCTGTATC CTCAGAAAAA AAGTTTGTTA TAAAGCATTC 4350
ACACCCATAA AAAGATAGAT TTAAATATTC CAGCTATAGG AAAGAAAGTG 4400
CGTCTGCTCT TCACTCTAGT CTCAGTTGGC TCCTTCACAT GCATGCTTCT 4450
TTATTTCTCC TATTTTGTCA AGAAAATAAT AGGTCACGTC TTGTTCTCAC 4500
TTATGTCCTG CCTAGCATGG CTCAGATGCA CGTTGTAGAT ACAAGAAGGA 4550
TCAAATGAAA CAGACTTCTG GTCTGTTACT ACAACCATAG TAATAAGCAC 4600
ACTAACTAAT AATTGCTAAT TATGTTTTCC ATCTCTAAGG TTCCCACATT 4650
TTTCTGTTTT CTTAAAGATC CCATTATCTG GTTGTAACTG AAGCTCAATG 4700
GAACATGAGC AATATTTCCC AGTCTTCTCT CCCATCCAAC AGTCCTGATG 4750
GATTAGCAGA ACAGGCAGAA AACACATTGT TACCCAGAAT TAAAAACTAA 4800
TATTTGCTCT CCATTCAATC CAAAATGGAC CTATTGAAAC TAAAATCTAA 4850
CCCAATCCCA TTAAATGATT TCTATGGCGT CAAAGGTCAA ACTTCTGAAG 4900
GGAACCTGTG GGTGGGTCAC AATTCAGGCT ATATATTCCC CAGGGCTCAG 4950
CGGATCCATG GGCTCCATCG GCGCAGCAAG CATGGAATTT TGTTTTGATG 5000
TATTCAAGGA GCTCAAAGTC CACCATGCCA ATGAGAACAT CTTCTACTGC 5050
CCCATTGCCA TCATGTCAGC TCTAGCCATG GTATACCTGG GTGCAAAAGA 5100
CAGCACCAGG ACACAGATAA ATAAGGTTGT TCGCTTTGAT AAACTTCCAG 5150
GATTCGGAGA CAGTATTGAA GCTCAGTGTG GCACATCTGT AAACGTTCAC 5200
TCTTCACTTA GAGACATCCT CAACCAAATC ACCAAACCAA ATGATGTTTA 5250
TTCGTTCAGC CTTGCCAGTA GACTTTATGC TGAAGAGAGA TACCCAATCC 5300
TGCCAGAATA CTTGCAGTGT GTGAAGGAAC TGTATAGAGG AGGCTTGGAA 5350
CCTATCAACT TTCAAACAGC TGCAGATCAA GCCAGAGAGC TCATCAATTC 5400
CTGGGTAGAA AGTCAGACAA ATGGAATTAT CAGAAATGTC CTTCAGCCAA 5450
GCTCCGTGGA TTCTCAAACT GCAATGGTTC TGGTTAATGC CATTGTCTTC 5500
AAAGGACTGT GGGAGAAAAC ATTTAAGGAT GAAGACACAC AAGCAATGCC 5550
TTTCAGAGTG ACTGAGCAAG AAAGCAAACC TGTGCAGATG ATGTACCAGA 5600
TTGGTTTATT TAGAGTGGCA TCAATGGCTT CTGAGAAAAT GAAGATCCTG 5650
GAGCTTCCAT TTGCCAGTGG GACAATGAGC ATGTTGGTGC TGTTGCCTGA 5700
TGAAGTCTCA GGCCTTGAGC AGCTTGAGAG TATAATCAAC TTTGAAAAAC 5750
TGACTGAATG GACCAGTTCT AATGTTATGG AAGAGAGGAA GATCAAAGTG 5800
TACTTACCTC GCATGAAGAT GGAGGAAAAA TACAACCTCA CATCTGTCTT 5850
AATGGCTATG GGCATTACTG ACGTGTTTAG CTCTTCAGCC AATCTGTCTG 5900
GCATCTCCTC AGCAGAGAGC CTGAAGATAT CTCAAGCTGT CCATGCAGCA 5950
CATGCAGAAA TCAATGAAGC AGGCAGAGAG GTGGTAGGGT CAGCAGAGGC 6000
TGGAGTGGAT GCTGCAAGCG TCTCTGAAGA ATTTAGGGCT GACCATCCAT 6050
TCCTCTTCTG TATCAAGCAC ATCGCAACCA ACGCCGTTCT CTTCTTTGGC 6100
AGATGTGTTT CCCCTCCGCG GCCAGCAGAT GACGCACCAG CAGATGACGC 6150
ACCAGCAGAT GACGCACCAG CAGATGACGC ACCAGCAGAT GACGCACCAG 6200
CAGATGACGC AACAACATGT ATCCTGAAAG GCTCTTGTGG CTGGATCGGC 6250
CTGCTGGATG ACGATGACAA ATTTGTGAAC CAACACCTGT GCGGCTCACA 6300
 
CCTGGTGGAA GCTCTCTACC TAGTGTGCGG GGAACGAGGC TTCTTCTACA 6350
CACCCAAGAC CCGCCGGGAG GCAGAGGACC TGCAGGTGGG GCAGGTGGAG 6400
CTGGCCGGGG GCCCTGGTGC AGGCAGCCTG CAGCCCTTGG CCCTGGAGGG 6450
GTCCCTGCAG AAGCGTGGCA TTGTGGAACA ATGCTGTACC AGCATCTGCT 6500
CCCTCTACCA GCTGGAGAAC TACTGCAACT AGGGCGCCTG GATCCAGATC 6550
ACTTCTGGCT AATAAAAGAT CAGAGCTCTA GAGATCTGTG TGTTGGTTTT 6600
TTGTGGATCT GCTGTGCCTT CTAGTTGCCA GCCATCTGTT GTTTGCCCCT 6650
CCCCCGTGCC TTCCTTGACC CTGGAAGGTG CCACTCCCAC TGTCCTTTCC 6700
TAATAAAATG AGGAAATTGC ATCGCATTGT CTGAGTAGGT GTCATTCTAT 6750
TCTGGGGGGT GGGGTGGGGC AGCACAGCAA GGGGGAGGAT TGGGAAGACA 6800
ATAGCAGGCA TGCTGGGGAT GCGGTGGGCT CTATGGGTAC CTCTCTCTCT 6850
CTCTCTCTCT CTCTCTCTCT CTCTCTCTCT CGGTACCTCT CTCGAGGGGG 6900
GGCCCGGTAC CCAATTCGCC CTATAGTGAG TCGTATTACG CGCGCTCACT 6950
GGCCGTCGTT TTACAACGTC GTGACTGGGA AAACCCTGGC GTTACCCAAC 7000
TTAATCGCCT TGCAGCACAT CCCCCTTTCG CCAGCTGGCG TAATAGCGAA 7050
GAGGCCCGCA CCGATCGCCC TTCCCAACAG TTGCGCAGCC TGAATGGCGA 7100
ATGGAAATTG TAAGCGTTAA TATTTTGTTA AAATTCGCGT TAAATTTTTG 7150
TTAAATCAGC TCATTTTTTA ACCAATAGGC CGAAATCGGC AAAATCCCTT 7200
ATAAATCAAA AGAATAGACC GAGATAGGGT TGAGTGTTGT TCCAGTTTGG 7250
AACAAGAGTC CACTATTAAA GAACGTGGAC TCCAACGTCA AAGGGCGAAA 7300
AACCGTCTAT CAGGGCGATG GCCCACTACT CCGGGATCAT ATGACAAGAT 7350
GTGTATCCAC CTTAACTTAA TGATTTTTAC CAAAATCATT AGGGGATTCA 7400
TCAGTGCTCA GGGTCAACGA GAATTAACAT TCCGTCAGGA AAGCTTATGA 7450
TGATGATGTG CTTAAAAACT TACTCAATGG CTGGTTATGC ATATCGCAAT 7500
ACATGCGAAA AACCTAAAAG AGCTTGCCGA TAAAAAAGGC CAATTTATTG 7550
CTATTTACCG CGGCTTTTTA TTGAGCTTGA AAGATAAATA AAATAGATAG 7600
GTTTTATTTG AAGCTAAATC TTCTTTATCG TAAAAAATGC CCTCTTGGGT 7650
TATCAAGAGG GTCATTATAT TTCGCGGAAT AACATCATTT GGTGACGAAA 7700
TAACTAAGCA CTTGTCTCCT GTTTACTCCC CTGAGCTTGA GGGGTTAACA 7750
TGAAGGTCAT CGATAGCAGG ATAATAATAC AGTAAAACGC TAAACCAATA 7800
ATCCAAATCC AGCCATCCCA AATTGGTAGT GAATGATTAT AAATAACAGC 7850
AAACAGTAAT GGGCCAATAA CACCGGTTGC ATTGGTAAGG CTCACCAATA 7900
ATCCCTGTAA AGCACCTTGC TGATGACTCT TTGTTTGGAT AGACATCACT 7950
CCCTGTAATG CAGGTAAAGC GATCCCACCA CCAGCCAATA AAATTAAAAC 8000
AGGGAAAACT AACCAACCTT CAGATATAAA CGCTAAAAAG GCAAATGCAC 8050
TACTATCTGC AATAAATCCG AGCAGTACTG CCGTTTTTTC GCCCCATTTA 8100
GTGGCTATTC TTCCTGCCAC AAAGGCTTGG AATACTGAGT GTAAAAGACC 8150
AAGACCCGCT AATGAAAAGC CAACCATCAT GCTATTCCAT CCAAAACGAT 8200
TTTCGGTAAA TAGCACCCAC ACCGTTGCGG GAATTTGGCC TATCAATTGC 8250
GCTGAAAAAT AAATAATCAA CAAAATGGCA TCGTTTTAAA TAAAGTGATG 8300
TATACCGAAT TCAGCTTTTG TTCCCTTTAG TGAGGGTTAA TTGCGCGCTT 8350
GGCGTAATCA TGGTCATAGC TGTTTCCTGT GTGAAATTGT TATCCGCTCA 8400
CAATTCCACA CAACATACGA GCCGGAAGCA TAAAGTGTAA AGCCTGGGGT 8450
GCCTAATGAG TGAGCTAACT CACATTAATT GCGTTGCGCT CACTGCCCGC 8500
TTTCCAGTCG GGAAACCTGT CGTGCCAGCT GCATTAATGA ATCGGCCAAC 8550
GCGCGGGGAG AGGCGGTTTG CGTATTGGGC GCTCTTCCGC TTCCTCGCTC 8600
ACTGACTCGC TGCGCTCGGT CGTTCGGCTG CGGCGAGCGG TATCAGCTCA 8650
CTCAAAGGCG GTAATACGGT TATCCACAGA ATCAGGGGAT AACGCAGGAA 8700
AGAACATGTG AGCAAAAGGC CAGCAAAAGG CCAGGAACCG TAAAAAGGCC 8750
GCGTTGCTGG CGTTTTTCCA TAGGCTCCGC CCCCCTGACG AGCATCACAA 8800
AAATCGACGC TCAAGTCAGA GGTGGCGAAA CCCGACAGGA CTATAAAGAT 8850
ACCAGGCGTT TCCCCCTGGA AGCTCCCTCG TGCGCTCTCC TGTTCCGACC 8900
CTGCCGCTTA CCGGATACCT GTCCGCCTTT CTCCCTTCGG GAAGCGTGGC 8950
GCTTTCTCAT AGCTCACGCT GTAGGTATCT CAGTTCGGTG TAGGTCGTTC 9000
GCTCCAAGCT GGGCTGTGTG CACGAACCCC CCGTTCAGCC CGACCGCTGC 9050
GCCTTATCCG GTAACTATCG TCTTGAGTCC AACCCGGTAA GACACGACTT 9100
ATCGCCACTG GCAGCAGCCA CTGGTAACAG GATTAGCAGA GCGAGGTATG 9150
TAGGCGGTGC TACAGAGTTC TTGAAGTGGT GGCCTAACTA CGGCTACACT 9200
AGAAGGACAG TATTTGGTAT CTGCGCTCTG CTGAAGCCAG TTACCTTCGG 9250
AAAAAGAGTT GGTAGCTCTT GATCCGGCAA ACAAACCACC GCTGGTAGCG 9300
GTGGTTTTTT TGTTTGCAAG CAGCAGATTA CGCGCAGAAA AAAAGGATCT 9350
 
CAAGAAGATC CTTTGATCTT TTCTACGGGG TCTGACGCTC AGTGGAACGA 9400
AAACTCACGT TAAGGGATTT TGGTCATGAG ATTATCAAAA AGGATCTTCA 9450
CCTAGATCCT TTTAAATTAA AAATGAAGTT TTAAATCAAT CTAAAGTATA 9500
TATGAGTAAA CTTGGTCTGA CAGTTACCAA TGCTTAATCA GTGAGGCACC 9550
TATCTCAGCG ATCTGTCTAT TTCGTTCATC CATAGTTGCC TGACTCCCCG 9600
TCGTGTAGAT AACTACGATA CGGGAGGGCT TACCATCTGG CCCCAGTGCT 9650
GCAATGATAC CGCGAGACCC ACGCTCACCG GCTCCAGATT TATCAGCAAT 9700
AAACCAGCCA GCCGGAAGGG CCGAGCGCAG AAGTGGTCCT GCAACTTTAT 9750
CCGCCTCCAT CCAGTCTATT AATTGTTGCC GGGAAGCTAG AGTAAGTAGT 9800
TCGCCAGTTA ATAGTTTGCG CAACGTTGTT GCCATTGCTA CAGGCATCGT 9850
GGTGTCACGC TCGTCGTTTG GTATGGCTTC ATTCAGCTCC GGTTCCCAAC 9900
GATCAAGGCG AGTTACATGA TCCCCCATGT TGTGCAAAAA AGCGGTTAGC 9950
TCCTTCGGTC CTCCGATCGT TGTCAGAAGT AAGTTGGCCG CAGTGTTATC 10000
ACTCATGGTT ATGGCAGCAC TGCATAATTC TCTTACTGTC ATGCCATCCG 10050
TAAGATGCTT TTCTGTGACT GGTGAGTACT CAACCAAGTC ATTCTGAGAA 10100
TAGTGTATGC GGCGACCGAG TTGCTCTTGC CCGGCGTCAA TACGGGATAA 10150
TACCGCGCCA CATAGCAGAA CTTTAAAAGT GCTCATCATT GGAAAACGTT 10200
CTTCGGGGCG AAAACTCTCA AGGATCTTAC CGCTGTTGAG ATCCAGTTCG 10250
ATGTAACCCA CTCGTGCACC CAACTGATCT TCAGCATCTT TTACTTTCAC 10300
CAGCGTTTCT GGGTGAGCAA AAACAGGAAG GCAAAATGCC GCAAAAAAGG 10350
GAATAAGGGC GACACGGAAA TGTTGAATAC TCATACTCTT CCTTTTTCAA 10400
TATTATTGAA GCATTTATCA GGGTTATTGT CTCATGAGCG GATACATATT 10450
TGAATGTATT TAGAAAAATA AACAAATAGG GGTTCCGCGC ACATTTCCCC 10500
GAAAAGTGCC AC 10512

SEQ ID NO:32 (pTnMod(Oval/ENT tag/Proins/PA)- QUAIL)

CTGACGCGCC CTGTAGCGGC GCATTAAGCG CGGCGGGTGT GGTGGTTACG 50
CGCAGCGTGA CCGCTACACT TGCCAGCGCC CTAGCGCCCG CTCCTTTCGC 100
TTTCTTCCCT TCCTTTCTCG CCACGTTCGC CGGCATCAGA TTGGCTATTG 150
GCCATTGCAT ACGTTGTATC CATATCATAA TATGTACATT TATATTGGCT 200
CATGTCCAAC ATTACCGCCA TGTTGACATT GATTATTGAC TAGTTATTAA 250
TAGTAATCAA TTACGGGGTC ATTAGTTCAT AGCCCATATA TGGAGTTCCG 300
CGTTACATAA CTTACGGTAA ATGGCCCGCC TGGCTGACCG CCCAACGACC 350
CCCGCCCATT GACGTCAATA ATGACGTATG TTCCCATAGT AACGCCAATA 400
GGGACTTTCC ATTGACGTCA ATGGGTGGAG TATTTACGGT AAACTGCCCA 450
CTTGGCAGTA CATCAAGTGT ATCATATGCC AAGTACGCCC CCTATTGACG 500
TCAATGACGG TAAATGGCCC GCCTGGCATT ATGCCCAGTA CATGACCTTA 550
TGGGACTTTC CTACTTGGCA GTACATCTAC GTATTAGTCA TCGCTATTAC 600
CATGGTGATG CGGTTTTGGC AGTACATCAA TGGGCGTGGA TAGCGGTTTG 650
ACTCACGGGG ATTTCCAAGT CTCCACCCCA TTGACGTCAA TGGGAGTTTG 700
TTTTGGCACC AAAATCAACG GGACTTTCCA AAATGTCGTA ACAACTCCGC 750
CCCATTGACG CAAATGGGCG GTAGGCGTGT ACGGTGGGAG GTCTATATAA 800
GCAGAGCTCG TTTAGTGAAC CGTCAGATCG CCTGGAGACG CCATCCACGC 850
TGTTTTGACC TCCATAGAAG ACACCGGGAC CGATCCAGCC TCCGCGGCCG 900
GGAACGGTGC ATTGGAACGC GGATTCCCCG TGCCAAGAGT GACGTAAGTA 950
CCGCCTATAG ACTCTATAGG CACACCCCTT TGGCTCTTAT GCATGCTATA 1000
CTGTTTTTGG CTTGGGGCCT ATACACCCCC GCTTCCTTAT GCTATAGGTG 1050
ATGGTATAGC TTAGCCTATA GGTGTGGGTT ATTGACCATT ATTGACCACT 1100
CCCCTATTGG TGACGATACT TTCCATTACT AATCCATAAC ATGGCTCTTT 1150
GCCACAACTA TCTCTATTGG CTATATGCCA ATACTCTGTC CTTCAGAGAC 1200
TGACACGGAC TCTGTATTTT TACAGGATGG GGTCCCATTT ATTATTTACA 1250
AATTCACATA TACAACAACG CCGTCCCCCG TGCCCGCAGT TTTTATTAAA 1300
CATAGCGTGG GATCTCCACG CGAATCTCGG GTACGTGTTC CGGACATGGG 1350
CTCTTCTCCG GTAGCGGCGG AGCTTCCACA TCCGAGCCCT GGTCCCATGC 1400
CTCCAGCGGC TCATGGTCGC TCGGCAGCTC CTTGCTCCTA ACAGTGGAGG 1450
CCAGACTTAG GCACAGCACA ATGCCCACCA CCACCAGTGT GCCGCACAAG 1500
GCCGTGGCGG TAGGGTATGT GTCTGAAAAT GAGCGTGGAG ATTGGGCTCG 1550
CACGGCTGAC GCAGATGGAA GACTTAAGGC AGCGGCAGAA GAAGATGCAG 1600
GCAGCTGAGT TGTTGTATTC TGATAAGAGT CAGAGGTAAC TCCCGTTGCG 1650
 
GTGCTGTTAA CGGTGGAGGG CAGTGTAGTC TGAGCAGTAC TCGTTGCTGC 1700
CGCGCGCGCC ACCAGACATA ATAGCTGACA GACTAACAGA CTGTTCCTTT 1750
CCATGGGTCT TTTCTGCAGT CACCGTCGGA CCATGTGTGA ACTTGATATT 1800
TTACATGATT CTCTTTACCA ATTCTGCCCC GAATTACACT TAAAACGACT 1850
CAACAGCTTA ACGTTGGCTT GCCACGCATT ACTTGACTGT AAAACTCTCA 1900
CTCTTACCGA ACTTGGCCGT AACCTGCCAA CCAAAGCGAG AACAAAACAT 1950
AACATCAAAC GAATCGACCG ATTGTTAGGT AATCGTCACC TCCACAAAGA 2000
GCGACTCGCT GTATACCGTT GGCATGCTAG CTTTATCTGT TCGGGAATAC 2050
GATGCCCATT GTACTTGTTG ACTGGTCTGA TATTCGTGAG CAAAAACGAC 2100
TTATGGTATT GCGAGCTTCA GTCGCACTAC ACGGTCGTTC TGTTACTCTT 2150
TATGAGAAAG CGTTCCCGCT TTCAGAGCAA TGTTCAAAGA AAGCTCATGA 2200
CCAATTTCTA GCCGACCTTG CGAGCATTCT ACCGAGTAAC ACCACACCGC 2250
TCATTGTCAG TGATGCTGGC TTTAAAGTGC CATGGTATAA ATCCGTTGAG 2300
AAGCTGGGTT GGTACTGGTT AAGTCGAGTA AGAGGAAAAG TACAATATGC 2350
AGACCTAGGA GCGGAAAACT GGAAACCTAT CAGCAACTTA CATGATATGT 2400
CATCTAGTCA CTCAAAGACT TTAGGCTATA AGAGGCTGAC TAAAAGCAAT 2450
CCAATCTCAT GCCAAATTCT ATTGTATAAA TCTCGCTCTA AAGGCCGAAA 2500
AAATCAGCGC TCGACACGGA CTCATTGTCA CCACCCGTCA CCTAAAATCT 2550
ACTCAGCGTC GGCAAAGGAG CCATGGGTTC TAGCAACTAA CTTACCTGTT 2600
GAAATTCGAA CACCCAAACA ACTTGTTAAT ATCTATTCGA AGCGAATGCA 2650
GATTGAAGAA ACCTTCCGAG ACTTGAAAAG TCCTGCCTAC GGACTAGGCC 2700
TACGCCATAG CCGAACGAGC AGCTCAGAGC GTTTTGATAT CATGCTGCTA 2750
ATCGCCCTGA TGCTTCAACT AACATGTTGG CTTGCGGGCG TTCATGCTCA 2800
GAAACAAGGT TGGGACAAGC ACTTCCAGGC TAACACAGTC AGAAATCGAA 2850
ACGTACTCTC AACAGTTCGC TTAGGCATGG AAGTTTTGCG GCATTCTGGC 2900
TACACAATAA CAAGGGAAGA CTTACTCGTG GCTGCAACCC TACTAGCTCA 2950
AAATTTATTC ACACATGGTT ACGCTTTGGG GAAATTATGA TAATGATCCA 3000
GATCACTTCT GGCTAATAAA AGATCAGAGC TCTAGAGATC TGTGTGTTGG 3050
TTTTTTGTGG ATCTGCTGTG CCTTCTAGTT GCCAGCCATC TGTTGTTTGC 3100
CCCTCCCCCG TGCCTTCCTT GACCCTGGAA GGTGCCACTC CCACTGTCCT 3150
TTCCTAATAA AATGAGGAAA TTGCATCGCA TTGTCTGAGT AGGTGTCATT 3200
CTATTCTGGG GGGTGGGGTG GGGCAGCACA GCAAGGGGGA GGATTGGGAA 3250
GACAATAGCA GGCATGCTGG GGATGCGGTG GGCTCTATGG GTACCTCTCT 3300
CTCTCTCTCT CTCTCTCTCT CTCTCTCTCT CTCTCGGTAC CTCTCTCTCT 3350
CTCTCTCTCT CTCTCTCTCT CTCTCTCTCT CGGTACCAGG TGCTGAAGAA 3400
TTGACCCGGT GACCAAAGGT GCCTTTTATC ATCACTTTAA AAATAAAAAA 3450
CAATTACTCA GTGCCTGTTA TAAGCAGCAA TTAATTATGA TTGATGCCTA 3500
CATCACAACA AAAACTGATT TAACAAATGG TTGGTCTGCC TTAGAAAGTA 3550
TATTTGAACA TTATCTTGAT TATATTATTG ATAATAATAA AAACCTTATC 3600
CCTATCCAAG AAGTGATGCC TATCATTGGT TGGAATGAAC TTGAAAAAAA 3650
TTAGCCTTGA ATACATTACT GGTAAGGTAA ACGCCATTGT CAGCAAATTG 3700
ATCCAAGAGA ACCAACTTAA AGCTTTCCTG ACGGAATGTT AATTCTCGTT 3750
GACCCTGAGC ACTGATGAAT CCCCTAATGA TTTTGGTAAA AATCATTAAG 3800
TTAAGGTGGA TACACATCTT GTCATATGAT CCCGGTAATG TGAGTTAGCT 3850
CACTCATTAG GCACCCCAGG CTTTACACTT TATGCTTCCG GCTCGTATGT 3900
TGTGTGGAAT TGTGAGCGGA TAACAATTTC ACACAGGAAA CAGCTATGAC 3950
CATGATTACG CCAAGCGCGC AATTAACCCT CACTAAAGGG AACAAAAGCT 4000
GGAGCTCCAC CGCGGTGGCG GCCGCTCTAG AACTAGTGGA TCCCCCGGGG 4050
AGGTCAGAAT GGTTTCTTTA CTGTTTGTCA ATTCTATTAT TTCAATACAG 4100
AACAAAAGCT TCTATAACTG AAATATATTT GCTATTGTAT ATTATGATTG 4150
TCCCTCGAAC CATGAACACT CCTCCAGCTG AATTTCACAA TTCCTCTGTC 4200
ATCTGCCAGG CTGGAAGATC ATGGAAGATC TCTGAGGAAC ATTGCAAGTT 4250
CATACCATAA ACTCATTTGG AATTGAGTAT TATTTTGCTT TGAATGGAGC 4300
TATGTTTTGC AGTTCCCTCA GAAGAAAAGC TTGTTATAAA GCGTCTACAC 4350
CCATCAAAAG ATATATTTAA ATATTCCAAC TACAGAAAGA TTTTGTCTGC 4400
TCTTCACTCT GATCTCAGTT GGTTTCTTCA CGTACATGCT TCTTTATTTG 4450
CCTATTTTGT CAAGAAAATA ATAGGTCAAG TCCTGTTCTC ACTTATCTCC 4500
TGCCTAGCAT GGCTTAGATG CACGTTGTAC ATTCAAGAAG GATCAAATGA 4550
AACAGACTTC TGGTCTGTTA CAACAACCAT AGTAATAAAC AGACTAACTA 4600
ATAATTGCTA ATTATGTTTT CCATCTCTAA GGTTCCCACA TTTTTCTGTT 4650
TTAAGATCCC ATTATCTGGT TGTAACTGAA GCTCAATGGA ACATGAACAG 4700
 
TATTTCTCAG TCTTTTCTCC AGCAATCCTG ACGGATTAGA AGAACTGGCA 4750
GAAAACACTT TGTTACCCAG AATTAAAAAC TAATATTTGC TCTCCCTTCA 4800
ATCCAAAATG GACCTATTGA AACTAAAATC TGACCCAATC CCATTAAATT 4850
ATTTCTATGG CGTCAAAGGT CAAACTTTTG AAGGGAACCT GTGGGTGGGT 4900
CCCAATTCAG GCTATATATT CCCCAGGGCT CAGCCAGTGG ATCCATGGGC 4950
TCCATCGGTG CAGCAAGCAT GGAATTTTGT TTTGATGTAT TCAAGGAGCT 5000
CAAAGTCCAC CATGCCAATG ACAACATGCT CTACTCCCCC TTTGCCATCT 5050
TGTCAACTCT GGCCATGGTC TTCCTAGGTG CAAAAGACAG CACCAGGACC 5100
CAGATAAATA AGGTTGTTCA CTTTGATAAA CTTCCAGGAT TCGGAGACAG 5150
TATTGAAGCT CAGTGTGGCA CATCTGTAAA TGTTCACTCT TCACTTAGAG 5200
ACATACTCAA CCAAATCACC AAACAAAATG ATGCTTATTC GTTCAGCCTT 5250
GCCAGTAGAC TTTATGCTCA AGAGACATAC ACAGTCGTGC CGGAATACTT 5300
GCAATGTGTG AAGGAACTGT ATAGAGGAGG CTTAGAATCC GTCAACTTTC 5350
AAACAGCTGC AGATCAAGCC AGAGGCCTCA TCAATGCCTG GGTAGAAAGT 5400
CAGACAAACG GAATTATCAG AAACATCCTT CAGCCAAGCT CCGTGGATTC 5450
TCAAACTGCA ATGGTCCTGG TTAATGCCAT TGCCTTCAAG GGACTGTGGG 5500
AGAAAGCATT TAAGGCTGAA GACACGCAAA CAATACCTTT CAGAGTGACT 5550
GAGCAAGAAA GCAAACCTGT GCAGATGATG TACCAGATTG GTTCATTTAA 5600
AGTGGCATCA ATGGCTTCTG AGAAAATGAA GATCCTGGAG CTTCCATTTG 5650
CCAGTGGAAC AATGAGCATG TTGGTGCTGT TGCCTGATGA TGTCTCAGGC 5700
CTTGAGCAGC TTGAGAGTAT AATCAGCTTT GAAAAACTGA CTGAATGGAC 5750
CAGTTCTAGT ATTATGGAAG AGAGGAAGGT CAAAGTGTAC TTACCTCGCA 5800
TGAAGATGGA GGAGAAATAC AACCTCACAT CTCTCTTAAT GGCTATGGGA 5850
ATTACTGACC TGTTCAGCTC TTCAGCCAAT CTGTCTGGCA TCTCCTCAGT 5900
AGGGAGCCTG AAGATATCTC AAGCTGTCCA TGCAGCACAT GCAGAAATCA 5950
ATGAAGCGGG CAGAGATGTG GTAGGCTCAG CAGAGGCTGG AGTGGATGCT 6000
ACTGAAGAAT TTAGGGCTGA CCATCCATTC CTCTTCTGTG TCAAGCACAT 6050
CGAAACCAAC GCCATTCTCC TCTTTGGCAG ATGTGTTTCT CCGCGGCCAG 6100
CAGATGACGC ACCAGCAGAT GACGCACCAG CAGATGACGC ACCAGCAGAT 6150
GACGCACCAG CAGATGACGC ACCAGCAGAT GACGCAACAA CATGTATCCT 6200
GAAAGGCTCT TGTGGCTGGA TCGGCCTGCT GGATGACGAT GACAAATTTG 6250
TGAACCAACA CCTGTGCGGC TCACACCTGG TGGAAGCTCT CTACCTAGTG 6300
TGCGGGGAAC GAGGCTTCTT CTACACACCC AAGACCCGCC GGGAGGCAGA 6350
GGACCTGCAG GTGGGGCAGG TGGAGCTGGG CGGGGGCCCT GGTGCAGGCA 6400
GCCTGCAGCC CTTGGCCCTG GAGGGGTCCC TGCAGAAGCG TGGCATTGTG 6450
GAACAATGCT GTACCAGCAT CTGCTCCCTC TACCAGCTGG AGAACTACTG 6500
CAACTAGGGC GCCTGGATCC AGATCACTTC TGGCTAATAA AAGATCAGAG 6550
CTCTAGAGAT CTGTGTGTTG GTTTTTTGTG GATCTGCTGT GCCTTCTAGT 6600
TGCCAGCCAT CTGTTGTTTG CCCCTCCCCC GTGCCTTCCT TGACCCTGGA 6650
AGGTGCCACT CCCACTGTCC TTTCCTAATA AAATGAGGAA ATTGCATCGC 6700
ATTGTCTGAG TAGGTGTCAT TCTATTCTGG GGGGTGGGGT GGGGCAGCAC 6750
AGCAAGGGGG AGGATTGGGA AGACAATAGC AGGCATGCTG GGGATGCGGT 6800
GGGCTCTATG GGTACCTCTC TCTCTCTCTC TCTCTCTCTC TCTCTCTCTC 6850
TCTCTCGGTA CCTCTCTCGA GGGGGGGCCC GGTACCCAAT TCGCCCTATA 6900
GTGAGTCGTA TTACGCGCGC TCACTGGCCG TCGTTTTACA ACGTCGTGAC 6950
TGGGAAAACC CTGGCGTTAC CCAACTTAAT CGCCTTGCAG CACATCCCCC 7000
TTTCGCCAGC TGGCGTAATA GCGAAGAGGC CCGCACCGAT CGCCCTTCCC 7050
AACAGTTGCG CAGCCTGAAT GGCGAATGGA AATTGTAAGC GTTAATATTT 7100
TGTTAAAATT CGCGTTAAAT TTTTGTTAAA TCAGCTCATT TTTTAACCAA 7150
TAGGCCGAAA TCGGCAAAAT CCCTTATAAA TCAAAAGAAT AGACCGAGAT 7200
AGGGTTGAGT GTTGTTCCAG TTTGGAACAA GAGTCCACTA TTAAAGAACG 7250
TGGACTCCAA CGTCAAAGGG CGAAAAACCG TCTATCAGGG CGATGGCCCA 7300
CTACTCCGGG ATCATATGAC AAGATGTGTA TCCACCTTAA CTTAATGATT 7350
TTTACCAAAA TCATTAGGGG ATTCATCAGT GCTCAGGGTC AACGAGAATT 7400
AACATTCCGT CAGGAAAGCT TATGATGATG ATGTGCTTAA AAACTTACTC 7450
AATGGCTGGT TATGCATATC GCAATACATG CGAAAAACCT AAAAGAGCTT 7500
GCCGATAAAA AAGGCCAATT TATTGCTATT TACCGCGGCT TTTTATTGAG 7550
CTTGAAAGAT AAATAAAATA GATAGGTTTT ATTTGAAGCT AAATCTTCTT 7600
TATCGTAAAA AATGCCCTCT TGGGTTATCA AGAGGGTCAT TATATTTCGC 7650
GGAATAACAT CATTTGGTGA CGAAATAACT AAGCACTTGT CTCCTGTTTA 7700
CTCCCCTGAG CTTGAGGGGT TAACATGAAG GTCATCGATA GCAGGATAAT 7750
 
AATACAGTAA AACGCTAAAC CAATAATCCA AATCCAGCCA TCCCAAATTG 7800
GTAGTGAATG ATTATAAATA ACAGCAAACA GTAATGGGCC AATAACACCG 7850
GTTGCATTGG TAAGGCTCAC CAATAATCCC TGTAAAGCAC CTTGCTGATG 7900
ACTCTTTGTT TGGATAGACA TCACTCCCTG TAATGCAGGT AAAGCGATCC 7950
CACCACCAGC CAATAAAATT AAAACAGGGA AAACTAACCA ACCTTCAGAT 8000
ATAAACGCTA AAAAGGCAAA TGCACTACTA TCTGCAATAA ATCCGAGCAG 8050
TACTGCCGTT TTTTCGCCCC ATTTAGTGGC TATTCTTCCT GCCACAAAGG 8100
CTTGGAATAC TGAGTGTAAA AGACCAAGAC CCGCTAATGA AAAGCCAACC 8150
ATCATGCTAT TCCATCCAAA ACGATTTTCG GTAAATAGCA CCCACACCGT 8200
TGCGGGAATT TGGCCTATCA ATTGCGCTGA AAAATAAATA ATCAACAAAA 8250
TGGCATCGTT TTAAATAAAG TGATGTATAC CGAATTCAGC TTTTGTTCCC 8300
TTTAGTGAGG GTTAATTGCG CGCTTGGCGT AATCATGGTC ATAGCTGTTT 8350
CCTGTGTGAA ATTGTTATCC GCTCACAATT CCACACAACA TACGAGCCGG 8400
AAGCATAAAG TGTAAAGCCT GGGGTGCCTA ATGAGTGAGC TAACTCACAT 8450
TAATTGCGTT GCGCTCACTG CCCGCTTTCC AGTCGGGAAA CCTGTCGTGC 8500
CAGCTGCATT AATGAATCGG CCAACGCGCG GGGAGAGGCG GTTTGCGTAT 8550
TGGGCGCTCT TCCGCTTCCT CGCTCACTGA CTCGCTGCGC TCGGTCGTTC 8600
GGCTGCGGCG AGCGGTATCA GCTCACTCAA AGGCGGTAAT ACGGTTATCC 8650
ACAGAATCAG GGGATAACGC AGGAAAGAAC ATGTGAGCAA AAGGCCAGCA 8700
AAAGGCCAGG AACCGTAAAA AGGCCGCGTT GCTGGCGTTT TTCCATAGGC 8750
TCCGCCCCCC TGACGAGCAT CACAAAAATC GACGCTCAAG TCAGAGGTGG 8800
CGAAACCCGA CAGGACTATA AAGATACCAG GCGTTTCCCC CTGGAAGCTC 8850
CCTCGTGCGC TCTCCTGTTC CGACCCTGCC GCTTACCGGA TACCTGTCCG 8900
CCTTTCTCCC TTCGGGAAGC GTGGCGCTTT CTCATAGCTC ACGCTGTAGG 8950
TATCTCAGTT CGGTGTAGGT CGTTCGCTCC AAGCTGGGCT GTGTGCACGA 9000
ACCCCCCGTT CAGCCCGACC GCTGCGCCTT ATCCGGTAAC TATCGTCTTG 9050
AGTCCAACCC GGTAAGACAC GACTTATCGC CACTGGCAGC AGCCACTGGT 9100
AACAGGATTA GCAGAGCGAG GTATGTAGGC GGTGCTACAG AGTTCTTGAA 9150
GTGGTGGCCT AACTACGGCT ACACTAGAAG GACAGTATTT GGTATCTGCG 9200
CTCTGCTGAA GCCAGTTACC TTCGGAAAAA GAGTTGGTAG CTCTTGATCC 9250
GGCAAACAAA CCACCGCTGG TAGCGGTGGT TTTTTTGTTT GCAAGCAGCA 9300
GATTACGCGC AGAAAAAAAG GATCTCAAGA AGATCCTTTG ATCTTTTCTA 9350
CGGGGTCTGA CGCTCAGTGG AACGAAAACT CACGTTAAGG GATTTTGGTC 9400
ATGAGATTAT CAAAAAGGAT CTTCACCTAG ATCCTTTTAA ATTAAAAATG 9450
AAGTTTTAAA TCAATCTAAA GTATATATGA GTAAACTTGG TCTGACAGTT 9500
ACCAATGCTT AATCAGTGAG GCACCTATCT CAGCGATCTG TCTATTTCGT 9550
TCATCCATAG TTGCCTGACT CCCCGTCGTG TAGATAACTA CGATACGGGA 9600
GGGCTTACCA TCTGGCCCCA GTGCTGCAAT GATACCGCGA GACCCACGCT 9650
CACCGGCTCC AGATTTATCA GCAATAAACC AGCCAGCCGG AAGGGCCGAG 9700
CGCAGAAGTG GTCCTGCAAC TTTATCCGCC TCCATCCAGT CTATTAATTG 9750
TTGCCGGGAA GCTAGAGTAA GTAGTTCGCC AGTTAATAGT TTGCGCAACG 9800
TTGTTGCCAT TGCTACAGGC ATCGTGGTGT CACGCTCGTC GTTTGGTATG 9850
GCTTCATTCA GCTCCGGTTC CCAACGATCA AGGCGAGTTA CATGATCCCC 9900
CATGTTGTGC AAAAAAGCGG TTAGCTCCTT CGGTCCTCCG ATCGTTGTCA 9950
GAAGTAAGTT GGCCGCAGTG TTATCACTCA TGGTTATGGC AGCACTGCAT 10000
AATTCTCTTA CTGTCATGCC ATCCGTAAGA TGCTTTTCTG TGACTGGTGA 10050
GTACTCAACC AAGTCATTCT GAGAATAGTG TATGCGGCGA CCGAGTTGCT 10100
CTTGCCCGGC GTCAATACGG GATAATACCG CGCCACATAG CAGAACTTTA 10150
AAAGTGCTCA TCATTGGAAA ACGTTCTTCG GGGCGAAAAC TCTCAAGGAT 10200
CTTACCGCTG TTGAGATCCA GTTCGATGTA ACCCACTCGT GCACCCAACT 10250
GATCTTCAGC ATCTTTTACT TTCACCAGCG TTTCTGGGTG AGCAAAAACA 10300
GGAAGGCAAA ATGCCGCAAA AAAGGGAATA AGGGCGACAC GGAAATGTTG 10350
AATACTCATA CTCTTCCTTT TTCAATATTA TTGAAGCATT TATCAGGGTT 10400
ATTGTCTCAT GAGCGGATAC ATATTTGAAT GTATTTAGAA AAATAAACAA 10450
ATAGGGGTTC CGCGCACATT TCCCCGAAAA GTGCCAC 10487

SEQ ID NO:33 (conalbumin polyA)
tctgccattg ctgcttcctc tgcccttcct cgtcactctg aatgtggctt cttcgctact
gccacagcaa gaaataaaat ctcaacatct aaatgggttt cctgaggttt ttcaagagtc
gttaagcaca ttccttcccc agcacccctt gctgcaggcc agtgccaggc accaacttgg
 
ctactgctgc ccatgagaga aatccagttc aatattttcc aaagcaaaat ggattacata
tgccctagat cctgattaac aggcgtttgt attatctagt gctttcgctt cacccagatt
atcccattgc ctccc

SEQ ID NO:34 (exemplary antibody light chain sequence)
1 gagctcgtga tgacccagac tccatcctcc ctgtctgcct ctctgggaga cagagtcacc
61 atcagttgca gggcaaatca ggacattagc aattatttaa actggtatca gcagaaacca
121 gatggaactg ttaaactcct gatctactac acatcaagat tacactcagg ggtcccatca
181 aggttcagtg gcagtgggtc tggaacagat tattctctca ccattagcaa cctggagcaa
241 caagattttg ccacttactt ttgccaacag ggtaatacgc ttccgtggac gttcggtgga
301 ggcaccaacc tggaaatcaa acgggctgat gctgcaccaa ctgtatccat cttcccacca
361 tccagtgagc agttaacatc tggaggtgcc tcagtcgtgt gcttcttgaa caacttctac
421 cccaaagaca tcaatgtcaa gtggaagatt gatggcagtg aacgacaaaa tggcgtcctg
481 aacagttgga ctgatcagga cagcaaagac agcacctaca gcatgagcag caccctcacg
541 ttgaccaagg acgagtatga acgacataac agctatacct gtgaggccac tcacaagaca
601 tcaacttcac ccattgtcaa gagcttcaac aggaatgagt gttaa

SEQ ID NO:35 (exemplary antibody heavy chain sequence)
1 ctcgagtcag gacctggcct ggtggcgccc tcacagaacc tgtccatcac ttgcactgtc
61 tctgggtttt cattaaccag ctatggtgta cactgggttc gccagcctcc aggaaagggt
121 ctggaatggc tgggagtaat atggactggt agaagcacaa cttataattc ggctctcatg
181 tccagactga gcatcagcaa agacaactcc aagagccaag ttttcttaaa aatgaacagt
241 ctgcaaactg atgacacagc catttactac tgtggcagag ggggtctgat tacgtccttt
301 gctatggact actggggtca aggaacctca gtcaccgtct cctcagccaa aacgacaccc
361 ccatctgtct atccactggc ccctggatct gctgcccaaa ctaactccat ggtgaccctg
421 ggatgcctgg tcaagggcta tttccctgag ccagtgacag tgacctggaa ctctggatcc
481 ctgtccagcg gtgtgcacac cttcccagct gtcctgcagt ctgacctcta cactctgagc
541 agctcagtga ctgtcccctc cagcacctgg cccagcgaga ccgtcacctg caacgttgcc
601 cacccggcca gcagcaccaa ggtggacaag aaaattgtgc ccagggattg tactagt

SEQ ID NO:36 (pTnMCS)

1 ctgacgcgcc ctgtagcggc gcattaagcg cggcgggtgt ggtggttacg cgcagcgtga
61 ccgctacact tgccagcgcc ctagcgcccg ctcctttcgc tttcttccct tcctttctcg
121 ccacgttcgc cggcatcaga ttggctattg gccattgcat acgttgtatc catatcataa
181 tatgtacatt tatattggct catgtccaac attaccgcca tgttgacatt gattattgac
241 tagttattaa tagtaatcaa ttacggggtc attagttcat agcccatata tggagttccg
301 cgttacataa cttacggtaa atggcccgcc tggctgaccg cccaacgacc cccgcccatt
361 gacgtcaata atgacgtatg ttcccatagt aacgccaata gggactttcc attgacgtca
421 atgggtggag tatttacggt aaactgccca cttggcagta catcaagtgt atcatatgcc
481 aagtacgccc cctattgacg tcaatgacgg taaatggccc gcctggcatt atgcccagta
541 catgacctta tgggactttc ctacttggca gtacatctac gtattagtca tcgctattac
601 catggtgatg cggttttggc agtacatcaa tgggcgtgga tagcggtttg actcacgggg
661 atttccaagt ctccacccca ttgacgtcaa tgggagtttg ttttggcacc aaaatcaacg
721 ggactttcca aaatgtcgta acaactccgc cccattgacg caaatgggcg gtaggcgtgt
781 acggtgggag gtctatataa gcagagctcg tttagtgaac cgtcagatcg cctggagacg
 
841 ccatccacgc tgttttgacc tccatagaag acaccgggac cgatccagcc tccgcggccg
901 ggaacggtgc attggaacgc ggattccccg tgccaagagt gacgtaagta ccgcctatag
961 actctatagg cacacccctt tggctcttat gcatgctata ctgtttttgg cttggggcct
1021 atacaccccc gcttccttat gctataggtg atggtatagc ttagcctata ggtgtgggtt
1081 attgaccatt attgaccact cccctattgg tgacgatact ttccattact aatccataac
1141 atggctcttt gccacaacta tctctattgg ctatatgcca atactctgtc cttcagagac
1201 tgacacggac tctgtatttt tacaggatgg ggtcccattt attatttaca aattcacata
1261 tacaacaacg ccgtcccccg tgcccgcagt ttttattaaa catagcgtgg gatctccacg
1321 cgaatctcgg gtacgtgttc cggacatggg ctcttctccg gtagcggcgg agcttccaca
1381 tccgagccct ggtcccatgc ctccagcggc tcatggtcgc tcggcagctc cttgctccta
1441 acagtggagg ccagacttag gcacagcaca atgcccacca ccaccagtgt gccgcacaag
1501 gccgtggcgg tagggtatgt gtctgaaaat gagcgtggag attgggctcg cacggctgac
1561 gcagatggaa gacttaaggc agcggcagaa gaagatgcag gcagctgagt tgttgtattc
1621 tgataagagt cagaggtaac tcccgttgcg gtgctgttaa cggtggaggg cagtgtagtc
1681 tgagcagtac tcgttgctgc cgcgcgcgcc accagacata atagctgaca gactaacaga
1741 ctgttccttt ccatgggtct tttctgcagt caccgtcgga ccatgtgcga actcgatatt
1801 ttacacgact ctctttacca attctgcccc gaattacact taaaacgact caacagctta
1861 acgttggctt gccacgcatt acttgactgt aaaactctca ctcttaccga acttggccgt
1921 aacctgccaa ccaaagcgag aacaaaacat aacatcaaac gaatcgaccg attgttaggt
1981 aatcgtcacc tccacaaaga gcgactcgct gtataccgtt ggcatgctag ctttatctgt
2041 tcgggcaata cgatgcccat tgtacttgtt gactggtctg atattcgtga gcaaaaacga
2101 cttatggtat tgcgagcttc agtcgcacta cacggtcgtt ctgttactct ttatgagaaa
2161 gcgttcccgc tttcagagca atgttcaaag aaagctcatg accaatttct agccgacctt
2221 gcgagcattc taccgagtaa caccacaccg ctcattgtca gtgatgctgg ctttaaagtg
2281 ccatggtata aatccgttga gaagctgggt tggtactggt taagtcgagt aagaggaaaa
2341 gtacaatatg cagacctagg agcggaaaac tggaaaccta tcagcaactt acatgatatg
2401 tcatctagtc actcaaagac tttaggctat aagaggctga ctaaaagcaa tccaatctca
2461 tgccaaattc tattgtataa atctcgctct aaaggccgaa aaaatcagcg ctcgacacgg
2521 actcattgtc accacccgtc acctaaaatc tactcagcgt cggcaaagga gccatgggtt
2581 ctagcaacta acttacctgt tgaaattcga acacccaaac aacttgttaa tatctattcg
2641 aagcgaatgc agattgaaga aaccttccga gacttgaaaa gtcctgccta cggactaggc
2701 ctacgccata gccgaacgag cagctcagag cgttttgata tcatgctgct aatcgccctg
2761 atgcttcaac taacatgttg gcttgcgggc gttcatgctc agaaacaagg ttgggacaag
2821 cacttccagg ctaacacagt cagaaatcga aacgtactct caacagttcg cttaggcatg
2881 gaagttttgc ggcattctgg ctacacaata acaagggaag acttactcgt ggctgcaacc
2941 ctactagctc aaaatttatt cacacatggt tacgctttgg ggaaattatg aggggatcgc
3001 tctagagcga tccgggatct cgggaaaagc gttggtgacc aaaggtgcct tttatcatca
3061 ctttaaaaat aaaaaacaat tactcagtgc ctgttataag cagcaattaa ttatgattga
3121 tgcctacatc acaacaaaaa ctgatttaac aaatggttgg tctgccttag aaagtatatt
3181 tgaacattat cttgattata ttattgataa taataaaaac cttatcccta tccaagaagt
3241 gatgcctatc attggttgga atgaacttga aaaaaattag ccttgaatac attactggta
3301 aggtaaacgc cattgtcagc aaattgatcc aagagaacca acttaaagct ttcctgacgg
3361 aatgttaatt ctcgttgacc ctgagcactg atgaatcccc taatgatttt ggtaaaaatc
3421 attaagttaa ggtggataca catcttgtca tatgatcccg gtaatgtgag ttagctcact
3481 cattaggcac cccaggcttt acactttatg cttccggctc gtatgttgtg tggaattgtg
3541 agcggataac aatttcacac aggaaacagc tatgaccatg attacgccaa gcgcgcaatt
3601 aaccctcact aaagggaaca aaagctggag ctccaccgcg gtggcggccg ctctagaact
3661 agtggatccc ccgggctgca ggaattcgat atcaagctta tcgataccgc tgacctcgag
3721 ggggggcccg gtacccaatt cgccctatag tgagtcgtat tacgcgcgct cactggccgt
3781 cgttttacaa cgtcgtgact gggaaaaccc tggcgttacc caacttaatc gccttgcagc
3841 acatccccct ttcgccagct ggcgtaatag cgaagaggcc cgcaccgatc gcccttccca
3901 acagttgcgc agcctgaatg gcgaatggaa attgtaagcg ttaatatttt gttaaaattc
3961 gcgttaaatt tttgttaaat cagctcattt tttaaccaat aggccgaaat cggcaaaatc
4021 ccttataaat caaaagaata gaccgagata gggttgagtg ttgttccagt ttggaacaag
4081 agtccactat taaagaacgt ggactccaac gtcaaagggc gaaaaaccgt ctatcagggc
4141 gatggcccac tactccggga tcatatgaca agatgtgtat ccaccttaac ttaatgattt
4201 ttaccaaaat cattagggga ttcatcagtg ctcagggtca acgagaatta acattccgtc
4261 aggaaagctt atgatgatga tgtgcttaaa aacttactca atggctggtt atgcatatcg
4321 caatacatgc gaaaaaccta aaagagcttg ccgataaaaa aggccaattt attgctattt
4381 accgcggctt tttattgagc ttgaaagata aataaaatag ataggtttta tttgaagcta
4441 aatcttcttt atcgtaaaaa atgccctctt gggttatcaa gagggtcatt atatttcgcg
4501 gaataacatc atttggtgac gaaataacta agcacttgtc tcctgtttac tcccctgagc
4561 ttgaggggtt aacatgaagg tcatcgatag caggataata atacagtaaa acgctaaacc
4621 aataatccaa atccagccat cccaaattgg tagtgaatga ttataaataa cagcaaacag
4681 taatgggcca ataacaccgg ttgcattggt aaggctcacc aataatccct gtaaagcacc
4741 ttgctgatga ctctttgttt ggatagacat cactccctgt aatgcaggta aagcgatccc
4801 accaccagcc aataaaatta aaacagggaa aactaaccaa ccttcagata taaacgctaa
4861 aaaggcaaat gcactactat ctgcaataaa tccgagcagt actgccgttt tttcgcccat
 
4921 ttagtggcta ttcttcctgc cacaaaggct tggaatactg agtgtaaaag accaagaccc
4981 gtaatgaaaa gccaaccatc atgctattca tcatcacgat ttctgtaata gcaccacacc
5041 gtgctggatt ggctatcaat gcgctgaaat aataatcaac aaatggcatc gttaaataag
5101 [?]gatgtatac cgatcagctt ttgttccctt tagtgagggt taattgcgcg cttggcgtaa
5161 [?]catggtcat agctgtttcc tgtgtgaaat tgttatccgc tcacaattcc acacaacata
5221 cgagccggaa gcataaagtg taaagcctgg ggtgcctaat gagtgagcta actcacatta
5281 attgcgttgc gctcactgcc cgctttccag tcgggaaacc tgtcgtgcca gctgcattaa
5341 tgaatcggcc aacgcgcggg gagaggcggt ttgcgtattg ggcgctcttc cgcttcctcg
5401 ctcactgact cgctgcgctc ggtcgttcgg ctgcggcgag cggtatcagc tcactcaaag
5461 gcggtaatac ggttatccac agaatcaggg gataacgcag gaaagaacat gtgagcaaaa
5521 ggccagcaaa aggccaggaa ccgtaaaaag gccgcgttgc tggcgttttt ccataggctc
5581 cgcccccctg acgagcatca caaaaatcga cgctcaagtc agaggtggcg aaacccgaca
5641 ggactataaa gataccaggc gtttccccct ggaagctccc tcgtgcgctc tcctgttccg
5701 accctgccgc ttaccggata cctgtccgcc tttctccctt cgggaagcgt ggcgctttct
5761 catagctcac gctgtaggta tctcagttcg gtgtaggtcg ttcgctccaa gctgggctgt
5821 gtgcacgaac cccccgttca gcccgaccgc tgcgccttat ccggtaacta tcgtcttgag
5881 tccaacccgg taagacacga cttatcgcca ctggcagcag ccactggtaa caggattagc
5941 agagcgaggt atgtaggcgg tgctacagag ttcttgaagt ggtggcctaa ctacggctac
6001 actagaagga cagtatttgg tatctgcgct ctgctgaagc cagttacctt cggaaaaaga
6061 gttggtagct cttgatccgg caaacaaacc accgctggta gcggtggttt ttttgtttgc
6121 aagcagcaga ttacgcgcag aaaaaaagga tctcaagaag atcctttgat cttttctacg
6181 gggtctgacg ctcagtggaa cgaaaactca cgttaaggga ttttggtcat gagattatca
6241 aaaaggatct tcacctagat ccttttaaat taaaaatgaa gttttaaatc aatctaaagt
6301 atatatgagt aaacttggtc tgacagttac caatgcttaa tcagtgaggc acctatctca
6361 gcgatctgtc tatttcgttc atccatagtt gcctgactcc ccgtcgtgta gataactacg
6421 atacgggagg gcttaccatc tggccccagt gctgcaatga taccgcgaga cccacgctca
6481 ccggctccag atttatcagc aataaaccag ccagccggaa gggccgagcg cagaagtggt
6541 cctgcaactt tatccgcctc catccagtct attaattgtt gccgggaagc tagagtaagt
6601 agttcgccag ttaatagttt gcgcaacgtt gttgccattg ctacaggcat cgtggtgtca
6661 cgctcgtcgt ttggtatggc ttcattcagc tccggttccc aacgatcaag gcgagttaca
6721 tgatccccca tgttgtgcaa aaaagcggtt agctccttcg gtcctccgat cgttgtcaga
6781 agtaagttgg ccgcagtgtt atcactcatg gttatggcag cactgcataa ttctcttact
6841 gtcatgccat ccgtaagatg cttttctgtg actggtgagt actcaaccaa gtcattctga
6901 gaatagtgta tgcggcgacc gagttgctct tgcccggcgt caatacggga taataccgcg
6961 ccacatagca gaactttaaa agtgctcatc attggaaaac gttcttcggg gcgaaaactc
7021 tcaaggatct taccgctgtt gagatccagt tcgatgtaac ccactcgtgc acccaactga
7081 tcttcagcat cttttacttt caccagcgtt tctgggtgag caaaaacagg aaggcaaaat
7141 gccgcaaaaa agggaataag ggcgacacgg aaatgttgaa tactcatact cttccttttt
7201 caatattatt gaagcattta tcagggttat tgtctcatga gcggatacat atttgaatgt
7261 atttagaaaa ataaacaaat aggggttccg cgcacatttc cccgaaaagt gccac

SEQ ID NO:37 (chicken ovalbumin ehancer)
ccgggctgca gaaaaatgcc aggtggacta tgaactcaca tccaaaggag
cttgacctga tacctgattt tcttcaaact ggggaaacaa cacaatccca caaaacagct
cagagagaaa ccatcactga tggctacagc accaaggtat gcaatggcaa tccattcgac
attcatctgt gacctgagca aaatgattta tctctccatg aatggttgct tctttccctc
atgaa[?]aggc aatttccaca ctcacaatat gcaacaaaga caaacagaga acaattaatg
tgctc[?]ttcc taatgtcaaa attgtagtgg caaagaggag aacaaaatct caagttctga
gtaggtttta gtgattggat aagaggcttt gacctgtgag ctcacctgga cttcatatcc
ttttggataa aaagtgcttt tataactttc aggtctccga gtctttattc atgagactgt
tggtttaggg acagacccac aatgaaatgc ctggcatagg aaagggcagc agagccttag
ctgacctttt cttgggacaa gcattgtcaa acaatgtgtg acaaaactat ttgtactgct
ttgcacagct gtgctgggca gggcaatcca ttgccaccta tcccaggtaa ccttccaact
gcaagaagat tgttgcttac tctctctaga

SEQ ID NO:38 (5' untranslated region)
GTGGATCAACATACAGCTAGAAAGCTGTATTGCCTTTAGCACTCAAGCTCAAAAGACAACTCAGAGTTC
ACC
SEQ ID NO:39 (putative cap site)
   ACATACAGCTAG AAAGCTGTAT TGCCTTTAGC ACTCAAGCTC AAAAGACAAC TCAGAGTTCA

SEQ I[?] NO:40 (fragment of ovalbumin promoter - chicken)
GAGGTCAGAAT GGTTTCTTTA CTGTTTGTCA ATTCTATTAT TTCAATACAG
AACAATAGCT TCTATAACTG AAATATATTT GCTATTGTAT ATTATGATTG

TCCCTCGAAC CATGAACACT CCTCCAGCTG AATTTCACAA TTCCTCTGTC
ATCTGCCAGG CCATTAAGTT ATTCATGGAA GATCTTTGAG GAACACTGCA
AGTTCATATC ATAAACACAT TTGAAATTGA GTATTGTTTT GCATTGTATG
GAGCTATGTT TTGCTGTATC CTCAGAAAAA AAGTTTGTTA TAAAGCATTC
ACACCCATAA AAAGATAGAT TTAAATATTC CAGCTATAGG AAAGAAAGTG
CGTCTGCTCT TCACTCTAGT CTCAGTTGGC TCCTTCACAT GCATGCTTCT
TTATTTCTCC TATTTTGTCA AGAAAATAAT AGGTCACGTC TTGTTCTCAC
TTATGTCCTG CCTAGCATGG CTCAGATGCA CGTTGTAGAT ACAAGAAGGA
TCAAATGAAA CAGACTTCTG GTCTGTTACT ACAACCATAG TAATAAGCAC
ACTAACTAAT AATTGCTAAT TATGTTTTCC ATCTCTAAGG TTCCCACATT
TTTCTGTTTT CTTAAAGATC CCATTATCTG GTTGTAACTG AAGCTCAATG
GAACATGAGC AATATTTCCC AGTCTTCTCT CCCATCCAAC AGTCCTGATG
GATTAGCAGA ACAGGCAGAA AACACATTGT TACCCAGAAT TAAAAACTAA
TATTTGCTCT CCATTCAATC CAAAATGGAC CTATTGAAAC TAAAATCTAA
CCCAATCCCA TTAAATGATT TCTATGGCGT CAAAGGTCAA ACTTCTGAAG
GGAACCTGTG GGTGGGTCAC AATTCAGGCT ATATATTCCC CAGGGCTCAG
C

SEQ ID NO:41 pTnMCS (CMV-CHOVg-ent-ProInsulin-synPA)
1 ctgacgcgcc ctgtagcggc gcattaagcg cggcgggtgt ggtggttacg cgcagcgtga
61 ccgctacact tgccagcgcc ctagcgcccg ctcctttcgc tttcttccct tcctttctcg
121 ccacgttcgc cggcatcaga ttggctattg gccattgcat acgttgtatc catatcataa
181 tatgtacatt tatattggct catgtccaac attaccgcca tgttgacatt gattattgac
241 tagttattaa tagtaatcaa ttacggggtc attagttcat agcccatata tggagttccg
301 cgttacataa cttacggtaa atggcccgcc tggctgaccg cccaacgacc cccgcccatt
361 gacgtcaata atgacgtatg ttcccatagt aacgccaata gggactttcc attgacgtca
421 atgggtggag tatttacggt aaactgccca cttggcagta catcaagtgt atcatatgcc
481 aagtacgccc cctattgacg tcaatgacgg taaatggccc gcctggcatt atgcccagta
541 catgacctta tgggactttc ctacttggca gtacatctac gtattagtca tcgctattac
601 catggtgatg cggttttggc agtacatcaa tgggcgtgga tagcggtttg actcacgggg
661 atttccaagt ctccacccca ttgacgtcaa tgggagtttg ttttggcacc aaaatcaacg
721 ggactttcca aaatgtcgta acaactccgc cccattgacg caaatgggcg gtaggcgtgt
781 acggtgggag gtctatataa gcagagctcg tttagtgaac cgtcagatcg cctggagacg
841 ccatccacgc tgttttgacc tccatagaag acaccgggac cgatccagcc tccgcggccg
901 ggaacggtgc attggaacgc ggattccccg tgccaagagt gacgtaagta ccgcctatag
961 actctatagg cacacccctt tggctcttat gcatgctata ctgtttttgg cttggggcct
1021 atacaccccc gcttccttat gctataggtg atggtatagc ttagcctata ggtgtgggtt
1081 attgaccatt attgaccact cccctattgg tgacgatact ttccattact aatccataac
1141 atggctcttt gccacaacta tctctattgg ctatatgcca atactctgtc cttcagagac
1201 tgacacggac tctgtatttt tacaggatgg ggtcccattt attatttaca aattcacata
1261 tacaacaacg ccgtcccccg tgcccgcagt ttttattaaa catagcgtgg gatctccacg
1321 cgaatctcgg gtacgtgttc cggacatggg ctcttctccg gtagcggcgg agcttccaca
1381 tccgagccct ggtcccatgc ctccagcggc tcatggtcgc tcggcagctc cttgctccta
1441 acagtggagg ccagacttag gcacagcaca atgcccacca ccaccagtgt gccgcacaag
1501 gccgtggcgg tagggtatgt gtctgaaaat gagcgtggag attgggctcg cacggctgac
1561 gcagatggaa gacttaaggc agcggcagaa gaagatgcag gcagctgagt tgttgtattc
1621 tgataagagt cagaggtaac tcccgttgcg gtgctgttaa cggtggaggg cagtgtagtc
1681 tgagcagtac tcgttgctgc cgcgcgcgcc accagacata atagctgaca gactaacaga
1741 ctgttccttt ccatgggtct tttctgcagt caccgtcgga ccatgtgcga actcgatatt
1801 ttacacgact ctctttacca attctgcccc gaattacact taaaacgact caacagctta
1861 acgttggctt gccacgcatt acttgactgt aaaactctca ctcttaccga acttggccgt
1921 aacctgccaa ccaaagcgag aacaaaacat aacatcaaac gaatcgaccg attgttaggt
1981 aatcgtcacc tccacaaaga gcgactcgct gtataccgtt ggcatgctag ctttatctgt
2041 tcgggcaata cgatgcccat tgtacttgtt gactggtctg atattcgtga gcaaaaacga
2101 cttatggtat tgcgagcttc agtcgcacta cacggtcgtt ctgttactct ttatgagaaa
2161 gcgttcccgc tttcagagca atgttcaaag aaagctcatg accaatttct agccgacctt
2221 gcgagcattc taccgagtaa caccacaccg ctcattgtca gtgatgctgg ctttaaagtg
2281 ccatggtata aatccgttga gaagctgggt tggtactggt taagtcgagt aagaggaaaa
2341 gtacaatatg cagacctagg agcggaaaac tggaaaccta tcagcaactt acatgatatg
2401 tcatctagtc actcaaagac tttaggctat aagaggctga ctaaaagcaa tccaatctca
2461 tgccaaattc tattgtataa atctcgctct aaaggccgaa aaaatcagcg ctcgacacgg
2521 actcattgtc accacccgtc acctaaaatc tactcagcgt cggcaaagga gccatgggtt
2581 ctagcaacta acttacctgt tgaaattcga acacccaaac aacttgttaa tatctattcg
2641 aagcgaatgc agattgaaga aaccttccga gacttgaaaa gtcctgccta cggactaggc
2701 ctacgccata gccgaacgag cagctcagag cgttttgata tcatgctgct aatcgccctg
2761 atgcttcaac taacatgttg gcttgcgggc gttcatgctc agaaacaagg ttgggacaag

2821 cacttccagg ctaacacagt cagaaatcga aacgtactct caacagttcg cttaggcatg
2881 gaagttttgc ggcattctgg ctacacaata acaagggaag acttactcgt ggctgcaacc
2941 ctactagctc aaaatttatt cacacatggt tacgctttgg ggaaattatg aggggatcgc
3001 tctagagcga tccgggatct cgggaaaagc gttggtgacc aaaggtgcct tttatcatca
3061 ctttaaaaat aaaaaacaat tactcagtgc ctgttataag cagcaattaa ttatgattga
3121 tgcctacatc acaacaaaaa ctgatttaac aaatggttgg tctgccttag aaagtatatt
3181 tgaacattat cttgattata ttattgataa taataaaaac cttatcccta tccaagaagt
3241 gatgcctatc attggttgga atgaacttga aaaaaattag ccttgaatac attactggta
3301 aggtaaacgc cattgtcagc aaattgatcc aagagaacca acttaaagct ttcctgacgg
3361 aatgttaatt ctcgttgacc ctgagcactg atgaatcccc taatgatttt ggtaaaaatc
3421 attaagttaa ggtggataca catcttgtca tatgatcccg gtaatgtgag ttagctcact
3481 cattaggcac cccaggcttt acactttatg cttccggctc gtatgttgtg tggaattgtg
3541 agcggataac aatttcacac aggaaacagc tatgaccatg attacgccaa gcgcgcaatt
3601 aaccctcact aaagggaaca aaagctggag ctccaccgcg gtggcggccg ctctagaact
3661 agtggatccc ccgggcatca gattggctat tggccattgc atacgttgta tccatatcat
3721 aatatgtaca tttatattgg ctcatgtcca acattaccgc catgttgaca ttgattattg
3781 actagttatt aatagtaatc aattacgggg tcattagttc atagcccata tatggagttc
3841 cgcgttacat aacttacggt aaatggcccg cctggctgac cgcccaacga cccccgccca
3901 ttgacgtcaa taatgacgta tgttcccata gtaacgccaa tagggacttt ccattgacgt
3961 caatgggtgg agtatttacg gtaaactgcc cacttggcag tacatcaagt gtatcatatg
4021 ccaagtacgc cccctattga cgtcaatgac ggtaaatggc ccgcctggca ttatgcccag
4081 tacatgacct tatgggactt tcctacttgg cagtacatct acgtattagt catcgctatt
4141 accatggtga tgcggttttg gcagtacatc aatgggcgtg gatagcggtt tgactcacgg
4201 ggatttccaa gtctccaccc cattgacgtc aatgggagtt tgttttggca ccaaaatcaa
4261 cgggactttc caaaatgtcg taacaactcc gccccattga cgcaaatggg cggtaggcgt
4321 gtacggtggg aggtctatat aagcagagct cgtttagtga accgtcagat cgcctggaga
4381 cgccatccac gctgttttga cctccataga agacaccggg accgatccag cctccgcggc
4441 cgggaacggt gcattggaac gcggattccc cgtgccaaga gtgacgtaag taccgcctat
4501 agactctata ggcacacccc tttggctctt atgcatgcta tactgttttt ggcttggggc
4561 ctatacaccc ccgcttcctt atgctatagg tgatggtata gcttagccta taggtgtggg
4621 ttattgacca ttattgacca ctcccctatt ggtgacgata ctttccatta ctaatccata
4681 acatggctct ttgccacaac tatctctatt ggctatatgc caatactctg tccttcagag
4741 actgacacgg actctgtatt tttacaggat ggggtcccat ttattattta caaattcaca
4801 tatacaacaa cgccgtcccc cgtgcccgca gtttttatta aacatagcgt gggatctcca
4861 cgcgaatctc gggtacgtgt tccggacatg ggctcttctc cggtagcggc ggagcttcca
4921 catccgagcc ctggtcccat gcctccagcg gctcatggtc gctcggcagc tccttgctcc
4981 taacagtgga ggccagactt aggcacagca caatgcccac caccaccagt gtgccgcaca
5041 aggccgtggc ggtagggtat gtgtctgaaa atgagcgtgg agattgggct cgcacggctg
5101 acgcagatgg aagacttaag gcagcggcag aagaagatgc aggcagctga gttgttgtat
5161 tctgataaga gtcagaggta actcccgttg cggtgctgtt aacggtggag ggcagtgtag
5221 tctgagcagt actcgttgct gccgcgcgcg ccaccagaca taatagctga cagactaaca
5281 gactgttcct ttccatgggt cttttctgca gtcaccgtcg ggatccatgg gctccatcgg
5341 cgcagcaagc atggaatttt gttttgatgt attcaaggag ctcaaagtcc accatgccaa
5401 tgagaacatc ttctactgcc ccattgccat catgtcagct ctagccatgg tatacctggg
5461 tgcaaaagac agcaccagga cacagataaa taaggttgtt cgctttgata aacttccagg
5521 attcggagac agtattgaag ctcagtgtgg cacatctgta aacgttcact cttcacttag
5581 agacatcctc aaccaaatca ccaaaccaaa tgatgtttat tcgttcagcc ttgccagtag
5641 actttatgct gaagagagat acccaatcct gccagaatac ttgcagtgtg tgaaggaact
5701 gtatagagga ggcttggaac ctatcaactt tcaaacagct gcagatcaag ccagagagct
5761 catcaattcc tgggtagaaa gtcagacaaa tggaattatc agaaatgtcc ttcagccaag
5821 ctccgtggat tctcaaactg caatggttct ggttaatgcc attgtcttca aaggactgtg
5881 ggagaaaaca tttaaggatg aagacacaca agcaatgcct ttcagagtga ctgagcaaga
5941 aagcaaacct gtgcagatga tgtaccagat tggtttattt agagtggcat caatggcttc
6001 tgagaaaatg aagatcctgg agcttccatt tgccagtggg acaatgagca tgttggtgct
6061 gttgcctgat gaagtctcag gccttgagca gcttgagagt ataatcaact ttgaaaaact
6121 gactgaatgg accagttcta atgttatgga agagaggaag atcaaagtgt acttacctcg
6181 catgaagatg gaggaaaaat acaacctcac atctgtctta atggctatgg gcattactga
6241 cgtgtttagc tcttcagcca atctgtctgg catctcctca gcagagagcc tgaagatatc
6301 tcaagctgtc catgcagcac atgcagaaat caatgaagca ggcagagagg tggtagggtc
6361 agcagaggct ggagtggatg ctgcaagcgt ctctgaagaa tttagggctg accatccatt
6421 cctcttctgt atcaagcaca tcgcaaccaa cgccgttctc ttctttggca gatgtgtttc
6481 ccgcggccag cagatgacgc accagcagat gacgcaccag cagatgacgc accagcagat
6541 gacgcaccag cagatgacgc accagcagat gacgcaacaa catgtatcct gaaaggctct
6601 tgtggctgga tcggcctgct ggatgacgat gacaaatttg tgaaccaaca cctgtgcggc
6661 tcacacctgg tggaagctct ctacctagtg tgcggggaac gaggcttctt ctacacaccc
6721 aagacccgcc gggaggcaga ggacctgcag gtggggcagg tggagctggg cgggggccct
6781 ggtgcaggca gcctgcagcc cttggccctg gaggggtccc tgcagaagcg tggcattgtg
6841 gaacaatgct gtaccagcat ctgctccctc taccagctgg agaactactg caactagggc

6901 gcctaaaggg cgaattatcg cggccgctct agaccaggcg cctggatcca gatcacttct
6961 ggctaataaa agatcagagc tctagagatc tgtgtgttgg ttttttgtgg atctgctgtg
7021 ccttctagtt gccagccatc tgttgtttgc ccctcccccg tgccttcctt gaccctggaa
7081 ggtgccactc ccactgtcct ttcctaataa aatgaggaaa ttgcatcgca ttgtctgagt
7141 aggtgtcatt ctattctggg gggtggggtg gggcagcaca gcaaggggga ggattgggaa
7201 gacaatagca ggcatgctgg ggatgcggtg ggctctatgg gtacctctct ctctctctct
7261 ctctctctct ctctctctct ctctcggtac ctctctcgag ggggggcccg gtacccaatt
7321 cgccctatag tgagtcgtat tacgcgcgct cactggccgt cgttttacaa cgtcgtgact
7381 gggaaaaccc tggcgttacc caacttaatc gccttgcagc acatccccct ttcgccagct
7441 ggcgtaatag cgaagaggcc cgcaccgatc gcccttccca acagttgcgc agcctgaatg
7501 gcgaatggaa attgtaagcg ttaatatttt gttaaaattc gcgttaaatt tttgttaaat
7561 cagctcattt tttaaccaat aggccgaaat cggcaaaatc ccttataaat caaaagaata
7621 gaccgagata gggttgagtg ttgttccagt ttggaacaag agtccactat taaagaacgt
7681 ggactccaac gtcaaagggc gaaaaaccgt ctatcagggc gatggcccac tactccggga
7741 tcatatgaca agatgtgtat ccaccttaac ttaatgattt ttaccaaaat cattagggga
7801 ttcatcagtg ctcagggtca acgagaatta acattccgtc aggaaagctt atgatgatga
7861 tgtgcttaaa aacttactca atggctggtt atgcatatcg caatacatgc gaaaaaccta
7921 aaagagcttg ccgataaaaa aggccaattt attgctattt accgcggctt tttattgagc
7981 ttgaaagata aataaaatag ataggtttta tttgaagcta aatcttcttt atcgtaaaaa
8041 atgccctctt gggttatcaa gagggtcatt atatttcgcg gaataacatc atttggtgac
8101 gaaataacta agcacttgtc tcctgtttac tcccctgagc ttgaggggtt aacatgaagg
8161 tcatcgatag caggataata atacagtaaa acgctaaacc aataatccaa atccagccat
8221 cccaaattgg tagtgaatga ttataaataa cagcaaacag taatgggcca ataacaccgg
8281 ttgcattggt aaggctcacc aataatccct gtaaagcacc ttgctgatga ctctttgttt
8341 ggatagacat cactccctgt aatgcaggta aagcgatccc accaccagcc aataaaatta
8401 aaacagggaa aactaaccaa ccttcagata taaacgctaa aaaggcaaat gcactactat
8461 ctgcaataaa tccgagcagt actgccgttt tttcgcccat ttagtggcta ttcttcctgc
8521 cacaaaggct tggaatactg agtgtaaaag accaagaccc gtaatgaaaa gccaaccatc
8581 atgctattca tcatcacgat ttctgtaata gcaccacacc gtgctggatt ggctatcaat
8641 gcgctgaaat aataatcaac aaatggcatc gttaaataag tgatgtatac cgatcagctt
8701 ttgttccctt tagtgagggt taattgcgcg cttggcgtaa tcatggtcat agctgtttcc
8761 tgtgtgaaat tgttatccgc tcacaattcc acacaacata cgagccggaa gcataaagtg
8821 taaagcctgg ggtgcctaat gagtgagcta actcacatta attgcgttgc gctcactgcc
8881 cgctttccag tcgggaaacc tgtcgtgcca gctgcattaa tgaatcggcc aacgcgcggg
8941 gagaggcggt ttgcgtattg ggcgctcttc cgcttcctcg ctcactgact cgctgcgctc
9001 ggtcgttcgg ctgcggcgag cggtatcagc tcactcaaag gcggtaatac ggttatccac
9061 agaatcaggg gataacgcag gaaagaacat gtgagcaaaa ggccagcaaa aggccaggaa
9121 ccgtaaaaag gccgcgttgc tggcgttttt ccataggctc cgcccccctg acgagcatca
9181 caaaaatcga cgctcaagtc agaggtggcg aaacccgaca ggactataaa gataccaggc
9241 gtttccccct ggaagctccc tcgtgcgctc tcctgttccg accctgccgc ttaccggata
9301 cctgtccgcc tttctccctt cgggaagcgt ggcgctttct catagctcac gctgtaggta
9361 tctcagttcg gtgtaggtcg ttcgctccaa gctgggctgt gtgcacgaac cccccgttca
9421 gcccgaccgc tgcgccttat ccggtaacta tcgtcttgag tccaacccgg taagacacga
9481 cttatcgcca ctggcagcag ccactggtaa caggattagc agagcgaggt atgtaggcgg
9541 tgctacagag ttcttgaagt ggtggcctaa ctacggctac actagaagga cagtatttgg
9601 tatctgcgct ctgctgaagc cagttacctt cggaaaaaga gttggtagct cttgatccgg
9661 caaacaaacc accgctggta gcggtggttt ttttgtttgc aagcagcaga ttacgcgcag
9721 aaaaaaagga tctcaagaag atcctttgat cttttctacg gggtctgacg ctcagtggaa
9781 cgaaaactca cgttaaggga ttttggtcat gagattatca aaaaggatct tcacctagat
9841 ccttttaaat taaaaatgaa gttttaaatc aatctaaagt atatatgagt aaacttggtc
9901 tgacagttac caatgcttaa tcagtgaggc acctatctca gcgatctgtc tatttcgttc
9961 atccatagtt gcctgactcc ccgtcgtgta gataactacg atacgggagg gcttaccatc
10021 tggccccagt gctgcaatga taccgcgaga cccacgctca ccggctccag atttatcagc
10081 aataaaccag ccagccggaa gggccgagcg cagaagtggt cctgcaactt tatccgcctc
10141 catccagtct attaattgtt gccgggaagc tagagtaagt agttcgccag ttaatagttt
10201 gcgcaacgtt gttgccattg ctacaggcat cgtggtgtca cgctcgtcgt ttggtatggc
10261 ttcattcagc tccggttccc aacgatcaag gcgagttaca tgatccccca tgttgtgcaa
10321 aaaagcggtt agctccttcg gtcctccgat cgttgtcaga agtaagttgg ccgcagtgtt
10381 atcactcatg gttatggcag cactgcataa ttctcttact gtcatgccat ccgtaagatg
10441 cttttctgtg actggtgagt actcaaccaa gtcattctga gaatagtgta tgcggcgacc
10501 gagttgctct tgcccggcgt caatacggga taataccgcg ccacatagca gaactttaaa
10561 agtgctcatc attggaaaac gttcttcggg gcgaaaactc tcaaggatct taccgctgtt
10621 gagatccagt tcgatgtaac ccactcgtgc acccaactga tcttcagcat cttttacttt
10681 caccagcgtt tctgggtgag caaaaacagg aaggcaaaat gccgcaaaaa agggaataag
10741 ggcgacacgg aaatgttgaa tactcatact cttccttttt caatattatt gaagcattta
10801 tcagggttat tgtctcatga gcggatacat atttgaatgt atttagaaaa ataaacaaat
10861 aggggttccg cgcacatttc cccgaaaagt gccac

SEQ ID NO:42(pTnMOD (CMV-CHOVg-ent-ProInsulin-synPA))

1 ctgacgcgcc ctgtagcggc gcattaagcg cggcgggtgt ggtggttacg cgcagcgtga
61 ccgctacact tgccagcgcc ctagcgcccg ctcctttcgc tttcttccct tcctttctcg
121 ccacgttcgc cggcatcaga ttggctattg gccattgcat acgttgtatc catatcataa
181 tatgtacatt tatattggct catgtccaac attaccgcca tgttgacatt gattattgac
241 tagttattaa tagtaatcaa ttacggggtc attagttcat agcccatata tggagttccg
301 cgttacataa cttacggtaa atggcccgcc tggctgaccg cccaacgacc cccgcccatt
361 gacgtcaata atgacgtatg ttcccatagt aacgccaata gggactttcc attgacgtca
421 atgggtggag tatttacggt aaactgccca cttggcagta catcaagtgt atcatatgcc
481 aagtacgccc cctattgacg tcaatgacgg taaatggccc gcctggcatt atgcccagta
541 catgacctta tgggactttc ctacttggca gtacatctac gtattagtca tcgctattac
601 catggtgatg cggttttggc agtacatcaa tgggcgtgga tagcggtttg actcacgggg
661 atttccaagt ctccacccca ttgacgtcaa tgggagtttg ttttggcacc aaaatcaacg
721 ggactttcca aaatgtcgta acaactccgc cccattgacg caaatgggcg gtaggcgtgt
781 acggtgggag gtctatataa gcagagctcg tttagtgaac cgtcagatcg cctggagacg
841 ccatccacgc tgttttgacc tccatagaag acaccgggac cgatccagcc tccgcggccg
901 ggaacggtgc attggaacgc ggattccccg tgccaagagt gacgtaagta ccgcctatag
961 actctatagg cacacccctt tggctcttat gcatgctata ctgtttttgg cttggggcct
1021 atacaccccc gcttccttat gctataggtg atggtatagc ttagcctata ggtgtgggtt
1081 attgaccatt attgaccact cccctattgg tgacgatact ttccattact aatccataac
1141 atggctcttt gccacaacta tctctattgg ctatatgcca atactctgtc cttcagagac
1201 tgacacggac tctgtatttt tacaggatgg ggtcccattt attatttaca aattcacata
1261 tacaacaacg ccgtcccccg tgcccgcagt ttttattaaa catagcgtgg gatctccacg
1321 cgaatctcgg gtacgtgttc cggacatggg ctcttctccg gtagcggcgg agcttccaca
1381 tccgagccct ggtcccatgc ctccagcggc tcatggtcgc tcggcagctc cttgctccta
1441 acagtggagg ccagacttag gcacagcaca atgcccacca ccaccagtgt gccgcacaag
1501 gccgtggcgg tagggtatgt gtctgaaaat gagcgtggag attgggctcg cacggctgac
1561 gcagatggaa gacttaaggc agcggcagaa gaagatgcag gcagctgagt tgttgtattc
1621 tgataagagt cagaggtaac tcccgttgcg gtgctgttaa cggtggaggg cagtgtagtc
1681 tgagcagtac tcgttgctgc cgcgcgcgcc accagacata atagctgaca gactaacaga
1741 ctgttccttt ccatgggtct tttctgcagt caccgtcgga ccatgtgtga acttgatatt
1801 ttacatgatt ctctttacca attctgcccc gaattacact taaaacgact caacagctta
1861 acgttggctt gccacgcatt acttgactgt aaaactctca ctcttaccga acttggccgt
1921 aacctgccaa ccaaagcgag aacaaaacat aacatcaaac gaatcgaccg attgttaggt
1981 aatcgtcacc tccacaaaga gcgactcgct gtataccgtt ggcatgctag ctttatctgt
2041 tcgggcaata cgatgcccat tgtacttgtt gactggtctg atattcgtga gcaaaaacga
2101 cttatggtat tgcgagcttc agtcgcacta cacggtcgtt ctgttactct ttatgagaaa
2161 gcgttcccgc tttcagagca atgttcaaag aaagctcatg accaatttct agccgacctt
2221 gcgagcattc taccgagtaa caccacaccg ctcattgtca gtgatgctgg ctttaaagtg
2281 ccatggtata aatccgttga gaagctgggt tggtactggt taagtcgagt aagaggaaaa
2341 gtacaatatg cagacctagg agcggaaaac tggaaaccta tcagcaactt acatgatatg
2401 tcatctagtc actcaaagac tttaggctat aagaggctga ctaaaagcaa tccaatctca
2461 tgccaaattc tattgtataa atctcgctct aaaggccgaa aaaatcagcg ctcgacacgg
2521 actcattgtc accacccgtc acctaaaatc tactcagcgt cggcaaagga gccatgggtt
2581 ctagcaacta acttacctgt tgaaattcga acacccaaac aacttgttaa tatctattcg
2641 aagcgaatgc agattgaaga aaccttccga gacttgaaaa gtcctgccta cggactaggc
2701 ctacgccata gccgaacgag cagctcagag cgttttgata tcatgctgct aatcgccctg
2761 atgcttcaac taacatgttg gcttgcgggc gttcatgctc agaaacaagg ttgggacaag
2821 cacttccagg ctaacacagt cagaaatcga aacgtactct caacagttcg cttaggcatg
2881 gaagttttgc ggcattctgg ctacacaata acaagggaag acttactcgt ggctgcaacc
2941 ctactagctc aaaatttatt cacacatggt tacgctttgg ggaaattatg ataatgatcc
3001 agatcacttc tggctaataa aagatcagag ctctagagat ctgtgtgttg gttttttgtg
3061 gatctgctgt gccttctagt tgccagccat ctgttgtttg cccctccccc gtgccttcct
3121 tgaccctgga aggtgccact cccactgtcc tttcctaata aaatgaggaa attgcatcgc
3181 attgtctgag taggtgtcat tctattctgg ggggtggggt ggggcagcac agcaaggggg
3241 aggattggga agacaatagc aggcatgctg gggatgcggt gggctctatg ggtacctctc
3301 tctctctctc tctctctctc tctctctctc tctctcggta cctctctctc tctctctctc
3361 tctctctctc tctctctctc tcggtaccag gtgctgaaga attgacccgg tgaccaaagg
3421 tgccttttat catcacttta aaaataaaaa acaattactc agtgcctgtt ataagcagca
3481 attaattatg attgatgcct acatcacaac aaaaactgat ttaacaaatg gttggtctgc
3541 cttagaaagt atatttgaac attatcttga ttatattatt gataataata aaaaccttat
3601 ccctatccaa gaagtgatgc ctatcattgg ttggaatgaa cttgaaaaaa attagccttg
3661 aatacattac tggtaaggta aacgccattg tcagcaaatt gatccaagag aaccaactta
3721 aagctttcct gacggaatgt taattctcgt tgaccctgag cactgatgaa tcccctaatg
3781 attttggtaa aaatcattaa gttaaggtgg atacacatct tgtcatatga tcccggtaat
3841 gtgagttagc tcactcatta ggcaccccag gctttacact ttatgcttcc ggctcgtatg

3901 ttgtgtggaa ttgtgagcgg ataacaattt cacacaggaa acagctatga ccatgattac
3961 gccaagcgcg caattaaccc tcactaaagg gaacaaaagc tggagctcca ccgcggtggc
4021 ggccgctcta gaactagtgg atcccccggg catcagattg gctattggcc attgcatacg
4081 ttgtatccat atcataatat gtacatttat attggctcat gtccaacatt accgccatgt
4141 tgacattgat tattgactag ttattaatag taatcaatta cggggtcatt agttcatagc
4201 ccatatatgg agttccgcgt tacataactt acggtaaatg gcccgcctgg ctgaccgccc
4261 aacgaccccc gcccattgac gtcaataatg acgtatgttc ccatagtaac gccaataggg
4321 actttccatt gacgtcaatg ggtggagtat ttacggtaaa ctgcccactt ggcagtacat
4381 caagtgtatc atatgccaag tacgccccct attgacgtca atgacggtaa atggcccgcc
4441 tggcattatg cccagtacat gaccttatgg gactttccta cttggcagta catctacgta
4501 ttagtcatcg ctattaccat ggtgatgcgg ttttggcagt acatcaatgg gcgtggatag
4561 cggtttgact cacggggatt tccaagtctc caccccattg acgtcaatgg gagtttgttt
4621 tggcaccaaa atcaacggga ctttccaaaa tgtcgtaaca actccgcccc attgacgcaa
4681 atgggcggta ggcgtgtacg gtgggaggtc tatataagca gagctcgttt agtgaaccgt
4741 cagatcgcct ggagacgcca tccacgctgt tttgacctcc atagaagaca ccgggaccga
4801 tccagcctcc gcggccggga acggtgcatt ggaacgcgga ttccccgtgc caagagtgac
4861 gtaagtaccg cctatagact ctataggcac acccctttgg ctcttatgca tgctatactg
4921 tttttggctt ggggcctata cacccccgct tccttatgct ataggtgatg gtatagctta
4981 gcctataggt gtgggttatt gaccattatt gaccactccc ctattggtga cgatactttc
5041 cattactaat ccataacatg gctctttgcc acaactatct ctattggcta tatgccaata
5101 ctctgtcctt cagagactga cacggactct gtatttttac aggatggggt cccatttatt
5161 atttacaaat tcacatatac aacaacgccg tcccccgtgc ccgcagtttt tattaaacat
5221 agcgtgggat ctccacgcga atctcgggta cgtgttccgg acatgggctc ttctccggta
5281 gcggcggagc ttccacatcc gagccctggt cccatgcctc cagcggctca tggtcgctcg
5341 gcagctcctt gctcctaaca gtggaggcca gacttaggca cagcacaatg cccaccacca
5401 ccagtgtgcc gcacaaggcc gtggcggtag ggtatgtgtc tgaaaatgag cgtggagatt
5461 gggctcgcac ggctgacgca gatggaagac ttaaggcagc ggcagaagaa gatgcaggca
5521 gctgagttgt tgtattctga taagagtcag aggtaactcc cgttgcggtg ctgttaacgg
5581 tggagggcag tgtagtctga gcagtactcg ttgctgccgc gcgcgccacc agacataata
5641 gctgacagac taacagactg ttcctttcca tgggtctttt ctgcagtcac cgtcgggatc
5701 catgggctcc atcggcgcag caagcatgga attttgtttt gatgtattca aggagctcaa
5761 agtccaccat gccaatgaga acatcttcta ctgccccatt gccatcatgt cagctctagc
5821 catggtatac ctgggtgcaa aagacagcac caggacacag ataaataagg ttgttcgctt
5881 tgataaactt ccaggattcg gagacagtat tgaagctcag tgtggcacat ctgtaaacgt
5941 tcactcttca cttagagaca tcctcaacca aatcaccaaa ccaaatgatg tttattcgtt
6001 cagccttgcc agtagacttt atgctgaaga gagataccca atcctgccag aatacttgca
6061 gtgtgtgaag gaactgtata gaggaggctt ggaacctatc aactttcaaa cagctgcaga
6121 tcaagccaga gagctcatca attcctgggt agaaagtcag acaaatggaa ttatcagaaa
6181 tgtccttcag ccaagctccg tggattctca aactgcaatg gttctggtta atgccattgt
6241 cttcaaagga ctgtgggaga aaacatttaa ggatgaagac acacaagcaa tgcctttcag
6301 agtgactgag caagaaagca aacctgtgca gatgatgtac cagattggtt tatttagagt
6361 ggcatcaatg gcttctgaga aaatgaagat cctggagctt ccatttgcca gtgggacaat
6421 gagcatgttg gtgctgttgc ctgatgaagt ctcaggcctt gagcagcttg agagtataat
6481 caactttgaa aaactgactg aatggaccag ttctaatgtt atggaagaga ggaagatcaa
6541 agtgtactta cctcgcatga agatggagga aaaatacaac ctcacatctg tcttaatggc
6601 tatgggcatt actgacgtgt ttagctcttc agccaatctg tctggcatct cctcagcaga
6661 gagcctgaag atatctcaag ctgtccatgc agcacatgca gaaatcaatg aagcaggcag
6721 agaggtggta gggtcagcag aggctggagt ggatgctgca agcgtctctg aagaatttag
6781 ggctgaccat ccattcctct tctgtatcaa gcacatcgca accaacgccg ttctcttctt
6841 tggcagatgt gtttcccgcg gccagcagat gacgcaccag cagatgacgc accagcagat
6901 gacgcaccag cagatgacgc accagcagat gacgcaccag cagatgacgc aacaacatgt
6961 atcctgaaag gctcttgtgg ctggatcggc ctgctggatg acgatgacaa atttgtgaac
7021 caacacctgt gcggctcaca cctggtggaa gctctctacc tagtgtgcgg ggaacgaggc
7081 ttcttctaca cacccaagac ccgccgggag gcagaggacc tgcaggtggg gcaggtggag
7141 ctgggcgggg gccctggtgc aggcagcctg cagcccttgg ccctggaggg gtccctgcag
7201 aagcgtggca ttgtggaaca atgctgtacc agcatctgct ccctctacca gctggagaac
7261 tactgcaact agggcgccta aagggcgaat tatcgcggcc gctctagacc aggcgcctgg
7321 atccagatca cttctggcta ataaaagatc agagctctag agatctgtgt gttggttttt
7381 tgtggatctg ctgtgccttc tagttgccag ccatctgttg tttgcccctc ccccgtgcct
7441 tccttgaccc tggaaggtgc cactcccact gtcctttcct aataaaatga ggaaattgca
7501 tcgcattgtc tgagtaggtg tcattctatt ctggggggtg gggtggggca gcacagcaag
7561 ggggaggatt gggaagacaa tagcaggcat gctggggatg cggtgggctc tatgggtacc
7621 tctctctctc tctctctctc tctctctctc tctctctctc ggtacctctc ctcgaggggg
7681 ggcccggtac ccaattcgcc ctatagtgag tcgtattacg cgcgctcact ggccgtcgtt
7741 ttacaacgtc gtgactggga aaaccctggc gttacccaac ttaatcgcct tgcagcacat
7801 ccccctttcg ccagctggcg taatagcgaa gaggcccgca ccgatcgccc ttcccaacag
7861 ttgcgcagcc tgaatggcga atggaaattg taagcgttaa tattttgtta aaattcgcgt
7921 taaatttttg ttaaatcagc tcatttttta accaataggc cgaaatcggc aaaatccctt

7981 ataaatcaaa agaatagacc gagatagggt tgagtgttgt tccagtttgg aacaagagtc
8041 cactattaaa gaacgtggac tccaacgtca aagggcgaaa aaccgtctat cagggcgatg
8101 gcccactact ccgggatcat atgacaagat gtgtatccac cttaacttaa tgatttttac
8161 caaaatcatt aggggattca tcagtgctca gggtcaacga gaattaacat tccgtcagga
8221 aagcttatga tgatgatgtg cttaaaaact tactcaatgg ctggttatgc atatcgcaat
8281 acatgcgaaa aacctaaaag agcttgccga taaaaaaggc caatttattg ctatttaccg
8341 cggcttttta ttgagcttga aagataaata aaatagatag gttttatttg aagctaaatc
8401 ttctttatcg taaaaaatgc cctcttgggt tatcaagagg gtcattatat ttcgcggaat
8461 aacatcattt ggtgacgaaa taactaagca cttgtctcct gtttactccc ctgagcttga
8521 ggggttaaca tgaaggtcat cgatagcagg ataataatac agtaaaacgc taaaccaata
8581 atccaaatcc agccatccca aattggtagt gaatgattat aaataacagc aaacagtaat
8641 gggccaataa caccggttgc attggtaagg ctcaccaata atccctgtaa agcaccttgc
8701 tgatgactct ttgtttggat agacatcact ccctgtaatg caggtaaagc gatcccacca
8761 ccagccaata aaattaaaac agggaaaact aaccaacctt cagatataaa cgctaaaaag
8821 gcaaatgcac tactatctgc aataaatccg agcagtactg ccgttttttc gcccatttag
8881 tggctattct tcctgccaca aaggcttgga atactgagtg taaaagacca agacccgtaa
8941 tgaaaagcca accatcatgc tattcatcat cacgatttct gtaatagcac cacaccgtgc
9001 tggattggct atcaatgcgc tgaaataata atcaacaaat ggcatcgtta aataagtgat
9061 gtataccgat cagcttttgt tccctttagt gagggttaat tgcgcgcttg gcgtaatcat
9121 ggtcatagct gtttcctgtg tgaaattgtt atccgctcac aattccacac aacatacgag
9181 ccggaagcat aaagtgtaaa gcctggggtg cctaatgagt gagctaactc acattaattg
9241 cgttgcgctc actgcccgct ttccagtcgg gaaacctgtc gtgccagctg cattaatgaa
9301 tcggccaacg cgcggggaga ggcggtttgc gtattgggcg ctcttccgct tcctcgctca
9361 ctgactcgct gcgctcggtc gttcggctgc ggcgagcggt atcagctcac tcaaaggcgg
9421 taatacggtt atccacagaa tcaggggata acgcaggaaa gaacatgtga gcaaaaggcc
9481 agcaaaaggc caggaaccgt aaaaaggccg cgttgctggc gtttttccat aggctccgcc
9541 cccctgacga gcatcacaaa aatcgacgct caagtcagag gtggcgaaac ccgacaggac
9601 tataaagata ccaggcgttt ccccctggaa gctccctcgt gcgctctcct gttccgaccc
9661 tgccgcttac cggatacctg tccgcctttc tcccttcggg aagcgtggcg ctttctcata
9721 gctcacgctg taggtatctc agttcggtgt aggtcgttcg ctccaagctg ggctgtgtgc
9781 acgaaccccc cgttcagccc gaccgctgcg ccttatccgg taactatcgt cttgagtcca
9841 acccggtaag acacgactta tcgccactgg cagcagccac tggtaacagg attagcagag
9901 cgaggtatgt aggcggtgct acagagttct tgaagtggtg gcctaactac ggctacacta
9961 gaaggacagt atttggtatc tgcgctctgc tgaagccagt taccttcgga aaaagagttg
10021 gtagctcttg atccggcaaa caaaccaccg ctggtagcgg tggttttttt gtttgcaagc
10081 agcagattac gcgcagaaaa aaaggatctc aagaagatcc tttgatcttt tctacggggt
10141 ctgacgctca gtggaacgaa aactcacgtt aagggatttt ggtcatgaga ttatcaaaaa
10201 ggatcttcac ctagatcctt ttaaattaaa aatgaagttt taaatcaatc taaagtatat
10261 atgagtaaac ttggtctgac agttaccaat gcttaatcag tgaggcacct atctcagcga
10321 tctgtctatt tcgttcatcc atagttgcct gactccccgt cgtgtagata actacgatac
10381 gggagggctt accatctggc cccagtgctg caatgatacc gcgagaccca cgctcaccgg
10441 ctccagattt atcagcaata aaccagccag ccggaagggc cgagcgcaga agtggtcctg
10501 caactttatc cgcctccatc cagtctatta attgttgccg ggaagctaga gtaagtagtt
10561 cgccagttaa tagtttgcgc aacgttgttg ccattgctac aggcatcgtg gtgtcacgct
10621 cgtcgtttgg tatggcttca ttcagctccg gttcccaacg atcaaggcga gttacatgat
10681 cccccatgtt gtgcaaaaaa gcggttagct ccttcggtcc tccgatcgtt gtcagaagta
10741 agttggccgc agtgttatca ctcatggtta tggcagcact gcataattct cttactgtca
10801 tgccatccgt aagatgcttt tctgtgactg gtgagtactc aaccaagtca ttctgagaat
10861 agtgtatgcg gcgaccgagt tgctcttgcc cggcgtcaat acgggataat accgcgccac
10921 atagcagaac tttaaaagtg ctcatcattg gaaaacgttc ttcggggcga aaactctcaa
10981 ggatcttacc gctgttgaga tccagttcga tgtaacccac tcgtgcaccc aactgatctt
11041 cagcatcttt tactttcacc agcgtttctg ggtgagcaaa aacaggaagg caaaatgccg
11101 caaaaaaggg aataagggcg acacggaaat gttgaatact catactcttc ctttttcaat
11161 attattgaag catttatcag ggttattgtc tcatgagcgg atacatattt gaatgtattt
11221 agaaaaataa acaaataggg gttccgcgca catttccccg aaaagtgcca c

SEQ ID NO:43 (pTnMOD(Chicken OVep+OVg'+ENT+proins+syn polyA))

1 ctgacgcgcc ctgtagcggc gcattaagcg cggcgggtgt ggtggttacg cgcagcgtga
61 ccgctacact tgccagcgcc ctagcgcccg ctcctttcgc tttcttccct tcctttctcg
121 ccacgttcgc cggcatcaga ttggctattg gccattgcat acgttgtatc catatcataa
181 tatgtacatt tatattggct catgtccaac attaccgcca tgttgacatt gattattgac
241 tagttattaa tagtaatcaa ttacggggtc attagttcat agcccatata tggagttccg
301 cgttacataa cttacggtaa atggcccgcc tggctgaccg cccaacgacc cccgcccatt
361 gacgtcaata atgacgtatg ttcccatagt aacgccaata gggactttcc attgacgtca

421 atgggtggag tatttacggt aaactgccca cttggcagta catcaagtgt atcatatgcc
481 aagtacgccc cctattgacg tcaatgacgg taaatggccc gcctggcatt atgcccagta
541 catgacctta tgggactttc ctacttggca gtacatctac gtattagtca tcgctattac
601 catggtgatg cggttttggc agtacatcaa tgggcgtgga tagcggtttg actcacgggg
661 atttccaagt ctccacccca ttgacgtcaa tgggagtttg ttttggcacc aaaatcaacg
721 ggactttcca aaatgtcgta acaactccgc cccattgacg caaatgggcg gtaggcgtgt
781 acggtgggag gtctatataa gcagagctcg tttagtgaac cgtcagatcg cctggagacg
841 ccatccacgc tgttttgacc tccatagaag acaccgggac cgatccagcc tccgcggccg
901 ggaacggtgc attggaacgc ggattccccg tgccaagagt gacgtaagta ccgcctatag
961 actctatagg cacacccctt tggctcttat gcatgctata ctgtttttgg cttggggcct
1021 atacaccccc gcttccttat gctataggtg atggtatagc ttagcctata ggtgtgggtt
1081 attgaccatt attgaccact cccctattgg tgacgatact ttccattact aatccataac
1141 atggctcttt gccacaacta tctctattgg ctatatgcca atactctgtc cttcagagac
1201 tgacacggac tctgtatttt tacaggatgg ggtcccattt attatttaca aattcacata
1261 tacaacaacg ccgtcccccg tgcccgcagt ttttattaaa catagcgtgg gatctccacg
1321 cgaatctcgg gtacgtgttc cggacatggg ctcttctccg gtagcggcgg agcttccaca
1381 tccgagccct ggtcccatgc ctccagcggc tcatggtcgc tcggcagctc cttgctccta
1441 acagtggagg ccagacttag gcacagcaca atgcccacca ccaccagtgt gccgcacaag
1501 gccgtggcgg tagggtatgt gtctgaaaat gagcgtggag attgggctcg cacggctgac
1561 gcagatggaa gacttaaggc agcggcagaa gaagatgcag gcagctgagt tgttgtattc
1621 tgataagagt cagaggtaac tcccgttgcg gtgctgttaa cggtggaggg cagtgtagtc
1681 tgagcagtac tcgttgctgc cgcgcgcgcc accagacata atagctgaca gactaacaga
1741 ctgttccttt ccatgggtct tttctgcagt caccgtcgga ccatgtgcga actcgatatt
1801 ttacacgact ctctttacca attctgcccc gaattacact taaaacgact caacagctta
1861 acgttggctt gccacgcatt acttgactgt aaaactctca ctcttaccga acttggccgt
1921 aacctgccaa ccaaagcgag aacaaaacat aacatcaaac gaatcgaccg attgttaggt
1981 aatcgtcacc tccacaaaga gcgactcgct gtataccgtt ggcatgctag ctttatctgt
2041 tcgggcaata cgatgcccat tgtacttgtt gactggtctg atattcgtga gcaaaaacga
2101 cttatggtat tgcgagcttc agtcgcacta cacggtcgtt ctgttactct ttatgagaaa
2161 gcgttcccgc tttcagagca atgttcaaag aaagctcatg accaatttct agccgacctt
2221 gcgagcattc taccgagtaa caccacaccg ctcattgtca gtgatgctgg ctttaaagtg
2281 ccatggtata aatccgttga gaagctgggt tggtactggt taagtcgagt aagaggaaaa
2341 gtacaatatg cagacctagg agcggaaaac tggaaaccta tcagcaactt acatgatatg
2401 tcatctagtc actcaaagac tttaggctat aagaggctga ctaaaagcaa tccaatctca
2461 tgccaaattc tattgtataa atctcgctct aaaggccgaa aaaatcagcg ctcgacacgg
2521 actcattgtc accacccgtc acctaaaatc tactcagcgt cggcaaagga gccatgggtt
2581 ctagcaacta acttacctgt tgaaattcga acacccaaac aacttgttaa tatctattcg
2641 aagcgaatgc agattgaaga aaccttccga gacttgaaaa gtcctgccta cggactaggc
2701 ctacgccata gccgaacgag cagctcagag cgttttgata tcatgctgct aatcgccctg
2761 atgcttcaac taacatgttg gcttgcgggc gttcatgctc agaaacaagg ttgggacaag
2821 cacttccagg ctaacacagt cagaaatcga aacgtactct caacagttcg cttaggcatg
2881 gaagttttgc ggcattctgg ctacacaata acaagggaag acttactcgt ggctgcaacc
2941 ctactagctc aaaatttatt cacacatggt tacgctttgg ggaaattatg aggggatcgc
3001 tctagagcga tccgggatct cgggaaaagc gttggtgacc aaaggtgcct tttatcatca
3061 ctttaaaaat aaaaaacaat tactcagtgc ctgttataag cagcaattaa ttatgattga
3121 tgcctacatc acaacaaaaa ctgatttaac aaatggttgg tctgccttag aaagtatatt
3181 tgaacattat cttgattata ttattgataa taataaaaac cttatcccta tccaagaagt
3241 gatgcctatc attggttgga atgaacttga aaaaaattag ccttgaatac attactggta
3301 aggtaaacgc cattgtcagc aaattgatcc aagagaacca acttaaagct ttcctgacgg
3361 aatgttaatt ctcgttgacc ctgagcactg atgaatcccc taatgatttt ggtaaaaatc
3421 attaagttaa ggtggataca catcttgtca tatgatcccg gtaatgtgag ttagctcact
3481 cattaggcac cccaggcttt acactttatg cttccggctc gtatgttgtg tggaattgtg
3541 agcggataac aatttcacac aggaaacagc tatgaccatg attacgccaa gcgcgcaatt
3601 aaccctcact aaagggaaca aaagctggag ctccaccgcg gtggcggccg ctctagaact
3661 agtggatccc ccgggctgca gaaaaatgcc aggtggacta tgaactcaca tccaaaggag
3721 cttgacctga tacctgattt tcttcaaact ggggaaacaa cacaatccca caaaacagct
3781 cagagagaaa ccatcactga tggctacagc accaaggtat gcaatggcaa tccattcgac
3841 attcatctgt gacctgagca aaatgattta tctctccatg aatggttgct tctttccctc
3901 atgaaaaggc aatttccaca ctcacaatat gcaacaaaga caaacagaga acaattaatg
3961 tgctccttcc taatgtcaaa attgtagtgg caaagaggag aacaaaatct caagttctga
4021 gtaggtttta gtgattggat aagaggcttt gacctgtgag ctcacctgga cttcatatcc
4081 ttttggataa aaagtgcttt tataactttc aggtctccga gtctttattc atgagactgt
4141 tggtttaggg acagacccac aatgaaatgc ctggcatagg aaagggcagc agagccttag
4201 ctgacctttt cttgggacaa gcattgtcaa acaatgtgtg acaaaactat ttgtactgct
4261 ttgcacagct gtgctgggca gggcaatcca ttgccaccta tcccaggtaa ccttccaact
4321 gcaagaagat tgttgcttac tctctctaga aagcttctgc agactgacat gcatttcata
4381 ggtagagata acatttactg ggaagcacat ctatcatcat aaaaagcagg caagattttc
4441 agactttctt agtggctgaa atagaagcaa aagacgtgat taaaaacaaa atgaaacaaa

4501 aaaaatcagt tgatacctgt ggtgtagaca tccagcaaaa aaatattatt tgcactacca
4561 tcttgtctta agtcctcaga cttggcaagg agaatgtaga tttctacagt atatatgttt
4621 tcacaaaagg aaggagagaa acaaaagaaa atggcactga ctaaacttca gctagtggta
4681 taggaaagta attctgctta acagagattg cagtgatctc tatgtatgtc ctgaagaatt
4741 atgttgtact tttttccccc atttttaaat caaacagtgc tttacagagg tcagaatggt
4801 ttctttactg tttgtcaatt ctattatttc aatacagaac aatagcttct ataactgaaa
4861 tatatttgct attgtatatt atgattgtcc ctcgaaccat gaacactcct ccagctgaat
4921 ttcacaattc ctctgtcatc tgccaggcca ttaagttatt catggaagat ctttgaggaa
4981 cactgcaagt tcatatcata aacacatttg aaattgagta ttgttttgca ttgtatggag
5041 ctatgttttg ctgtatcctc agaaaaaaag tttgttataa agcattcaca cccataaaaa
5101 gatagattta aatattccag ctataggaaa gaaagtgcgt ctgctcttca ctctagtctc
5161 agttggctcc ttcacatgca tgcttcttta tttctcctat tttgtcaaga aaataatagg
5221 tcacgtcttg ttctcactta tgtcctgcct agcatggctc agatgcacgt tgtagataca
5281 agaaggatca aatgaaacag acttctggtc tgttactaca accatagtaa taagcacact
5341 aactaataat tgctaattat gttttccatc tctaaggttc ccacattttt ctgttttctt
5401 aaagatccca ttatctggtt gtaactgaag ctcaatggaa catgagcaat atttcccagt
5461 cttctctccc atccaacagt cctgatggat tagcagaaca ggcagaaaac acattgttac
5521 ccagaattaa aaactaatat ttgctctcca ttcaatccaa aatggaccta ttgaaactaa
5581 aatctaaccc aatcccatta aatgatttct atggcgtcaa aggtcaaact tctgaaggga
5641 acctgtgggt gggtcacaat tcaggctata tattccccag ggctcagcca gtggatcaac
5701 atacagctag aaagctgtat tgcctttagc actcaagctc aaaagacaac tcagagttca
5761 ccatgggctc catcggcgca gcaagcatgg aattttgttt tgatgtattc aaggagctca
5821 aagtccacca tgccaatgag aacatcttct actgccccat tgccatcatg tcagctctag
5881 ccatggtata cctgggtgca aaagacagca ccaggacaca gataaataag gttgttcgct
5941 ttgataaact tccaggattc ggagacagta ttgaagctca gtgtggcaca tctgtaaacg
6001 ttcactcttc acttagagac atcctcaacc aaatcaccaa accaaatgat gtttattcgt
6061 tcagccttgc cagtagactt tatgctgaag agagataccc aatcctgcca gaatacttgc
6121 agtgtgtgaa ggaactgtat agaggaggct tggaacctat caactttcaa acagctgcag
6181 atcaagccag agagctcatc aattcctggg tagaaagtca gacaaatgga attatcagaa
6241 atgtccttca gccaagctcc gtggattctc aaactgcaat ggttctggtt aatgccattg
6301 tcttcaaagg actgtgggag aaaacattta aggatgaaga cacacaagca atgcctttca
6361 gagtgactga gcaagaaagc aaacctgtgc agatgatgta ccagattggt ttatttagag
6421 tggcatcaat ggcttctgag aaaatgaaga tcctggagct tccatttgcc agtgggacaa
6481 tgagcatgtt ggtgctgttg cctgatgaag tctcaggcct tgagcagctt gagagtataa
6541 tcaactttga aaaactgact gaatggacca gttctaatgt tatggaagag aggaagatca
6601 aagtgtactt acctcgcatg aagatggagg aaaaatacaa cctcacatct gtcttaatgg
6661 ctatgggcat tactgacgtg tttagctctt cagccaatct gtctggcatc tcctcagcag
6721 agagcctgaa gatatctcaa gctgtccatg cagcacatgc agaaatcaat gaagcaggca
6781 gagaggtggt agggtcagca gaggctggag tggatgctgc aagcgtctct gaagaattta
6841 gggctgacca tccattcctc ttctgtatca agcacatcgc aaccaacgcc gttctcttct
6901 ttggcagatg tgtttctccg cggccagcag atgacgcacc agcagatgac gcaccagcag
6961 atgacgcacc agcagatgac gcaccagcag atgacgcacc agcagatgac gcaacaacat
7021 gtatcctgaa aggctcttgt ggctggatcg gcctgctgga tgacgatgac aaatttgtga
7081 accaacacct gtgcggctca cacctggtgg aagctctcta cctagtgtgc ggggaacgag
7141 gcttcttcta cacacccaag acccgccggg aggcagagga cctgcaggtg gggcaggtgg
7201 agctgggcgg gggccctggt gcaggcagcc tgcagccctt ggccctggag gggtccctgc
7261 agaagcgtgg cattgtggaa caatgctgta ccagcatctg ctccctctac cagctggaga
7321 actactgcaa ctagggcgcc taaagggcga attatcgcgg ccgctctaga ccaggcgcct
7381 ggatccagat cacttctggc taataaaaga tcagagctct agagatctgt gtgttggttt
7441 tttgtggatc tgctgtgcct tctagttgcc agccatctgt tgtttgcccc tcccccgtgc
7501 cttccttgac cctggaaggt gccactccca ctgtcctttc ctaataaaat gaggaaattg
7561 catcgcattg tctgagtagg tgtcattcta ttctgggggg tggggtgggg cagcacagca
7621 agggggagga ttgggaagac aatagcaggc atgctgggga tgcggtgggc tctatgggta
7681 cctctctctc tctctctctc tctctctctc tctctctctc tcggtacctc tctcgagggg
7741 gggcccggta cccaattcgc cctatagtga gtcgtattac gcgcgctcac tggccgtcgt
7801 tttacaacgt cgtgactggg aaaaccctgg cgttacccaa cttaatcgcc ttgcagcaca
7861 tccccctttc gccagctggc gtaatagcga agaggcccgc accgatcgcc cttcccaaca
7921 gttgcgcagc ctgaatggcg aatggaaatt gtaagcgtta atattttgtt aaaattcgcg
7981 ttaaattttt gttaaatcag ctcatttttt aaccaatagg ccgaaatcgg caaaatccct
8041 tataaatcaa aagaatagac cgagataggg ttgagtgttg ttccagtttg gaacaagagt
8101 ccactattaa agaacgtgga ctccaacgtc aaagggcgaa aaaccgtcta tcagggcgat
8161 ggcccactac tccgggatca tatgacaaga tgtgtatcca ccttaactta atgattttta
8221 ccaaaatcat taggggattc atcagtgctc agggtcaacg agaattaaca ttccgtcagg
8281 aaagcttatg atgatgatgt gcttaaaaac ttactcaatg gctggttatg catatcgcaa
8341 tacatgcgaa aaacctaaaa gagcttgccg ataaaaaagg ccaatttatt gctatttacc
8401 gcggcttttt attgagcttg aaagataaat aaaatagata ggttttattt gaagctaaat
8461 cttctttatc gtaaaaaatg ccctcttggg ttatcaagag ggtcattata tttcgcggaa
8521 taacatcatt tggtgacgaa ataactaagc acttgtctcc tgtttactcc cctgagcttg

8581 aggggttaac atgaaggtca tcgatagcag gataataata cagtaaaacg ctaaaccaat
8641 aatccaaatc cagccatccc aaattggtag tgaatgatta taaataacag caaacagtaa
8701 tgggccaata acaccggttg cattggtaag gctcaccaat aatccctgta aagcaccttg
8761 ctgatgactc tttgtttgga tagacatcac tccctgtaat gcaggtaaag cgatcccacc
8821 accagccaat aaaattaaaa cagggaaaac taaccaacct tcagatataa acgctaaaaa
8881 ggcaaatgca ctactatctg caataaatcc gagcagtact gccgtttttt cgcccattta
8941 gtggctattc ttcctgccac aaaggcttgg aatactgagt gtaaaagacc aagacccgta
9001 atgaaaagcc aaccatcatg ctattcatca tcacgatttc tgtaatagca ccacaccgtg
9061 ctggattggc tatcaatgcg ctgaaataat aatcaacaaa tggcatcgtt aaataagtga
9121 tgtataccga tcagcttttg ttccctttag tgagggttaa ttgcgcgctt ggcgtaatca
9181 tggtcatagc tgtttcctgt gtgaaattgt tatccgctca caattccaca caacatacga
9241 gccggaagca taaagtgtaa agcctggggt gcctaatgag tgagctaact cacattaatt
9301 gcgttgcgct cactgcccgc tttccagtcg ggaaacctgt cgtgccagct gcattaatga
9361 atcggccaac gcgcggggag aggcggtttg cgtattgggc gctcttccgc ttcctcgctc
9421 actgactcgc tgcgctcggt cgttcggctg cggcgagcgg tatcagctca ctcaaaggcg
9481 gtaatacggt tatccacaga atcaggggat aacgcaggaa agaacatgtg agcaaaaggc
9541 cagcaaaagg ccaggaaccg taaaaaggcc gcgttgctgg cgtttttcca taggctccgc
9601 ccccctgacg agcatcacaa aaatcgacgc tcaagtcaga ggtggcgaaa cccgacagga
9661 ctataaagat accaggcgtt tccccctgga agctccctcg tgcgctctcc tgttccgacc
9721 ctgccgctta ccggatacct gtccgccttt ctcccttcgg gaagcgtggc gctttctcat
9781 agctcacgct gtaggtatct cagttcggtg taggtcgttc gctccaagct gggctgtgtg
9841 cacgaacccc ccgttcagcc cgaccgctgc gccttatccg gtaactatcg tcttgagtcc
9901 aacccggtaa gacacgactt atcgccactg gcagcagcca ctggtaacag gattagcaga
9961 gcgaggtatg taggcggtgc tacagagttc ttgaagtggt ggcctaacta cggctacact
10021 agaaggacag tatttggtat ctgcgctctg ctgaagccag ttaccttcgg aaaaagagtt
10081 ggtagctctt gatccggcaa acaaaccacc gctggtagcg gtggtttttt tgtttgcaag
10141 cagcagatta cgcgcagaaa aaaaggatct caagaagatc ctttgatctt ttctacgggg
10201 tctgacgctc agtggaacga aaactcacgt taagggattt tggtcatgag attatcaaaa
10261 aggatcttca cctagatcct tttaaattaa aaatgaagtt ttaaatcaat ctaaagtata
10321 tatgagtaaa cttggtctga cagttaccaa tgcttaatca gtgaggcacc tatctcagcg
10381 atctgtctat ttcgttcatc catagttgcc tgactccccg tcgtgtagat aactacgata
10441 cgggagggct taccatctgg ccccagtgct gcaatgatac cgcgagaccc acgctcaccg
10501 gctccagatt tatcagcaat aaaccagcca gccggaaggg ccgagcgcag aagtggtcct
10561 gcaactttat ccgcctccat ccagtctatt aattgttgcc gggaagctag agtaagtagt
10621 tcgccagtta atagtttgcg caacgttgtt gccattgcta caggcatcgt ggtgtcacgc
10681 tcgtcgtttg gtatggcttc attcagctcc ggttcccaac gatcaaggcg agttacatga
10741 tcccccatgt tgtgcaaaaa agcggttagc tccttcggtc ctccgatcgt tgtcagaagt
10801 aagttggccg cagtgttatc actcatggtt atggcagcac tgcataattc tcttactgtc
10861 atgccatccg taagatgctt ttctgtgact ggtgagtact caaccaagtc attctgagaa
10921 tagtgtatgc ggcgaccgag ttgctcttgc ccggcgtcaa tacgggataa taccgcgcca
10981 catagcagaa ctttaaaagt gctcatcatt ggaaaacgtt cttcggggcg aaaactctca
11041 aggatcttac cgctgttgag atccagttcg atgtaaccca ctcgtgcacc caactgatct
11101 tcagcatctt ttactttcac cagcgtttct gggtgagcaa aaacaggaag gcaaaatgcc
11161 gcaaaaaagg gaataagggc gacacggaaa tgttgaatac tcatactctt cctttttcaa
11221 tattattgaa gcatttatca gggttattgt ctcatgagcg gatacatatt tgaatgtatt
11281 tagaaaaata aacaaatagg ggttccgcgc acatttcccc gaaaagtgcc ac

## Claims

1. A vector comprising:
a) a modified transposase gene operably linked to a first promoter, wherein the nucleic acid sequence 3' to the first promoter comprises the sequence as set forth in SEQ ID NO: 13, wherein SEQ ID NO: 13 contains the Kozak sequence and a start codon for the transposase, and wherein at least one of the first twenty codons of the transposase gene are modified from the wild-type sequence by changing a nucleotide at a third base position of the codon to an adenine or thymine without modifying the amino acid encoded by the codon, and
b) one or more genes of interest operably-linked to one or more additional promoters, and wherein the one or more genes of interest and their operably-linked promoters are flanked by transposase insertion sequences recognized by the transposase encoded by the modified transposase gene, wherein the promoter which is operably linked to the gene of interest is selected from the group consisting of an ovalbumin promoter, a conalbumin promoter, a vitellogenin promoter or an ovomucoid promoter.

2. The vector of claim 1, wherein the modified transposase gene comprises an adenine or thymine at the third position in each of codons 2-10 of the modified transposase gene.

3. The vector of claim 1, comprising the sequence as set forth in SEQ ID NO: 1.

4. The vector of claim 1, wherein the transposase is a Tn10 transposase.

5. The vector of claim 1 or claim 4, wherein the first promoter is selected from the group consisting of a constitutive promoter and an inducible promoter.

6. The vector of claim 5 wherein the inducible promoter is selected from the group consisting of an ovalbumin, promoter, a conalbumin promoter, a vitellogenin promoter or an ovomucoid promoter.

7. The vector of claim 1, further comprising a polyA sequence operably linked to the transposase gene.

8. The vector of claim 7, wherein the polyA sequence is a conalbumin polyA sequence.

9. The vector of claim 1 or claim 7 further comprising two stop codons operably-linked to the transposase gene.

10. The vector of claim 1, wherein a first gene of interest is operably-linked to a second promoter and a second gene of interest is operably-linked to a third promoter.

11. The vector of claim 1, wherein a first and a second gene of interest are operably-linked to a second promoter.

12. The vector of claim 1, further comprising an enhancer operably-linked to the one or more genes of interest.

13. The vector of claim 12 wherein the enhancer comprises at least a portion of an ovalbumin enhancer.

14. The vector of claim 1, further comprising an egg directing sequence operably-linked to the one or more genes of interest.

15. The vector of claim 14 wherein the egg directing sequence is an ovalbumin signal sequence, an ovomucoid signal sequence or a vitellogenin targeting sequence.

16. Use of a vector according to any one of claims 1-15 for producing a non-human transgenic animal.

17. The use of claim 16, wherein the vector is administered via an intratesticular, intraarterial, intraoviductal or intraembryonic route.

18. The use of claim 16, wherein the animal is an avian animal.

19. The use of claim 18, wherein the avian animal is a chicken or a quail.

20. Use of a vector according to any of the claims 1-15 to be administered to an animal for producing a desired protein.

21. The use of claim 20, wherein the animal is an egg-laying animal, and the administration is intraoviductal, such that the desired protein produced by the at least one gene of interest is isolated from the egg white of eggs laid by the egg-laying animal.

22. The use of claim 20, wherein the vector further comprises a TAG sequence and wherein the desired protein can be purified using the TAG sequence.

23. The use of claim 22, wherein the TAG sequence comprises: (i) a sequence that encodes polypeptide that functions as a purification handle; (ii) a cleavage site; and (iii) a polynucleotide spacer.

24. The use of claim 22, wherein the TAG sequence comprises a polynucleotide sequence shown in SEQ ID NO: 22.

25. The use of claim 20, wherein the desired protein is a lytic protein, proinsulin, or a human growth hormone.

26. The use of claim 20, wherein the vector further comprises a second gene of interest operably-linked to a third promoter and wherein the genes of interest encode antibody polypeptides.

27. The vector of claim 1 or 6, wherein the inducible promoter comprises the sequence as set forth in SEQ ID NO. 17, SEQ ID NO: 40, or nucleic acids 4050-4938 of SEQ ID NO: 30.

28. The vector of claim 14 wherein the egg directing sequence comprises at least one of the sequences as set forth in SEQ ID NO: 18, nucleic acids 4960-5112 of SEQ ID NO: 3, nucleic acids 4943-5092 of SEQ ID NO: 4, nucleic acids 4958-6115 of SEQ ID NO: 29, or nucleic acids 4945-6092 of SEQ ID NO: 30.

29. The vector of claim 7, wherein the polyA sequence comprises at least one of the sequences as set forth in SEQ ID NO: 28, SEQ ID NO: 33, or nucleic acids 2995-3410 of SEQ ID NO: 1.

30. The vector of claims 1-15 wherein the modified transposase gene comprises an A or a T at the third position in each of codons 2-10 of the modified transposase gene.

31. The use of claim 20, wherein the vector is to be administered via an intratesticular, intraarterial, intraperitoneal, intravenous, intraoviductal, intraembryonic, nasal, or pronuclear route.

## Patentansprüche

1. Vektor umfassend:
a) ein modifiziertes Transposasegen, das operativ mit einem ersten Promotor verbunden ist, wobei die Nukleinsäuresequenz 3' von dem ersten Promotor die in SEQ ID NO: 13 dargelegte Sequenz umfasst, wobei SEQ ID NO: 13 die Kozak-Sequenz und ein Startcodon für die Transposase enthält, und wobei mindestens eines der ersten zwanzig Codons des Transposasegens gegenüber der Wildtypsequenz verändert ist, indem ein Nukleotid an der Position einer dritten Base des Codons gegen ein Adenin oder Thymin ausgetauscht ist, ohne dass die von dem Codon kodierte Aminosäuresequenz verändert ist, und
b) ein oder mehrere Gen(e) von Interesse, das/die operativ mit einem oder mehreren zusätzlichen Promotor(en) verbunden ist/sind, und wobei das eine oder die mehreren Gene von Interesse und ihre operativ verbundenen Promotoren durch Transposaselnsertionssequenzen flankiert werden, die von der Transposase, die von dem veränderten Transposasegen kodiert wird, erkannt werden, wobei der Promotor, der operativ mit dem Gen von Interesse verbunden ist, aus der Gruppe bestehend aus einem Ovalbuminpromotor, einem Conalbuminpromotor, einem Vitellogeninpromotor oder einem Ovomucoidpromotor ausgewählt ist.

2. Vektor nach Anspruch 1, wobei das modifzierte Transposasegen ein Adenin oder Thymin an der dritten Position in jedem der Codons 2-10 des veränderten Transposasegens umfasst.

3. Vektor nach Anspruch 1, umfassend die in SEQ ID NO: 1 dargelegte Sequenz.

4. Vektor nach Anspruch 1, wobei die Transposase eine Tn10-Transposase ist.

5. Vektor nach Anspruch 1 oder nach Anspruch 4, wobei der erste Promotor aus der Gruppe bestehend aus einem konstitutiven Promotor und einem induzierbaren Promotor ausgewählt ist.

6. Vektor nach Anspruch 5, wobei der induzierbare Promotor aus der Gruppe bestehend aus einem Ovalbuminpromotor, einem Conalbuminpromotor, einem Vitellogeninpromotor oder einem Ovomucoidpromotor ausgewählt ist.

7. Vektor nach Anspruch 1, weiterhin umfassend eine Poly-A-Sequenz, die operativ mit dem Transposasegen verbunden ist.

8. Vektor nach Anspruch 7, wobei die Poly-A-Sequenz eine Conalbumin-Poly-A-Sequenz ist.

9. Vektor nach Anspruch 1 oder nach Anspruch 7, weiterhin umfassend zwei Stopcodons, die operativ mit dem Transposasegen verbunden sind.

10. Vektor nach Anspruch 1, wobei ein erstes Gen von Interesse operativ mit einem zweiten Promotor verbunden ist und ein zweites Gen von Interesse operativ mit einem dritten Promotor verbunden ist.

11. Vektor nach Anspruch 1, wobei ein erstes und ein zweites Gen von Interesse operativ mit einem zweiten Promotor verbunden sind.

12. Vektor nach Anspruch 1, weiterhin umfassend einen Enhancer, der operativ mit dem einen oder den mehreren Gen(en) von Interesse verbunden ist.

13. Vektor nach Anspruch 12, wobei der Enhancer mindestens einen Teil eines Ovalbumin-Enhancers umfasst.

14. Vektor nach Anspruch 1, weiterhin umfassend eine Ei-dirigierende Sequenz, die operativ mit dem einen oder den mehreren Gen(en) von Interesse verbunden ist.

15. Vektor nach Anspruch 14, wobei die Ei-dirigierende Sequenz eine Ovalbumin-Signalsequenz, eine Ovomucoid-Signalsequenz oder eine Vitellogenin-Zielsequenz ist.

16. Verwendung eines Vektors nach einem der Ansprüche 1-15 zur Herstellung eines nicht-humanen transgenen Tieres.

17. Verwendung nach Anspruch 16, wobei der Vektor über einen intratestikulären, intraarteriellen, intraoviductalen oder intraembryonalen Weg verabreicht wird.

18. Verwendung nach Anspruch 16, wobei das Tier ein Vogel ist.

19. Verwendung nach Anspruch 18, wobei der Vogel ein Hühnchen oder eine Wachtel ist.

20. Verwendung eines Vektors nach einem der Ansprüche 1-15 zur Verabreichung an ein Tier zur Herstellung eines gewünschten Proteins.

21. Verwendung nach Anspruch 20, wobei das Tier ein eierlegendes Tier ist, und die Verabreichung intraoviductal ist, so dass das gewünschte Protein, das von dem mindestens einen Gen von Interesse hergestellt wurde, aus dem Eiweiß von Eiern, die von dem eierlegenden Tier gelegt wurden, isoliert wird.

22. Verwendung nach Anspruch 20, wobei der Vektor weiterhin eine TAG-Sequenz umfasst und wobei das gewünschte Protein unter Verwendung der TAG-Sequenz aufgereinigt werden kann.

23. Verwendung nach Anspruch 22, wobei die TAG-Sequenz umfasst: (i) eine Sequenz, die ein Polypeptid kodiert, das als Mittel für die Aufreinigung fungiert; (ii) eine Spaltstelle; und (iii) einen Polynukleotidspacer.

24. Verwendung nach Anspruch 22, wobei die TAG-Sequenz eine in SEQ ID NO: 22 gezeigte Polynukleotidsequenz umfasst.

25. Verwendung nach Anspruch 20, wobei das gewünschte Protein ein lytisches Protein, Proinsulin oder ein humanes Wachstumshormon ist.

26. Verwendung nach Anspruch 20, wobei der Vektor weiterhin ein zweites Gen von Interesse umfasst, das operativ mit einem dritten Promotor verbunden ist, und worin die Gene von Interesse Antikörperpolypeptide kodieren.

27. Vektor nach Anspruch 1 oder 6, wobei der induzierbare Promotor die Sequenz, wie in SEQ ID NO: 17, SEQ ID NO: 40 oder den Nukleinsäuren 4050-4938 der SEQ ID NO: 30 dargelegt, umfasst.

28. Vektor nach Anspruch 14, wobei die Ei-dirigierende Sequenz mindestens eine der Sequenzen, wie in SEQ ID NO: 18, den Nukleinsäuren 4960-5112 der SEQ ID NO: 3, den Nukleinsäuren 4943-5092 der SEQ ID NO: 4, den Nukleinsäuren 4958-6115 der SEQ ID NO: 29 oder den Nukleinsäuren 4945-6092 der SEQ ID NO: 30 dargelegt, umfasst.

29. Vektor nach Anspruch 7, wobei die Poly-A-Sequenz mindestens eine der Sequenzen wie in SEQ ID NO: 28, SEQ 10 NO: 33 oder den Nukleinsäuren 2995-3410 der SEQ ID NO: 1 dargelegt, umfasst.

30. Vektor nach den Ansprüchen 1-15, wobei das modifzierte Transposasegen ein A oder ein T an der dritten Position in jedem der Codons 2-10 des modifzierten Transposasegens umfasst.

31. Verwendung nach Anspruch 20, wobei der Vektor über einen intratestikulären, intraarteriellen, intraperitonealen, intravenösen, intraoviductalen, intraembryonalen, nasalen oder pronukleären Weg verabreicht werden soll.

## Revendications

1. Vecteur comprenant :
a) un gène de transposase modifié lié de manière fonctionnelle à un premier promoteur, où la séquence d'acide nucléique en 3' par rapport au premier promoteur comprend la séquence telle que représentée par SEQ ID NO : 13, où SEQ ID NO : 13 contient la séquence Kozak et un codon de départ pour la transposase, et où au moins l'un des vingt premiers codons du gène de transposase est modifié par rapport à la séquence de type sauvage en changeant un nucléotide au niveau d'une troisième position de base du codon en une adénine ou une thymine sans modifier l'acide aminé codé par le codon, et
b) un ou plusieurs gènes d'intérêt liés de manière fonctionnelle à un ou plusieurs promoteurs supplémentaires, et où les un ou plusieurs gènes d'intérêt et leurs promoteurs liés de manière fonctionnelle sont flanqués par des séquences d'insertion de transposase reconnues par la transposase codée par le gène de transposase modifié, où le promoteur qui est lié de manière fonctionnelle au gène d'intérêt est choisi dans le groupe constitué d'un promoteur de l'ovalbumine, d'un promoteur de la conalbumine, d'un promoteur de la vitellogénine ou d'un promoteur de l'ovomucoïde.

2. Vecteur selon la revendication 1, dans lequel le gène de transposase modifié comprend une adénine ou une thymine au niveau de la troisième position dans chacun des codons 2 à 10 du gène de transposase modifié.

3. Vecteur selon la revendication 1, comprenant la séquence telle que représentée par SEQ ID NO : 1.

4. Vecteur selon la revendication 1, dans lequel la transposase est une transposase Tn10.

5. Vecteur selon la revendication 1 ou la revendication 4, dans lequel le premier promoteur est choisi dans le groupe constitué d'un promoteur constitutif et d'un promoteur inductible.

6. Vecteur selon la revendication 5, dans lequel le promoteur inductible est choisi dans le groupe constitué d'un promoteur de l'ovalbumine, d'un promoteur de la conalbumine, d'un promoteur de la vitellogénine ou d'un promoteur de l'ovomucoïde.

7. Vecteur selon la revendication 1, comprenant en outre une séquence polyA liée de manière fonctionnelle au gène de transposase.

8. Vecteur selon la revendication 7, dans lequel la séquence polyA est une séquence polyA de la conalbumine.

9. Vecteur selon la revendication 1 ou la revendication 7, comprenant en outre deux codons stop liés de manière fonctionnelle au gène de transposase.

10. Vecteur selon la revendication 1, dans lequel un premier gène d'intérêt est lié de manière fonctionnelle à un deuxième promoteur et un deuxième gène d'intérêt est lié de manière fonctionnelle à un troisième promoteur.

11. Vecteur selon la revendication 1, dans lequel un premier et un deuxième gènes d'intérêt sont liés de manière fonctionnelle à un deuxième promoteur.

12. Vecteur selon la revendication 1, comprenant en outre un amplificateur lié de manière fonctionnelle à l'au moins un gène d'intérêt.

13. Vecteur selon la revendication 12, dans lequel l'amplificateur comprend au moins une partie d'un amplificateur de l'ovalbumine.

14. Vecteur selon la revendication 1, comprenant en outre une séquence dirigeante de l'oeuf liée de manière fonctionnelle à l'au moins un gène d'intérêt.

15. Vecteur selon la revendication 14, dans lequel la séquence dirigeante de l'oeuf est une séquence signal de l'ovalbumine, une séquence signal de l'ovomucoïde ou une séquence de ciblage de la vitellogénine.

16. Utilisation d'un vecteur selon l'une quelconque des revendications 1 à 15, pour produire un animal transgénique non humain.

17. Utilisation selon la revendication 16, où le vecteur est administré par une voie intratesticulaire, intra-artérielle, intra-oviductale ou intra-embryonnaire.

18. Utilisation selon la revendication 16, où l'animal est un animal aviaire.

19. Utilisation selon la revendication 18, où l'animal aviaire est un poulet ou une caille.

20. Utilisation d'un vecteur selon l'une quelconque des revendications 1 à 15, pour être administré à un animal pour produire une protéine souhaitée.

21. Utilisation selon la revendication 20, où l'animal est un animal qui pond des oeufs et l'administration est intra-oviductale, de telle manière que la protéine souhaitée produite par l'au moins un gène d'intérêt est isolée du blanc des oeufs pondus par l'animal pondant des oeufs.

22. Utilisation selon la revendication 20, où le vecteur comprend en outre une séquence TAG et où la protéine souhaitée peut être purifiée en utilisant la séquence TAG.

23. Utilisation selon la revendication 22, où la séquence TAG comprend : (i) une séquence qui code pour le polypeptide qui fonctionne comme une poignée de purification ; (ii) un site de clivage ; et (iii) un espaceur polynucléotidique.

24. Utilisation selon la revendication 22, où la séquence TAG comprend une séquence polynucléotidique représentée par SEQ ID NO : 22.

25. Utilisation selon la revendication 20, où la protéine souhaitée est une protéine lytique, la proinsuline ou une hormone de croissance humaine.

26. Utilisation selon la revendication 20, où le vecteur comprend en outre un deuxième gène d'intérêt lié de manière fonctionnelle à un troisième promoteur et où les gènes d'intérêt codent pour des polypeptides d'anticorps.

27. Vecteur selon la revendication 1 ou 6, où le promoteur inductible comprend la séquence telle que représentée par SEQ ID NO : 17, SEQ ID NO : 40 ou les acides nucléiques 4050 à 4938 de SEQ ID NO : 30.

28. Vecteur selon la revendication 14, dans lequel la séquence dirigeante de l'oeuf comprend au moins l'une des séquences telles que représentées par SEQ ID NO : 18, les acides nucléiques 4960 à 5112 de SEQ ID NO : 3, les acides nucléiques 4943 à 5092 de SEQ ID NO : 4, les acides nucléiques 4958 à 6115 de SEQ ID NO : 29 ou les acides nucléiques 4945 à 6092 de SEQ ID NO : 30.

29. Vecteur selon la revendication 7, dans lequel la séquence polyA comprend au moins l'une des séquences telles que représentées par SEQ ID NO: 28, SEQ ID NO : 33 ou les acides nucléiques 2995 à 3410 de SEQ ID NO : 1.

30. Vecteur selon les revendications 1 à 15, dans lequel le gène de transposase modifié comprend un A ou un T au niveau de la troisième position dans chacun des codons 2 à 10 du gène de transposase modifié.

31. Utilisation selon la revendication 20, où le vecteur doit être administré par une voie intratesticulaire, intra-artérielle, intrapéritonéale, intraveineuse, intra-oviductale, intra-embryonnaire, nasale ou pronucléaire.
